# EUROPEAN PATENT SPECIFICATION

(11) **EP 2 882 460 B1**
(45) Date of publication and mention of the grant of the patent: **24.04.2019**
(21) Application number: 13827396.6
(22) Date of filing: 08.08.2013
(51) Int. Cl.: A61K 48/00, C12N 15/00, C12N 5/00, A61K 35/28, C12N 5/0789, C12N 15/113, C12N 15/86

(54) **CD25 PRE-SELECTIVE COMBINATION ANTI-HIV VECTORS, TARGETING VECTORS, AND METHODS OF USE**
PRÄSELEKTIVE CD25-ANTI-HIV-KOMBINATIONSVEKTOREN, TARGETING-VEKTOREN UND ANWENDUNGSVERFAHREN
VECTEURS CD25 ANTI-VIH À COMBINAISON PRÉ-SÉLECTIVE, VECTEURS DE CIBLAGE ET PROCÉDÉS D'UTILISATION

(30) Priority: 09.08.2012 US 201261681586 P
(43) Date of publication of application: 17.06.2015
(73) Proprietor: The Regents of the University of California, Oakland, CA 94607 (US)
(72) Inventor: ANDERSON, Joseph, Sacramento, California 95817 (US)
(74) Representative: Teall, Charlotte
(86) International application number: PCT/US2013/054217
(87) International publication number: WO 2014/026053

(56) References cited:
- WO-A1-2011/060218
- WO-A1-2011/066578
- US-A1- 2011 243 849
- US-A1- 2012 076 763
- US-A1- 2012 076 763
- STEFANOS KALOMOIRIS ET AL: "CD25 Preselective Anti-HIV Vectors for Improved HIV Gene Therapy", HUMAN GENE THERAPY METHODS, vol. 23, no. 6, 1 December 2012 (2012-12-01), pages 366-375, XP055197758, ISSN: 1946-6536, DOI: 10.1089/hgtb.2012.142
- SANG-SOO KIM ET AL: "RNAi-mediated CCR5 Silencing by LFA-1-targeted Nanoparticles Prevents HIV Infection in BLT Mice", MOLECULAR THERAPY, vol. 18, no. 2, 8 December 2009 (2009-12-08), pages 370-376, XP055249739, GB ISSN: 1525-0016, DOI: 10.1038/mt.2009.271
- JOSEPH S ANDERSON ET AL: "Preintegration HIV-1 Inhibition by a Combination Lentiviral Vector Containing a Chimeric TRIM5[alpha] Protein, a CCR5 shRNA, and a TAR Decoy", MOLECULAR THERAPY, vol. 17, no. 12, 1 December 2009 (2009-12-01), pages 2103-2114, XP055038621, ISSN: 1525-0016, DOI: 10.1038/mt.2009.187
- HUBNER ET AL.: 'Lentivirus Transduction of Murine Embryonic Stem Cells with Truncated Human Low-Affinity Nerve Growth Factor Permits Efficient Purification of Genetically Modified Cells without Loss of Stem Cell Characteristics Molecular' THERAPY vol. 13, no. SUPPL, 2006, XP005675448
- KALOMOIRIS ET AL.: 'CD25 preselective anti-HIV vectors for improved HIV gene therapy' HUM GENE THER METHODS vol. 23, no. 6, December 2012, pages 366 - 375, XP055197758

## Description

### BACKGROUND

Throughout this disclosure, various publications, patents and published patent specifications are referenced by an identifying citation and in some aspects, the complete bibliographic of the citation is found in the section immediately preceding the claims.

HIV infections continue to spread worldwide in both developed and underdeveloped countries with no effective vaccine available (Barouch et al., 2008; Edgeworth et al., 2002). Although antiretroviral therapy (ART) is effective in the majority of HIV infected patients, challenges to therapeutic and curative success include the continuing emergence of drug-resistant HIV variants, drug toxicity, and incomplete viral suppression (Baldanti et al., 2010; Domingo et al., 2012; Johnson et al., 2010; Kuritzkes, 2011; Lewden et al., 2007; Macias et al., 2006; Tilton et al., 2010). ART also fails to eradicate viral reservoirs which are established early in infection, leading to viral persistence and incomplete immune restoration (Gazzola et al., 2009; Mehandru et al., 2006). Interruption of ART results in rapid viral resurgence, the generation of escape mutants, and CD4+ T cell loss in the peripheral blood of HIV infected patients (Graham et al., 2012; Kalmar et al., 2012). US 2012/0076763 discloses a lentiviral vector comprising TRIM5-alpha, CCR5 RNAi, TAR and the selectable marker EGFP, and applications in HIV therapy.

These challenges highlight the need for the further development of innovative HIV therapies with broad mechanisms of action. This invention satisfies this need and provides related advantages as well.

### SUMMARY

The present invention is defined in the claims. HIV-1 gene therapy offers a promising alternative to small molecule antiretroviral treatments and current vaccination strategies by transferring, into HIV-1 susceptible cells, the genetic ability to resist infection. The need for novel and innovative strategies to prevent and treat HIV-1 infection is critical due to devastating effects of the virus in developing countries, high cost, toxicity, and generation of escape mutants from antiretroviral therapies and the failure of past and current vaccination efforts. Described herein are DNA vectors, viral packaging systems, and methods useful for HIV stem cell gene therapy with an enriched population of HIV-resistant cells compared to unpurified cells. This was achieved by a triple combination anti-HIV vector which incorporates a selectable marker, e.g., human CD25, which is expressed on the surface of transduced cells. Human CD25, the low affinity IL-2 receptor alpha subunit, is an example of a selectable marker and is useful because of its normal characteristics of not being expressed on the surface of HPCs or HSCs and its lack of intracellular signaling (Grant et al., 1992; Kuziel et al., 1990; Minami et al., 1993). Upon expressing CD25 on the surface of HPCs and purification of the transduced cells, safety of the enriched population of anti-HIV vector transduced HPCs was observed along with potent HIV-1 inhibition. This demonstrates the use of this strategy for HIV stem cell gene therapy to improve the efficacy of future HIV stem cell gene therapy clinical trials.

Thus, this invention provides a vector comprising, or alternatively consisting essentially of, or yet further consisting of: a vector backbone comprising or alternatively consisting essentially of, or yet further consisting of essential sequences for integration of exongenous genes into a target cell's genome; a nucleic acid encoding a CCR5 RNAi that, in one aspect, inhibits integration of a human immunodeficiency virus (HIV) into a mammalian cell; a first expression control element that regulates expression of the nucleic acid encoding the CCR5 RNAi element and operatively linked to the CCR5 RNAi element; a nucleic acid encoding at least the extracellular domain of CD25 or an equivalent thereof; and a second expression control element that regulates expression of the nucleic acid encoding at least the extracellular domain of CD25 or an equivalent thereof and operatively linked to it. In a further aspect, the vector also contains a nucleic acid encoding a TRIM5 alpha sequence and an HIV TAR sequence.

The invention also provides a vector as described above, wherein the backbone is derived from a virus; and a packaging plasmid which contains the nucleoside, capsid and matrix proteins. In a further aspect, the invention further comprise an envelope plasmid and in a further aspect, a packaging cell line is provided.

Also provided are pseudotyped viral particles that are optionally conjugated to cell-specific targeting antibodies. The particles optionally can be conjugated to cells. This invention also provides a cell or an enriched population of HIV-resistant cells, wherein the cell(s) expresses CD25 extracellular domain on the surface of the cell and comprise an HIV CCR5 RNAi element. In one aspect, the cell(s) further comprises a TRIM5alpha and/or an HIV TAR polynucleotide(s). In one aspect the cell is a stem cell such as a hematopoietc progenitor cell (HPC) or a hematopoietic stem cell (HSC).

The vectors, particles and cells are useful to inhibit, *ex vivo* and *in vivo,* the replication of HIV in a cell system, such as a cell culture, or in a subject in need thereof by administering an effective amount of the vector, the particle, the cell, the enriched population of cells, or the cell conjugated to the partice. In one aspect, the methods not only inhibit HIV replication but also prevent replication in a subject infected with the virus. Specific details of the various embodiments of this invention are provided herein.

### BRIEF DESCRIPTION OF THE DRAWINGS

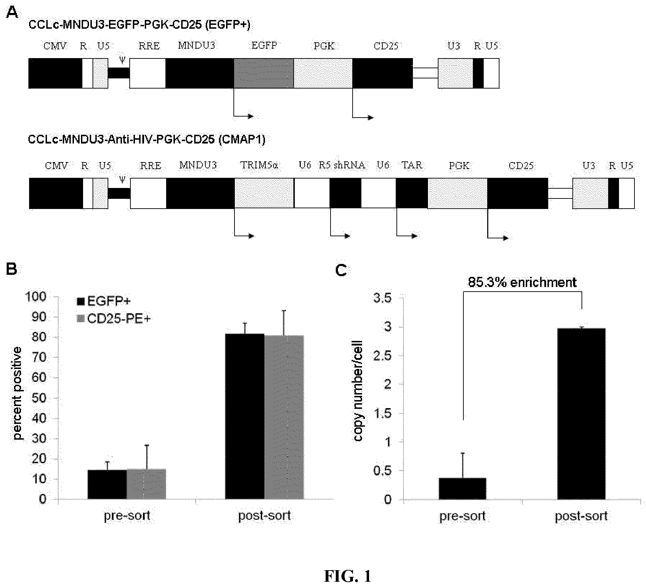

**FIGS. 1A-1C** depict pre-selective lentiviral vectors and purification of transduced HPCs. **FIG. 1A****:** A self-inactivating third generation lentiviral vector, CCLc-MNDU3-X-PGK-X2, was utilized to derive the pre-selective vectors. The control EGFP+ vector contains an EGFP reporter gene under the control of the MNDU3 promoter and a human CD25 gene under the control of the PGK promoter. The CMAP1 anti-HIV vector contains a triple combination of anti-HIV genes, a human/rhesus macaque chimeric TRIM5α under the control of the MNDU3 promoter, a pol-III U6 promoter driven CCR5 shRNA, a pol-III U6 promoter driven TAR decoy, and a human CD25 gene under the control of the PGK promoter. **FIG. 1B****:** CD34+ HPCs were transduced with the control EGFP+ pre-selective vector, purified by CD25 immunomagnetic beads, and analyzed by flow cytometry for EGFP and CD25 expression. **FIG. 1C****:** CD34+ HPCs were transduced with the anti-HIV CMAP1 vector, purified by CD25 immunomagnetic beads, and analyzed by QPCR for vector copy number. All experiments were performed in triplicate.

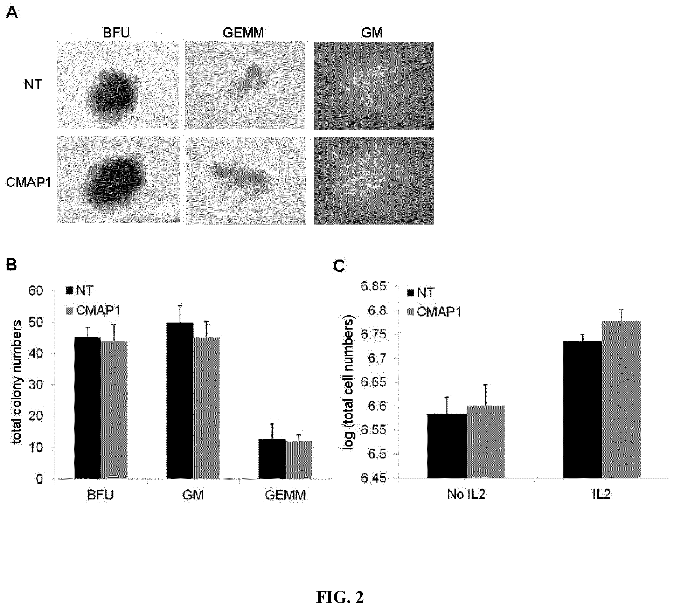

**FIG. 2A-2C** show the safety analysis of purified CMAP1 transduced HPCs. **FIG. 2A****:** HPCs, either nontransduced (NT) or purified CMAP1 vector transduced, were cultured in semi-solid methylcellulose medium for 10 days and CFUs (blood forming colonies (BFU), granulocyte/erythrocyte/megakaryocyte/monocyte colonies (GEMM), and granulocyte/monocyte colonies (GM)) were visualized by microscopy under 10x magnification. **FIG. 2B****:** Total BFU, GM, and GEMM colonies were counted on day 10 for the NT and purified CMAP1 cultures. **FIG. 2C****:** Total cells were counted in HPC methylcellulose NT and purified CMAP1 vector transduced cultures in the absence or presence of IL-2. All experiments were performed in triplicate. Representative cell pictures are displayed.

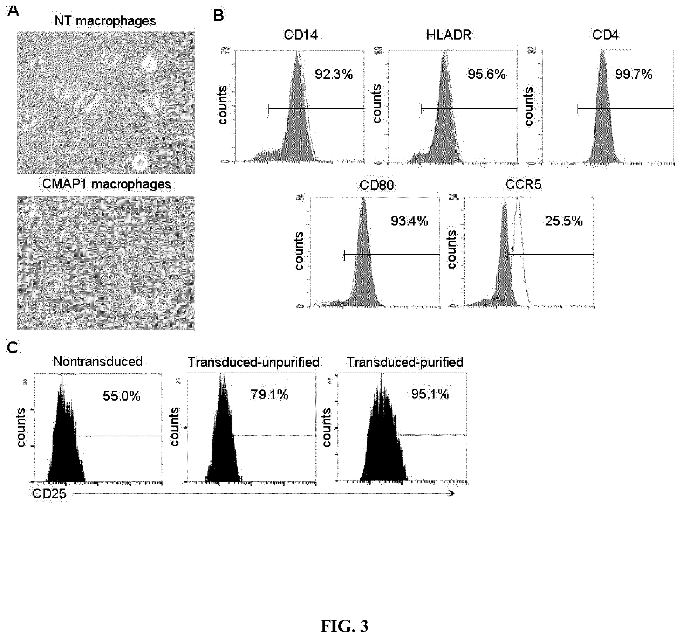

**FIGS. 3A and B** depict the derivation of phenotypically normal macrophages. **FIG. 3A****:** Macrophages derived from the nontransduced (NT) and purified CMAP1 HPC cultures were visualized by microscopy under 10x magnification. **FIG. 3B****:** The NT (unshaded) and purified CMAP1 (shaded) macrophages were analyzed by flow cytometry for the cell surface markers CD14, HLADR, CD4, CD80, and CCR5. FIG. 3C: Macrophages, NT, unpurified CMAP1 transduced, and purified CMAP1 transduced, were also analyzed by flow cytometry for the expression of CD25. All experiments were performed in triplicate. Representative cell pictures and flow cytometry histogram overlays are displayed.

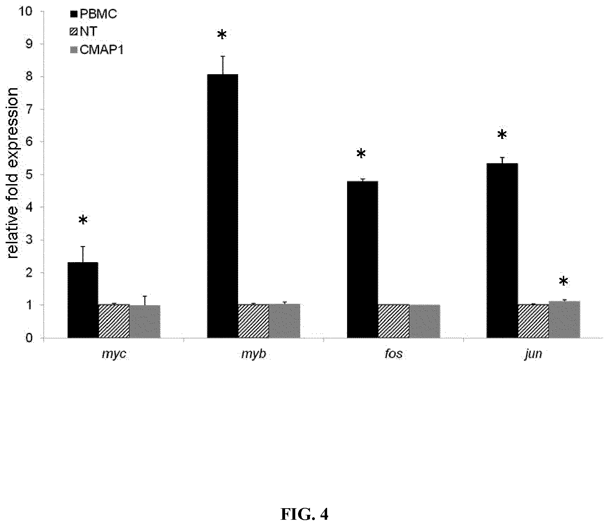

**FIG. 4** shows the expression of proto-oncogenes in purified CMAP1 macrophages. Cell cultures containing either peripheral blood mononuclear cells (PBMCs), nontransduced (NT) HPC derived macrophages, or purified CMAP1 vector transduced HPC derived macrophages were evaluated by QPCR for their expression of the proto-oncogenes myc, myb, fos, and jun in the presence of IL-2. Experiments were performed in triplicate. Statistical significance (p<0.05) is represented by an asterisk.

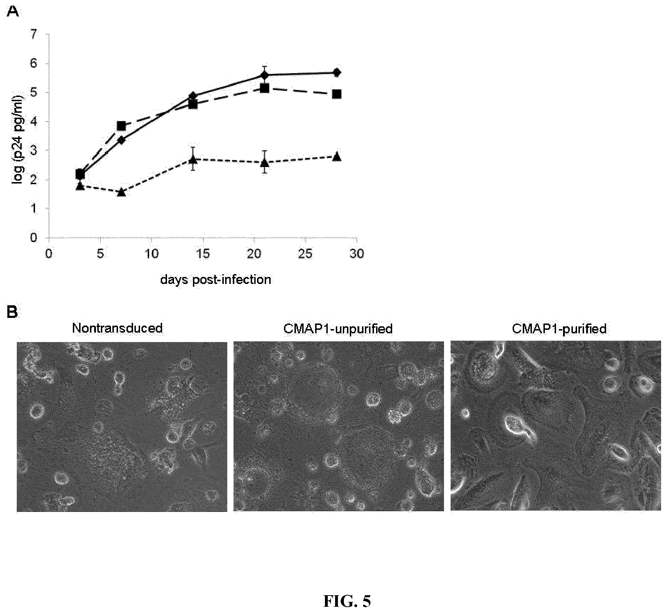

**FIGS. 5A and B** depict HIV-1 challenge of CMAP1 HPC derived macrophages. **FIG. 5A****:** HPC derived macrophages, either nontransduced (NT) (◆), unpurified CMAP1 transduced (CMAP1-UP) (■), or purified CMAP1 (CMAP1-P) (A) transduced, were challenged with an R5-tropic BaL-1 strain of HIV-1 at an MOI of 0.05. On various days post-infection, culture supernatants were analyzed for HIV-1 replication by p24 ELISA. **FIG. 5B****:** On day 28 post-infection infected cultures were visualized by microscopy under 10x magnification. All experiments were performed in triplicate. Representative cell pictures are displayed.

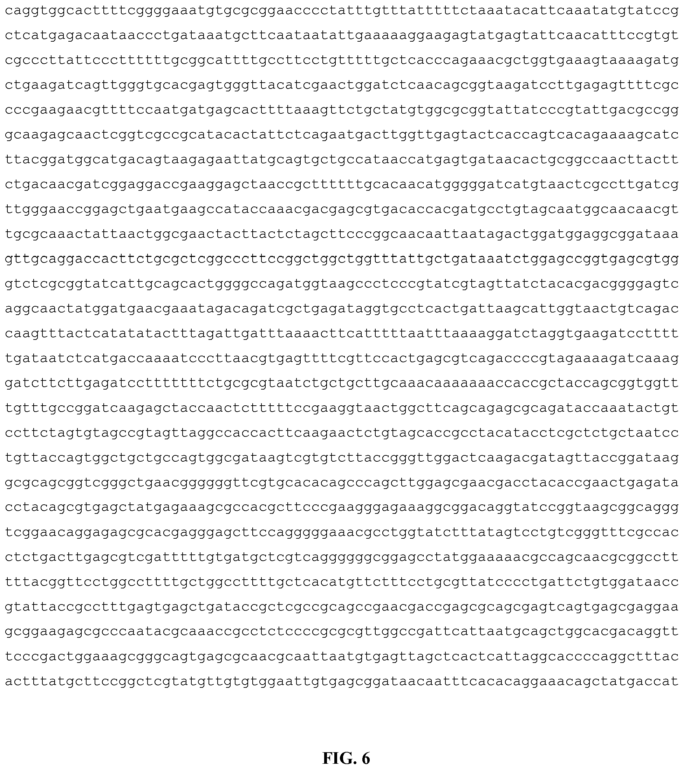

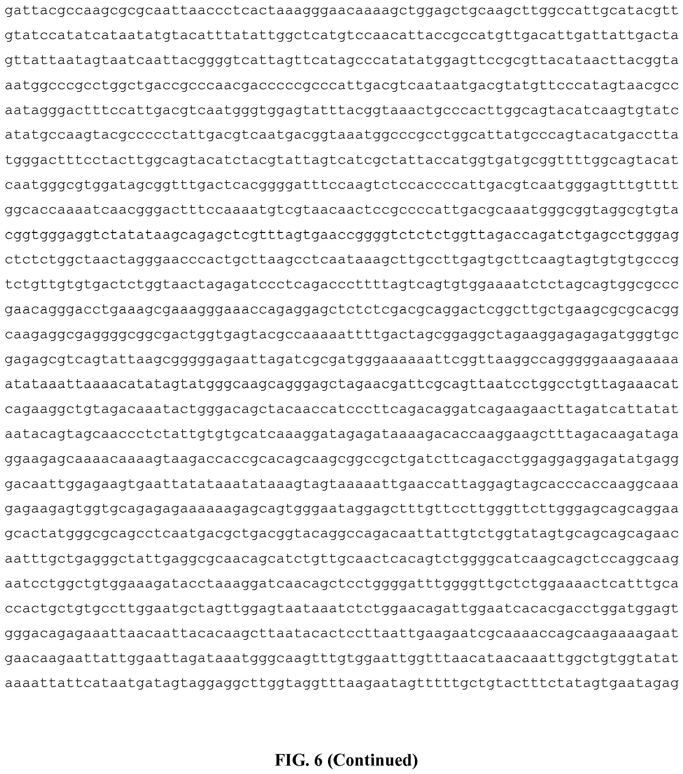

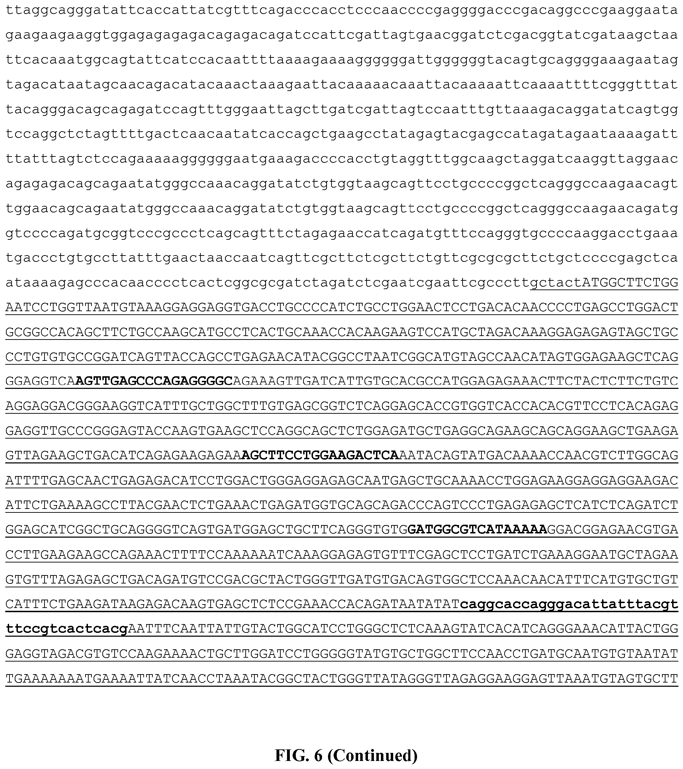

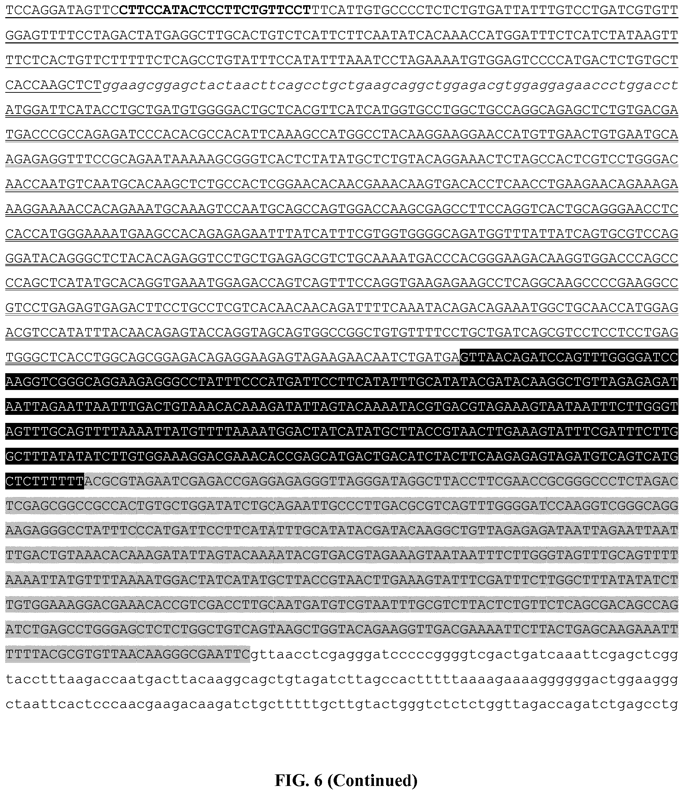

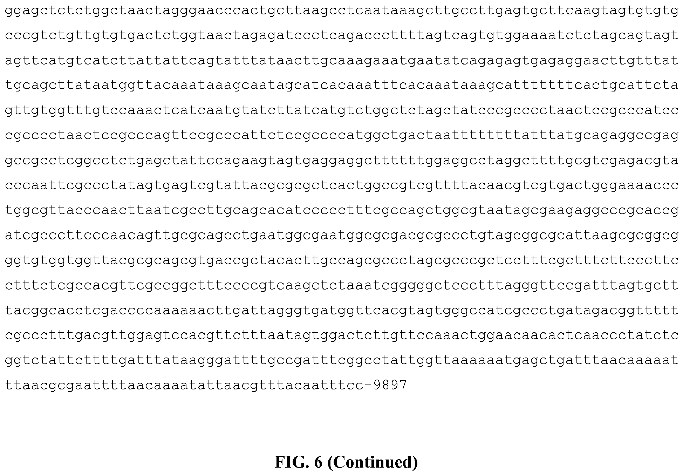

**FIG. 6** shows the nucleotide sequence of the full CD25 pre-selective anti-HIV lentiviral vector backbone (9897 base pairs). The sequence is annotated as follows: 1) the underline portion is human/rhesus macaque TRIM5alpha; 2) the italic portion is the 2A protease sequence; 3) the double underline protein is full CD25 sequence; 4) the black background/white type portion is the CCR5 shRNA; and 5) the shaded portion is TAR decoy sequence. This nucleotide sequence represends SEQ ID NO: 1.

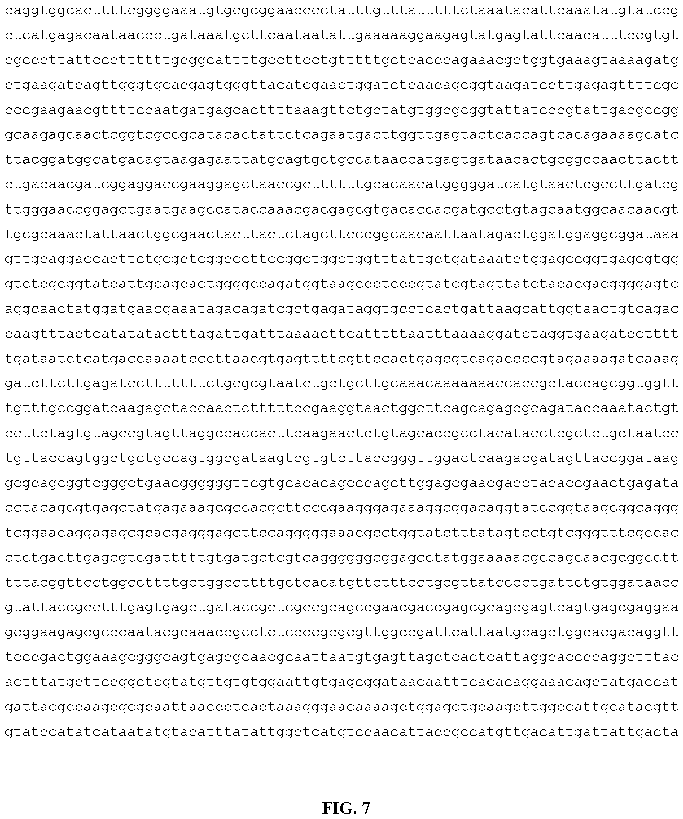

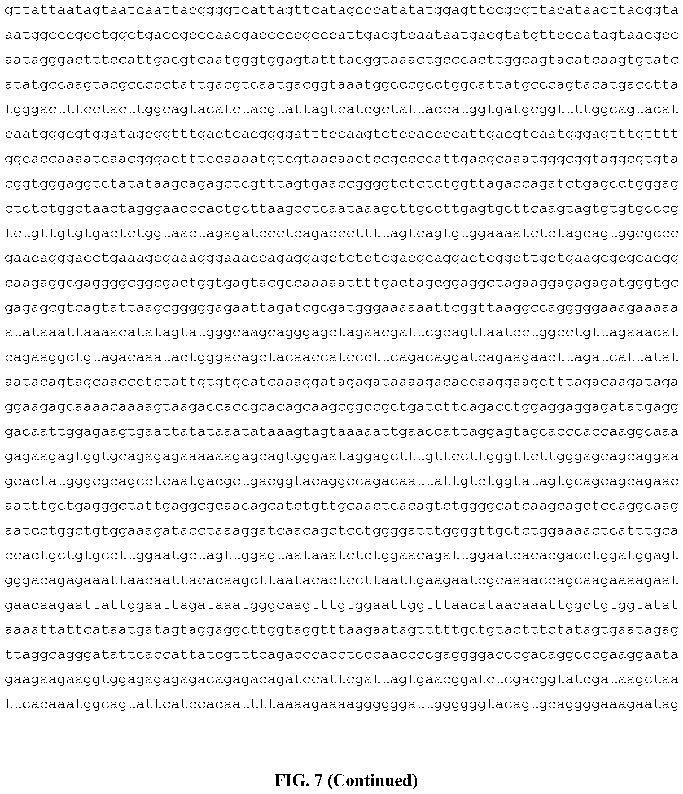

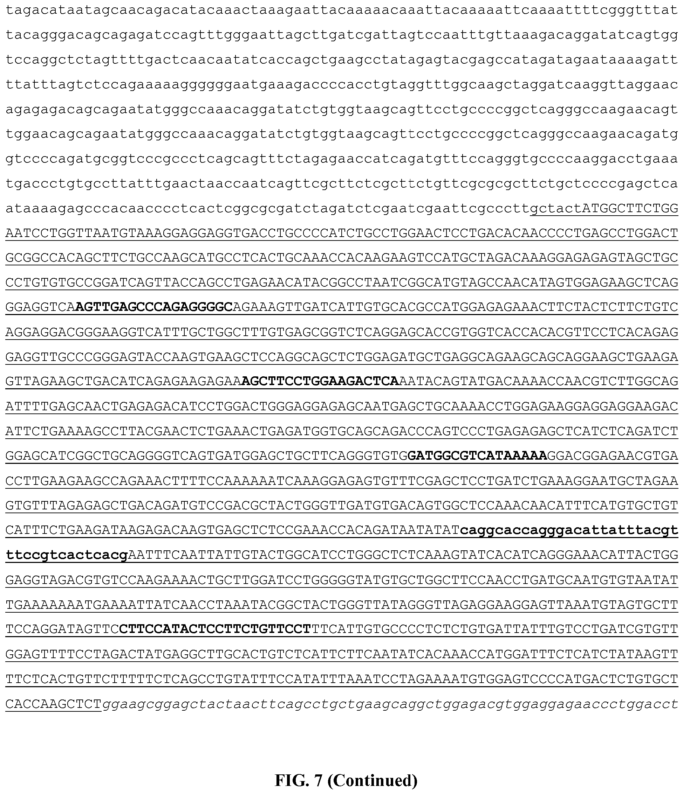

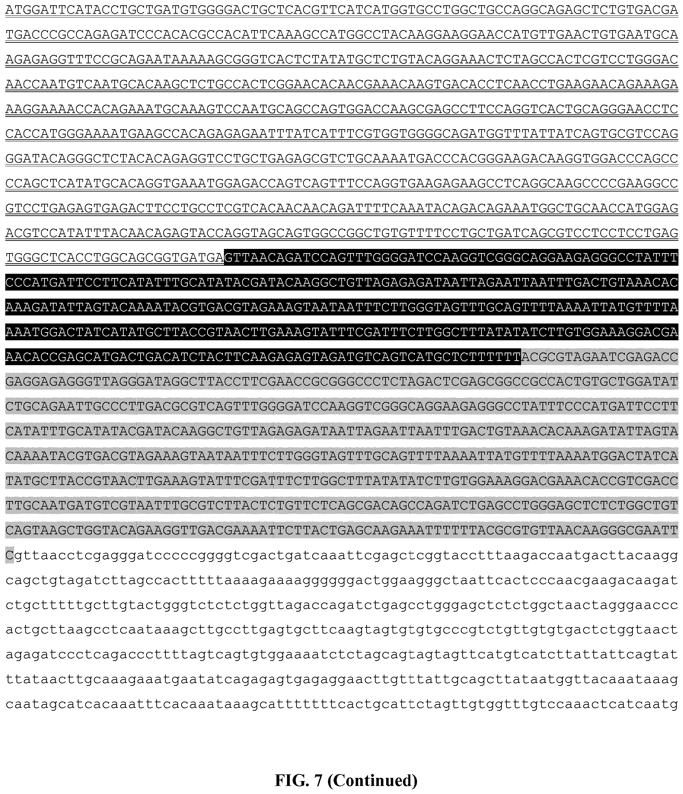

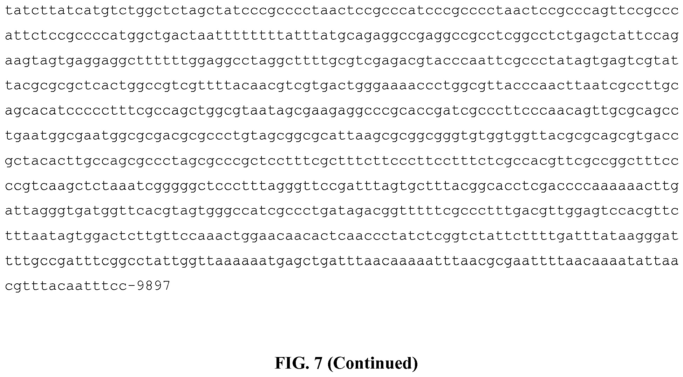

**FIG. 7** shows the nucleotide sequence of the partial truncated CD25 pre-selective anti-HIV lentiviral vector backbone (9870 base pairs). The sequence is annotated as follows: 1) the underline portion is human/rhesus macaque TRIM5alpha sequence; 2) the italic portion is the2A protease sequence; 3) the double underline portion is partial truncated CD25 sequence; 4) the black background/white type portion is the CCR5 shRNA; and 5) the shaded portion is TAR decoy sequence. This nucleotide sequence represends SEQ ID NO: 2.

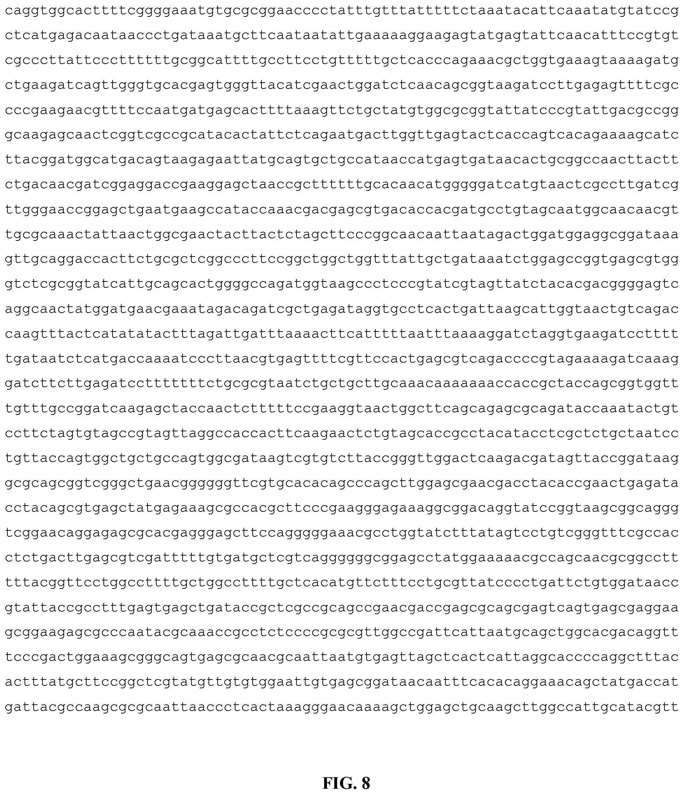

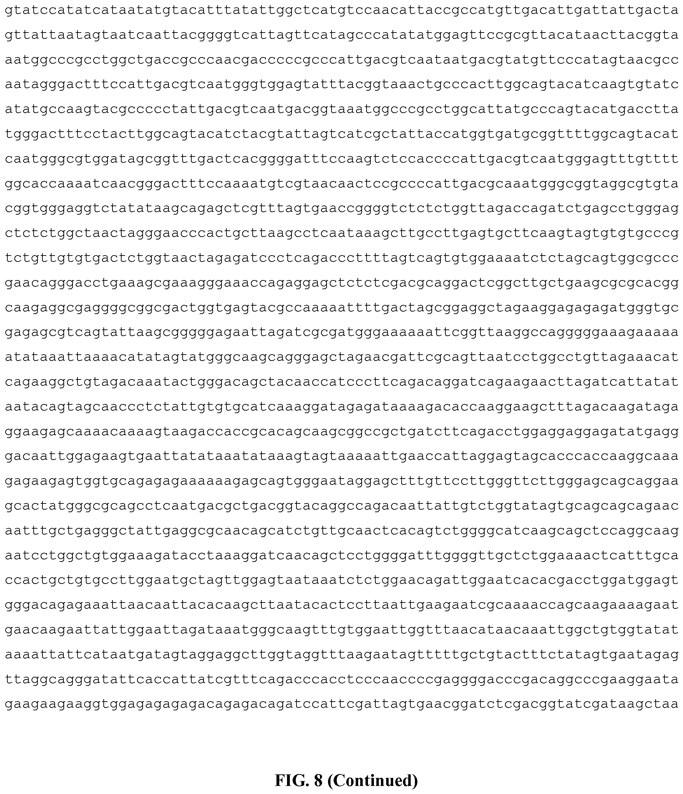

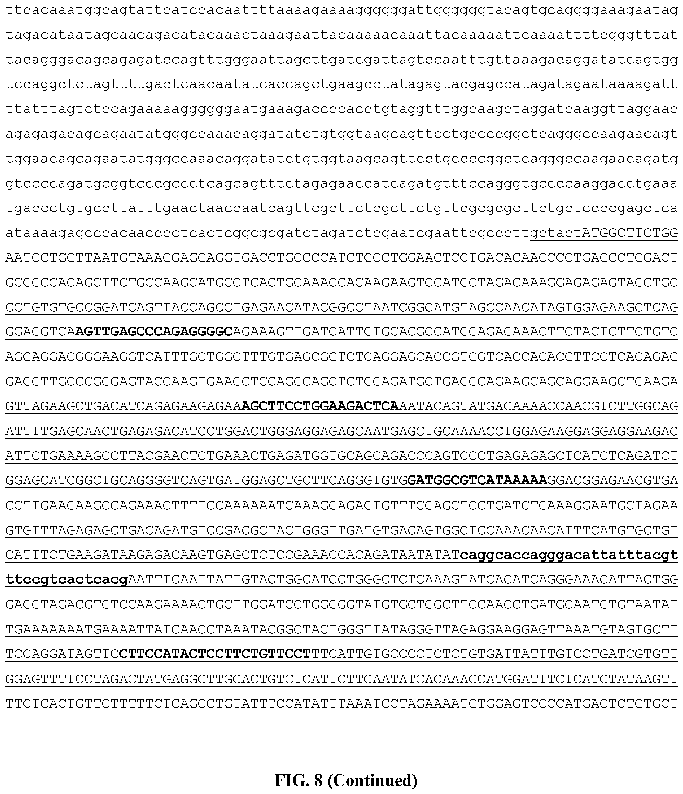

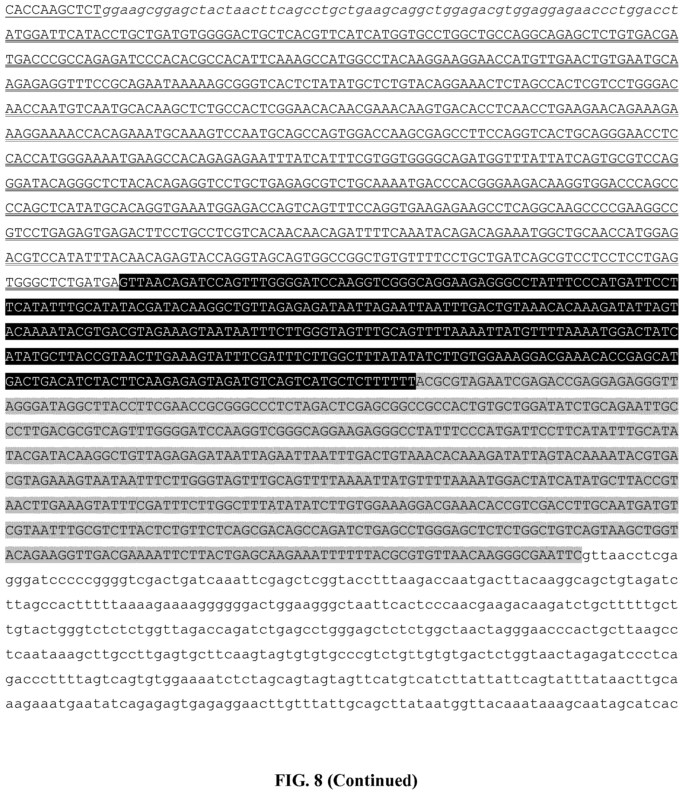

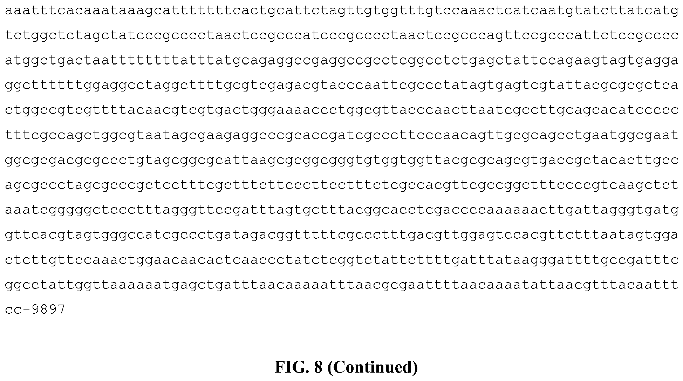

**FIG. 8** shows the nucleotide sequence of the truncated CD25 pre-selective anti-HIV lentiviral vector backbone (9858 base pairs). The sequence is annotated as follows: 1) the underline portion is human/rhesus macaque TRIM5alpha sequence; 2) the italic portion is the 2A protease sequence; 3) the double underline portion is truncated CD25 sequence; 4) the black background/white type portion is the CCR5 shRNA sequence; and 5) the shaded portion is TAR decoy sequence. This nucleotide sequence represends SEQ ID NO: 3.

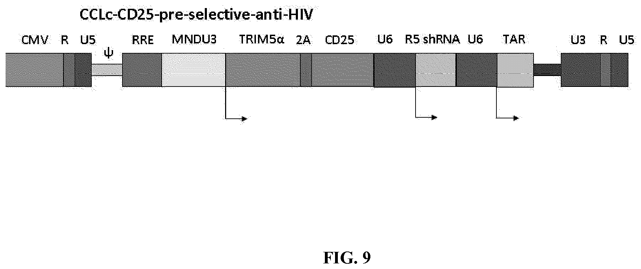

**FIG. 9** depicts a vector map of a CD25-pre-selective-anti-HIV lentiviral vector that was constructed by the method of Example 11.

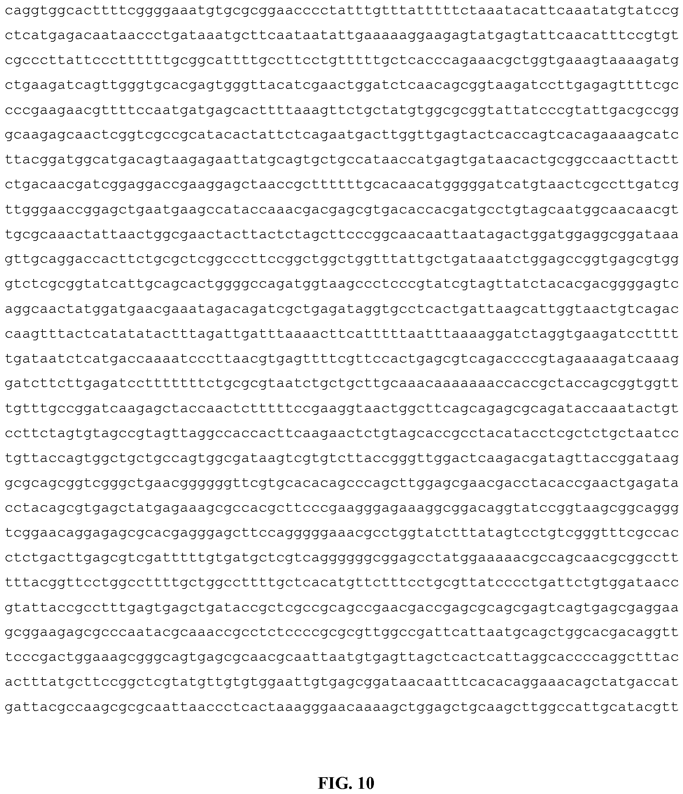

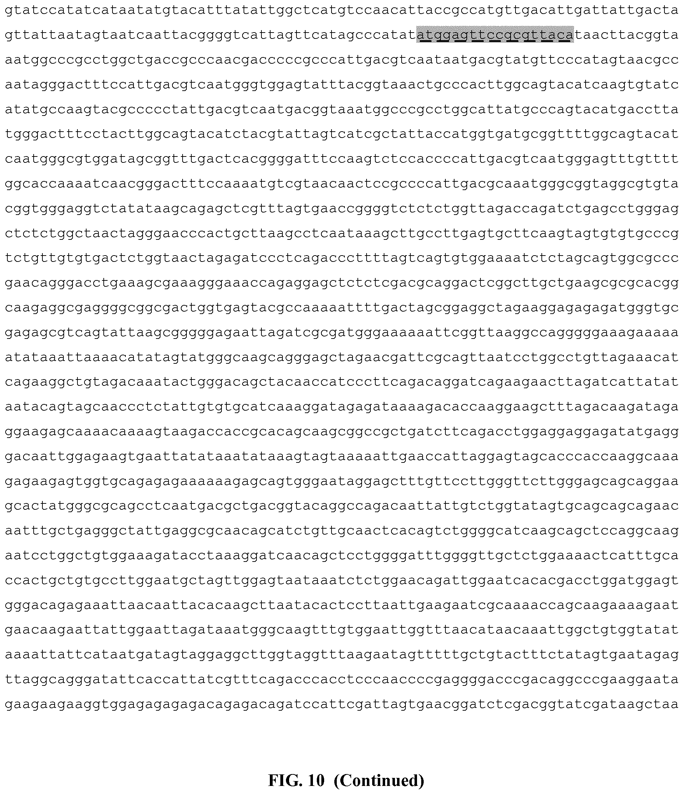

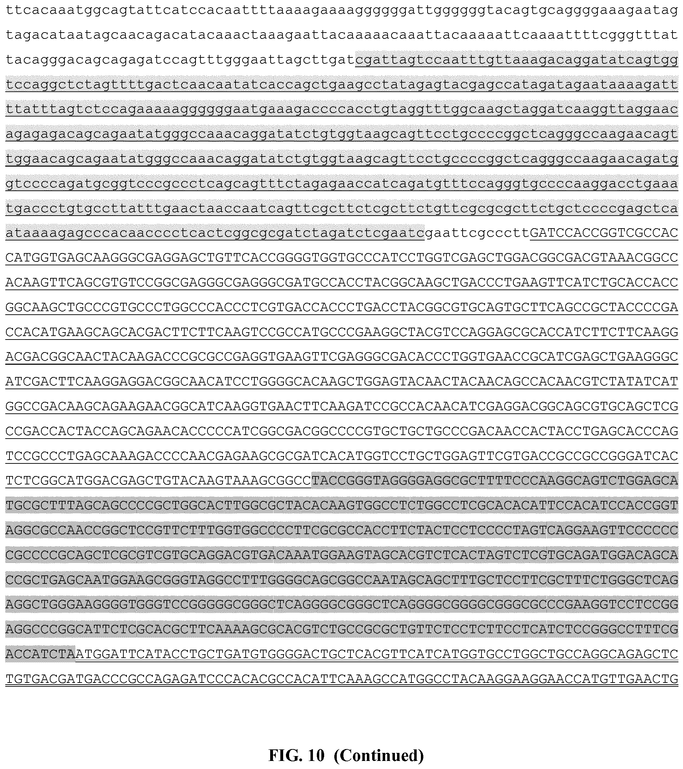

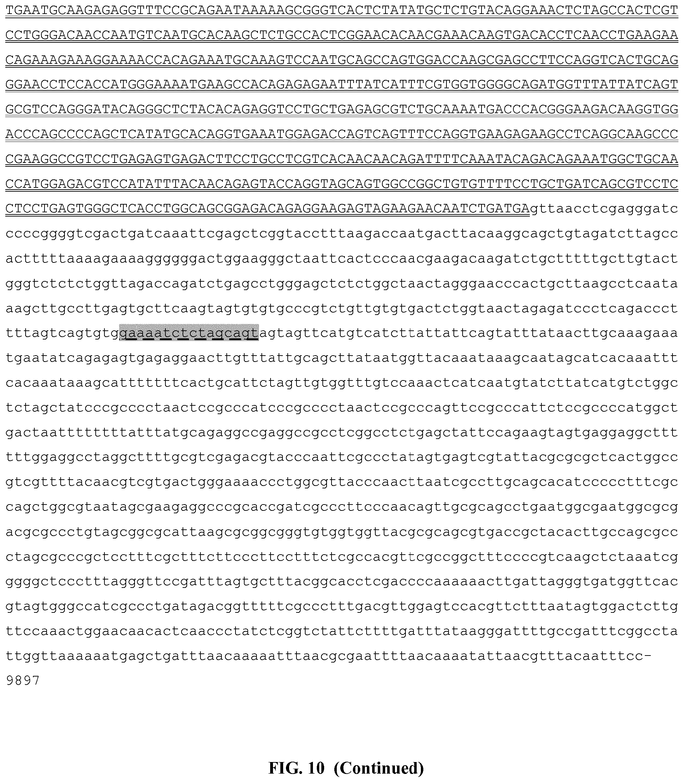

**FIG. 10** depicts SEQ ID NO: 21 which is the sequence of the control vector with EGFP from Figure 1A: CCLc-MNDU3-EGFP-PGK-CD25. The MNDU3 promoter is double underlined and shaded. EGFP is underlined, the PGK promoter is shaded grey, and the full CD25 sequence is in double underline. The 5' and 3' ends of the viral backbone are highlighted and underlined with a broken line.

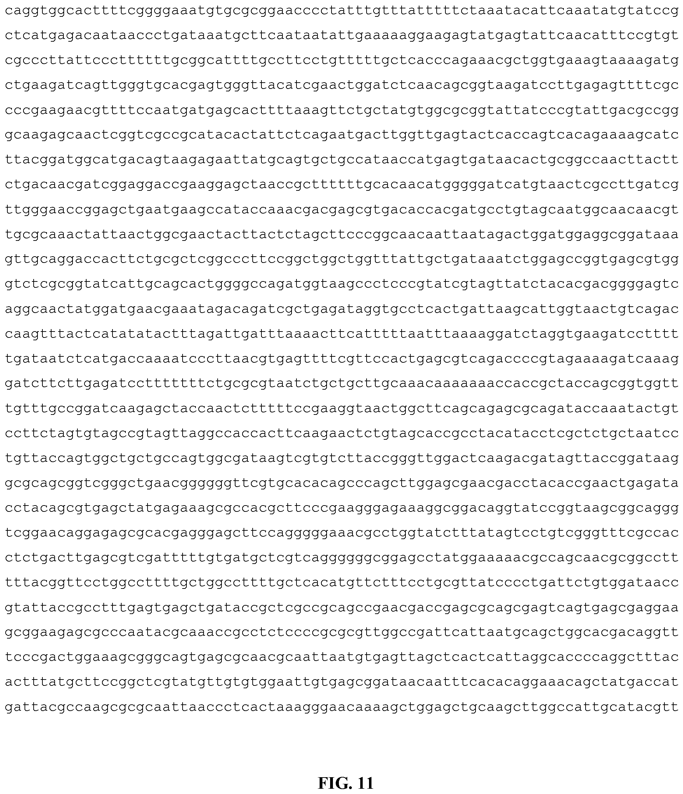

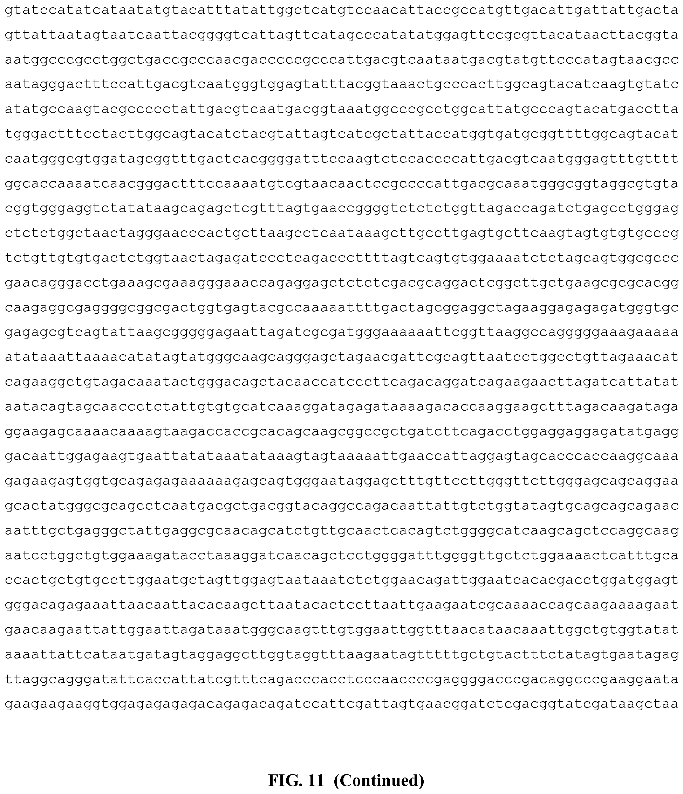

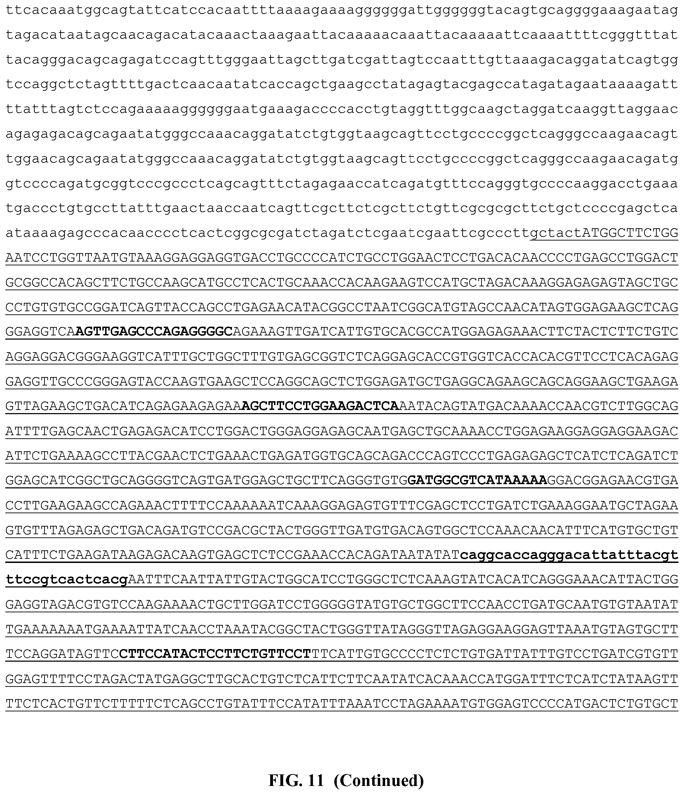

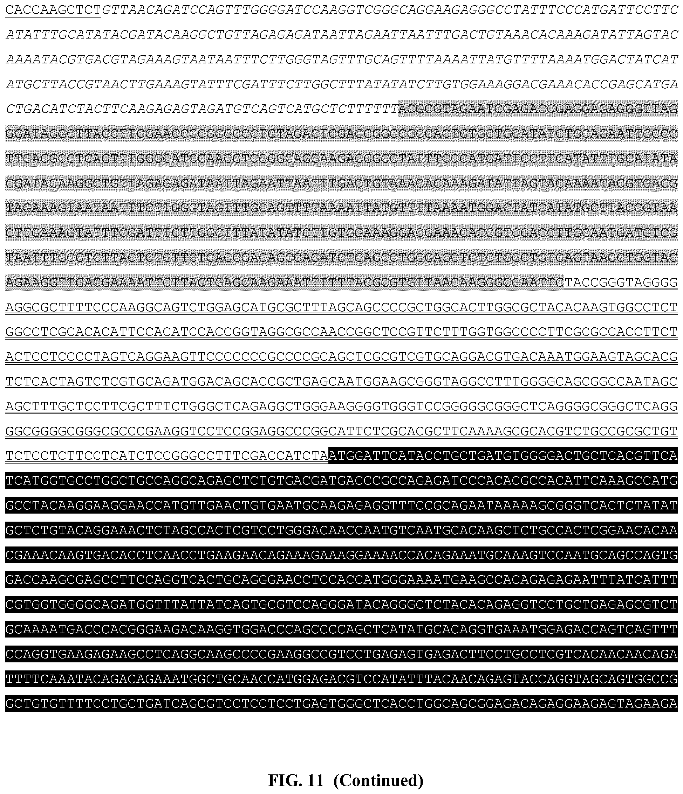

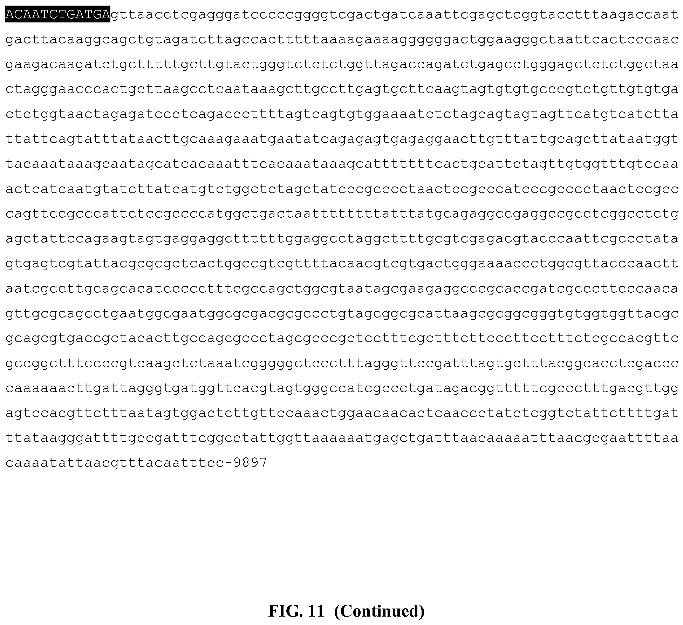

**FIG. 11** depicts SEQ ID NO: 22 which is the sequence of the vector from Figure 1A: CClc-MNDU3-antiHIV-PGK-fullCD25. Human/rhesus macaque TRIM5alpha is underlined. CCR5 shRNA is in italics. TAR decoy is shaded grey. The PGK promoter is double underlined. The full CD25 sequence is shown with white letters on a black background.

### Brief Description of Selected Sequence Listings

SEQ ID NO: 1 is the nucleotide sequence of the full CD25 pre-selective anti-HIV lentiviral vector backbone (9897 base pairs). The sequence is annotated as follows: 1) the underline portion is human/rhesus macaque TRIM5alpha sequence; 2) the italic portion is the 2A protease sequence; 3) the double underline portion is full CD25 sequence; 4) the black background/white type portion is the CCR5 shRNA sequence; and 5) the shaded portion is TAR decoy sequence.
SEQ ID NO: 2 is the nucleotide sequence of the partial truncated CD25 pre-selective anti-HIV lentiviral vector backbone (9870 base pairs). The sequence is annotated as follows: 1) the underline portion is human/rhesus macaque TRIM5alpha sequence; 2) the italic portion is the2A protease sequence; 3) the double underline portion is partial truncated CD25 sequence; 4) the black background/white type portion is the CCR5 shRNA; and 5) the shaded portion is TAR decoy sequence.
SEQ ID NO: 3 is the nucleotide sequence of the truncated CD25 pre-selective anti-HIV lentiviral vector backbone (9858 base pairs). The sequence is annotated as follows: 1) the underline portion is human/rhesus macaque TRIM5alpha sequence; 2) the italic portion is the 2A protease sequence; 3) the double underline portion is the truncated CD25 sequence; 4) the black background/white type portion is the CCR5 shRNA; and 5) the shaded portion is TAR decoy sequence.
SEQ ID NO: 4 is the nucleotide sequence of the full length CD25.
SEQ ID NO: 5 is the nucleotide sequence of the partial truncated CD25.
SEQ ID NO: 6 is the nucleotide sequence of the truncated CD25.
Alternative CCR5 RNAi for use in this invention are shown in SEQ ID NOS: 7-10, as well as a full length coding sequence for Human G-Protein Chemokine Receptor (CCR5), SEQ ID NO: 11 that is reproduced from GenBank Accession No. DM068065, last accessed on April 29, 2009.
SEQ ID NOS: 12-14 are additional TAR sequences for use in this invention.
SEQ ID NO: 15 is the sequence of a packaging plasmid sequence for use in this invention.
SEQ ID NO: 16 is an embodiment of polynucleotides encoding the pSINDBIS-ZZ envelope plasmid and transcribed by the polymerase-II CMV promoter into one messenger RNA. The pSINDBIS-ZZ plasmid comprises the E3 gene (SEQ ID NO: 17), the E2 gene (SEQ ID NO: 18) (the ZZ domain is between nucleotides 220 and 597), the 6K gene (SEQ ID NO: 19) and the E1 gene (SEQ ID NO: 20).
SEQ ID NO: 21 shows the sequence of the control vector with EGFP from Figure 1A: CCLc-MNDU3-EGFP-PGK-CD25, also shown in Figure 10. In Figure 10, the MNDU3 promoter is double underlined and shaded. EGFP is underlined, the PGK promoter is shaded grey, and the full CD25 sequence is in double underline. The 5' and 3' ends of the viral backbone are highlighted and underlined with a broken line.
SEQ ID NO: 22 shows the sequence of the vector from Figure 1A: CClc-MNDU3-antiHIV-PGK-fullCD25. Human/rhesus macaque TRIM5alpha is underlined. CCR5 shRNA is in italics. TAR decoy is shaded grey. The PGK promoter is double underlined. The full length CD25 is shown with white letters on a black background.
SEQ ID NO: 23 is an example of a CCR5 shRNA.
SEQ ID NO: 24 is an example of a human TRIM5alpha 11-aa patch. SEQ ID NO: 25 shows an example of a rhesus macaque TRIM5alpha 13-aa patch.
SEQ ID NO: 26 is a polynucleotide encoding an example of a human/rhesus macaque chimeric TRIM5alpha sequence. The first six nucleotides are the Kozak sequence followed by the ATG start codon. The nucleotides correspond to the rhesus macaque 13 amino acids inserted into the human TRIM5alpha sequence to make the chimeric protein (994-1032). The last 39 nucleotides at the end of the sequence (1492-1530) correspond to a hemmaglutinin tag which was put on the end of the protein coding sequence for detection of expression. These 39 nucleotides are followed by the TGA stop codon.
SEQ ID NO: 27 is an HIV TAR decoy sequence for use in the embodiments of this invention. The polymeraseIII U6 promoter is shown as nucleotides 1-283 followed by the TAR decoy sequence (284-415). The TAR decoy sequence is followed by a string of 6 thymidines which is the "transcriptional stop signal" for the U6 promoter.
SEQ ID NOS: 28-29 show examples of quantitative PCR (QPCR) primers sequences for the TRIM5α gene.
SEQ ID NOS: 30-31 show examples of quantitative PCR (QPCR) primers sequences for the myc gene.
SEQ ID NOS: 32-33 show examples of quantitative PCR (QPCR) primers sequences for the myb gene.
SEQ ID NOS: 34-35 show examples of quantitative PCR (QPCR) primers sequences for the fos gene.
SEQ ID NOS: 36-37 show examples of quantitative PCR (QPCR) primers sequences for the jun gene.
SEQ ID NO: 38 shows the nucleotide sequence of an equivalent truncated human CD25 nucleic acid. The nucleotide sequence has 80% or more sequence identity to SEQ ID NO: 6 or alternatively, it hybridizes under conditions of high stringency to the complement of SEQ ID NO: 6. Mutations in the sequence as compared to SEQ ID NO: 6 are underlined and in lowercase.

### MODES FOR CARRYING OUT THE INVENTION

### Definitions

Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods, devices, and materials are now described. Nothing herein is to be construed as an admission that the invention is not entitled to antedate such disclosure by virtue of prior invention.

The practice of the present invention will employ, unless otherwise indicated, conventional techniques of tissue culture, immunology, molecular biology, microbiology, cell biology and recombinant DNA, which are within the skill of the art. See, e.g., Sambrook and Russell eds. (2001) Molecular Cloning: A Laboratory Manual, 3rd edition; the series Ausubel et al. eds. (2007) Current Protocols in Molecular Biology; the series Methods in Enzymology (Academic Press, Inc., N.Y.); MacPherson et al. (1991) PCR 1: A Practical Approach (IRL Press at Oxford University Press); MacPherson et al. (1995) PCR 2: A Practical Approach; Harlow and Lane eds. (1999) Antibodies, A Laboratory Manual; Freshney (2005) Culture of Animal Cells: A Manual of Basic Techique, 5th edition; Gait ed. (1984) Oligonucleotide Synthesis; U.S. Patent No. 4,683,195; Hames and Higgins eds. (1984) Nucleic Acid Hybridization; Anderson (1999) Nucleic Acid Hybridization; Hames and Higgins eds. (1984) Transcription and Translation; Immobilized Cells and Enzymes (IRL Press (1986)); Perbal (1984) A Practical Guide to Molecular Cloning; Miller and Calos eds. (1987) Gene Transfer Vectors for Mammalian Cells (Cold Spring Harbor Laboratory); Makrides ed. (2003) Gene Transfer and Expression in Mammalian Cells; Mayer and Walker eds. (1987) Immunochemical Methods in Cell and Molecular Biology (Academic Press, London); Herzenberg et al. eds (1996) Weir's Handbook of Experimental Immunology; Manipulating the Mouse Embryo: A Laboratory Manual, 3rd edition (Cold Spring Harbor Laboratory Press (2002)); Sohail (ed.) (2004) Gene Silencing by RNA Interference: Technology and Application (CRC Press).

All numerical designations, e.g., pH, temperature, time, concentration, and molecular weight, including ranges, are approximations which are varied (+) or (-) by increments of 0.1 or 1.0, where appropriate. It is to be understood, although not always explicitly stated that all numerical designations are preceded by the term "about." It also is to be understood, although not always explicitly stated, that the reagents described herein are merely exemplary and that equivalents of such are known in the art.

As used in the specification and claims, the singular form "a", "an" and "the" include plural references unless the context clearly dictates otherwise. For example, the term "a cell" includes a plurality of cells, including mixtures thereof.

As used herein, the term "comprising" or "comprises" is intended to mean that the compositions and methods include the recited elements, but not excluding others. "Consisting essentially of' when used to define compositions and methods, shall mean excluding other elements of any essential significance to the combination for the stated purpose. Thus, a composition consisting essentially of the elements as defined herein would not exclude trace contaminants from the isolation and purification method and pharmaceutically acceptable carriers, such as phosphate buffered saline, preservatives and the like. "Consisting of' shall mean excluding more than trace elements of other ingredients and substantial method steps for administering the compositions of this invention or process steps to produce a composition or achieve an intended result. Embodiments defined by each of these transition terms are within the scope of this invention.

The term "isolated" as used herein with respect to nucleic acids, such as DNA or RNA, refers to molecules separated from other DNAs or RNAs, respectively that are present in the natural source of the macromolecule. The term "isolated nucleic acid" is meant to include nucleic acid fragments which are not naturally occurring as fragments and would not be found in the natural state. The term "isolated" is also used herein to refer to polypeptides, proteins and/or host cells that are isolated from other cellular proteins and is meant to encompass both purified and recombinant polypeptides. In other embodiments, the term "isolated" means separated from constituents, cellular and otherwise, in which the cell, tissue, polynucleotide, peptide, polypeptide, protein, antibody or fragment(s) thereof, which are normally associated in nature. For example, an isolated cell is a cell that is separated form tissue or cells of dissimilar phenotype or genotype. As is apparent to those of skill in the art, a non-naturally occurring polynucleotide, peptide, polypeptide, protein, antibody or fragment(s) thereof, does not require "isolation" to distinguish it from its naturally occurring counterpart.

As is known to those of skill in the art, there are 6 classes of viruses. The DNA viruses constitute classes I and II. The RNA viruses and retroviruses make up the remaining classes. Class III viruses have a double-stranded RNA genome. Class IV viruses have a positive single-stranded RNA genome, the genome itself acting as mRNA Class V viruses have a negative single-stranded RNA genome used as a template for mRNA synthesis. Class VI viruses have a positive single- stranded RNA genome but with a DNA intermediate not only in replication but also in mRNA synthesis. Retroviruses carry their genetic information in the form of RNA; however, once the virus infects a cell, the RNA is reverse-transcribed into the DNA form which integrates into the genomic DNA of the infected cell. The integrated DNA form is called a provirus.

The terms "polynucleotide", "nucleic acid" and "oligonucleotide" are used interchangeably and refer to a polymeric form of nucleotides of any length, either deoxyribonucleotides or ribonucleotides or analogs thereof. Polynucleotides can have any three-dimensional structure and may perform any function, known or unknown. The following are non-limiting examples of polynucleotides: a gene or gene fragment (for example, a probe, primer, EST or SAGE tag), exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes and primers. A polynucleotide can comprise modified nucleotides, such as methylated nucleotides and nucleotide analogs. If present, modifications to the nucleotide structure can be imparted before or after assembly of the polynucleotide. The sequence of nucleotides can be interrupted by non-nucleotide components. A polynucleotide can be further modified after polymerization, such as by conjugation with a labeling component. The term also refers to both double- and single-stranded molecules. Unless otherwise specified or required, any embodiment of this invention that is a polynucleotide encompasses both the double-stranded form and each of two complementary single-stranded forms known or predicted to make up the double-stranded form.

A polynucleotide is composed of a specific sequence of four nucleotide bases: adenine (A); cytosine (C); guanine (G); thymine (T); and uracil (U) for thymine when the polynucleotide is RNA. Thus, the term "polynucleotide sequence" is the alphabetical representation of a polynucleotide molecule. This alphabetical representation can be input into databases in a computer having a central processing unit and used for bioinformatics applications such as functional genomics and homology searching.

"Homology" or "identity" or "similarity" refers to sequence similarity between two peptides or between two nucleic acid molecules. Homology can be determined by comparing a position in each sequence which may be aligned for purposes of comparison. When a position in the compared sequence is occupied by the same base or amino acid, then the molecules are homologous at that position. A degree of homology between sequences is a function of the number of matching or homologous positions shared by the sequences. An "unrelated" or "non-homologous" sequence shares less than 40% identity, or alternatively less than 25% identity, with one of the sequences of the present invention.

A polynucleotide or polynucleotide region (or a polypeptide or polypeptide region) has a certain percentage (for example, 70%, 75%, 80%, 85%, 90%, 95%, 98% or 99%) of "sequence identity" to another sequence means that, when aligned, that percentage of bases (or amino acids) are the same in comparing the two sequences. This alignment and the percent homology or sequence identity can be determined using software programs known in the art, for example those described in Ausubel et al. eds. (2007) Current Protocols in Molecular Biology. Preferably, default parameters are used for alignment. One alignment program is BLAST, using default parameters. In particular, programs are BLASTN and BLASTP, using the following default parameters: Genetic code = standard; filter = none; strand = both; cutoff = 60; expect = 10; Matrix = BLOSUM62; Descriptions = 50 sequences; sort by = HIGH SCORE; Databases = non-redundant, GenBank + EMBL + DDBJ + PDB + GenBank CDS translations + SwissProtein + SPupdate + PIR. Details of these programs can be found at the following Internet address: http://www.ncbi.nlm.nih.gov/cgi-bin/BLAST.

An equivalent nucleic acid, polynucleotide or oligonucleotide is one having at least 80 % sequence identity, or alternatively at least 85 % sequence identity, or alternatively at least 90 % sequence identity, or alternatively at least 92 % sequence identity, or alternatively at least 95 % sequence identity, or alternatively at least 97 % sequence identity, or alternatively at least 98 % sequence identity to the reference nucleic acid, polynucleotide, or oligonucleotide, or alternatively an equivalent nucleic acid hybridizes under conditions of high stringency to a reference polynucleotide or its complement.

An equivalent polypeptide or protein is one having at least 80 % sequence identity, or alternatively at least 85 % sequence identity, or alternatively at least 90 % sequence identity, or alternatively at least 92 % sequence identity, or alternatively at least 95 % sequence identity, or alternatively at least 97 % sequence identity, or alternatively at least 98 % sequence identity to the reference polypeptide or protein, or alternatively an equivalent polypeptide or protein is one encoded by nucleic acid that hybridizes under conditions of high stringency to a polynucleotide or its complement that encodes the reference polypeptide or protein.

The expression "amplification of polynucleotides" includes methods such as PCR, ligation amplification (or ligase chain reaction, LCR) and amplification methods. These methods are known and widely practiced in the art. See, e.g., U.S. Pat. Nos. 4,683,195 and 4,683,202 and Innis et al., 1990 (for PCR); and Wu et al. (1989) Genomics 4:560-569 (for LCR). In general, the PCR procedure describes a method of gene amplification which is comprised of (i) sequence-specific hybridization of primers to specific genes within a DNA sample (or library), (ii) subsequent amplification involving multiple rounds of annealing, elongation, and denaturation using a DNA polymerase, and (iii) screening the PCR products for a band of the correct size. The primers used are oligonucleotides of sufficient length and appropriate sequence to provide initiation of polymerization, i.e. each primer is specifically designed to be complementary to each strand of the genomic locus to be amplified.

Reagents and hardware for conducting PCR are commercially available. Primers useful to amplify sequences from a particular gene region are preferably complementary to, and hybridize specifically to sequences in the target region or its flanking regions. Nucleic acid sequences generated by amplification may be sequenced directly. Alternatively the amplified sequence(s) may be cloned prior to sequence analysis. A method for the direct cloning and sequence analysis of enzymatically amplified genomic segments is known in the art.

A "gene" refers to a polynucleotide containing at least one open reading frame (ORF) that is capable of encoding a particular polypeptide or protein after being transcribed and translated.

The term "express" refers to the production of a gene product.

As used herein, "expression" refers to the process by which polynucleotides are transcribed into mRNA and/or the process by which the transcribed mRNA is subsequently being translated into peptides, polypeptides, or proteins. If the polynucleotide is derived from genomic DNA, expression may include splicing of the mRNA in a eukaryotic cell.

A "gene product" or alternatively a "gene expression product" refers to the amino acid (e.g., peptide or polypeptide) generated when a gene is transcribed and translated.

"Under transcriptional control" is a term well understood in the art and indicates that transcription of a polynucleotide sequence, usually a DNA sequence, depends on its being operatively linked to an element which contributes to the initiation of, or promotes, transcription. "Operatively linked" intends the polynucleotides are arranged in a manner that allows them to function in a cell. In one aspect, this invention provides promoters operatively linked to the downstream sequences, e.g., HIV TAR, CCR5, siRNA and TRIM5alpha.

The term "encode" as it is applied to polynucleotides refers to a polynucleotide which is said to "encode" a polypeptide if, in its native state or when manipulated by methods well known to those skilled in the art, it can be transcribed and/or translated to produce the mRNA for the polypeptide and/or a fragment thereof. The antisense strand is the complement of such a nucleic acid, and the encoding sequence can be deduced therefrom.

A "probe" when used in the context of polynucleotide manipulation refers to an oligonucleotide that is provided as a reagent to detect a target potentially present in a sample of interest by hybridizing with the target. Usually, a probe will comprise a detectable label or a means by which a label can be attached, either before or subsequent to the hybridization reaction. Alternatively, a "probe" can be a biological compound such as a polypeptide, antibody, or fragments thereof that is capable of binding to the target potentially present in a sample of interest.

"Detectable labels" or "markers" include, but are not limited to radioisotopes, fluorochromes, chemiluminescent compounds, dyes, and proteins, including enzymes. Detectable labels can also be attached to a polynucleotide, polypeptide, antibody or composition described herein.

A "primer" is a short polynucleotide, generally with a free 3' -OH group that binds to a target or "template" potentially present in a sample of interest by hybridizing with the target, and thereafter promoting polymerization of a polynucleotide complementary to the target. A "polymerase chain reaction" ("PCR") is a reaction in which replicate copies are made of a target polynucleotide using a "pair of primers" or a "set of primers" consisting of an "upstream" and a "downstream" primer, and a catalyst of polymerization, such as a DNA polymerase, and typically a thermally-stable polymerase enzyme. Methods for PCR are well known in the art, and taught, for example in MacPherson et al. (1991) PCR 1: A Practical Approach (IRL Press at Oxford University Press). All processes of producing replicate copies of a polynucleotide, such as PCR or gene cloning, are collectively referred to herein as "replication." A primer can also be used as a probe in hybridization reactions, such as Southern or Northern blot analyses. Sambrook and Russell (2001), infra.

"Hybridization" refers to a reaction in which one or more polynucleotides react to form a complex that is stabilized via hydrogen bonding between the bases of the nucleotide residues. The hydrogen bonding may occur by Watson-Crick base pairing, Hoogstein binding, or in any other sequence-specific manner. The complex may comprise two strands forming a duplex structure, three or more strands forming a multi-stranded complex, a single self-hybridizing strand, or any combination of these. A hybridization reaction may constitute a step in a more extensive process, such as the initiation of a PCR reaction, or the enzymatic cleavage of a polynucleotide by a ribozyme.

Hybridization reactions can be performed under conditions of different "stringency". In general, a low stringency hybridization reaction is carried out at about 40 °C in 10 x SSC or a solution of equivalent ionic strength/temperature. A moderate stringency hybridization is typically performed at about 50 °C in 6 x SSC, and a high stringency hybridization reaction is generally performed at about 60 °C in 1 x SSC. Hybridization reactions can also be performed under "physiological conditions" which is well known to one of skill in the art. A non-limiting example of a physiological condition is the temperature, ionic strength, pH and concentration of Mg²⁺ normally found in a cell.

When hybridization occurs in an antiparallel configuration between two single-stranded polynucleotides, the reaction is called "annealing" and those polynucleotides are described as "complementary". A double-stranded polynucleotide can be "complementary" or "homologous" to another polynucleotide, if hybridization can occur between one of the strands of the first polynucleotide and the second. "Complementarity" or "homology" (the degree that one polynucleotide is complementary with another) is quantifiable in terms of the proportion of bases in opposing strands that are expected to form hydrogen bonding with each other, according to generally accepted base-pairing rules.

The term "propagate" means to grow a cell or population of cells. The term "growing" also refers to the proliferation of cells in the presence of supporting media, nutrients, growth factors, support cells, or any chemical or biological compound necessary for obtaining the desired number of cells or cell type.

The term "culturing" refers to the *in vitro* propagation of cells or organisms on or in media of various kinds. It is understood that the descendants of a cell grown in culture may not be completely identical (i.e., morphologically, genetically, or phenotypically) to the parent cell.

A "viral vector" is defined as a recombinantly produced virus or viral particle that comprises a polynucleotide to be delivered into a host cell, either *in vivo, ex vivo* or *in vitro.* Examples of viral vectors include retroviral vectors, lentiviral vectors, adenovirus vectors, adeno-associated virus vectors, alphavirus vectors and the like. Alphavirus vectors, such as Semliki Forest virus-based vectors and Sindbis virus-based vectors, have also been developed for use in gene therapy and immunotherapy. See, Schlesinger and Dubensky (1999) Curr. Opin. Biotechnol. 5:434-439 and Ying, et al. (1999) Nat. Med. 5(7):823-827.

In aspects where gene transfer is mediated by a lentiviral vector, a vector construct refers to the polynucleotide comprising the lentiviral genome or part thereof, and a therapeutic gene. As used herein, "lentiviral mediated gene transfer" or "lentiviral transduction" carries the same meaning and refers to the process by which a gene or nucleic acid sequences are stably transferred into the host cell by virtue of the virus entering the cell and integrating its genome into the host cell genome. The virus can enter the host cell via its normal mechanism of infection or be modified such that it binds to a different host cell surface receptor or ligand to enter the cell. Retroviruses carry their genetic information in the form of RNA; however, once the virus infects a cell, the RNA is reverse-transcribed into the DNA form which integrates into the genomic DNA of the infected cell. The integrated DNA form is called a provirus. As used herein, lentiviral vector refers to a viral particle capable of introducing exogenous nucleic acid into a cell through a viral or viral-like entry mechanism. A "lentiviral vector" is a type of retroviral vector well-known in the art that has certain advantages in transducing nondividing cells as compared to other retroviral vectors. See, Trono D. (2002) Lentiviral vectors, New York: Spring-Verlag Berlin Heidelberg.

Lentiviral vectors of this invention are based on or derived from oncoretroviruses (the sub-group of retroviruses containing MLV), and lentiviruses (the sub-group of retroviruses containing HIV). Examples include ASLV, SNV and RSV all of which have been split into packaging and vector components for lentiviral vector particle production systems. The lentiviral vector particle according to the invention may be based on a genetically or otherwise (e.g. by specific choice of packaging cell system) altered version of a particular retrovirus.

That the vector particle according to the invention is "based on" a particular retrovirus means that the vector is derived from that particular retrovirus. The genome of the vector particle comprises components from that retrovirus as a backbone. The vector particle contains essential vector components compatible with the RNA genome, including reverse transcription and integration systems. Usually these will include gag and pol proteins derived from the particular retrovirus. Thus, the majority of the structural components of the vector particle will normally be derived from that retrovirus, although they may have been altered genetically or otherwise so as to provide desired useful properties. However, certain structural components and in particular the env proteins, may originate from a different virus. The vector host range and cell types infected or transduced can be altered by using different env genes in the vector particle production system to give the vector particle a different specificity.

As used herein, "stem cell" defines a cell with the ability to divide for indefinite periods in culture and give rise to specialized cells. At this time and for convenience, stem cells are categorized as somatic (adult) or embryonic. A somatic stem cell is an undifferentiated cell found in a differentiated tissue that can renew itself (clonal) and (with certain limitations) differentiate to yield all the specialized cell types of the tissue from which it originated. An embryonic stem cell is a primitive (undifferentiated) cell from the embryo that has the potential to become a wide variety of specialized cell types. An embryonic stem cell is one that has been cultured under in vitro conditions that allow proliferation without differentiation for months to years. A clone is a line of cells that is genetically identical to the originating cell; in this case, a stem cell.

As used herein, an "antibody" includes whole antibodies and any antigen binding fragment or a single chain thereof. Thus the term "antibody" includes any protein or peptide containing molecule that comprises at least a portion of an immunoglobulin molecule. Examples of such include, but are not limited to a complementarity determining region (CDR) of a heavy or light chain or a ligand binding portion thereof, a heavy chain or light chain variable region, a heavy chain or light chain constant region, a framework (FR) region, or any portion thereof, or at least one portion of a binding protein, any of which can be incorporated into an antibody of the present invention. The term "antibody" is further intended to encompass digestion fragments, specified portions, derivatives and variants thereof, including antibody mimetics or comprising portions of antibodies that mimic the structure and/or function of an antibody or specified fragment or portion thereof, including single chain antibodies and fragments thereof. Examples of binding fragments encompassed within the term "antigen binding portion" of an antibody include a Fab fragment, a monovalent fragment consisting of the V_{L}, V_{H}, C_{L} and CH, domains; a F(ab')2 fragment, a bivalent fragment comprising two Fab fragments linked by a disulfide bridge at the hinge region; a Fd fragment consisting of the V_{H} and C_{H}, domains; a Fv fragment consisting of the V_{L} and V_{H} domains of a single arm of an antibody, a dAb fragment (Ward et al. (1989) Nature 341:544-546), which consists of a V_{H} domain; and an isolated complementarity determining region (CDR). Furthermore, although the two domains of the Fv fragment, V_{L} and V_{H}, are coded for by separate genes, they can be joined, using recombinant methods, by a synthetic linker that enables them to be made as a single protein chain in which the V_{L} and V_{H} regions pair to form monovalent molecules (known as single chain Fv (scFv)). Bird et al. (1988) Science 242:423-426 and Huston et al. (1988) Proc. Natl. Acad Sci. USA 85:5879-5883. Single chain antibodies are also intended to be encompassed within the term "fragment of an antibody." Any of the above-noted antibody fragments are obtained using conventional techniques known to those of skill in the art, and the fragments are screened for binding specificity and neutralization activity in the same manner as are intact antibodies.

As used herein, an "antibody" includes whole antibodies and any antigen binding fragment or a single chain thereof. Thus the term "antibody" includes any protein or peptide containing molecule that comprises at least a portion of an immunoglobulin molecule. Examples of such include, but are not limited to a complementarity determining region (CDR) of a heavy or light chain or a ligand binding portion thereof, a heavy chain or light chain variable region, a heavy chain or light chain constant region, a framework (FR) region, or any portion thereof, or at least one portion of a binding protein, any of which can be incorporated into an antibody of the present invention. The term "antibody" is further intended to encompass digestion fragments, specified portions, derivatives and variants thereof, including antibody mimetics or comprising portions of antibodies that mimic the structure and/or function of an antibody or specified fragment or portion thereof, including single chain antibodies and fragments thereof. It also includes in some aspects, antibody variants, polyclonal antibodies, human antibodies, humanized antibodies, chimeric antibodies, antibody derivatives, a bispecific molecule, a multispecific molecule, a heterospecific molecule, heteroantibodies and human monoclonal antibodies.

Examples of binding fragments encompassed within the term "antigen binding portion" of an antibody include a Fab fragment, a monovalent fragment consisting of the V_{L}, V_{H}, C_{L} and CH, domains; a F(ab')2 fragment, a bivalent fragment comprising two Fab fragments linked by a disulfide bridge at the hinge region; a Fd fragment consisting of the V_{H} and C_{H}, domains; a Fv fragment consisting of the V_{L} and V_{H} domains of a single arm of an antibody, a dAb fragment (Ward et al. (1989) Nature 341:544-546), which consists of a V_{H} domain; and an isolated complementarity determining region (CDR). Furthermore, although the two domains of the Fv fragment, V_{L} and V_{H}, are coded for by separate genes, they can be joined, using recombinant methods, by a synthetic linker that enables them to be made as a single protein chain in which the V_{L} and V_{H} regions pair to form monovalent molecules (known as single chain Fv (scFv)). Bird et al. (1988) Science 242:423-426 and Huston et al. (1988) Proc. Natl. Acad Sci. USA 85:5879-5883. Single chain antibodies are also intended to be encompassed within the term "fragment of an antibody." Any of the above-noted antibody fragments are obtained using conventional techniques known to those of skill in the art, and the fragments are screened for binding specificity and neutralization activity in the same manner as are intact antibodies.

The term "antibody variant" is intended to include antibodies produced in a species other than a mouse. It also includes antibodies containing post-translational modifications to the linear polypeptide sequence of the antibody or fragment. It further encompasses fully human antibodies.

The term "antibody derivative" is intended to encompass molecules that bind an epitope as defined above and which are modifications or derivatives of a native monoclonal antibody of this invention. Derivatives include, but are not limited to, for example, bispecific, multispecific, heterospecific, trispecific, tetraspecific, multispecific antibodies, diabodies, chimeric, recombinant and humanized.

The term "bispecific molecule" is intended to include any agent, e.g., a protein, peptide, or protein or peptide complex, which has two different binding specificities. The term "multispecific molecule" or "heterospecific molecule" is intended to include any agent, e.g. a protein, peptide, or protein or peptide complex, which has more than two different binding specificities.

The term "heteroantibodies" refers to two or more antibodies, antibody binding fragments (e.g., Fab), derivatives thereof, or antigen binding regions linked together, at least two of which have different specificities.

The term "human antibody" as used herein, is intended to include antibodies having variable and constant regions derived from human germline immunoglobulin sequences. The human antibodies of the invention may include amino acid residues not encoded by human germline immunoglobulin sequences (e.g., mutations introduced by random or site-specific mutagenesis *in vitro* or by somatic mutation *in vivo*). However, the term "human antibody" as used herein, is not intended to include antibodies in which CDR sequences derived from the germline of another mammalian species, such as a mouse, have been grafted onto human framework sequences. Thus, as used herein, the term "human antibody" refers to an antibody in which substantially every part of the protein (e.g., CDR, framework, C_{L}, C_{H} domains (e.g., C_{H1}, C_{H2}, C_{H3}), hinge, (VL, VH)) is substantially non-immunogenic in humans, with only minor sequence changes or variations. Similarly, antibodies designated primate (monkey, baboon, chimpanzee, etc.), rodent (mouse, rat, rabbit, guinea pig, hamster, and the like) and other mammals designate such species, sub-genus, genus, sub-family, family specific antibodies. Further, chimeric antibodies include any combination of the above. Such changes or variations optionally and preferably retain or reduce the immunogenicity in humans or other species relative to non-modified antibodies. Thus, a human antibody is distinct from a chimeric or humanized antibody. It is pointed out that a human antibody can be produced by a non-human animal or prokaryotic or eukaryotic cell that is capable of expressing functionally rearranged human immunoglobulin (e.g., heavy chain and/or light chain) genes. Further, when a human antibody is a single chain antibody, it can comprise a linker peptide that is not found in native human antibodies. For example, an Fv can comprise a linker peptide, such as two to about eight glycine or other amino acid residues, which connects the variable region of the heavy chain and the variable region of the light chain. Such linker peptides are considered to be of human origin.

As used herein, a human antibody is "derived from" a particular germline sequence if the antibody is obtained from a system using human immunoglobulin sequences, e.g., by immunizing a transgenic mouse carrying human immunoglobulin genes or by screening a human immunoglobulin gene library. A human antibody that is "derived from" a human germline immunoglobulin sequence can be identified as such by comparing the amino acid sequence of the human antibody to the amino acid sequence of human germline immunoglobulins. A selected human antibody typically is at least 90% identical in amino acids sequence to an amino acid sequence encoded by a human germline immunoglobulin gene and contains amino acid residues that identify the human antibody as being human when compared to the germline immunoglobulin amino acid sequences of other species (e.g., murine germline sequences). In certain cases, a human antibody may be at least 95%, or even at least 96%, 97%, 98%, or 99% identical in amino acid sequence to the amino acid sequence encoded by the germline immunoglobulin gene. Typically, a human antibody derived from a particular human germline sequence will display no more than 10 amino acid differences from the amino acid sequence encoded by the human germline immunoglobulin gene. In certain cases, the human antibody may display no more than 5, or even no more than 4, 3, 2, or 1 amino acid difference from the amino acid sequence encoded by the germline immunoglobulin gene.

The terms "monoclonal antibody" or "monoclonal antibody composition" as used herein refer to a preparation of antibody molecules of single molecular composition. A monoclonal antibody composition displays a single binding specificity and affinity for a particular epitope.

A "human monoclonal antibody" refers to antibodies displaying a single binding specificity which have variable and constant regions derived from human germline immunoglobulin sequences.

The term "recombinant human antibody", as used herein, includes all human antibodies that are prepared, expressed, created or isolated by recombinant means, such as antibodies isolated from an animal (e.g., a mouse) that is transgenic or transchromosomal for human immunoglobulin genes or a hybridoma prepared therefrom, antibodies isolated from a host cell transformed to express the antibody, e.g., from a transfectoma, antibodies isolated from a recombinant, combinatorial human antibody library, and antibodies prepared, expressed, created or isolated by any other means that involve splicing of human immunoglobulin gene sequences to other DNA sequences. Such recombinant human antibodies have variable and constant regions derived from human germline immunoglobulin sequences. In certain embodiments, however, such recombinant human antibodies can be subjected to *in vitro* mutagenesis (or, when an animal transgenic for human Ig sequences is used, *in vivo* somatic mutagenesis) and thus the amino acid sequences of the VH and VL regions of the recombinant antibodies are sequences that, while derived from and related to human germline VH and VL sequences, may not naturally exist within the human antibody germline repertoire *in vivo.*

"RNA interference" (RNAi) refers to sequence-specific or gene specific suppression of gene expression (protein synthesis) that is mediated by short interfering RNA (siRNA).

"Short interfering RNA" (siRNA) refers to double-stranded RNA molecules (dsRNA), generally, from about 10 to about 30 nucleotides in length that are capable of mediating RNA interference (RNAi), or 11 nucleotides in length, 12 nucleotides in length, 13 nucleotides in length, 14 nucleotides in length, 15 nucleotides in length, 16 nucleotides in length, 17 nucleotides in length, 18 nucleotides in length, 19 nucleotides in length, 20 nucleotides in length, 21 nucleotides in length, 22 nucleotides in length, 23 nucleotides in length, 24 nucleotides in length, 25 nucleotides in length, 26 nucleotides in length, 27 nucleotides in length, 28 nucleotides in length, or 29 nucleotides in length. As used herein, the term siRNA includes short hairpin RNAs (shRNAs).

"Double stranded RNA" (dsRNA) refer to double stranded RNA molecules that may be of any length and may be cleaved intracellularly into smaller RNA molecules, such as siRNA. In cells that have a competent interferon response, longer dsRNA, such as those longer than about 30 base pair in length, may trigger the interferon response. In other cells that do not have a competent interferon response, dsRNA may be used to trigger specific RNAi.

The term siRNA includes short hairpin RNAs (shRNAs). shRNAs comprise a single strand of RNA that forms a stem-loop structure, where the stem consists of the complementary sense and antisense strands that comprise a double-stranded siRNA, and the loop is a linker of varying size. The stem structure of shRNAs generally is from about 10 to about 30 nucleotides in length. For example, the stem can be 10-30 nucleotides in length, or alternatively, 12-28 nucleotides in length, or alternatively, 15-25 nucleotides in length, or alternatively, 19-23 nucleotides in length, or alternatively, 21-23 nucleotides in length.

Tools to assist siRNA design are readily available to the public. For example, a computer-based siRNA design tool is available on the internet at www.dharmacon.com, Ambion-www.ambion.com/jp/techlib/misc/siRNA_finder.html; Thermo Scientific-Dharmacon-www.dharmacon.com/DesignCenter/DesignCenterPage.aspx; Bioinformatics Research Center-sysbio.kribb.re.kr:8080/AsiDesigner/menuDesigner.jsf; and Invitrogen-rnaidesigner.invitrogen.com/rnaiexpress/.

Without being bound by theory, it is generally believed that the primary cellular receptor for HIV entry is the CD4 receptor. In addition to CD4 expression, CXCR4, and CCR5 are necessary co-factors that allow HIV entry when co-expressed with CD4 on a cell surface.

CXCR4, or fusin, is expressed on T cells (Feng et al. (1996) Science 10:272(5263):872-7. Co-expression of CXCR4 and CD4 on a cell allow T-tropic HIV isolates to fuse with and infect the cell. HIV gp120 interacts with both CD4 and CXCR4 to adhere to the cell and to effect conformational changes in the gp120/gp41 complex that allow membrane fusion by gp41.

CCR5 is another co-receptor that is expressed on macrophages and on some populations of T cells, can also function in concert with CD4 to allow HIV membrane fusion (Deng et al. (1996) Nature, Jun 20;381(6584):661-6.

TRIM5alpha is 493 amino acid protein that is found in most primate cells that appears to act to interfere with the replication of retrovirus in infected cells. The human protein sequence is published in GenBank (Accession number NP_149023) and the mRNA sequence also has been published (NM_033034). Murine protein sequence is available at NP_783608 and mRNA is available at NM_175677. (All last accessed April 29, 2009).

HIV is a retrovirus that is rougly spherical with a diameter of about 120 nm. HIV is composed of two copies of positive single-stranded RNA that codes for the virus' nine genes enclosed by a conical capsid composed of 2,000 copies of the viral protein p24. The single-stranded RNA is tightly bound to nucleocapsid proteins, p7 and enzymes needed for the development of the virion such as reverse transcriptase, proteases, ribonuclease and integrase. A matrix composed of the viral protein p17 surrounds the capsid ensuring the integrity of the virion particle. The RNA genome of HIV consists of at least seven structural landmarks (LTR,TAR, RRE, PE, SLIP, CRS, and INS) and nine genes (gag, pol, and env, tat, rev, nef, vif, vpr, vpu, and tev) encoding 19 proteins. Three of these genes, gag, pol, and env, contain information needed to make the structural proteins for new virus particles. For example, env codes for a protein called gp160 that is broken down by a viral enzyme to form gp120 and gp41. The six remaining genes, tat, rev, nef, vif, vpr, and vpu (or vpx in the case of HIV-2), are regulatory genes for proteins that control the ability of HIV to infect cells, produce new copies of virus (replicate), or cause disease. The two Tat proteins (p16 and p14) are transcriptional transactivators for the LTR promoter acting by binding the TAR RNA element. Activation of HIV-1 gene expression by the transactivator Tat is dependent on the RNA regulatory element (TAR) located downstream of the transcription initiation site. This element forms a stable stem-loop structure and can be bound by either the protein encoded by this gene or by RNA polymerase II. This protein may act to disengage RNA polymerase II from TAR during transcriptional elongation. Alternatively spliced transcripts of this gene may exist, but their full-length natures have not been determined. The mRNA sequence is known in the art and reported at NM_005646, last accessed on August 3, 2013.

An "shRNA CCR5" is an interfering RNA that down regulates or suppresses expression of the CCR5 polynucleotide, examples of which are shown in SEQ ID NO: 11. CCR5 is also known as Human G-protein Chemokine Receptor HDGNR10. Isolated polynucleotides encoding this protein are known in the art and described, for example in US Patent No. 7,501,123.

The term "CD25" refers to the low affinity IL-2 receptor alpha subunit transmembrane protein. CD25 is the alpha chain of the IL-2 receptor, and is not normally found on CD34+ hematopoietic progenitor cells (HPCs). In addition to the CD25 sequences described herein, the GenBank Accession numbers: NP_000408.1 and NP_032393.3 are examples of the human and mouse CD25 protein sequences, respectively. The extracellular domain of CD25 refers to the first 657 nucleotides of the CD25. For example, the extracellular domain of CD25 is shown in the first 657 nucleotides of SEQ ID NO: 4. SEQ ID NOS: 6 and 38 depict truncated versions of CD25. SEQ ID NO: 38 is an example of an equivalent of SEQ ID NO: 6.

The term "an expression control element" as used herein, intends a polynucleotide that is operatively linked to a target polynucleotide to be transcribed, and facilitates the expression of the target polynucleotide. A promoter is an example of an expression control element.

A promoter is a regulatory polynucleotide, usually located 5' or upstream of a gene or other polynucleotide, that provides a control point for regulated gene transcription. Polymerase II and III are examples of promoters.

A polymerase II or "pol II" promoter catalyzes the transcription of DNA to synthesize precursors of mRNA, and most shRNA and microRNA. Examples of pol II promoters are known in the art and include without limitation, the phosphoglycerate kinase ("PGK") promoter; EF1-alpha; CMV (minimal cytomegalovirus promoter); and LTRs from retroviral and lentiviral vectors.

A polymerase III or "pol III" promoter is a polynucleotide found in eukaryotic cells that transcribes DNA to synthesize ribosomal 5S rRNA, tRNA and other small RNAs. Examples of pol III promoters include without limitation a U6 promoter or an MNDU3 promoter.

A "target cell" as used herein, shall intend a cell containing the genome into which polynucleotides that are operatively linked to an expression control element are to be integrated. Cells that are infected with HIV or susceptible to HIV infection are examples of target cells.

As used herein, the term "reporter marker" intends a polynucleotide, detectable label or other molecule that allows for the identification of a preselected composition. Non-limiting examples of reporter markers include, without limitation CD25, a hemmaglutinin tag, an enhanced green fluorescent protein (EGFP), a red flouresence protein (RFP), a green fluorescent protein (GFP) and yellow fluorescent protein (YFP) or the like. These are commercially available and described in the technical art.

A "composition" is intended to mean a combination of active polypeptide, polynucleotide or antibody and another compound or composition, inert (e.g. a detectable label) or active (e.g. a gene delivery vehicle).

A "pharmaceutical composition" is intended to include the combination of an active polypeptide, polynucleotide or antibody with a carrier, inert or active such as a solid support, making the composition suitable for diagnostic or therapeutic use *in vitro, in vivo* or *ex vivo.*

As used herein, the term "pharmaceutically acceptable carrier" encompasses any of the standard pharmaceutical carriers, such as a phosphate buffered saline solution, water, and emulsions, such as an oil/water or water/oil emulsion, and various types of wetting agents. The compositions also can include stabilizers and preservatives. For examples of carriers, stabilizers and adjuvants, see Martin (1975) Remington's Pharm. Sci., 15th Ed. (Mack Publ. Co., Easton).

A "subject," "individual" or "patient" is used interchangeably herein, and refers to a vertebrate, preferably a mammal, more preferably a human. Mammals include, but are not limited to, murines, rats, rabbit, simians, bovines, ovine, porcine, canines, feline, farm animals, sport animals, pets, equine, and primate, particularly human. Besides being useful for human treatment, the present invention is also useful for veterinary treatment of companion mammals, exotic animals and domesticated animals, including mammals, rodents, and the like which is susceptible to RNA and in particular, HIV viral infection. In one embodiment, the mammals include horses, dogs, and cats. In another embodiment of the present invention, the human is an adolescent or infant under the age of eighteen years of age.

"Host cell" refers not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

An "enriched population" of cells intends a substantially homogenous population of cells having certain defined characteristics. The cells are greater than 70 %, or alternatively greater than 75 %, or alternatively greater than 80 %, or alternatively greater than 85 %, or alternatively greater than 90 %, or alternatively greater than 95 %, or alternatively greater than 98% identical in the defined characteristics. In one aspect, the substantially homogenous population of cells express CD25 on the surface and contain an exogenous CCR5 polynucleotide. In a further aspect, the cells further comprise an exogenous TRIM5alpha polynucleotide and/or HIV TAR polyncleotide.

The terms "disease," "disorder," and "condition" are used inclusively and refer to any condition mediated at least in part by infection by an RNA virus such as HIV.

"Treating" or "treatment" of a disease includes: (1) preventing the disease, i.e., causing the clinical symptoms of the disease not to develop in a patient that may be predisposed to the disease but does not yet experience or display symptoms of the disease; (2) inhibiting the disease, i.e., arresting or reducing the development of the disease or its clinical symptoms; or (3) relieving the disease, i.e., causing regression of the disease or its clinical symptoms.

The term "suffering" as it related to the term "treatment" refers to a patient or individual who has been diagnosed with or is predisposed to infection or a disease incident to infection. A patient may also be referred to being "at risk of suffering" from a disease because of active or latent infection. This patient has not yet developed characteristic disease pathology.

An "effective amount" is an amount sufficient to effect beneficial or desired results. An effective amount can be administered in one or more administrations, applications or dosages. Such delivery is dependent on a number of variables including the time period for which the individual dosage unit is to be used, the bioavailability of the therapeutic agent, the route of administration, etc. It is understood, however, that specific dose levels of the therapeutic agents of the present invention for any particular subject depends upon a variety of factors including the activity of the specific compound employed, the age, body weight, general health, sex, and diet of the subject, the time of administration, the rate of excretion, the drug combination, and the severity of the particular disorder being treated and form of administration. Treatment dosages generally may be titrated to optimize safety and efficacy. Typically, dosage-effect relationships from *in vitro* and/or *in vivo* tests initially can provide useful guidance on the proper doses for patient administration. In general, one will desire to administer an amount of the compound that is effective to achieve a serum level commensurate with the concentrations found to be effective in vitro. Determination of these parameters is well within the skill of the art. These considerations, as well as effective formulations and administration procedures are well known in the art and are described in standard textbooks. Consistent with this definition, as used herein, the term "therapeutically effective amount" is an amount sufficient to inhibit RNA virus replication *ex vivo, in vitro* or *in vivo.*

The term administration shall include without limitation, administration by oral, parenteral (e.g., intramuscular, intraperitoneal, intravenous, ICV, intracisternal injection or infusion, subcutaneous injection, or implant), by inhalation spray nasal, vaginal, rectal, sublingual, urethral (e.g., urethral suppository) or topical routes of administration (e.g., gel, ointment, cream, aerosol, etc.) and can be formulated, alone or together, in suitable dosage unit formulations containing conventional non-toxic pharmaceutically acceptable carriers, adjuvants, excipients, and vehicles appropriate for each route of administration. The invention is not limited by the route of administration, the formulation or dosing schedule.

### Descriptive Embodiments

### Vectors

The invention is defined in the claims. This invention provides a vector comprising, or alternatively consisting essentially of, or yet further consisting of a viral backbone. In one aspect, the viral backbone contains essential nucleic acids or sequences for integration into a target cell's genome. In one aspect, the essential nucleice acids necessary for integration of the genome of the target cell include at the 5' and 3' ends the minimal LTR regions required for integration of the vector. The vector also comprises, or alternatively consists essentially of, or yet further consists of a nucleic acid encoding a CCR5 RNAi; an operatively linked first expression control element that regulates expression of the nucleic acid encoding the CCR5 RNAi element; a nucleic acid encoding at least the extracellular domain of CD25 or an equivalent thereof; and an operatively linked second expression control element that regulates expression of the nucleic acid encoding at least the extracellular domain of CD25 or an equivalent thereof operatively linked to it.

In one aspect, the term "vector" intends a recombinant vector that retains the ability to infect and transduce non-dividing and/or slowly-dividing cells and integrate into the target cell's genome. In several aspects, the vector is derived from or based on a wild-type virus. In further aspects, the vector is derived from or based on a wild-type lentivirus. Examples of such, include without limitation, human immunodeficiency virus (HIV), equine infectious anaemia virus (EIAV), simian immunodeficiency virus (SIV) and feline immunodeficiency virus (FIV). Alternatively, it is contemplated that other retrovirus can be used as a basis for a vector backbone such murine leukemia virus (MLV). It will be evident that a viral vector according to the invention need not be confined to the components of a particular virus. The viral vector may comprise components derived from two or more different viruses, and may also comprise synthetic components. Vector components can be manipulated to obtain desired characteristics, such as target cell specificity.

The recombinant vectors of this invention are derived from primates and non-primates. Examples of primate lentiviruses include the human immunodeficiency virus (HIV), the causative agent of human acquired immunodeficiency syndrome (AIDS), and the simian immunodeficiency virus (SIV). The non-primate lentiviral group includes the prototype "slow virus" visna/maedi virus (VMV), as well as the related caprine arthritis-encephalitis virus (CAEV), equine infectious anaemia virus (EIAV) and the more recently described feline immunodeficiency virus (FIV) and bovine immunodeficiency virus (BIV). Prior art recombinant lentiviral vectors are known in the art, e.g., see US Patent Nos. 6,924,123; 7,056,699; 7,07,993; 7,419,829 and 7,442,551.

U.S. Patent No. 6,924,123 discloses that certain retroviral sequence facilitate integration into the target cell genome. This patent teaches that each retroviral genome comprises genes called gag, pol and env which code for virion proteins and enzymes. These genes are flanked at both ends by regions called long terminal repeats (LTRs). The LTRs are responsible for proviral integration, and transcription. They also serve as enhancer-promoter sequences. In other words, the LTRs can control the expression of the viral genes. Encapsidation of the retroviral RNAs occurs by virtue of a psi sequence located at the 5' end of the viral genome. The LTRs themselves are identical sequences that can be divided into three elements, which are called U3, R and U5. U3 is derived from the sequence unique to the 3' end of the RNA. R is derived from a sequence repeated at both ends of the RNA, and U5 is derived from the sequence unique to the 5'end of the RNA. The sizes of the three elements can vary considerably among different retroviruses. For the viral genome. and the site of poly (A) addition (termination) is at the boundary between R and U5 in the right hand side LTR. U3 contains most of the transcriptional control elements of the provirus, which include the promoter and multiple enhancer sequences responsive to cellular and in some cases, viral transcriptional activator proteins.

With regard to the structural genes gag, pol and env themselves, gag encodes the internal structural protein of the virus. Gag protein is proteolytically processed into the mature proteins MA (matrix), CA (capsid) and NC (nucleocapsid). The pol gene encodes the reverse transcriptase (RT), which contains DNA polymerase, associated RNase H and integrase (IN), which mediate replication of the genome.

For the production of viral vector particles, the vector RNA genome is expressed from a DNA construct encoding it, in a host cell. The components of the particles not encoded by the vector genome are provided in trans by additional nucleic acid sequences (the "packaging system", which usually includes either or both of the gag/pol and env genes) expressed in the host cell. The set of sequences required for the production of the viral vector particles may be introduced into the host cell by transient transfection, or they may be integrated into the host cell genome, or they may be provided in a mixture of ways. The techniques involved are known to those skilled in the art.

Retroviral vectors for use in this invention include, but are not limited to Invitrogen's pLenti series versions 4, 6, and 6.2 "ViraPower" system. Manufactured by Lentigen Corp.; pHIV-7-GFP, lab generated and used by the City of Hope Research Institute; "Lenti-X" lentiviral vector, pLVX, manufactured by Clontech; pLKO.1-puro, manufactured by Sigma-Aldrich; pLemiR, manufactured by Open Biosystems; and pLV, lab generated and used by Charité Medical School, Institute of Virology (CBF), Berlin, Germany.

The vector also comprises, or alternatively consists essentially of, or yet further consists of, a nucleic acid encoding a CCR5 RNAi. The RNAi may be any one or more of RNA interfering molecules as described herein, e.g., shRNA, siRNA, miRNA or dsRNA.. In one particular aspect, the RNAi is one or more of a shRNA or siRNA sequence shown in SEQ ID NO: 23 or an equivalent thereof, e.g., a polynucleotide having at least 80 % sequence identity, or alternatively at least 85 % sequence identity, or alternatively at least 90 % sequence identity, or alternatively at least 92 % sequence identity, or alternatively at least 95 % sequence identity, or alternatively at least 97 % sequence identity, or alternatively at least 98 % sequence identity. Alternative sequences for use in this invention are available using the publicly available CCR5 polynucleotide sequence (see U.S. Patent No. 7,501,123 or those disclosed in SEQ ID NOS: 7-11) and a computer-based siRNA design tool available on the internet at one or more of www.dharmacon.com, Ambion-www.ambion.com/jp/techlib/ misc/siRNA_finder.html; Thermo Scientific-Dharmacon-www.dharmacon.com/DesignCenter/ DesignCenterPage.aspx; Bioinformatics Research Center-sysbio.kribb.re.kr:8080/AsiDesigner/ menuDesigner.jsf; and Invitrogen-rnaidesigner.invitrogen.com/rnaiexpress/.

To produce RNAi for use in this invention, one can follow conventional techniques as described in the art using published sequences or those provided herein. dsRNA and siRNA can be synthesized chemically or enzymatically *in vitro* using the methods disclosed in Micura, R. (2002) Agnes Chem. Int. Ed. Emgl. 41: 2265-9; Betz, N. (2003) Promega Notes 85:15-18; Paddison, P.J. and Hannon, G.J. (2002) Cancer Cell. 2:17-23. Chemical synthesis can be performed via manual or automated methods, both of which are well known in the art. Micura, R. (2002) Agnes Chem. Int. Ed. Emgl. 41: 2265-9. siRNA can also be endogenously expressed inside the cells in the form of shRNAs. Yu, J-Y. et al. (2002) Proc. Natl. Acad. Sci. USA 99: 6047-52; McManus, M.T. et al. (2002) RNA 8:842-50. Endogenous expression has been achieved using plasmid-based expression systems using small nuclear RNA promoters, such as RNA polymerase III U6 or H1, or RNA polymerase II U1. Brummelkamp, T.R. et al. (2002) Science 296:550-3; Novarino, G. et al. (2004) J. Neurosci. 24:5322-30.

*In vitro* enzymatic dsRNA and siRNA synthesis can be performed using an RNA polymerase mediated process to produce individual sense and antisense strands that are annealed *in vitro* prior to delivery into the cells of choice. Fire A. et al. (1998) Nature 391:806-811; Donze, O. and Picard, D. (2002) Nucl. Acids Res. 30 (10):e46; Yu, J-Y. et al. (2002) Proc. Natl. Acad. Sci. USA 99: 6047-52; Shim, E.Y. et al. (2002) J. Biol. Chem. 277:30413-6. Several manufacturers (Promega Corp. (Madison, WI); Ambion, Inc. (Austin, TX); New England Biolabs (Ipswich, MA); and Stragene (La Jolla, CA) provide transcription kits useful in performing the *in vitro* synthesis.

Alternatively, one can use a Polymerase II promoter to express CCR5 miRNA. For this embodiment, microRNAs are initially transcribed from Pol II promoters as long transcripts called primary-miRNAs which are processed into small pre-miRNAs. These pre-miRNAs are further processed intracellularly into miRNAs which are the mediators of gene regulation. CCR5 shRNAs or siRNAs can be converted to miRNAs by swapping in the exact CCR5 siRNA sequence in place of the original miRNA sequence. Hence, the CCR5 siRNA can be expressed via a Pol II promoter in the context of a miRNA backbone for efficient processing and regulation/knockdown of gene expression. The original CCR5 siRNA sequence will be found within the entire miRNA sequence but will be surrounded by the original miRNA secondary structure generated from the extra nucleotides found in the miRNA backbone. Alternative Polymerase II promoters include, but are not limited to EF1-alpha; PGK (phosphoglycerate kinase promoter); CMV (minimal cytomegalovirus promoter) and LTRs from retroviral and lentiviral vectors.

In other embodiments, the vector further comprise, or alternatively consists essentially of, or yet further consists of a nucleic acid encoding at least the extracellular domain of CD25 or an equivalent thereof and an operatively linked second expression control element that regulates expression of the nucleic acid encoding at least the extracellular domain of CD25 or an equivalent thereof. In some embodiments, the first or second expression control element is a promoter. In related embodiments, the first or second expression control element comprises a Polymerase III promoter or a Polymerase II promoter. The Polymerase II promoter may comprise, for example, the phosphoglycerate kinase promoter (PGK). In yet further embodiments, the vector backbone is derived from a virus, such as a lentivirus. In certain embodiments, the CD25 comprises a portion of the polynucleotide of SEQ ID NO: 1, or SEQ ID NO: 4, or SEQ ID NOS: 5, 6 or 38, or an equivalent of each thereof, e.g. a polynucleotide having at least 80 % identity thereto. In other embodiments, the nucleic acid encoding CD25 comprises a portion of the polynucleotide of SEQ ID NO: 3 or an equivalent thereof, e.g., a polynucleotide having at least 80 % identity thereto. In a further embodiment, the nucleic acid encoding CD25 comprises a portion of the polynucleotide of SEQ ID NO: 2 or an equivalent thereof, e.g. a polynucleotide having at least 80 % identity thereto.

In a further embodiment, the vector further comprise, or alternatively consists essentially of, or yet further consists of a nucleic acid encoding a TRIM5alpha polynucleotide and a sequence the regulates expression of the TRIM5alpha sequence operatively linked to it. For the purpose of illustration only, a nucleic acid that encodes TRIM5alpha for use in this invention is one that encodes either of the amino acid sequences for human TRIM5α 11-amino acid patch (GARGTRYQTFV (SEQ ID NO: 24)) or the rhesus macaque TRIM5α 13-amino acid patch (QAPGTLFTFPSLT (SEQ ID NO: 25)) or equivalents to these sequences. A TRIM5 expression cassette having the TRIM5alpha sequence operatively linked to a polymerase-III promoter is provided in SEQ ID NOS: 1-3 and 22. Alternative nucleic acids include, but are not limited to an equivalent polynucleotide as defined above, e.g., a polynucleotide having at least 80 % sequence identity, or alternatively at least 85 % sequence identity, or alternatively at least 90 % sequence identity, or alternatively at least 92 % sequence identity, or alternatively at least 95 % sequence identity, or alternatively at least 97 % sequence identity, or alternatively at least 98 % sequence identity to the TRIM5alpha sequence shown in SEQ ID NO: 26 as long as the 11 amino acid (human) or the 13 amino acid sequence (rhesus macaque) remains.

Another embodiment of the disclosure includes vectors wherein the one or more expression control element comprises a Polymerase II promoter that regulates expression of a polynucleotide comprising the nucleic acid encoding the TRIM5alpha sequence and the nucleic acid encoding at least the extracellular domain of CD25 or an equivalent thereof. In this instance, the TRIM5alpha protein and the CD25 protein or an equivalent thereof is expressed as a fusion protein, and the expression is controlled by the same promoter. The fusion protein may further comprise, or alternatively consists essentially of, or yet further consists of a protease cleavage site between the TRIM5alpha sequence and the CD25 sequence or an equivalent thereof. In some embodiments, the protease cleavage site is the 2A protease cleavage site. Other protease cleavage sites useful in the aspects described herein include, for example, E2A, F2A, and T2A. In other embodiments, the Polymerase II promoter controlling the expression of the TRIM5alpha-CD25 fusion protein is the MNDU3 promoter. Embodiments of the vector which include this fusion protein include, for example, the vectors of SEQ ID NOS: 1-3.

In a yet further embodiment, the vector further comprise, or alternatively consists essentially of, or yet further consists of a nucleic acid encoding an HIV TAR decoy polynucleotide. For the purpose of illustration only, a nucleic acid for use in this invention are provided in a portion of SEQ ID NO: 27 or an equivalent thereof, along with the sequence of a suitable regulation element such as polymerase-II promoter operatively linked to the sequence. Alternative nucleic acids include, but are not limited to a polynucleotide having at least 80 % sequence identity, or alternatively at least 85 % sequence identity, or alternatively at least 90 % sequence identity, or alternatively at least 92 % sequence identity, or alternatively at least 95 % sequence identity, or alternatively at least 97 % sequence identity, or alternatively at least 98 % sequence identity to the TAR decoy sequence shown in SEQ ID NO: 27 or one that hybridizes under stringent conditions to SEQ ID NO: 27 or its complement. Alternative sequences for use in this invention are disclosed in U.S. Patent Publication Nos. 2004/013167 and 2003/0013669 and U.S. Patent Nos. 6,995,258; 5,994,108; and 5,693,508. HIV TAR mRNA sequences are also available on the GenBank database, available under Accession Nos. NM_134324 (Homo sapiens TAR (HIV-1) RNA binding protein 2, transcript variant 2) and NM_004178 ((Homo sapiens TAR (HIV-1) RNA binding protein 2, transcript variant 3), each last accessed on April 29, 2009. Further additional sequences include: a) 5'-cgacttaaaatcgctagccagatctgagcctgggagctctctggctag-3' (SEQ ID NO: 12) or b) 5'-gggtctctctggttagaccagatttgagcctgggagctctctggctaactagggaaccc-3' (SEQ ID NO: 13) or c) 5'-acgaagcttgatcccgtttgccggtcgatcgcttcga-3' (SEQ ID NO: 14).

In a further aspect, the vector further comprises a marker or detectable label such as a gene encoding an enhanced green fluorescent protein (EGFP), red flouresence protein (RFP), green fluorescent protein (GFP) and yellow fluorescent protein (YFP) or the like. These are commercially available and described in the technical art.

### Packaging Systems

The invention is defined in the claims. The invention also provides a viral packaging system comprising: the vector as described above, wherein the backbone is derived from a virus; a packaging plasmid; and an envelope plasmid. The packaging plasmid contains polynucleotides encoding the nucleoside, capsid and matrix proteins. As an example, SEQ ID NO: 15 provides the sequence encoding a packaging plasmid that can be used in this invention. Alternatives include, but are not limited to a polynucleotide having at least 80 % sequence identity, or alternatively at least 85 % sequence identity, or alternatively at least 90 % sequence identity, or alternatively at least 92 % sequence identity, or alternatively at least 95 % sequence identity, or alternatively at least 97 % sequence identity, or alternatively at least 98 % sequence identity to SEQ ID NO: 15 or one that hybridizes under stringent conditions to SEQ ID NO: 15 or its complement. Alternatives are also described in the patent literature, e.g., U.S. Patent Nos. 7,262,049; 6,995,258; 7,252,991 and 5,710,037.

The system also contains a plasmid encoding a pseudotyped envelope protein provided by an envelope plasmid. Pseudotyped viral vectors consist of vector particles bearing glycoproteins derived from other enveloped viruses or alternatively constaining functional portions. See, for example U.S. Patent No. 7,262,049. In a preferred aspect, the envelope plasmid encodes an envelope protein that does not cause the viral particle to unspecifically bind to a cell or population of cells. The specificity of the viral particle is conferred by the antibody binding domain that is inserted into the particle. Examples of suitable envelope proteins include, but are not limited to those containing the *Staph. aureus* ZZ domain, the encoding sequence of which is provided in SEQ ID NO: 16 or a polynucleotide having at least 80 % sequence identity, or alternatively at least 85 % sequence identity, or alternatively at least 90 % sequence identity, or alternatively at least 92 % sequence identity, or alternatively at least 95 % sequence identity, or alternatively at least 97 % sequence identity, or alternatively at least 98 % sequence identity to that shown in SEQ ID NO: 16 or one that hybridizes under stringent conditions to SEQ ID NO: 16 or its complement. The choice of glycoprotein for use in the envelope is determined in part, by the antibody to which the particle may be conjugated.

This invention also describes the suitable packaging cell line. In one aspect, the packaging cell line is the HEK-293 cell line. Other suitable cell lines are known in the art, for example, described in the patent literature within U.S. Patent Nos. 7,070,994; 6,995,919; 6,475,786; 6,372,502; 6,365,150 and 5,591,624.

### Pseudotyped Viral Particles

The invention is defined in the claims. This invention further provides a method for producing a pseudotyped viral particle, comprising, or alternatively consisting essentially of, or yet further consisting of, transducing a packaging cell line with the viral system as described above, under conditions suitable to package the viral vector. Such conditions are known in the art and briefly described herein. The pseudotyped viral particle can be isolated from the cell supernatant, using methods known to those of skill in the art, e.g., centrifugation. Such isolated particles are further provided by this invention.

This invention further provides the isolated pseudotyped viral particle produced by this method. The pseudotyped viral particle comprises, or alternatively consists essentially of, or yet further consists of a polynucleotide encoding a CCR5 RNAi and envelope protein comprising the pre-selected domain such as the ZZ *S. aureus* domain alone or in combination with a TRIM5alpha sequence and an HIV TAR decoy polynucleotide.

The isolated pseudotyped particles can be conjugated to one or more of an antibody or an antibody fragment (e.g. an fragment containing at least the Fc domain) that retains the ability to bind a pre-selected cell receptor selected from the group consisting of CCR5, CD4, CD34 and CXCR4. Such antibodies include anti-CCR5 antibody, an anti-CD4 antibody, an anti-CD34 antibody and a CXCR4 antibody. Anti-CCR5 antibodies are commercially available from Invitrogen; abcam; ProSci Inc. and eptitomics, and described in the patent literature within U.S. Patent Nos. 7,122,185; 7,175,988; 7,160,546; and 6,930,174. Anti-CD34 antibodies are commercially available from R&D Systems, Invitrogen; Biolegend; and Miltenyi Biotec, and described in the patent literature within U.S. Patent No. 4,965,204. Anti-CD4 antibodies are commercially available from R&D Systems, Invitrogen; Biolegend; and Miltenyi Biotec, and described in the patent literature within U.S. Patent No. 4,965,204. Anti-CXCR4 antibodies are commercially available from Leinco Technologies, Capralogics and BioLegend, and described in the patent literature within U.S. Patent Nos. 7,521,045; 6,949,243 and 6,485,929, and U.S. Patent Publication No. 2005/0271665.

The antibodies are not species specific. In other words, the antibodies can be polyclonal or monoclonal and can be murine, ovine, human or other species. In addition, they can be chimeric or humanized.

When used in combination, the particles can be combination of CD4/CCR5 or CD4/CXCR4 or CD4/CCR5/CXCR4 or CD4/CD34 or CD34/CCR5 or CD34/CXCR4 or CD4/CD34/CCR4/CXCR4, which target multiple populations of HIV susceptible cells.

### Methods to Produce the Pseudotyped Particles

The invention is defined in the claims. This invention also provides methods to prepare a pseudotyped viral particle by transducing a packaging cell line, as described herein with the vector, the envelope plasmid and the packaging plasmid under conditions that facilitate packaging of the vector into the envelope particle. In one aspect, the pseudotyped viral particle is a pseudotyped viral particle. In a further aspect, the particles are separated from the cellular supernatant and conjugated to an antibody for cell-specific targeting.

In one aspect, the complete vector particle is a viral, or alternatively a retroviral vector pseudotyped with a Sindbis virus glycoprotein envelope containing the ZZ domain of Protein A from *Staphylococcus aureus.*

The genetic information of the viral vector particle is RNA which contains, on the 5' and 3' ends, the minimal LTR regions required for integration of the vector. In between the two LTR regions is the psi region which is required for packaging of the vector RNA into the particle. This region is followed by the RRE and cPPT sequences which enhance vector production by transporting the full length vector transcript out of the nucleus for efficient packaging into the vector particle. Next is the polymerase-II promoter MNDU3 which drives the expression of the chimeric TRIM5alpha gene. The polymerase-III U6 promoter driven CCR5 shRNA gene follows immediately downstream. Next is the polymerase-III U6 promoter driven TAR decoy gene. The last gene in the vector is an EGFP gene (enhanced Green Fluorescent Protein) which is driven by the polymerase-II PGK promoter. The EGFP gene is used as a reporter gene to detect transduced cells. The above listed genetic elements are transcribed into a full length RNA molecule which is packaged into the vector particle and contains all of the genetic information that will be integrated into the transduced cells.

The full length RNA transcript is packaged inside the capsid of the vector particle which contains the nucleocapsid, capsid, and matrix proteins which are generated from the packaging plasmid delta-8.91. The reverse transcriptase polymerse which is generated from the packaging plasmid delta-8.91 is also located within the capsid with the RNA transcript. The capsid encases and protects the full length RNA transcript.

Surrounding the capsid/RNA complex is the Sindbis-ZZ glycoprotein envelope which is generated from the Sindbis-ZZ plasmid. This envelope, when conjugated with a specific monoclonal antibody, will direct the vector particle to specifically transduce a cell of interest that expresses a cell surface receptor recognized by the chosen monoclonal antibody.

The vector particle is generated by a transient transfection protocol which includes a packaging cell line (HEK-293T cells), a lipofection reagent (Transit-293T), and the three plasmids encoding the parts of the vector particle (delta-8.91 (packaging plasmid)), CD25-containing vectors described herein (viral vector plasmid), and Sindbis-ZZ (envelope plasmid).

HEK-293T cells are plated at 75% confluency in complete DMEM media 24 hours prior to transfection. After at least 24 hours post-plating of cells, the transfection mixture should be prepared. Three milliliters of serum free media is incubated with 150ul of the lipofection reagent for 20 minutes at room temperature. The three plasmids are then added to the media/lipofection reagent mixture at a ratio of 5:5:2 (packaging plasmid: viral vector plasmid: envelope plasmid) and incubated for 30 minutes. After this final incubation period, the media/lipofection reagent/DNA mixture is then added to the HEK-293T cells and left overnight for the transfection to occur. The next day, the transfection media is removed and fresh complete DMEM is added. Seventy-two hours later, the cell culture supernatant is collected and concentrated by ultracentrifugation at 20,000 rpm for 1.5 hours.

In one aspect, the 13-amino acid patch sequence required for the TRIM5alpha molecule to inhibit HIV infection is N terminal---QAPGTLFTFPSLT---C terminal. Thus, any TRIM5alpha polynucleotide must encode this primary amino acid sequence.

To construct the ZZ domain containing Sindbis virus glycoprotein, the ZZ domain from *S. aureus* was inserted in between the amino acids #71-#74 of the E2 region of the Sindbis glycoprotein gene. In the wild-type Sindbis glycoprotein E2 region, this is where normal cell surface receptor recognition occurs. By inserting the ZZ domain here, normal cell binding is abolished. After the ZZ domain was inserted into the E2 region in a BsteII restriction enzyme site, the entire E3-E2ZZ-6K-E1 glycoprotein gene was PCR amplified and TOPO cloned into the pcDNA3.1 expression plasmid. By inserting the glycoprotein genes into this expression plasmid, the genes are under the control of the highly active polymerase-II CMV promoter.

Once the vector particle buds from the packaging cells and is released into the supernatant, this vector particle is conjugated to an antibody as defined herein.

### Isolated Host Cells

The invention is defined in the claims. Yet further provided is an isolated cell or population of cells, comprising, or alternatively consisting essentially of, or yet further consisting of, a retroviral particle of this invention, which in one aspect, is a viral particle. In one aspect, the isolated host cell is a packaging cell line.

In another aspect, the invention provides a pseudotyped viral particle conjugated to an antibody as described herein which is further conjugated to a host cell expressing a receptor to which the viral particle as described herein. In one aspect, the host cell is a cell expressing one or more of CD4, CD34, CXCR4 and/or CCR5. The cell can be any of a cell of a species of the group of: murine, rats, rabbit, simians, bovines, ovine, porcine, canines, feline, farm animals, sport animals, pets, equine, and primate, and in particular a human cell. Cells that are known to express such receptors include blood cell and in particular lymphocyte cells such as peripheral blood lymphocytes and mobilized blood lymphocytes. In another aspect, the host cell is an adult stem cell such as an hematopoietic stem cell "HSC" (CD34⁺ and/or CD34⁺/Thy-1⁻ HSC).

In a further aspect is an isolated host cell expressing on its cell surface CD25 extracellular domain or an equivalent thereof and comprising, or alternatively consisting of, or yet further consisting of CCR5 RNAi or an equivalent thereof. In a further aspect, the cell further comprises, or alternatively consists essentially of, or yet further consists of, a TRIM5alpha polynucleotic and/or HIR TAR polynucleotide or an equivalent of each thereof. The host cell can further comprise a detectable label. In one aspect the cell is a stem cell, such as a hematopoietic progenitor cell or a hematopoietic stem cell, e.g., a CD34+ cell. When used therapeutically, the cells can be allogeneic or autologous to the subject to be treated. The subjects can be mammalian, e.g., murine, canine, bovine, equine, ovine, feline or a human subject or patient.

This invention also provides an enriched population of cells as described above.

This invention further describes an isolated cell or an enriched population of cells that are derived from the stem cell described above. In one aspect, the derived cell or cell population is a macrophage. These cells are useful to treat and/or prevent HIV infection in a subject in need thereof.

### Compositions, Screens and Therapeutic Uses

The invention is defined in the claims. Also provided by this invention is a composition or kit comprising any one or more of the compositions described above and a carrier, e.g., an isolated cell, an enriched population of cells, vectors, packaging system, pseudotyped viral, viral particle conjugated to an antibody or fragment thereof which in turn may optionally be conjugated to a cell and a carrier. In one aspect, the carrier is a pharmaceutically acceptable carrier. These compositions can be used diagnostically or therapeutically as described herein and can be used in combination with other known anti-HIV therapies.

The compositions can be used *in vitro* to screen for small molecules and other agents that may modify HIV infectivity and replication by adding to the composition varying amounts of the agent to be tested and comparing it to a companion system that does not have the agent but which exhibits the desired therapeutic effect. For example, if it is known that the viral particle or cell inhibits HIV infection in a system, then the system can be used to test alternative therapies to determine if it is a substitute to the viral particle or cell. Alternatively, one can test agents in the viral particle system itself to determine if the agent acts competitively, additively or synergistically with the viral particle system. After an *in vitro* screen, the test agent or combination therapy can be assayed in an appropriate animal model.

When the cells, particles and/or antibody conjugated cells (to the particles) are administered to an appropriate animal subject, the animal subject can be used as an animal model to test alternative therapies in the same manner as the *in vitro* screen. This invention also describes the isolated cell, enriched population of cells, pseudotyped viral particle or the pseudotyped viral particle conjugated to the antibody as described herein for use in a method to inhibit HIV replication *in vivo* or *ex vivo,* comprising, or alternatively consisting essentially of, or yet further consisting of administering to a subject in need thereof an effective amount of the isolated cell, enriched population of cells, pseudotyped viral particle or the pseudotyped viral particle conjugated to the antibody as described herein. In another aspect of the disclosure, the isolated cell, enriched population of cells, pseudotyped viral particle or the pseudotyped viral particle conjugated to the antibody as described herein is for use in a method to prevent HIV replication *in vivo* or *ex vivo* comprising, or alternatively consisting essentially of, or yet further consisting of administering to a subject in need thereof an effective amount of the isolated cell, enriched population of cells, pseudotyped viral particle or the pseudotyped viral particle conjugated to the antibody as described herein. The cell, enriched population of cells, pseudotyped viral particle or the pseudotyped viral particle conjugated to the antibody as describe herein can be combined with other anti-viral therapies that are known in the art. When combined with other therapies, administration of the therapies can be concurrent or sequential as determined by the treating physician. In one aspect, bone marrow, mobilized bone marrow cells or peripheral blood lymphocytes are removed from the patient to be treated and cultured with the pseudotyped viral particle conjugated to the one or more antibodies. After an appropriate amount of time to allow for the particle to bind to the appropriate receptor, the cells are then re-administered to the subject or patient to which they were isolated. As noted above, this therapy can be combined with other anti-viral therapies or the like.

This invention also describes the compositions as described herein for use in a method to treat a subject at risk of developing an active infection or infected with HIV (AIDs) by administrering to the subject an effective amount of one of the compositions as described herein. For the purpose of this aspect, a subject is as described herein and therefore includes mammals, animals and humans, for example. Additional effective therapies can combined with this invention and/or added as necessary.

Further described are an isolated cell, an enriched population of cells, a pseudotyped viral vector particle as described herein or or the psuetotyped viral vector as described herein for use in methods to inhibit or prevent HIV replication in a cell infected with HIV, by contacting the cell with an effective amount of one or more of an isolated cell, an enriched population of cells, a pseudotyped viral vector particle as described herein or or the psuetotyped viral vector as described herein. In one aspect, the contacting is *in vitro.* In another aspect it is *in vivo.*

This invention also describes the use of compositions as described herein to prevent or treat an HIV infection and/or AIDs by administering to a subject an effective amount of one or more compositions described herein. Further provided is the use of a composition as described herein in the manufacture of a medicament to treat or prevent HIV infection and/or AIDs. Additional effective therapies can combined with this invention and/or added as necessary.

Having been generally described herein, the follow examples are provided to further illustrate this invention.

### Examples

### CD25 Pre-selective Anti-HIV Vectors for Improved HIV Gene Therapy

HIV infections continue to spread worldwide in both developed and underdeveloped countries with no effective vaccine available (Barouch et al., 2008; Edgeworth et al., 2002). Although antiretroviral therapy (ART) is effective in the majority of HIV infected patients, challenges to therapeutic and curative success include the continuing emergence of drug-resistant HIV variants, drug toxicity, and incomplete viral suppression (Baldanti et al., 2010; Domingo et al., 2012; Johnson et al., 2010; Kuritzkes, 2011; Lewden et al., 2007; Macias et al., 2006; Tilton et al., 2010). ART also fails to eradicate viral reservoirs which are established early in infection, leading to viral persistence and incomplete immune restoration (Gazzola et al., 2009; Mehandru et al., 2006). Interruption of ART results in rapid viral resurgence, the generation of escape mutants, and CD4+ T cell loss in the peripheral blood of HIV infected patients (Graham et al., 2012; Kalmar et al., 2012).

These challenges highlight the need for the further development of innovative HIV therapies with broad mechanisms of action. HIV gene therapy has the potential as an alternative or complementary treatment strategy to ART, especially when hematopoietic stem cells (HSCs) are the cellular targets for genetic modification (Strayer et al., 2005). Advantages of HIV stem cell gene therapy include constitutive or controlled expression of anti-HIV genes, the generation of a durable and HIV-resistant immune system, and the possibility of a one-time treatment if enough anti-HIV vector transduced cells can be transplanted into patients (Strayer et al., 2005). Many potent anti-HIV genes have been developed and tested both in preclinical and clinical settings and the safety of some of these genes has been observed with engraftment of transduced stem cells and anti-HIV gene expression in transplanted patients (Bauer et al., 1997; DiGuisto et al., 2010; Kohn et al., 1999; Mitsuyasu et al., 2009; Podsakoff et al., 2005; Shimizu et al., 2010; ter Brake et al., 2009; Walker et al., 2012). However, efficacy in a clinical setting has been difficult to achieve due to low transduction efficiencies and low *in vivo* gene marking (DiGuisto et al., 2010; Mitsuyasu et al., 2009; Podsakoff et al., 2005).

*In vitro* HIV challenge experiments designed to evaluate the efficacy of anti-HIV genes in inhibiting HIV infection/replication rely on sorting or selection of the gene transduced cells resulting in a pure population of HIV-resistant cells prior to infection. However, this has not been feasible in a clinical setting as many reporter genes utilized for sorting may be immunoreactive. When unsorted/mixed populations of nontransduced and anti-HIV vector transduced cells are infected with HIV, a selective survival advantage and an increase in the percentage of total immune cells of the anti-HIV gene expressing cells has been observed (Anderson et al., 2009; Walker et al., 2012). These results highlight the ability of anti-HIV gene modified cells to survive and possibly replenish the immune system with functioning HIV-resistant immune cells. However, when translated into a clinical setting, a large percentage of nontransduced HSCs are transplanted along with the anti-HIV vector transduced cells due to low transduction efficiencies (DiGuisto et al., 2010; Mitsuyasu et al., 2009; Podsakoff et al., 2005). These nontransduced HSCs produce unprotected immune cells which are targets for HIV infection and replication. These infected cells may also replenish viral reservoirs. By transplanting an enriched population of anti-HIV vector transduced cells into patients where the majority of the cells express the anti-HIV genes, similar results observed with the Berlin patient may be achievable as this patient received a pure population of HIV-resistant HSCs from a donor who was homozygous for a CCR5 Δ32 bp allele (Hutter et al., 2009). Therefore, new methods need to be developed to increase the total percentage of anti-HIV vector transduced cells transplanted into patients.

In the studies described herein, *in vitro* safety and an improved efficacy of HIV stem cell gene therapy in the enriched population of HIV-resistant cells compared to unpurified cells are demonstrated. This was achieved by a triple combination anti-HIV vector which incorporated a selectable marker, human CD25, which is expressed on the surface of transduced cells. Human CD25, the low affinity IL-2 receptor alpha subunit, was chosen as the selectable marker because of its normal characteristics of not being expressed on the surface of HPCs or HSCs and its lack of intracellular signaling (Grant et al., 1992; Kuziel et al., 1990; Minami et al., 1993). Upon expressing CD25 on the surface of HPCs and purification of the transduced cells, safety of the enriched population of anti-HIV vector transduced HPCs was observed along with potent HIV-1 inhibition. These results highlight the potential use of this strategy for HIV stem cell gene therapy to improve the efficacy of future HIV stem cell gene therapy clinical trials.

### Example 1: Lentiviral vector design and production

A third-generation self-inactivating lentiviral vector was utilized in this study, CCLc-MNDU3-X-PGK-X2. To generate the control vector (named EGFP+), an EGFP reporter gene was inserted into position "X" under the control of the MNDU3 promoter and a human CD25 coding region was inserted into position "X2" of this vector under the control of a phosphoglycerate kinase (PGK) promoter (FIG. 1A). This vector was only used to initially test the strategy of utilizing CD25 as a selective protein in purifying transduced cells. Therefore, Applicant was able to compare EGFP% positive cells to CD25% positive cells. To generate the pre-selective anti-HIV vector (named CMAP1 (Cclc-Mndu3-Antihiv-Protein-1), a triple combination of anti-HIV genes was inserted into position "X" and a human CD25 coding region was inserted into position "X2" of this vector under the control of a PGK promoter (FIG. 1B). The triple combination of anti-HIV genes includes a chimeric human/rhesus macaque TRIM5α gene under the control of the MNDU3 promoter, a polymerase-III U6 promoter driven CCR5 shRNA expression cassette, and a polymerase III U6 promoter driven TAR decoy expression cassette (FIG. 1B). Sequencing of clones was confirmed by Laragen Inc., Los Angeles, CA.

Lentiviral vectors were generated in HEK-293T cells. Twenty-five micrograms of the packaging construct, Δ8.9 (packaging plasmid containing the gag and pol genes), 25µg of EGFP+ or CMAP1, and 5µg of VSVG (envelope) were transfected into cells in T225 flasks by lipofection. Vector supernatants were collected at 48 hours post-transfection and concentrated by ultrafiltration. Vector titers were calculated by transduction of HEK-293T cells. Forty-eight hours post-transduction, the HEK-293T cells were stained with a phycoerythrin (PE)-conjugated anti-human CD25 antibody (BD Biosciences, San Jose, CA) and analyzed by flow cytometry. All flow cytometry analyses were performed on a Beckman Coulter Cytomics FC500 using CXP software.

### Example 2: Transduction and purification of vector transduced primary human CD34+ HPCs

CD34+ hematopoietic progenitor cells (HPCs) were isolated from human umbilical cord blood (NDRI, Philadelphia, PA) by Ficoll-Paque (GE Healthcare, Piscataway, NJ) and purified by CD34+ magnetic bead column separation (Miltenyi Biotec, Auburn, CA). CD34+ cell isolation purity (>90%) was routinely obtained. Total CD34+ cells were cultured in complete IMDM media containing 10% FBS and supplemented with 50ng/ml SCF, Flt-3 ligand, and TPO. Cells were transduced with the lentiviral vectors EGFP+ or CMAP1 (MOI 15) overnight at 37°C with 8µg/ml protamine sulfate. Two days post-transduction, an aliquot of the EGFP+ vector transduced cells was stained with a PE-conjugated anti-human CD25 antibody (BD Biosciences, San Jose, CA) and analyzed by flow cytometry for both EGFP and CD25 percentages. Total genomic DNA was extracted from an aliquot of the CMAP1 vector transduced cells utilizing a Wizard Genomic DNA Isolation System (Promega, Madison, WI) and analyzed by quantitative PCR (QPCR) for vector copy number with a primer set specific for the chimeric TRIM5α gene: (forward) 5'-CTGGGTTGATGTGACAGTGG-3' (SEQ ID NO: 28) and (reverse) 5'-CGTGAGTGACGGAAACGTAA-3' (SEQ ID NO: 29). QPCR was performed using a SYBR Green PCR Master Mix Kit (Applied Biosystems, Foster City, CA). The rest of the transduced cells were labeled with CD25+ immunomagnetic beads (Miltenyi Biotec, Auburn, CA) according to the manufacturer's protocol and separated over a magnetic bead column. Purified cells were then utilized for subsequent experiments.

To evaluate the purity of the EGFP+ vector transduced cells after CD25+ immunomagnetic bead selection, purified cells were stained with a PE-conjugated anti-human CD25 antibody (BD Biosciences, San Jose, CA) and analyzed for both EGFP and CD25 percentages. To evaluate the purity of the CMAP1 vector transduced cells, total genomic DNA was extracted and analyzed by QPCR for vector copy number utilizing the chimeric TRIM5α primer set described above. QPCR was performed using a SYBR Green PCR Master Mix Kit (Applied Biosystems, Foster City, CA). GAPDH was used as an internal control. **Example 3: Colony forming unit assays**

CD34+ HPCs, either nontransduced (NT) or CD25 immunomagnetic bead purified CMAP1 vector transduced cells were cultured in semi-solid methylcellulose medium with growth factors (Stem Cell Technologies,Vancouver, BC, Canada) for 10 days. After 10 days, total blood forming colonies (BFU), granulocyte/erythrocyte/megakaryocyte/monocyte colonies (GEMM), and granulocyte/monocyte colonies (GM) were observed and counted.

To evaluate whether purified CMAP1 vector transduced CD34+ HPCs (10,000 cells/3ml methylcellulose) had an increased proliferation potential in the presence of IL-2, 1µg/ml of IL-2 was added to the methylcellulose medium. After 10 days, total cell numbers were counted.

To derive macrophages from the nontransduced and the purified CMAP1 vector transduced CD34+ HPCs, cells were removed from the methylcellulose and plated in 6-well plates in complete DMEM medium with 10% FBS supplemented with 10ng/ml of GM-CSF and M-CSF (R&D Systems, Minneapolis, MN). Media was changed every two days for four days to derive mature macrophages. Both nontransduced (NT) and purified CMAP1 vector transduced CD34+ cell derived macrophages were used for subsequent experiments.

### Example 4: Phenotypic and genotypic analysis of CD34+ cell derived macrophages

To determine if CMAP1 vector transduced CD34+ cells were able to mature into phenotypically normal macrophages, cells were visualized by microscopy and analyzed by flow cytometry. Macrophages were stained with antibodies to detect normal macrophage cell surface markers including, PE-conjugated CD14, allophycocyanin (APC)-conjugated HLADR, PE-conjugated CD4, PECY7-conjugated CD80, PE-conjugated CCR5, and PE-conjugated CD25 (BD Biosciences, San Jose, CA).

To determine if the addition of IL-2 induced the expression of proto-oncogenes in CMAP1 vector transduced macrophages, IL-2 (1µg/ml) was added to the macrophage cultures. On day three post-IL2 addition, total cellular RNA was extracted and QPCR was performed. Total cellular RNA was extracted from cells using RNA-STAT-60 (Tel-Test Inc., Friendswood, TX). First strand cDNA synthesis was performed using the High Capacity cDNA Reverse Transcription Kit (Applied Biosystems, Foster City, CA). QPCR was then performed using the SYBR Green PCR Master Mix Kit (Applied Biosystems, Foster City, CA) with primers: myc (forward) 5'-TCCATTCCGAGGCCACAGCAAG-3' (SEQ ID NO: 30); (reverse) 5'-TCAGCTCGTTCCTCCTCTGACG-3' (SEQ ID NO: 31); myb (forward) 5'-AAGACCCTGAGAAGGAAAAGCG-3' (SEQ ID NO: 32); (reverse) 5'-GTGTTGGTAATGCCTGCTGTCC-3' (SEQ ID NO: 33); fos (forward) 5'-ACTACCACTCACCCGCAGAC-3' (SEQ ID NO: 34); (reverse) 5'-GACGGGAAGCCAGCCTTAC-3' (SEQ ID NO: 35); jun (forward) 5'-CCCCAAGATCCTGAAACAGA-3' (SEQ ID NO: 36); and (reverse) 5'-CCGTTGCTGGACTGGATTAT-3' (SEQ ID NO: 37). Glyceraldehyde-6-phosphate was used as an internal control. Peripheral blood mononuclear cells (PBMCs) were cultured in complete RPMI media supplemented with 10% FBS and used as a positive control for proto-oncogene up-regulation upon IL-2 (1µg/ml) stimulation.

### Example 5: HIV-1 challenge of vector transduced macrophages

To determine whether the purified CMAP1 vector transduced macrophages were capable of improved inhibition of HIV-1 infection compared to unpurified CMAP1 vector transduced macrophages, cells were challenged with an R5-tropic BaL-1 strain of HIV-1 (MOI 0.05). On various days post-infection, supernatants were collected and analyzed for levels of HIV-1 replication by p24 antigen ELISA (Zeptometrix Corp., Buffalo, NY). On day 28 post-infection, the HIV-1 challenged cells were also visualized by microscopy.

Two-sample t-tests were used for statistical analyses. They were conducted in R (version 2.10.1) for Windows. A significance level of 0.05 was used for testing hypotheses.

### Example 6: Enrichment of cells transduced with the pre-selective vectors

A third generation self-inactivating lentiviral vector, CCLc-MNDU3-X-PGK-X2, was utilized to derive the control (EGFP+) and the anti-HIV (CMAP1) pre-selective vectors. A selectable marker, human CD25 which is not normally found on the surface of CD34+ HPCs, was incorporated into the control EGFP+ and CMAP1 vectors under the control of the PGK promoter and used to purify vector transduced cells (FIG. 1A). The EGFP+ control vector was only used to initially test the strategy of utilizing CD25 as a selective protein in purifying transduced cells and to compare the purification levels to CMAP1 vector transduced cells. By using EGFP in the same vector as CD25, we would be able to compare EGFP% positive cells to CD25% positive cells. To evaluate the levels of purification of vector transduced cells, flow cytometry and quantitative PCR (QPCR) were performed on EGFP+ and CMAP1 vector transduced CD34+ HPCs, respectively. As displayed in FIG. 1B, the percentage of control EGFP+ vector transduced cells in the total cell population increased on average from 14.5% EGFP+ unpurified to 81.9% EGFP+ after purification. This correlated with expression of CD25 on the surface of the transduced HPCs which increased on average from 15.1% CD25+ before purification to 81.0% CD25+ post-purification. As displayed in FIG. 1C, the levels of vector copy number per cell in the total cell population of CMAP1 vector transduced cells increased on average from 0.38 vector copies per cell unpurified to 2.97 vector copies per cell after purification. This amounted to an average enrichment percentage of 85.3%. These data successfully demonstrate that upon expression of CD25 on the surface of cells transduced with the pre-selective vectors, a high level of enrichment can be achieved.

### Example 7: Safety of CMAP1 transduced HPCs in CFU assays

CD25 is not normally found on the surface of human HPCs. Therefore, to evaluate the effect of CD25 expression on HPCs and their ability to form normal quantities and types of hematopoietic colonies, CFU assays were performed on purified CMAP1 vector transduced HPCs. As displayed in FIG. 2A, normal colony phenotypes of BFUs, GEMMs, and GMs formed in the purified CMAP1 cultures compared to nontransduced (NT) cultures. The representative pictures displayed were taken with a 10x objective. Total numbers of each type of colony were also counted. As displayed in FIG. 2B, no significant differences (p>0.05) in the numbers of each colony, BFUs, GMs, and GEMMs had formed, on average, in the purified CMAP1 cultures, 44.0 BFUs, 45.3 GMs, and 12.0 GEMMs with standard deviations of 5.29, 5.03 and 2.00, respectively compared to the NT cultures, 45.3 BFUs, 50.0 GMs, and 12.7 GEMMs with standard deviations of 3.06, 5.29 and 5.03, respectively.

As CD25 is part of the IL-2 receptor complex which is involved in the immune response and immune cell proliferation, we wanted to investigate whether the addition of IL-2 to the purified CMAP1 vector transduced HPC methylcellulose cultures resulted in an increase in HPC proliferation and in total cell numbers. As displayed in FIG. 2C, no significant differences (p>0.05) in cell numbers were observed, on average, in the NT and CMAP1 cultures with the addition of IL-2 (106.74 and 106.78 total cells, respectively). An increase in total cell numbers was observed in the CMAP1 cultures with IL-2 compared to the cultures without IL-2 (an increase of 0.18 log), however, a similar increase was observed in the NT cultures (an increase of 0.16 log). This was likely due to the presence of cells which normally respond to IL-2 since during the course of the 10 day culture, the majority of the cells have differentiated.

These results highlight the initial safety of expressing CD25 on the surface of vector transduced HPCs and demonstrated that no apparent aberrations in HPC differentiation or proliferation had occurred with over expression of CD25.

### Example 8: Derivation of phenotypically normal macrophages from CMAP1 transduced HPCs

To determine whether normal immune cells could be derived from the purified CMAP1 transduced cells, macrophages were differentiated from the HPCs. Macrophage cultures were visualized by microscopy under a 10x objective. As displayed in FIG. 3A, normal macrophage phenotypes were observed with the appearance of attached cells having a "fried-egg" appearance in both the NT and purified CMAP1 vector transduced cultures. These macrophages were then phenotypically analyzed by flow cytometry for normal macrophage cell surface markers. As displayed in FIG. 3B, a normal phenotype of purified CMAP1 macrophages (shaded histograms) was demonstrated as compared to NT macrophages (unshaded histograms). Representative overlay histograms are displayed (FIG. 3B). CMAP1 cells displayed 92.3% of CD14, 95.6% of HLADR, 99.7% of CD4, and 93.4% of CD80. CCR5 expression had decreased to 25.5% in the purified CMAP1 macrophages compared to NT macrophages which displayed a level of 94.6% of CCR5. The decrease in CCR5 expression in the CMAP1 cultures was due to the expression of the CCR5 shRNA.

Macrophages normally express a certain level of CD25 on their cell surface. To evaluate the increase in cell surface expression of CD25 on CMAP1 transduced macrophages, flow cytometry was performed. As displayed in FIG. 3C, a gradient increase was observed, on average, in CD25 expression on the surface of macrophages from NT macrophages (55.0%) to unpurified CMAP1 transduced macrophages (79.1%) to purified CMAP1 transduced macrophages (95.1%).

These results demonstrate that phenotypically normal macrophages can be derived from purified CMAP1 vector transduced HPCs and that overexpression of CD25 did not disrupt normal macrophage differentiation from HPCs. These results also demonstrate that the CMAP1 vector worked as hypothesized by displaying an increase in CD25 expression on purified populations of transduced cells.

### Example 9: Proto-oncogene expression in purified CMAP1 macrophages

As mentioned above, CD25 is part of the IL-2 receptor complex which is involved in the cell proliferation of immune cells. After IL-2 binds to the IL-2 receptor, expression of proto-oncogenes including myc, myb, fos, and jun are up-regulated to promote cell division. To evaluate whether overexpression of CD25 on the surface of purified CMAP1 macrophages up-regulated the expression of the mentioned proto-oncogenes, QPCR was performed on total RNA from macrophage cultures with the addition of IL-2. PBMCs were used as a positive control for proto-oncogene up-regulation in the presence of IL-2. As displayed in FIG. 4, similar levels of proto-oncogene expression were measured in NT and purified CMAP1 macrophage cultures. No significant difference (p>0.05) was observed with the expression of myc, myb, and fos in CMAP1 macrophages compared to NT macrophages in the presence of IL-2. However, an average relative expression level of 1.1 (standard deviation of 0.04) for jun (p=0.0121, statistically significant) was observed with CMAP1 macrophages compared to NT macrophages which were used as the reference cells with an average relative expression level of 1.0 (standard deviation of 0.04). As a positive control for proto-oncogene up-regulation, PBMCs were cultured in the presence of IL-2. A significant up-regulation in the expression of myc (2.3-fold) (p=0.0418), myb (8.1-fold) (p=0.0018), fos (4.8-fold) (p=0.0001), and jun (5.3-fold) (p=0.0004) was observed in the IL-2 stimulated PBMC cultures relative to NT macrophages.

These results demonstrate that even though the purified CMAP1 macrophages express an increased level of CD25, proto-oncogene expression levels remained similar to nontransduced cells.

### Example 10: HIV-1 inhibition of CMAP1 HPC derived macrophages

Previous challenge experiments performed with this triple combination of anti-HIV genes, both in vitro and in vivo, demonstrated strong viral inhibition to both CCR5 and CXCR4-tropic strains of HIV-1 (Anderson et al., 2009; Kohn et al., 1999). These experiments, however, relied on sorting the cells based on a non-clinically acceptable reporter gene, EGFP, prior to viral challenge. To evaluate whether purified CMAP1 macrophages displayed an increased efficacy of HIV-1 inhibition compared to NT and unpurified CMAP1 macrophages, cells were challenged with an R5-tropic BaL-1 strain of HIV-1. As displayed in FIG. 5A at the end of the challenge experiments, potent inhibition of HIV-1 infection was observed in the purified CMAP1 cultures compared to NT macrophages (2.9 log difference) and unpurified CMAP1 macrophages (2.2 log difference). A slight inhibition of HIV-1 infection was observed in the unpurified CMAP1 macrophage cultures (0.7 log difference) compared to NT macrophages due to the presence of anti-HIV gene expressing cells. On day 28-post infection, HIV-1 infected macrophages were also visualized by microscopy under 10x magnification. As displayed in FIG. 5B with representative pictures, cell death from HIV infection was observed throughout the cultures of infected NT and unpurified CMAP1 macrophages. This was in comparison to purified CMAP1 macrophages which displayed a small amount of cell death but where the majority of macrophages still appeared healthy.

These results highlight the increased efficacy of anti-HIV vector transduced cells when they are purified to a cell population where the majority of the cells express the anti-HIV genes and demonstrate the utility of this novel CMAP1 vector.

HIV gene therapy holds considerable promise as an alternative treatment strategy for HIV infected patients. As observed with the Berlin patient who received a pure population of HIV-resistant hematopoietic stem cells in a bone marrow transplant from a donor homozygous for a CCR5 Δ32 bp allele, HIV-resistant stem cells are capable of repopulating the immune system with cells which can inhibit HIV infection in the absence of ART for a prolonged period of time (Hutter et al., 2009). The safety of numerous anti-HIV genes has been demonstrated in previous HIV stem cell gene therapy clinical trials (DiGuisto et al., 2010; Mitsuyasu et al., 2009; Podsakoff et al., 2005). However, patients were given a mixed population of cells, with the vast majority not being transduced with anti-HIV genes. This led to the derivation of an immune system where the majority of the immune cells were still susceptible to HIV infection. A low level of efficacy was observed with a measureable increase in the levels of anti-HIV gene expressing cells in the presence of a viral load, however, initial transduction efficiencies and in vivo gene marking were too low for patients to remain off ART. If a pure population of anti-HIV gene transduced cells could be transplanted into patients, similar results observed with the Berlin patient may be achievable.

Applicant has recently demonstrated strong ex vivo resistance to infection to multiple strains of HIV-1 and a selective survival advantage of cells transduced with a triple combination anti-HIV lentiviral vector expressing a human/rhesus macaque TRIMS5α, a CCR5 shRNA, and a TAR decoy in vivo in a humanized mouse model (Walker et al., 2012). However, plasma viremia levels in HIV-1 infected mice transplanted with anti-HIV vector transduced cells did not decrease over time due to the transplantation of a majority of nontransduced HSCs which continually produced HIV susceptible target cells (Walker et al., 2012). If anti-HIV vector transduced cells could be enriched to a pure population or at least to a population where the majority of the cells express the anti-HIV genes, improved in vivo efficacy may be demonstrated.

Current reporter genes for cell sorting are not clinically relevant due to their foreign nature which would invoke an immune response against transduced cells. Another approach similar to the one presented here utilizes a P140K mutant methyl guanine methyltransferase (MGMT) transgene to select for transduced cells in vivo after transplantation into patients. This, however, would require another patient infusion with agents which would select for vector transduced cells (Trobridge et al., 2009). Therefore, as a first step to improve on the efficacy of HIV gene therapy, Applicant has developed pre-selective anti-HIV lentiviral vectors which express a normal human cell surface protein as to avoid rejection of transduced cells and allow for the purification of vector transduced cells prior to transplantation. Human CD25 was chosen as a selectable marker based on its characteristics of not being expressed on the surface of HPCs or HSCs, it is a normal immune cell surface protein, and it has been previously shown to have no direct intracellular signaling (Grant et al., 1992; Kuziel et al., 1990; Minami et al., 1993).

When over-expressing a protein on the surface of cells, especially cells which do not normally express the protein, the safety and function of the engineered cells is a concern. Upon transduction and high level purification (>85%) of CMAP1 vector transduced HPCs, phenotypically normal CFUs and the number of CFUs formed in methylcellulose medium were similar to nontransduced cells (FIG. 2). Safety was also observed with macrophages derived from the purified CMAP1 vector transduced HPCs as they appeared phenotypically normal compared to nontransduced macrophages (FIG. 3).

As CD25 is part of the IL-2 receptor complex, it was important to investigate whether the over expression of CD25 in purified vector transduced cells had any effect on cell proliferation or the up-regulation of proto-oncogenes in the addition of IL-2. In the presence of IL-2 no adverse effects were observed in purified CMAP1 vector transduced HPCs or macrophages. No increased cell proliferation of HPCs (FIG. 2) and no increase in the expression of the proto-oncogenes myc, myb, or fos (FIG. 4) was observed compared to nontransduced cells. A small increase in the expression of jun in CMAP1 vector transduced macrophages was observed (1.1) compared to NT cells (1.0). This difference was, however, significantly different to the positive control PBMCs which displayed a 5.3 relative increase in expression of jun. No other adverse effects were observed with purified CMAP1 vector transduced cells. Applicant's findings of a lack of cell proliferation and proto-oncogene up-regulation are likely due to CD25's normal function of being involved with the assembly of the IL-2 receptor but having no mitotic signaling capabilities (Grant et al., 1992; Kuziel et al., 1990; Minami et al., 1993).

The ultimate goal of HIV stem cell gene therapy is to provide an alternative therapeutic intervention for HIV infected patients and to provide a possibility for them to withdraw ART medications. For this to be achievable, an enriched population of anti-HIV gene transduced cells with little to no nontransduced cells needs to be transplanted. Upon HIV-1 challenge of purified CMAP1 vector transduced HPC derived macrophages, strong inhibition of HIV-1 infection was observed. CMAP1 vector transduced cells which were not purified displayed high levels of HIV-1 replication similar to nontransduced macrophage cultures. Even though unpurified CMAP1 cultures contained anti-HIV gene expressing cells, the majority of the cells were nontransduced and, thus, were capable of being infected and replicating HIV-1 at a high level. This highlights the improved efficacy of this pre-selective anti-HIV vector and allows for the purification of anti-HIV gene expressing cells to an enriched population.

### Example 11: CD25-pre-selective-anti-HIV lentiviral vector

Construction of the lentiviral vector depicted in FIG. 9 was performed according to the following. The lentiviral vector backbone CCLc was used to generate the CD25 pre-selective anti-HIV vectors. First, a human/rhesus macaque TRIM5alpha gene was fused via a 2A protease cleavage site to either the complete human CD25 gene or to a truncated version of the human CD25 gene (either a full truncation of the cytoplasmic domain or a truncation which leaves only four cytoplasmic amino acids). These fusion proteins were generated by fusion PCR and contained an HpaI site at the 3' end of the fusion gene for subcloning of the U6-CCR5 shRNA/U6-TAR decoy expression cassettes. The TRIM5alpha-2A-CD25 fusion genes were first cloned into a TOPO PCR cloning vector and the sequence was verified. Next, the U6-CCR5 shRNA/U6-TAR decoy expression cassettes were subcloned into an HpaI site downstream from the TRIM5alpha-2A-CD25 genes in their respective TOPO clones. Correct sequences were verified. Next, the TRIM5alpha-2A-CD25-U6-CCR5 shRNA/U6 TAR decoy expression clusters were subcloned into the CCLc lentiviral vector in an EcoRI site downstream of the MNDU3 promoter. Sequences were verified. This generated the CCLc lentiviral vector which contains the TRIM5alpha-2A-CD25 expression cassette under the control of the MNDU3 promoter, a CCR5 shRNA under the control of a U6 promoter, and a TAR decoy under the control of a U6 promoter. SEQ ID NOS 1-3 are representative sequences of this vector construct.

### Example 12: Derivation of macrophages from purified CMAP1 vector transduced HPCs

Macrophages normally express a measureable level of CD25 on their cell surface. To evaluate any increase in cell surface expression of CD25 on CMAP1-transduced macrophages, flow cytomotry was performed. A gradient increase was observed, on average, in CD25 expression on the surface of macrophages from NT macrophages (63.7%) to unpurified CMAP1-transduced macrophages (84.1%) to purified CMAP1-transduced macrophages (94.3 %). This increase in CD25 expression, which correlates to CMAP1 vector transduction, resulted in the downregulatin ofCCR5 due to the expression of the CCR5 shRNA. In purified CMAP1 macrophages, CCR5 expression was 12.5 %, which compared to NT macrophages (86.5%) and unpurified CMAP1 macrophages (78.9%).

These results demonstrate that phenotypically normal macrophages can be derived from purified CMAP1 vector-transduced HPCs and that overexpression of CD25 did not disrupt normal macrophage differentiation from HPCs.

It is to be understood that while the invention has been described in conjunction with the above embodiments, that the foregoing description and examples are intended to illustrate and not limit the scope of the invention. Other aspects, advantages and modifications within the scope of the invention will be apparent to those skilled in the art to which the invention pertains.

In addition, where features or aspects of the invention are described in terms of Markush groups, those skilled in the art will recognize that the invention is also thereby described in terms of any individual member or subgroup of members of the Markush group.

### Sequence Listing

SEQ ID NO: 1 
caggtggcacttttcggggaaatgtgcgcggaacccctatttgtttatttttctaaatacattcaaatatgtatccg
ctcatgagacaataaccctgataaatgcttcaataatattgaaaaaggaagagtatgagtattcaacatttccgtgt
cgcccttattcccttttttgcggcattttgccttcctgtttttgctcacccagaaacgctggtgaaagtaaaagatg
ctgaagatcagttgggtgcacgagtgggttacatcgaactggatctcaacagcggtaagatccttgagagttttcgc
cccgaagaacgttttccaatgatgagcacttttaaagttctgctatgtggcgcggtattatcccgtattgacgccgg
gcaagagcaactcggtcgccgcatacactattctcagaatgacttggttgagtactcaccagtcacagaaaagcatc
ttacggatggcatgacagtaagagaattatgcagtgctgccataaccatgagtgataacactgcggccaacttactt
ctgacaacgatcggaggaccgaaggagctaaccgcttttttgcacaacatgggggatcatgtaactcgccttgatcg
ttgggaaccggagctgaatgaagccataccaaacgacgagcgtgacaccacgatgcctgtagcaatggcaacaacgt
tgcgcaaactattaactggcgaactacttactctagcttcccggcaacaattaatagactggatggaggcggataaa
gttgcaggaccacttctgcgctcggcccttccggctggctggtttattgctgataaatctggagccggtgagcgtgg
gtctcgcggtatcattgcagcactggggccagatggtaagccctcccgtatcgtagttatctacacgacggggagtc
aggcaactatggatgaacgaaatagacagatcgctgagataggtgcctcactgattaagcattggtaactgtcagac
caagtttactcatatatactttagattgatttaaaacttcatttttaatttaaaaggatctaggtgaagatcctttt
tgataatctcatgaccaaaatcccttaacgtgagttttcgttccactgagcgtcagaccccgtagaaaagatcaaag
gatcttcttgagatcctttttttctgcgcgtaatctgctgcttgcaaacaaaaaaaccaccgctaccagcggtggtt
tgtttgccggatcaagagctaccaactctttttccgaaggtaactggcttcagcagagcgcagataccaaatactgt
ccttctagtgtagccgtagttaggccaccacttcaagaactctgtagcaccgcctacatacctcgctctgctaatcc
tgttaccagtggctgctgccagtggcgataagtcgtgtcttaccgggttggactcaagacgatagttaccggataag
gcgcagcggtcgggctgaacggggggttcgtgcacacagcccagcttggagcgaacgacctacaccgaactgagata
cctacagcgtgagctatgagaaagcgccacgcttcccgaagggagaaaggcggacaggtatccggtaagcggcaggg
tcggaacaggagagcgcacgagggagcttccagggggaaacgcctggtatctttatagtcctgtcgggtttcgccac
ctctgacttgagcgtcgatttttgtgatgctcgtcaggggggcggagcctatggaaaaacgccagcaacgcggcctt
tttacggttcctggccttttgctggccttttgctcacatgttctttcctgcgttatcccctgattctgtggataacc
gtattaccgcctttgagtgagctgataccgctcgccgcagccgaacgaccgagcgcagcgagtcagtgagcgaggaa
gcggaagagcgcccaatacgcaaaccgcctctccccgcgcgttggccgattcattaatgcagctggcacgacaggtt
tcccgactggaaagcgggcagtgagcgcaacgcaattaatgtgagttagctcactcattaggcaccccaggctttac
actttatgcttccggctcgtatgttgtgtggaattgtgagcggataacaatttcacacaggaaacagctatgaccat
gattacgccaagcgcgcaattaaccctcactaaagggaacaaaagctggagctgcaagcttggccattgcatacgtt
gtatccatatcataatatgtacatttatattggctcatgtccaacattaccgccatgttgacattgattattgacta
gttattaatagtaatcaattacggggtcattagttcatagcccatatatggagttccgcgttacataacttacggta
aatggcccgcctggctgaccgcccaacgacccccgcccattgacgtcaataatgacgtatgttcccatagtaacgcc
aatagggactttccattgacgtcaatgggtggagtatttacggtaaactgcccacttggcagtacatcaagtgtatc
 
atatgccaagtacgccccctattgacgtcaatgacggtaaatggcccgcctggcattatgcccagtacatgacctta
tgggactttcctacttggcagtacatctacgtattagtcatcgctattaccatggtgatgcggttttggcagtacat
caatgggcgtggatagcggtttgactcacggggatttccaagtctccaccccattgacgtcaatgggagtttgtttt
ggcaccaaaatcaacgggactttccaaaatgtcgtaacaactccgccccattgacgcaaatgggcggtaggcgtgta
cggtgggaggtctatataagcagagctcgtttagtgaaccggggtctctctggttagaccagatctgagcctgggag
ctctctggctaactagggaacccactgcttaagcctcaataaagcttgccttgagtgcttcaagtagtgtgtgcccg
tctgttgtgtgactctggtaactagagatccctcagacccttttagtcagtgtggaaaatctctagcagtggcgccc
gaacagggacctgaaagcgaaagggaaaccagaggagctctctcgacgcaggactcggcttgctgaagcgcgcacgg
caagaggcgaggggcggcgactggtgagtacgccaaaaattttgactagcggaggctagaaggagagagatgggtgc
gagagcgtcagtattaagcgggggagaattagatcgcgatgggaaaaaattcggttaaggccagggggaaagaaaaa
atataaattaaaacatatagtatgggcaagcagggagctagaacgattcgcagttaatcctggcctgttagaaacat
cagaaggctgtagacaaatactgggacagctacaaccatcccttcagacaggatcagaagaacttagatcattatat
aatacagtagcaaccctctattgtgtgcatcaaaggatagagataaaagacaccaaggaagctttagacaagataga
ggaagagcaaaacaaaagtaagaccaccgcacagcaagcggccgctgatcttcagacctggaggaggagatatgagg
gacaattggagaagtgaattatataaatataaagtagtaaaaattgaaccattaggagtagcacccaccaaggcaaa
gagaagagtggtgcagagagaaaaaagagcagtgggaataggagctttgttccttgggttcttgggagcagcaggaa
gcactatgggcgcagcctcaatgacgctgacggtacaggccagacaattattgtctggtatagtgcagcagcagaac
aatttgctgagggctattgaggcgcaacagcatctgttgcaactcacagtctggggcatcaagcagctccaggcaag
aatcctggctgtggaaagatacctaaaggatcaacagctcctggggatttggggttgctctggaaaactcatttgca
ccactgctgtgccttggaatgctagttggagtaataaatctctggaacagattggaatcacacgacctggatggagt
gggacagagaaattaacaattacacaagcttaatacactccttaattgaagaatcgcaaaaccagcaagaaaagaat
gaacaagaattattggaattagataaatgggcaagtttgtggaattggtttaacataacaaattggctgtggtatat
aaaattattcataatgatagtaggaggcttggtaggtttaagaatagtttttgctgtactttctatagtgaatagag
ttaggcagggatattcaccattatcgtttcagacccacctcccaaccccgaggggacccgacaggcccgaaggaata
gaagaagaaggtggagagagagacagagacagatccattcgattagtgaacggatctcgacggtatcgataagctaa
ttcacaaatggcagtattcatccacaattttaaaagaaaaggggggattggggggtacagtgcaggggaaagaatag
tagacataatagcaacagacatacaaactaaagaattacaaaaacaaattacaaaaattcaaaattttcgggtttat
tacagggacagcagagatccagtttgggaattagcttgatcgattagtccaatttgttaaagacaggatatcagtgg
tccaggctctagttttgactcaacaatatcaccagctgaagcctatagagtacgagccatagatagaataaaagatt
ttatttagtctccagaaaaaggggggaatgaaagaccccacctgtaggtttggcaagctaggatcaaggttaggaac
agagagacagcagaatatgggccaaacaggatatctgtggtaagcagttcctgccccggctcagggccaagaacagt
tggaacagcagaatatgggccaaacaggatatctgtggtaagcagttcctgccccggctcagggccaagaacagatg
gtccccagatgcggtcccgccctcagcagtttctagagaaccatcagatgtttccagggtgccccaaggacctgaaa
tgaccctgtgccttatttgaactaaccaatcagttcgcttctcgcttctgttcgcgcgcttctgctccccgagctca
ataaaagagcccacaacccctcactcggcgcgatctagatctcgaatcgaattcgcccttgctactATGGCTTCTGG
 
AATCCTGGTTAATGTAAAGGAGGAGGTGACCTGCCCCATCTGCCTGGAACTCCTGACACAACCCCTGAGCCTGGACT
GCGGCCACAGCTTCTGCCAAGCATGCCTCACTGCAAACCACAAGAAGTCCATGCTAGACAAAGGAGAGAGTAGCTGC
CCTGTGTGCCGGATCAGTTACCAGCCTGAGAACATACGGCCTAATCGGCATGTAGCCAACATAGTGGAGAAGCTCAG
GGAGGTCAAGTTGAGCCCAGAGGGGCAGAAAGTTGATCATTGTGCACGCCATGGAGAGAAACTTCTACTCTTCTGTC
AGGAGGACGGGAAGGTCATTTGCTGGCTTTGTGAGCGGTCTCAGGAGCACCGTGGTCACCACACGTTCCTCACAGAG
GAGGTTGCCCGGGAGTACCAAGTGAAGCTCCAGGCAGCTCTGGAGATGCTGAGGCAGAAGCAGCAGGAAGCTGAAGA
GTTAGAAGCTGACATCAGAGAAGAGAAAGCTTCCTGGAAGACTCAAATACAGTATGACAAAACCAACGTCTTGGCAG
ATTTTGAGCAACTGAGAGACATCCTGGACTGGGAGGAGAGCAATGAGCTGCAAAACCTGGAGAAGGAGGAGGAAGAC
ATTCTGAAAAGCCTTACGAACTCTGAAACTGAGATGGTGCAGCAGACCCAGTCCCTGAGAGAGCTCATCTCAGATCT
GGAGCATCGGCTGCAGGGGTCAGTGATGGAGCTGCTTCAGGGTGTGGATGGCGTCATAAAAAGGACGGAGAACGTGA
CCTTGAAGAAGCCAGAAACTTTTCCAAAAAATCAAAGGAGAGTGTTTCGAGCTCCTGATCTGAAAGGAATGCTAGAA
GTGTTTAGAGAGCTGACAGATGTCCGACGCTACTGGGTTGATGTGACAGTGGCTCCAAACAACATTTCATGTGCTGT
CATTTCTGAAGATAAGAGACAAGTGAGCTCTCCGAAACCACAGATAATATATcaggcaccagggacattatttacgt
ttccgtcactcacgAATTTCAATTATTGTACTGGCATCCTGGGCTCTCAAAGTATCACATCAGGGAAACATTACTGG
GAGGTAGACGTGTCCAAGAAAACTGCTTGGATCCTGGGGGTATGTGCTGGCTTCCAACCTGATGCAATGTGTAATAT
TGAAAAAAATGAAAATTATCAACCTAAATACGGCTACTGGGTTATAGGGTTAGAGGAAGGAGTTAAATGTAGTGCTT
TCCAGGATAGTTCCTTCCATACTCCTTCTGTTCCTTTCATTGTGCCCCTCTCTGTGATTATTTGTCCTGATCGTGTT
GGAGTTTTCCTAGACTATGAGGCTTGCACTGTCTCATTCTTCAATATCACAAACCATGGATTTCTCATCTATAAGTT
TTCTCACTGTTCTTTTTCTCAGCCTGTATTTCCATATTTAAATCCTAGAAAATGTGGAGTCCCCATGACTCTGTGCT
CACCAAGCTCTggaagcggagctactaacttcagcctgctgaagcaggctggagacgtggaggagaaccctggacct
ATGGATTCATACCTGCTGATGTGGGGACTGCTCACGTTCATCATGGTGCCTGGCTGCCAGGCAGAGCTCTGTGACGA
TGACCCGCCAGAGATCCCACACGCCACATTCAAAGCCATGGCCTACAAGGAAGGAACCATGTTGAACTGTGAATGCA
AGAGAGGTTTCCGCAGAATAAAAAGCGGGTCACTCTATATGCTCTGTACAGGAAACTCTAGCCACTCGTCCTGGGAC
AACCAATGTCAATGCACAAGCTCTGCCACTCGGAACACAACGAAACAAGTGACACCTCAACCTGAAGAACAGAAAGA
AAGGAAAACCACAGAAATGCAAAGTCCAATGCAGCCAGTGGACCAAGCGAGCCTTCCAGGTCACTGCAGGGAACCTC
CACCATGGGAAAATGAAGCCACAGAGAGAATTTATCATTTCGTGGTGGGGCAGATGGTTTATTATCAGTGCGTCCAG
GGATACAGGGCTCTACACAGAGGTCCTGCTGAGAGCGTCTGCAAAATGACCCACGGGAAGACAAGGTGGACCCAGCC
CCAGCTCATATGCACAGGTGAAATGGAGACCAGTCAGTTTCCAGGTGAAGAGAAGCCTCAGGCAAGCCCCGAAGGCC
GTCCTGAGAGTGAGACTTCCTGCCTCGTCACAACAACAGATTTTCAAATACAGACAGAAATGGCTGCAACCATGGAG
ACGTCCATATTTACAACAGAGTACCAGGTAGCAGTGGCCGGCTGTGTTTTCCTGCTGATCAGCGTCCTCCTCCTGAG
TGGGCTCACCTGGCAGCGGAGACAGAGGAAGAGTAGAAGAACAATCTGATGAGTTAACAGATCCAGTTTGGGGATCC
AAGGTCGGGCAGGAAGAGGGCCTATTTCCCATGATTCCTTCATATTTGCATATACGATACAAGGCTGTTAGAGAGAT
AATTAGAATTAATTTGACTGTAAACACAAAGATATTAGTACAAAATACGTGACGTAGAAAGTAATAATTTCTTGGGT
AGTTTGCAGTTTTAAAATTATGTTTTAAAATGGACTATCATATGCTTACCGTAACTTGAAAGTATTTCGATTTCTTG
GCTTTATATATCTTGTGGAAAGGACGAAACACCGAGCATGACTGACATCTACTTCAAGAGAGTAGATGTCAGTCATG
 
CTCTTTTTTACGCGTAGAATCGAGACCGAGGAGAGGGTTAGGGATAGGCTTACCTTCGAACCGCGGGCCCTCTAGAC
TCGAGCGGCCGCCACTGTGCTGGATATCTGCAGAATTGCCCTTGACGCGTCAGTTGGGGATCCAAGGTCGGGCAGG
AAGAGGGCCTATTTCCCATGATTCCTTCATATTTGCATATACGATACAAGGCTGTTAGAGAGATAATTAGAATTAAT
TTGACTGTAAACACAAAGATATTAGTACAAAATACGTGACGTAGAAAGTAATAATTTCTTGGGTAGTTTGCAGTTTT
AAAATTATGTTTTAAAATGGACTATCATATGCTTACCGTAACTTGAAAGTATTTCGATTTCTTGGCTTTATATATCT
TGTGGAAAGGACGAAACACCGTCGACCTTGCAATGATGTCGTAATTTGCGTCTTACTCTGTTCTCAGCGACAGCCAG
ATCTGAGCCTGGGAGCTCTCTGGCTGTCAGTAAGCTGGTACAGAAGGTTGACGAAAATTCTTACTGAGCAAGAAATT
TTTTACGCGTGTTAACAAGGGCGAATTCgttaacctcgaggatcccccgggtcgactgatcaaattcgagctcgg
tacctttaagaccaatgacttacaaggcagctgtagatcttagccactttttaaaagaaaaggggggactggaaggg
ctaattcactcccaacgaagacaagatctgctttttgcttgtactgggtctctctggttagaccagatctgagcctg
ggagctctctggctaactagggaacccactgcttaagcctcaataaagcttgccttgagtgcttcaagtagtgtgtg
cccgtctgttgtgtgactctggtaactagagatccctcagacccttttagtcagtgtggaaaatctctagcagtagt
agttcatgtcatcttattattcagtatttataacttgcaaagaaatgaatatcagagagtgagaggaacttgtttat
tgcagcttataatggttacaaataaagcaatagcatcacaaatttcacaaataaagcatttttttcactgcattcta
gttgtggtttgtccaaactcatcaatgtatcttatcatgtctggctctagctatcccgcccctaactccgcccatcc
cgcccctaactccgcccagttccgcccattctccgccccatggctgactaattttttttatttatgcagaggccgag
gccgcctcggcctctgagctattccagaagtagtgaggaggcttttttggaggcctaggcttttgcgtcgagacgta
cccaattcgccctatagtgagtcgtattacgcgcgctcactggccgtcgttttacaacgtcgtgactgggaaaaccc
tggcgttacccaacttaatcgccttgcagcacatccccctttcgccagctggcgtaatagcgaagaggcccgcaccg
atcgcccttcccaacagttgcgcagcctgaatggcgaatggcgcgacgcgccctgtagcggcgcattaagcgcggcg
ggtgtggtggttacgcgcagcgtgaccgctacacttgccagcgccctagcgcccgctcctttcgctttcttcccttc
ctttctcgccacgttcgccggctttccccgtcaagctctaaatcgggggctccctttagggttccgatttagtgctt
tacggcacctcgaccccaaaaaacttgattagggtgatggttcacgtagtgggccatcgccctgatagacggttttt
cgccctttgacgttggagtccacgttctttaatagtggactcttgttccaaactggaacaacactcaaccctatctc
ggtctattcttttgatttataagggattttgccgatttcggcctattggttaaaaaatgagctgatttaacaaaaat
ttaacgcgaattttaacaaaatattaacgtttacaatttcc-9897
SEQ ID NO: 2 
caggtggcacttttcggggaaatgtgcgcggaacccctatttgtttatttttctaaatacattcaaatatgtatccg
ctcatgagacaataaccctgataaatgcttcaataatattgaaaaaggaagagtatgagtattcaacatttccgtgt
cgcccttattcccttttttgcggcattttgccttcctgtttttgctcacccagaaacgctggtgaaagtaaaagatg
ctgaagatcagttgggtgcacgagtgggttacatcgaactggatctcaacagcggtaagatccttgagagttttcgc
cccgaagaacgttttccaatgatgagcacttttaaagttctgctatgtggcgcggtattatcccgtattgacgccgg
gcaagagcaactcggtcgccgcatacactattctcagaatgacttggttgagtactcaccagtcacagaaaagcatc
ttacggatggcatgacagtaagagaattatgcagtgctgccataaccatgagtgataacactgcggccaacttactt
 
ctgacaacgatcggaggaccgaaggagctaaccgcttttttgcacaacatgggggatcatgtaactcgccttgatcg
ttgggaaccggagctgaatgaagccataccaaacgacgagcgtgacaccacgatgcctgtagcaatggcaacaacgt
tgcgcaaactattaactggcgaactacttactctagcttcccggcaacaattaatagactggatggaggcggataaa
gttgcaggaccacttctgcgctcggcccttccggctggctggtttattgctgataaatctggagccggtgagcgtgg
gtctcgcggtatcattgcagcactggggccagatggtaagccctcccgtatcgtagttatctacacgacggggagtc
aggcaactatggatgaacgaaatagacagatcgctgagataggtgcctcactgattaagcattggtaactgtcagac
caagtttactcatatatactttagattgatttaaaacttcatttttaatttaaaaggatctaggtgaagatcctttt
tgataatctcatgaccaaaatcccttaacgtgagttttcgttccactgagcgtcagaccccgtagaaaagatcaaag
gatcttcttgagatcctttttttctgcgcgtaatctgctgcttgcaaacaaaaaaaccaccgctaccagcggtggtt
tgtttgccggatcaagagctaccaactctttttccgaaggtaactggcttcagcagagcgcagataccaaatactgt
ccttctagtgtagccgtagttaggccaccacttcaagaactctgtagcaccgcctacatacctcgctctgctaatcc
tgttaccagtggctgctgccagtggcgataagtcgtgtcttaccgggttggactcaagacgatagttaccggataag
gcgcagcggtcgggctgaacggggggttcgtgcacacagcccagcttggagcgaacgacctacaccgaactgagata
cctacagcgtgagctatgagaaagcgccacgcttcccgaagggagaaaggcggacaggtatccggtaagcggcaggg
tcggaacaggagagcgcacgagggagcttccagggggaaacgcctggtatctttatagtcctgtcgggtttcgccac
ctctgacttgagcgtcgatttttgtgatgctcgtcaggggggcggagcctatggaaaaacgccagcaacgcggcctt
tttacggttcctggccttttgctggccttttgctcacatgttctttcctgcgttatcccctgattctgtggataacc
gtattaccgcctttgagtgagctgataccgctcgccgcagccgaacgaccgagcgcagcgagtcagtgagcgaggaa
gcggaagagcgcccaatacgcaaaccgcctctccccgcgcgttggccgattcattaatgcagctggcacgacaggtt
tcccgactggaaagcgggcagtgagcgcaacgcaattaatgtgagttagctcactcattaggcaccccaggcttac
actttatgcttccggctcgtatgttgtgtggaattgtgagcggataacaatttcacacaggaaacagctatgaccat
gattacgccaagcgcgcaattaaccctcactaaagggaacaaaagctggagctgcaagcttggccattgcatacgtt
gtatccatatcataatatgtacatttatattggctcatgtccaacattaccgccatgttgacattgattattgacta
gttattaatagtaatcaattacggggtcattagttcatagcccatatatggagttccgcgttacataacttacggta
aatggcccgcctggctgaccgcccaacgacccccgcccattgacgtcaataatgacgtatgttcccatagtaacgcc
aatagggactttccattgacgtcaatgggtggagtatttacggtaaactgcccacttggcagtacatcaagtgtatc
atatgccaagtacgccccctattgacgtcaatgacggtaaatggcccgcctggcattatgcccagtacatgacctta
tgggactttcctacttggcagtacatctacgtattagtcatcgctattaccatggtgatgcggttttggcagtacat
caatgggcgtggatagcggtttgactcacggggatttccaagtctccaccccattgacgtcaatgggagtttgtttt
ggcaccaaaatcaacgggactttccaaaatgtcgtaacaactccgccccattgacgcaaatgggcggtaggcgtgta
cggtgggaggtctatataagcagagctcgtttagtgaaccggggtctctctggttagaccagatctgagcctgggag
ctctctggctaactagggaacccactgcttaagcctcaataaagcttgccttgagtgcttcaagtagtgtgtgcccg
tctgttgtgtgactctggtaactagagatccctcagacccttttagtcagtgtggaaaatctctagcagtggcgccc
gaacagggacctgaaagcgaaagggaaaccagaggagctctctcgacgcaggactcggcttgctgaagcgcgcacgg
caagaggcgaggggcggcgactggtgagtacgccaaaaattttgactagcggaggctagaaggagagagatgggtgc
 
gagagcgtcagtattaagcgggggagaattagatcgcgatgggaaaaaattcggttaaggccagggggaaagaaaaa
atataaattaaaacatatagtatgggcaagcagggagctagaacgattcgcagttaatcctggcctgttagaaacat
cagaaggctgtagacaaatactgggacagctacaaccatcccttcagacaggatcagaagaacttagatcattatat
aatacagtagcaaccctctattgtgtgcatcaaaggatagagataaaagacaccaaggaagctttagacaagataga
ggaagagcaaaacaaaagtaagaccaccgcacagcaagcggccgctgatcttcagacctggaggaggagatatgagg
gacaattggagaagtgaattatataaatataaagtagtaaaaattgaaccattaggagtagcacccaccaaggcaaa
gagaagagtggtgcagagagaaaaaagagcagtgggaataggagctttgttccttgggttcttgggagcagcaggaa
gcactatgggcgcagcctcaatgacgctgacggtacaggccagacaattattgtctggtatagtgcagcagcagaac
aatttgctgagggctattgaggcgcaacagcatctgttgcaactcacagtctggggcatcaagcagctccaggcaag
aatcctggctgtggaaagatacctaaaggatcaacagctcctggggatttggggttgctctggaaaactcatttgca
ccactgctgtgccttggaatgctagttggagtaataaatctctggaacagattggaatcacacgacctggatggagt
gggacagagaaattaacaattacacaagcttaatacactccttaattgaagaatcgcaaaaccagcaagaaaagaat
gaacaagaattattggaattagataaatgggcaagtttgtggaattggtttaacataacaaattggctgtggtatat
aaaattattcataatgatagtaggaggcttggtaggtttaagaatagtttttgctgtactttctatagtgaatagag
ttaggcagggatattcaccattatcgtttcagacccacctcccaaccccgaggggacccgacaggcccgaaggaata
gaagaagaaggtggagagagagacagagacagatccattcgattagtgaacggatctcgacggtatcgataagctaa
ttcacaaatggcagtattcatccacaattttaaaagaaaaggggggattggggggtacagtgcaggggaaagaatag
tagacataatagcaacagacatacaaactaaagaattacaaaaacaaattacaaaaattcaaaattttcgggtttat
tacagggacagcagagatccagtttgggaattagcttgatcgattagtccaatttgttaaagacaggatatcagtgg
tccaggctctagttttgactcaacaatatcaccagctgaagcctatagagtacgagccatagatagaataaaagatt
ttatttagtctccagaaaaaggggggaatgaaagaccccacctgtaggtttggcaagctaggatcaaggttaggaac
agagagacagcagaatatgggccaaacaggatatctgtggtaagcagttcctgccccggctcagggccaagaacagt
tggaacagcagaatatgggccaaacaggatatctgtggtaagcagttcctgccccggctcagggccaagaacagatg
gtccccagatgcggtcccgccctcagcagtttctagagaaccatcagatgtttccagggtgccccaaggacctgaaa
tgaccctgtgccttatttgaactaaccaatcagttcgcttctcgcttctgttcgcgcgcttctgctccccgagctca
ataaaagagcccacaacccctcactcggcgcgatctagatctcgaatcgaattcgcccttgctactATGGCTTCTGG
AATCCTGGTTAATGTAAAGGAGGAGGTGACCTGCCCCATCTGCCTGGAACTCCTGACACAACCCCTGAGCCTGGACT
GCGGCCACAGCTTCTGCCAAGCATGCCTCACTGCAAACCACAAGAAGTCCATGCTAGACAAAGGAGAGAGTAGCTGC
CCTGTGTGCCGGATCAGTTACCAGCCTGAGAACATACGGCCTAATCGGCATGTAGCCAACATAGTGGAGAAGCTCAG
GGAGGTCAAGTTGAGCCCAGAGGGGCAGAAAGTTGATCATTGTGCACGCCATGGAGAGAAACTTCTACTCTTCTGTC
AGGAGGACGGGAAGGTCATTTGCTGGCTTTGTGAGCGGTCTCAGGAGCACCGTGGTCACCACACGTTCCTCACAGAG
GAGGTTGCCCGGGAGTACCAAGTGAAGCTCCAGGCAGCTCTGGAGATGCTGAGGCAGAAGCAGCAGGAAGCTGAAGA
GTTAGAAGCTGACATCAGAGAAGAGAAAGCTTCCTGGAAGACTCAAATACAGTATGACAAAACCAACGTCTTGGCAG
ATTTTGAGCAACTGAGAGACATCCTGGACTGGGAGGAGAGCAATGAGCTGCAAAACCTGGAGAAGGAGGAGGAAGAC
ATTCTGAAAAGCCTTACGAACTCTGAAACTGAGATGGTGCAGCAGACCCAGTCCCTGAGAGAGCTCATCTCAGATCT
 
GGAGCATCGGCTGCAGGGGTCAGTGATGGAGCTGCTTCAGGGTGTGGATGGCGTCATAAAAAGGACGGAGAACGTGA
CCTTGAAGAAGCCAGAAACTTTTCCAAAAAATCAAAGGAGAGTGTTTCGAGCTCCTGATCTGAAAGGAATGCTAGAA
GTGTTTAGAGAGCTGACAGATGTCCGACGCTACTGGGTTGATGTGACAGTGGCTCCAAACAACATTTCATGTGCTGT
CATTTCTGAAGATAAGAGACAAGTGAGCTCTCCGAAACCACAGATAATATATcaggcaccagggacattatttacgt
ttccgtcactcacgAATTTCAATTATTGTACTGGCATCCTGGGCTCTCAAAGTATCACATCAGGGAAACATTACTGG
GAGGTAGACGTGTCCAAGAAAACTGCTTGGATCCTGGGGGTATGTGCTGGCTTCCAACCTGATGCAATGTGTAATAT
TGAAAAAAATGAAAATTATCAACCTAAATACGGCTACTGGGTTATAGGGTTAGAGGAAGGAGTTAAATGTAGTGCTT
TCCAGGATAGTTCCTTCCATACTCCTTCTGTTCCTTTCATTGTGCCCCTCTCTGTGATTATTTGTCCTGATCGTGTT
GGAGTTTTCCTAGACTATGAGGCTTGCACTGTCTCATTCTTCAATATCACAAACCATGGATTTCTCATCTATAAGTT
TTCTCACTGTTCTTTTTCTCAGCCTGTATTTCCATATTTAAATCCTAGAAAATGTGGAGTCCCCATGACTCTGTGCT
CACCAAGCTCTggaagcggagctactaacttcagcctgctgaagcaggctggagacgtggaggagaaccctggacct
ATGGATTCATACCTGCTGATGTGGGGACTGCTCACGTTCATCATGGTGCCTGGCTGCCAGGCAGAGCTCTGTGACGA
TGACCCGCCAGAGATCCCACACGCCACATTCAAAGCCATGGCCTACAAGGAAGGAACCATGTTGAACTGTGAATGCA
AGAGAGGTTTCCGCAGAATAAAAAGCGGGTCACTCTATATGCTCTGTACAGGAAACTCTAGCCACTCGTCCTGGGAC
AACCAATGTCAATGCACAAGCTCTGCCACTCGGAACACAACGAAACAAGTGACACCTCAACCTGAAGAACAGAAAGA
AAGGAAAACCACAGAAATGCAAAGTCCAATGCAGCCAGTGGACCAAGCGAGCCTTCCAGGTCACTGCAGGGAACCTC
CACCATGGGAAAATGAAGCCACAGAGAGAATTTATCATTTCGTGGTGGGGCAGATGGTTTATTATCAGTGCGTCCAG
GGATACAGGGCTCTACACAGAGGTCCTGCTGAGAGCGTCTGCAAAATGACCCACGGGAAGACAAGGTGGACCCAGCC
CCAGCTCATATGCACAGGTGAAATGGAGACCAGTCAGTTTCCAGGTGAAGAGAAGCCTCAGGCAAGCCCCGAAGGCC
GTCCTGAGAGTGAGACTTCCTGCCTCGTCACAACAACAGATTTTCAAATACAGACAGAAATGGCTGCAACCATGGAG
ACGTCCATATTTACAACAGAGTACCAGGTAGCAGTGGCCGGCTGTGTTTTCCTGCTGATCAGCGTCCTCCTCCTGAG
TGGGCTCACCTGGCAGCGGTGATGAGTTAACAGATCCAGTTTGGGGATCCAAGGTCGGGCAGGAAGAGGGCCTATTT
CCCATGATTCCTTCATATTTGCATATACGATACAAGGCTGTTAGAGAGATAATTAGAATTAATTTGACTGTAAACAC
AAAGATATTAGTACAAAATACGTGACGTAGAAAGTAATAATTTCTTGGGTAGTTTGCAGTTTTAAAATTATGTTTTA
AAATGGACTATCATATGCTTACCGTAACTTGAAAGTATTTCGATTTCTTGGCTTTATATATCTTGTGGAAAGGACGA
AACACCGAGCATGACTGACATCTACTTCAAGAGAGTAGATGTCAGTCATGCTCTTTTTTACGCGTAGAATCGAGACC
GAGGAGAGGGTTAGGGATAGGCTTACCTTCGAACCGCGGGCCCTCTAGACTCGAGCGGCCGCCACTGTGCTGGATAT
CTGCAGAATTGCCCTTGACGCGTCAGTTTGGGGATCCAAGGTCGGGCAGGAAGAGGGCCTATTTCCCATGATTCCTT
CATATTTGCATATACGATACAAGGCTGTTAGAGAGATAATTAGAATTAATTTGACTGTAAACACAAAGATATTAGTA
CAAAATACGTGACGTAGAAAGTAATAATTTCTTGGGTAGTTTGCAGTTTTAAAATTATGTTTTAAAATGGACTATCA
TATGCTTACCGTAACTTGAAAGTATTTCGATTTCTTGGCTTTATATATCTTGTGGAAAGGACGAAACACCGTCGACC
TTGCAATGATGTCGTAATTTGCGTCTTACTCTGTTCTCAGCGACAGCCAGATCTGAGCCTGGGAGCTCTCTGGCTGT
CAGTAAGCTGGTACAGAAGGTTGACGAAAATTCTTACTGAGCAAGAAATTTTTTACGCGTGTTAACAAGGGCGAATT
Cgttaacctcgagggatcccccggggtcgactgatcaaattcgagctcggtacctttaagaccaatgacttacaagg
cagctgtagatcttagccactttttaaaagaaaaggggggactggaagggctaattcactcccaacgaagacaagat
 
ctgctttttgcttgtactgggtctctctggttagaccagatctgagcctgggagctctctggctaactagggaaccc
actgcttaagcctcaataaagcttgccttgagtgcttcaagtagtgtgtgcccgtctgttgtgtgactctggtaact
agagatccctcagacccttttagtcagtgtggaaaatctctagcagtagtagttcatgtcatcttattattcagtat
ttataacttgcaaagaaatgaatatcagagagtgagaggaacttgtttattgcagcttataatggttacaaataaag
caatagcatcacaaatttcacaaataaagcatttttttcactgcattctagttgtggtttgtccaaactcatcaatg
tatcttatcatgtctggctctagctatcccgcccctaactccgcccatcccgcccctaactccgcccagttccgccc
attctccgccccatggctgactaattttttttatttatgcagaggccgaggccgcctcggcctctgagctattccag
aagtagtgaggaggcttttttggaggcctaggcttttgcgtcgagacgtacccaattcgccctatagtgagtcgtat
tacgcgcgctcactggccgtcgttttacaacgtcgtgactgggaaaaccctggcgttacccaacttaatcgccttgc
agcacatccccctttcgccagctggcgtaatagcgaagaggcccgcaccgatcgcccttcccaacagttgcgcagcc
tgaatggcgaatggcgcgacgcgccctgtagcggcgcattaagcgcggcgggtgtggtggttacgcgcagcgtgacc
gctacacttgccagcgccctagcgcccgctcctttcgctttcttcccttcctttctcgccacgttcgccggctttcc
ccgtcaagctctaaatcgggggctccctttagggttccgatttagtgctttacggcacctcgaccccaaaaaacttg
attagggtgatggttcacgtagtgggccatcgccctgatagacggtttttcgccctttgacgttggagtccacgttc
tttaatagtggactcttgttccaaactggaacaacactcaaccctatctcggtctattcttttgatttataagggat
tttgccgatttcggcctattggttaaaaaatgagctgatttaacaaaaatttaacgcgaattttaacaaaatattaa
cgtttacaatttcc-9897
SEQ ID NO: 3 
caggtggcacttttcggggaaatgtgcgcggaacccctatttgtttatttttctaaatacattcaaatatgtatccg
ctcatgagacaataaccctgataaatgcttcaataatattgaaaaaggaagagtatgagtattcaacatttccgtgt
cgcccttattcccttttttgcggcattttgccttcctgtttttgctcacccagaaacgctggtgaaagtaaaagatg
ctgaagatcagttgggtgcacgagtgggttacatcgaactggatctcaacagcggtaagatccttgagagttttcgc
cccgaagaacgttttccaatgatgagcacttttaaagttctgctatgtggcgcggtattatcccgtattgacgccgg
gcaagagcaactcggtcgccgcatacactattctcagaatgacttggttgagtactcaccagtcacagaaaagcatc
ttacggatggcatgacagtaagagaattatgcagtgctgccataaccatgagtgataacactgcggccaacttactt
ctgacaacgatcggaggaccgaaggagctaaccgcttttttgcacaacatgggggatcatgtaactcgccttgatcg
ttgggaaccggagctgaatgaagccataccaaacgacgagcgtgacaccacgatgcctgtagcaatggcaacaacgt
tgcgcaaactattaactggcgaactacttactctagcttcccggcaacaattaatagactggatggaggcggataaa
gttgcaggaccacttctgcgctcggcccttccggctggctggtttattgctgataaatctggagccggtgagcgtgg
gtctcgcggtatcattgcagcactggggccagatggtaagccctcccgtatcgtagttatctacacgacggggagtc
aggcaactatggatgaacgaaatagacagatcgctgagataggtgcctcactgattaagcattggtaactgtcagac
caagtttactcatatatactttagattgatttaaaacttcatttttaatttaaaaggatctaggtgaagatcctttt
tgataatctcatgaccaaaatcccttaacgtgagttttcgttccactgagcgtcagaccccgtagaaaagatcaaag
gatcttcttgagatcctttttttctgcgcgtaatctgctgcttgcaaacaaaaaaaccaccgctaccagcggtggtt
 
tgtttgccggatcaagagctaccaactctttttccgaaggtaactggcttcagcagagcgcagataccaaatactgt
ccttctagtgtagccgtagttaggccaccacttcaagaactctgtagcaccgcctacatacctcgctctgctaatcc
tgttaccagtggctgctgccagtggcgataagtcgtgtcttaccgggttggactcaagacgatagttaccggataag
gcgcagcggtcgggctgaacggggggttcgtgcacacagcccagcttggagcgaacgacctacaccgaactgagata
cctacagcgtgagctatgagaaagcgccacgcttcccgaagggagaaaggcggacaggtatccggtaagcggcaggg
tcggaacaggagagcgcacgagggagcttccagggggaaacgcctggtatctttatagtcctgtcgggtttcgccac
ctctgacttgagcgtcgatttttgtgatgctcgtcaggggggcggagcctatggaaaaacgccagcaacgcggcctt
tttacggttcctggccttttgctggccttttgctcacatgttctttcctgcgttatcccctgattctgtggataacc
gtattaccgcctttgagtgagctgataccgctcgccgcagccgaacgaccgagcgcagcgagtcagtgagcgaggaa
gcggaagagcgcccaatacgcaaaccgcctctccccgcgcgttggccgattcattaatgcagctggcacgacaggtt
tcccgactggaaagcgggcagtgagcgcaacgcaattaatgtgagttagctcactcattaggcaccccaggctttac
actttatgcttccggctcgtatgttgtgtggaattgtgagcggataacaatttcacacaggaaacagctatgaccat
gattacgccaagcgcgcaattaaccctcactaaagggaacaaaagctggagctgcaagcttggccattgcatacgtt
gtatccatatcataatatgtacatttatattggctcatgtccaacattaccgccatgttgacattgattattgacta
gttattaatagtaatcaattacggggtcattagttcatagcccatatatggagttccgcgttacataacttacggta
aatggcccgcctggctgaccgcccaacgacccccgcccattgacgtcaataatgacgtatgttcccatagtaacgcc
aatagggactttccattgacgtcaatgggtggagtatttacggtaaactgcccacttggcagtacatcaagtgtatc
atatgccaagtacgccccctattgacgtcaatgacggtaaatggcccgcctggcattatgcccagtacatgacctta
tgggactttcctacttggcagtacatctacgtattagtcatcgctattaccatggtgatgcggttttggcagtacat
caatgggcgtggatagcggtttgactcacggggatttccaagtctccaccccattgacgtcaatgggagtttgtttt
ggcaccaaaatcaacgggactttccaaaatgtcgtaacaactccgccccattgacgcaaatgggcggtaggcgtgta
cggtgggaggtctatataagcagagctcgtttagtgaaccggggtctctctggttagaccagatctgagcctgggag
ctctctggctaactagggaacccactgcttaagcctcaataaagcttgccttgagtgcttcaagtagtgtgtgcccg
tctgttgtgtgactctggtaactagagatccctcagacccttttagtcagtgtggaaaatctctagcagtggcgccc
gaacagggacctgaaagcgaaagggaaaccagaggagctctctcgacgcaggactcggcttgctgaagcgcgcacgg
caagaggcgaggggcggcgactggtgagtacgccaaaaattttgactagcggaggctagaaggagagagatgggtgc
gagagcgtcagtattaagcgggggagaattagatcgcgatgggaaaaaattcggttaaggccagggggaaagaaaaa
atataaattaaaacatatagtatgggcaagcagggagctagaacgattcgcagttaatcctggcctgttagaaacat
cagaaggctgtagacaaatactgggacagctacaaccatcccttcagacaggatcagaagaacttagatcattatat
aatacagtagcaaccctctattgtgtgcatcaaaggatagagataaaagacaccaaggaagctttagacaagataga
ggaagagcaaaacaaaagtaagaccaccgcacagcaagcggccgctgatcttcagacctggaggaggagatatgagg
gacaattggagaagtgaattatataaatataaagtagtaaaaattgaaccattaggagtagcacccaccaaggcaaa
gagaagagtggtgcagagagaaaaaagagcagtgggaataggagctttgttccttgggttcttgggagcagcaggaa
gcactatgggcgcagcctcaatgacgctgacggtacaggccagacaattattgtctggtatagtgcagcagcagaac
aatttgctgagggctattgaggcgcaacagcatctgttgcaactcacagtctggggcatcaagcagctccaggcaag
 
aatcctggctgtggaaagatacctaaaggatcaacagctcctggggatttggggttgctctggaaaactcatttgca
ccactgctgtgccttggaatgctagttggagtaataaatctctggaacagattggaatcacacgacctggatggagt
gggacagagaaattaacaattacacaagcttaatacactccttaattgaagaatcgcaaaaccagcaagaaaagaat
gaacaagaattattggaattagataaatgggcaagtttgtggaattggtttaacataacaaattggctgtggtatat
aaaattattcataatgatagtaggaggcttggtaggtttaagaatagtttttgctgtactttctatagtgaatagag
ttaggcagggatattcaccattatcgtttcagacccacctcccaaccccgaggggacccgacaggcccgaaggaata
gaagaagaaggtggagagagagacagagacagatccattcgattagtgaacggatctcgacggtatcgataagctaa
ttcacaaatggcagtattcatccacaattttaaaagaaaaggggggattggggggtacagtgcaggggaaagaatag
tagacataatagcaacagacatacaaactaaagaattacaaaaacaaattacaaaaattcaaaattttcgggtttat
tacagggacagcagagatccagtttgggaattagcttgatcgattagtccaatttgttaaagacaggatatcagtgg
tccaggctctagttttgactcaacaatatcaccagctgaagcctatagagtacgagccatagatagaataaaagatt
ttatttagtctccagaaaaaggggggaatgaaagaccccacctgtaggtttggcaagctaggatcaaggttaggaac
agagagacagcagaatatgggccaaacaggatatctgtggtaagcagttcctgccccggctcagggccaagaacagt
tggaacagcagaatatgggccaaacaggatatctgtggtaagcagttcctgccccggctcagggccaagaacagatg
gtccccagatgcggtcccgccctcagcagtttctagagaaccatcagatgtttccagggtgccccaaggacctgaaa
tgaccctgtgccttatttgaactaaccaatcagttcgcttctcgcttctgttcgcgcgcttctgctccccgagctca
ataaaagagcccacaacccctcactcggcgcgatctagatctcgaatcgaattcgcccttgctactATGGCTTCTGG
AATCCTGGTTAATGTAAAGGAGGAGGTGACCTGCCCCATCTGCCTGGAACTCCTGACACAACCCCTGAGCCTGGACT
GCGGCCACAGCTTCTGCCAAGCATGCCTCACTGCAAACCACAAGAAGTCCATGCTAGACAAAGGAGAGAGTAGCTGC
CCTGTGTGCCGGATCAGTTACCAGCCTGAGAACATACGGCCTAATCGGCATGTAGCCAACATAGTGGAGAAGCTCAG
GGAGGTCAAGTTGAGCCCAGAGGGGCAGAAAGTTGATCATTGTGCACGCCATGGAGAGAAACTTCTACTCTTCTGTC
AGGAGGACGGGAAGGTCATTTGCTGGCTTTGTGAGCGGTCTCAGGAGCACCGTGGTCACCACACGTTCCTCACAGAG
GAGGTTGCCCGGGAGTACCAAGTGAAGCTCCAGGCAGCTCTGGAGATGCTGAGGCAGAAGCAGCAGGAAGCTGAAGA
GTTAGAAGCTGACATCAGAGAAGAGAAAGCTTCCTGGAAGACTCAAATACAGTATGACAAAACCAACGTCTTGGCAG
ATTTTGAGCAACTGAGAGACATCCTGGACTGGGAGGAGAGCAATGAGCTGCAAAACCTGGAGAAGGAGGAGGAAGAC
ATTCTGAAAAGCCTTACGAACTCTGAAACTGAGATGGTGCAGCAGACCCAGTCCCTGAGAGAGCTCATCTCAGATCT
GGAGCATCGGCTGCAGGGGTCAGTGATGGAGCTGCTTCAGGGTGTGGATGGCGTCATAAAAAGGACGGAGAACGTGA
CCTTGAAGAAGCCAGAAACTTTTCCAAAAAATCAAAGGAGAGTGTTTCGAGCTCCTGATCTGAAAGGAATGCTAGAA
GTGTTTAGAGAGCTGACAGATGTCCGACGCTACTGGGTTGATGTGACAGTGGCTCCAAACAACATTTCATGTGCTGT
CATTTCTGAAGATAAGAGACAAGTGAGCTCTCCGAAACCACAGATAATATATcaggcaccagggacattatttacgt
ttccgtcactcacgAATTTCAATTATTGTACTGGCATCCTGGGCTCTCAAAGTATCACATCAGGGAAACATTACTGG
GAGGTAGACGTGTCCAAGAAAACTGCTTGGATCCTGGGGGTATGTGCTGGCTTCCAACCTGATGCAATGTGTAATAT
TGAAAAAAATGAAAATTATCAACCTAAATACGGCTACTGGGTTATAGGGTTAGAGGAAGGAGTTAAATGTAGTGCTT
TCCAGGATAGTTCCTTCCATACTCCTTCTGTTCCTTTCATTGTGCCCCTCTCTGTGATTATTTGTCCTGATCGTGTT
GGAGTTTTCCTAGACTATGAGGCTTGCACTGTCTCATTCTTCAATATCACAAACCATGGATTTCTCATCTATAAGTT
 
TTCTCACTGTTCTTTTTCTCAGCCTGTATTTCCATATTTAAATCCTAGAAAATGTGGAGTCCCCATGACTCTGTGCT
CACCAAGCTCTggaagcggagctactaacttcagcctgctgaagcaggctggagacgtggaggagaaccctggacct
ATGGATTCATACCTGCTGATGTGGGGACTGCTCACGTTCATCATGGTGCCTGGCTGCCAGGCAGAGCTCTGTGACGA
TGACCCGCCAGAGATCCCACACGCCACATTCAAAGCCATGGCCTACAAGGAAGGAACCATGTTGAACTGTGAATGCA
AGAGAGGTTTCCGCAGAATAAAAAGCGGGTCACTCTATATGCTCTGTACAGGAAACTCTAGCCACTCGTCCTGGGAC
AACCAATGTCAATGCACAAGCTCTGCCACTCGGAACACAACGAAACAAGTGACACCTCAACCTGAAGAACAGAAAGA
AAGGAAAACCACAGAAATGCAAAGTCCAATGCAGCCAGTGGACCAAGCGAGCCTTCCAGGTCACTGCAGGGAACCTC
CACCATGGGAAAATGAAGCCACAGAGAGAATTTATCATTTCGTGGTGGGGCAGATGGTTTATTATCAGTGCGTCCAG
GGATACAGGGCTCTACACAGAGGTCCTGCTGAGAGCGTCTGCAAAATGACCCACGGGAAGACAAGGTGGACCCAGCC
CCAGCTCATATGCACAGGTGAAATGGAGACCAGTCAGTTTCCAGGTGAAGAGAAGCCTCAGGCAAGCCCCGAAGGCC
GTCCTGAGAGTGAGACTTCCTGCCTCGTCACAACAACAGATTTTCAAATACAGACAGAAATGGCTGCAACCATGGAG
ACGTCCATATTTACAACAGAGTACCAGGTAGCAGTGGCCGGCTGTGTTTTCCTGCTGATCAGCGTCCTCCTCCTGAG
TGGGCTCTGATGAGTTAACAGATCCAGTTTGGGGATCCAAGGTCGGGCAGGAAGAGGGCCTATTTCCCATGATTCCT
TCATATTTGCATATACGATACAAGGCTGTTAGAGAGATAATTAGAATTAATTTGACTGTAAACACAAAGATATTAGT
ACAAAATACGTGACGTAGAAAGTAATAATTTCTTGGGTAGTTTGCAGTTTTAAAATTATGTTTTAAAATGGACTATC
ATATGCTTACCGTAACTTGAAAGTATTTCGATTTCTTGGCTTTATATATCTTGTGGAAAGGACGAAACACCGAGCAT
GACTGACATCTACTTCAAGAGAGTAGATGTCAGTCATGCTCTTTTTTACGCGTAGAATCGAGACCGAGGAGAGGGTT
AGGGATAGGCTTACCTTCGAACCGCGGGCCCTCTAGACTCGAGCGGCCGCCACTGTGCTGGATATCTGCAGAATTGC
CCTTGACGCGTCAGTTTGGGGATCCAAGGTCGGGCAGGAAGAGGGCCTATTTCCCATGATTCCTTCATATTTGCATA
TACGATACAAGGCTGTTAGAGAGATAATTAGAATTAATTTGACTGTAAACACAAAGATATTAGTACAAAATACGTGA
CGTAGAAAGTAATAATTTCTTGGGTAGTTTGCAGTTTTAAAATTATGTTTTAAAATGGACTATCATATGCTTACCGT
AACTTGAAAGTATTTCGATTTCTTGGCTTTATATATCTTGTGGAAAGGACGAAACACCGTCGACCTTGCAATGATGT
CGTAATTTGCGTCTTACTCTGTTCTCAGCGACAGCCAGATCTGAGCCTGGGAGCTCTCTGGCTGTCAGTAAGCTGGT
ACAGAAGGTTGACGAAAATTCTTACTGAGCAAGAAATTTTTACGCGTGTTAACAAGGGCGAATTCgttaacctcga
gggatcccccggggtcgactgatcaaattcgagctcggtacctttaagaccaatgacttacaaggcagctgtagatc
ttagccactttttaaaagaaaaggggggactggaagggctaattcactcccaacgaagacaagatctgctttttgct
tgtactgggtctctctggttagaccagatctgagcctgggagctctctggctaactagggaacccactgcttaagcc
tcaataaagcttgccttgagtgcttcaagtagtgtgtgcccgtctgttgtgtgactctggtaactagagatccctca
gacccttttagtcagtgtggaaaatctctagcagtagtagttcatgtcatcttattattcagtatttataacttgca
aagaaatgaatatcagagagtgagaggaacttgtttattgcagcttataatggttacaaataaagcaatagcatcac
aaatttcacaaataaagcatttttttcactgcattctagttgtggtttgtccaaactcatcaatgtatcttatcatg
tctggctctagctatcccgcccctaactccgcccatcccgcccctaactccgcccagttccgcccattctccgcccc
atggctgactaattttttttatttatgcagaggccgaggccgcctcggcctctgagctattccagaagtagtgagga
ggcttttttggaggcctaggcttttgcgtcgagacgtacccaattcgccctatagtgagtcgtattacgcgcgctca
ctggccgtcgttttacaacgtcgtgactgggaaaaccctggcgttacccaacttaatcgccttgcagcacatccccc
 
tttcgccagctggcgtaatagcgaagaggcccgcaccgatcgcccttcccaacagttgcgcagcctgaatggcgaat
ggcgcgacgcgccctgtagcggcgcattaagcgcggcgggtgtggtggttacgcgcagcgtgaccgctacacttgcc
agcgccctagcgcccgctcctttcgctttcttcccttcctttctcgccacgttcgccggctttccccgtcaagctct
aaatcgggggctccctttagggttccgatttagtgctttacggcacctcgaccccaaaaaacttgattagggtgatg
gttcacgtagtgggccatcgccctgatagacggtttttcgccctttgacgttggagtccacgttctttaatagtgga
ctcttgttccaaactggaacaacactcaaccctatctcggtctattcttttgatttataagggattttgccgatttc
ggcctattggttaaaaaatgagctgatttaacaaaaatttaacgcgaattttaacaaaatattaacgtttacaattt
cc-9897
SEQ ID NO: 4 
ATGGATTCATACCTGCTGATGTGGGGACTGCTCACGTTCATCATGGTGCCTGGCTGCCAGGCAGAGCTCTGTGACGA
TGACCCGCCAGAGATCCCACACGCCACATTCAAAGCCATGGCCTACAAGGAAGGAACCATGTTGAACTGTGAATGCA
AGAGAGGTTTCCGCAGAATAAAAAGCGGGTCACTCTATATGCTCTGTACAGGAAACTCTAGCCACTCGTCCTGGGAC
AACCAATGTCAATGCACAAGCTCTGCCACTCGGAACACAACGAAACAAGTGACACCTCAACCTGAAGAACAGAAAGA
AAGGAAAACCACAGAAATGCAAAGTCCAATGCAGCCAGTGGACCAAGCGAGCCTTCCAGGTCACTGCAGGGAACCTC
CACCATGGGAAAATGAAGCCACAGAGAGAATTTATCATTTCGTGGTGGGGCAGATGGTTTATTATCAGTGCGTCCAG
GGATACAGGGCTCTACACAGAGGTCCTGCTGAGAGCGTCTGCAAAATGACCCACGGGAAGACAAGGTGGACCCAGCC
CCAGCTCATATGCACAGGTGAAATGGAGACCAGTCAGTTTCCAGGTGAAGAGAAGCCTCAGGCAAGCCCCGAAGGCC
GTCCTGAGAGTGAGACTTCCTGCCTCGTCACAACAACAGATTTTCAAATACAGACAGAAATGGCTGCAACCATGGAG
ACGTCCATATTTACAACAGAGTACCAGGTAGCAGTGGCCGGCTGTGTTTTCCTGCTGATCAGCGTCCTCCTCCTGAG
TGGGCTCACCTGGCAGCGGAGACAGAGGAAGAGTAGAAGAACAATCTGATGA
SEQ ID NO: 5 
ATGGATTCATACCTGCTGATGTGGGGACTGCTCACGTTCATCATGGTGCCTGGCTGCCAGGCAGAGCTCTGTGACGA
TGACCCGCCAGAGATCCCACACGCCACATTCAAAGCCATGGCCTACAAGGAAGGAACCATGTTGAACTGTGAATGCA
AGAGAGGTTTCCGCAGAATAAAAAGCGGGTCACTCTATATGCTCTGTACAGGAAACTCTAGCCACTCGTCCTGGGAC
AACCAATGTCAATGCACAAGCTCTGCCACTCGGAACACAACGAAACAAGTGACACCTCAACCTGAAGAACAGAAAGA
AAGGAAAACCACAGAAATGCAAAGTCCAATGCAGCCAGTGGACCAAGCGAGCCTTCCAGGTCACTGCAGGGAACCTC
CACCATGGGAAAATGAAGCCACAGAGAGAATTTATCATTTCGTGGTGGGGCAGATGGTTTATTATCAGTGCGTCCAG
GGATACAGGGCTCTACACAGAGGTCCTGCTGAGAGCGTCTGCAAAATGACCCACGGGAAGACAAGGTGGACCCAGCC
CCAGCTCATATGCACAGGTGAAATGGAGACCAGTCAGTTTCCAGGTGAAGAGAAGCCTCAGGCAAGCCCCGAAGGCC
GTCCTGAGAGTGAGACTTCCTGCCTCGTCACAACAACAGATTTTCAAATACAGACAGAAATGGCTGCAACCATGGAG
ACGTCCATATTTACAACAGAGTACCAGGTAGCAGTGGCCGGCTGTGTTTTCCTGCTGATCAGCGTCCTCCTCCTGAG
TGGGCTCACCTGGCAGCGGTGATGA
SEQ ID NO: 6 
ATGGATTCATACCTGCTGATGTGGGGACTGCTCACGTTCATCATGGTGCCTGGCTGCCAGGCAGAGCTCTGTGACGA
TGACCCGCCAGAGATCCCACACGCCACATTCAAAGCCATGGCCTACAAGGAAGGAACCATGTTGAACTGTGAATGCA
AGAGAGGTTTCCGCAGAATAAAAAGCGGGTCACTCTATATGCTCTGTACAGGAAACTCTAGCCACTCGTCCTGGGAC
AACCAATGTCAATGCACAAGCTCTGCCACTCGGAACACAACGAAACAAGTGACACCTCAACCTGAAGAACAGAAAGA
AAGGAAAACCACAGAAATGCAAAGTCCAATGCAGCCAGTGGACCAAGCGAGCCTTCCAGGTCACTGCAGGGAACCTC
CACCATGGGAAAATGAAGCCACAGAGAGAATTTATCATTTCGTGGTGGGGCAGATGGTTTATTATCAGTGCGTCCAG
GGATACAGGGCTCTACACAGAGGTCCTGCTGAGAGCGTCTGCAAAATGACCCACGGGAAGACAAGGTGGACCCAGCC
CCAGCTCATATGCACAGGTGAAATGGAGACCAGTCAGTTTCCAGGTGAAGAGAAGCCTCAGGCAAGCCCCGAAGGCC
GTCCTGAGAGTGAGACTTCCTGCCTCGTCACAACAACAGATTTTCAAATACAGACAGAAATGGCTGCAACCATGGAG
ACGTCCATATTTACAACAGAGTACCAGGTAGCAGTGGCCGGCTGTGTTTTCCTGCTGATCAGCGTCCTCCTCCTGAG
TGGGCTCTGATGA
SEQ ID NO: 7
   gtgtcaagtccaatctatgacatcaattatatgtgaattgatgtcatagattggacttga
SEQ ID NO: 8
   gttcagaaactacctcttaatatgtgtaagaggtagtttctgaac
SEQ ID NO: 9
   gttcagaaactacctcttagtcttcttcatatgtggaagaagactaagagaac
SEQ ID NO: 10
   Gagcatgactgacatctacctgctcaacatatgtggttgagcaggtagatgtcagtcatgctc
SEQ ID NO: 11 
gacatccagatgacccagtctccatcctccctgtctgcatctgtaggagacagagtcaccatcacttgccaggcgag
tcagggcattcgcaaatatttaaattggtatcagcaaaaaccagggaaagtccctaagctcctgatctacgatgcat
ccaatttggaaacaggggtcccatcaaggttcagtggaagtggatcagggacagattttactttcgccatcagcagc
ctgcagccggaagatactgcaacatattactgtcaacaatatgatgatttccccttcaccttcggccaggggacacg
actggagattaaacgt
SEQ ID NO: 12
   5'-cgacttaaaatcgctagccagatctgagcctgggagctctctggctag-3'
SEQ ID NO: 13
   5'-gggtctctctggttagaccagatttgagcctgggagctctctggctaactagggaaccc-3'
SEQ ID NO: 14
   5'-acgaagcttgatcccgtttgccggtcgatcgcttcga-3'
SEQ ID NO: 15 
taattcgagc tcgcccgaca ttgattattg actagttatt aatagtaatc aattacgggg 60
tcattagttc atagcccata tatggagttc cgcgttacat aacttacggt aaatggcccg 120
cctggctgac cgcccaacga cccccgccca ttgacgtcaa taatgacgta tgttcccata 180
gtaacgccaa tagggacttt ccattgacgt caatgggtgg agtatttacg gtaaactgcc 240
cacttggcag tacatcaagt gtatcatatg ccaagtacgc cccctattga cgtcaatgac 300
ggtaaatggc ccgcctggca ttatgcccag tacatgacct tatgggactt tcctacttgg 360
cagtacatct acgtattagt catcgctatt accatggtga tgcggttttg gcagtacatc 420
aatgggcgtg gatagcggtt tgactcacgg ggattccaag tctcacccca ttgacgtcaa 480
tgggagtttg ttttggcacc aaaatcaacg ggactttcca aaatgtcgta acaactccgc 540
cccattgacg caaatgggcg gtaggcgtgt acggtgggag gtctatataa gcagagctcg 600
tttagtgaac cgtcagatcg cctggagacg ccatccacgc tgttttgacc tccatagaag 660
acaccgggac cgatcctagc ctccgcggcc gggaacggtg cattggaacg cggattcccc 720
gtgccaagag tgacgtaagt accgcctata gagtctatag gcccaccccc ttggcttctt 780
atgcgacgga tcgatcccgt aataagcttc gaggtccgcg gccggccgcg ttgacgcgca 840
cggcagaggc gatggggctg gcgactggtg agagatgggt gcgagagcgt cagtattaag 900
cgggggagaa ttagatcgat gggaaaaaat tcggttaagg ccagggggaa agaaaaaata 960
taaattaaaa catatagtat gggcaagcag ggagctagaa cgattcgcag ttaatcctgg 1020
cctgttagaa acatcagaag gctgtagaca aatactggga cagctacaac catcccttca 1080
gacaggatca gaagaactta gatcattata taatacagta gcaaccctct attgtgtgca 1140
tcaaaggata gagataaaag acaccaagga agctttagac aagatagagg aagagcaaaa 1200
caaaagtaag aaaaaagcac agcaagcagc agctgacaca ggacacagca atcaggtcag 1260
ccaaaattac cctatagtgc agaacatcca ggggcaaatg gtacatcagg ccatatcacc 1320
tagaacttta aatgcatggg taaaagtagt agaagagaag gctttcagcc cagaagtgat 1380
acccatgttt tcagcattat cagaaggagc caccccacaa gatttaaaca ccatgctaac 1440
acagtggggg gacatcaagc agccatgcaa atgttaaaag agaccatcaa tgaggaagct 1500
gcagaatggg atagagtgca tccagtgcat gcagggccta ttgcaccagg ccagatgaga 1560
gaaccaaggg gaagtgacat agcaggaact actagtaccc ttcaggaaca aataggatgg 1620
 
atgacacata atccacctat cccagtagga gaaatctata aaagatggat aatcctggga 1680
ttaaataaaa tagtaagaat gtatagccct accagcattc tggacataag acaaggacca 1740
aaggaaccct ttagagacta tgtagaccga ttctataaaa ctctaagagc cgagcaagct 1800
tcacaagagg taaaaaattg gatgacagaa accttgttgg tccaaaatgc gaacccagat 1860
tgtaagacta ttttaaaagc attgggacca ggagcgacac tagaagaaat gatgacagca 1920
tgtcagggag tggggggacc cggccataaa gcaagagttt tggctgaagc aatgagccaa 1980
gtaacaaatc cagctaccat aatgatacag aaaggcaatt ttaggaacca aagaaagact 2040
gttaagtgtt tcaattgtgg caaagaaggg cacatagcca aaaattgcag ggcccctagg 2100
aaaagggctg ttggaaatgt ggaaaggaag gacacccaat gaaagattgt actgagagac 2160
aggctatttt ttagggaaga tctggccttc cacagggaag gccagggaat tttcttcaga 2220
gcagaccaga gccaacagcc ccaccagaag agagcttcag gtttggggaa gagacaacaa 2280
ctccctctca gaagcaggag ccgatagaca aggaactgtg tcctttagct tccctcagat 2340
cactctttgg cagcgacccc tcgtcacaat aaagataggg gggcaattaa aggaagctct 2400
attagataca ggagcagatg atacagtatt agaagaaatg aatttgccag gaagatggaa 2460
accaaaaatg atagggggaa ttggaggttt tatcaaagta ggacagtatg atcagatact 2520
catagaaatc tgcggacata aagctatagg tacagtatta gtaggaccta cacctgtcaa 2580
cataattgga agaaatctgt tgactcagat tggctgcact ttaaattttc ccattagtcc 2640
tattgagact gtaccagtaa aattaaagcc aggaatggat ggcccaaaag ttaaacaatg 2700
gccattgaca gaagaaaaaa taaaagcatt agtagaaatt tgtacagaaa tggaaaagga 2760
aggaaaaatt tcaaaaattg ggcctgaaaa tccatacaat actccagtat ttgccataaa 2820
gaaaaaagac agtactaaat ggagaaaatt agtagatttc agagaactta ataagagaac 2880
tcaagatttc tgggaagttc aattaggaat accacatcct gcagggttaa aacagaaaaa 2940
atcagtaaca gtactggatg tgggcgatgc atatttttca gttcccttag ataaagactt 3000
caggaagtat actgcattta ccatacctag tataaacaat gagnacacca gggnattagn 3060
atatcagtac aatgtgcttc cacagggnat ggaaaggatc accagcaata ttccagtgta 3120
gcatgacaaa aatcttagag ccttttagaa aacaaaatcc agacatagtc atctatcaat 3180
acatggatga tttgtatgta ggatctgact tagaaatagg gcagcataga acaaaaatag 3240
aggaactgag acaacatctg ttgaggtggg gatttaccac accagacaaa aaacatcaga 3300
aagaacctcc attcctttgg atgggttatg aactccatcc tgataaatgg acagtacagc 3360
ctatagtgct gccagaaaag gacagctgga ctgtcaatga catacagaaa ttagtgggaa 3420
aattgaattg ggcaagtcag atttatgcag ggattaaagt aaggcaatta tgtaaacttc 3480
ttaggggaac caaagcacta acagaagtag taccactaac agaagaagca gagctagaac 3540
tggcagaaaa cagggagatt ctaaaagaac cggtacatgg agtgtattat gacccatcaa 3600
aagacttaat agcagaaata cagaagcagg ggcaaggcca atggacatat caaatttatc 3660
aagagccatt taaaaatctg aaaacaggaa aatatgcaag aatgaagggt gcccacacta 3720
 
atgatgtgaa acaattaaca gaggcagtac aaaaaatagc cacagaaagc atagtaatat 3780
ggggaaagac tcctaaattt aaattaccca tacaaaagga aacatgggaa gcatggtgga 3840
cagagtattg gcaagccacc tggattcctg agtgggagtt tgtcaatacc cctcccttag 3900
tgaagttatg gtaccagtta gagaaagaac ccataatagg agcagaaact ttctatgtag 3960
atggggcagc caatagggaa actaaattag gaaaagcagg atatgtaact gacagaggaa 4020
gacaaaaagt tgtcccccta acggacacaa caaatcagaa gactgagtta caagcaattc 4080
atctagcttt gcaggattcg ggattagaag taaacatagt gacagactca caatatgcat 4140
tgggaatcat tcaagcacaa ccagataaga gtgaatcaga gttagtcagt caaataatag 4200
agcagttaat aanaaaagga aaaagtctac ctggcatggg taccagcaca caaaggaatt 4260
ggaggaaatg aacagtagat aaattggtca gtgctggaat caggaaagta ctatttttag 4320
atggaataga taaggcccaa gaagaacatg agaaatatca cagtaattgg agagcaatgg 4380
ctagtgattt taacctacca cctgtagtag caaaagaaat agtagccagc tgtgataaat 4440
gtcagctaaa aggggaagcc atgcatggac aagtagactg tagcccagga atatggcagc 4500
tagattgtac acatttagaa ggaaaagtta tcttggtagc agttcatgta gccagtggat 4560
atatagaagc agaagtaatt ccagcagaga cagggcaaga aacagcatac ttcctcttaa 4620
aattagcagg aagatggcca gtaaaaacag tacatacaga caatggcagc aatttcacca 4680
gtactacagt taaggccgcc tgttggtggg cggggatcag caggaatttg gcattcccta 4740
caatccccaa agtcaaggag taatagaatc tatgaataaa gaattaaaga aaattatagg 4800
acaggtaaga gatcaggctg aacatcttaa gacagcagta caaatggcag tattcatcca 4860
caattttaaa agaaaagggg ggattggggg gtacagtgca ggggaaagaa tagtagacat 4920
aatagcaaca gacatacaaa ctaaagaatt acaaaaacaa attacaaaaa ttcaaaattt 4980
tcgggtttat tacagggaca gcagagatcc agtttggaaa ggaccagcaa agctcctctg 5040
gaaaggtgaa ggggcagtag taatacaaga taatagtgac ataaaagtag tgccaagaag 5100
aaaagcaaag atcatcaggg attatggaaa acagatggca ggtgatgatt gtgtggcaag 5160
tagacaggat gaggattaac acatggaatt ctgcaacaac tgctgtttat ccatttcaga 5220
attgggtgtc gacatagcag aataggcgtt actcgacaga ggagagcaag aaatggagcc 5280
agtagatcct agactagagc cctggaagca tccaggaagt cagcctaaaa ctgcttgtac 5340
caattgctat tgtaaaaagt gttgctttca ttgccaagtt tgtttcatga caaaagcctt 5400
aggcatctcc tatggcagga agaagcggag acagcgacga agagctcatc agaacagtca 5460
gactcatcaa gcttctctat caaagcagta agtagtacat gtaatgcaac ctataatagt 5520
agcaatagta gcattagtag tagcaataat aatagcaata gttgtgtggt ccatagtaat 5580
catagaatat aggaaaatgg ccgctgatct tcagacctgg aggaggagat atgagggaca 5640
attggagaag tgaattatat aaatataaag tagtaaaaat tgaaccatta ggagtagcac 5700
ccaccaaggc aaagagaaga gtggtgcaga gagaaaaaag agcagtggga ataggagctt 5760
tgttccttgg gttcttggga gcagcaggaa gcactatggg cgcagcgtca atgacgctga 5820
 
cggtacaggc cagacaatta ttgtctggtn tagtgcagca gcagaacaat ttgctgaggg 5880
ctattgaggc gcaacagcat ctgttgcaac tcacagtctg gggcatcaag cagctccagg 5940
caagaatcct ggctgtggaa agatacctaa aggatcaaca gctcctgggg atttggggtt 6000
gctctggaaa actcatttgc accactgctg tgccttggaa tgctagttgg agtaataaat 6060
ctctggaaca gatttggaat cacacgacct ggatggagtg ggacagagaa attaacaatt 6120
acacaagctt aatacactcc ttaattgaag aatcgcaaaa ccagcaagaa aagaatgaac 6180
aagaattatt ggaattagat gaatgggcaa gtttgtggaa ttggtttaac ataacaaatt 6240
ggctgtggta tataaaatta ttcataatga tagtaggagg cttggtaggt ttaagaatag 6300
tttttgctgt actttctata gtgaatagag ttaggcaggg atattcacca ttatcgtttc 6360
agacccacct cccaaccccg aggggacccg acaggcccga aggaatagaa gaagaaggtg 6420
gagagagaga cagagacaga tccattcgat tagtgaacgg atccttggca cttatctggg 6480
acgatctgcg gagcctgtgc ctcttcagct accaccgctt gagagactta ctcttgattg 6540
taacgaggat tgtggaactt ctgggacgca gggggtggga agccctcaaa tattggtgga 6600
atctcctaca atattggagt caggagctaa agaatagtgc tgttagcttg ctcaatgcca 6660
cagccatagc agtagctgag gggacagata gggttataga agtagtacaa ggagcttgta 6720
gagctattcg ccacatacct agaagaataa gacagggctt ggaaaggatt ttgctataag 6780
ctcgaggccg ccccggtgac cttcagacct tggcactgga ggtggcccgg cagaagcgcg 6840
gcatcgtgga tcagtgctgc accagcatct gctctctcta ccaactggag aactactgca 6900
actaggccca ccactaccct gtccacccct ctgcaatgaa taaaaccttt gaaagagcac 6960
tacaagttgt gtgtacatgc gtgcatgtgc atatgtggtg cggggggaac atgagtgggg 7020
ctggctggag tggcgatgat aagctgtcaa acatgagaat taattcttga agacgaaagg 7080
gcctcgtgat acgcctattt ttataggtta atgtcatgat aataatggtt tcttagtcta 7140
gaattaattc cgtgtattct atagtgncac ctaaatcgta tgtgtatgat acataaggtt 7200
atgtattaat tgtagccgcg ttctaacgac aatatgtaca agcctaattg tgtagcatct 7260
ggcttactga agcagaccct atcatctctc tcgtaaactg ccgtcagagt cggtttggtt 7320
ggacgaacct tctgagtttc tggtaacgcc gtcccgcacc cgcacccggn aaatggtcag 7380
cgaaccaatc agcagggtca tcgctagcca gatcctctac gccggacgca tcgtggccgg 7440
catcaccggc gccacaggtg cggttgcatg gcgcctatat cgccgacatc accgatgggg 7500
aagatcgggc tcgccacttc gggctcatga gcgcttgttt cggcgtgggt atggtggcag 7560
gccccgtggc cgggggactg ttgggcgcca tctccttgca tgcaccattc cttgcggcgg 7620
cggtgctcaa cggcctcaac ctactactgg gctgcttcct aatgcaggag tcgcataagg 7680
gagagcgtcg aatggtgcac tctcagtaca atctgctctg atgccgcata gttaagccag 7740
ccccgacacc cgccaacacc cgctgacgcg ccctgacggg cttgtctgct cccggcatcc 7800
gcttacagac aagctgtgac cgtctccggg agctgcatgt gtcagaggtn ntcaccgtca 7860
tcaccgaaac gcgcgagacg aaagggcctc gtgatacgcc tatttttata ggttaatgtc 7920
 
atgataataa tggtttctta gacgtcaggt ggcacttttc ggggaaatgt gcgcggaacc 7980
cctatttgtt tatttttcta aatacattca aatatgtatc cgctcatgag acaataaccc 8040
tgataaatgc ttcaataata ttgaaaaagg aagagtatga gtattcaaca tttccgtgtc 8100
gcccttattc ccttttttgc ggcattttgc cttcctgttt ttgctcaccc agaaacgctg 8160
gtgaaagtaa aagatgctga agatcagttg ggtgcacgag tgggttacat cgaactggat 8220
ctcaacagcg gtaagatcct tgagagtttt cgccccgaag aacgttttcc aatgatgagc 8280
acttttaaag ttctgctatg tggcgcggta ttatcccgta ttgacgccgg gcaagagcaa 8340
ctcggtcgcc gcatacacta ttctcagaat gacttggttg agtactcacc agtcacagaa 8400
aagcatctta cggatggcat gacagtaaga gaattatgca gtgctgccat aaccatgagt 8460
gataacactg cggccaactt acttctgaca acgatcggag gaccgaagga gctaaccgct 8520
tttttgcaca acatggggga tcatgtaact cgccttgatc gttgggaacc ggagctgaat 8580
gaagccatac caaacgacga gcgtgacacc acgatgcctg tagcaatggc aacaacgttg 8640
cgcaaactat taactggcga actacttact ctagcttccc ggcaacaatt aatagactgg 8700
atggaggcgg ataaagttgc aggaccactt ctgcgctcgg cccttccggc tggctggttt 8760
attgctgata aatctggagc cggtgagcgt gggtctcgcg gtatcattgc agcactgggg 8820
ccagatggta agccctcccg tatcgtagtt atctacacga cggggagtca ggcaactatg 8880
gatgaacgaa atagacagat cgctgagata ggtgcctcac tgattaagca ttggtaactg 8940
tcagaccaag tttactcata tatactttag attgatttaa aacttcattt ttaatttaaa 9000
aggatctagg tgaagatcct ttttgataat ctcatgacca aaatccctta acgtgagttt 9060
tcgttccact gagcgtcaga ccccgtagaa aagatcaaag gatcttcttg agatcctttt 9120
tttctgcgcg taatctgctg cttgcaaaca aaaaaaccac cgctaccagc ggtggtttgt 9180
ttgccggatc aagagctacc aactcttttt ccgaaggtaa ctggcttcag cagagcgcag 9240
ataccaaata ctgtccttct agtgtagccg tagttaggcc accacttcaa gaactctgta 9300
gcaccgccta catacctcgc tctgctaatc ctgttaccag tggctgctgc cagtggcgat 9360
aagtcgtgtc ttaccgggtt ggactcaaga cgatagttac cggataaggc gcagcggtcg 9420
ggctgaacgg ggggttcgtg cacacagccc agcttggagc gaacgaccta caccgaactg 9480
agatacctac agcgtgagca ttgagaaagc gccacgcttc ccgaagggag aaaggcggac 9540
aggtatccgg taagcggcag ggtcggaaca ggagagcgca cgagggagct tccaggggga 9600
aacgcctggt atctttatag tcctgtcggg tttcgccacc tctgacttga gcgtcgattt 9660
ttgtgatgct cgtcaggggg gcggagccta tggaaaaacg ccagcaacgc ggccttttta 9720
cggttcctgg ccttttgctg gccttttgct cacatgttct ttcctgcgtt atcccctgat 9780
tctgtggata accgtattac cgcctttgag tgagctgata ccgctcgccg cagccgaacg 9840
accgagcgca gcgagtcagt gagcgaggaa gcggaagagc gcccaatacg caaaccgcct 9900
ctccccgcgc gttggccgat tcattaatgc agctgtggaa tgtgtgtcag ttagggtgtg 9960
gaaagtcccc aggctcccca gcaggcagaa gtatgcaaag catgcatctc aattagtcag 10020
 
caaccaggtg tggaaagtcc ccaggctccc cagcaggcag aagtatgcaa agcatgcatc 10080
tcaattagtc agcaaccata gtcccgcccc taactccgcc catcccgccc ctaactccgc 10140
ccagttccgc ccattctccg ccccatggct gactaatttt ttttatttat gcagaggccg 10200
aggccgcctc ggcctctgag ctattccaga agtagtgagg aggctttttt ggaggcctag 10260
gcttttgcaa aaagcttgga cacaagacag gcttgcgaga tatgtttgag aataccactt 10320
tatcccgcgt cagggagagg cagtgcgtaa aaagacgcgg actcatgtga aatactggtt 10380
tttagtgcgc cagatctcta taatctcgcg caacctattt tcccctcgaa cactttttaa 10440
gccgtagata aacaggctgg gacacttcac atgagcgaaa aatacatcgt cacctgggac 10500
atgttgcaga tccatgcacg taaactcgca agccgactga tgccttctga acaatggaaa 10560
ggcattattg ccgtaagccg tggcggtctg taccgggtgc gttactggcg cgtgaactgg 10620
gtattcgtca tgtcgatacc gtttgtattt ccagctacga tcacgacaac cagcgcgagc 10680
ttaaagtgct gaaacgcgca gaaggcgatg gcgaaggctt catcgttatt gatgacctgg 10740
tggataccgg tggtactgcg gttgcgattc gtgaaatgta tccaaaagcg cactttgtca 10800
ccatcttcgc aaaaccggct ggtcgtccgc tggttgatga ctatgttgtt gatatcccgc 10860
aagatacctg gattgaacag ccgtgggata tgggcgtcgt attcgtcccg ccaatctccg 10920
gtcgctaatc ttttcaacgc ctggcactgc cgggcgttgt tctttttaac ttcaggcggg 10980
ttacaatagt ttccagtaag tattctggag gctgcatcca tgacacaggc aaacctgagc 11040
gaaaccctgt tcaaaccccg ctttaaacat cctgaaacct cgacgctagt ccgccgcttt 11100
aatcacggcg cacaaccgcc tgtgcagtcg gcccttgatg gtaaaaccat ccctcactgg 11160
tatcgcatga ttaaccgtct gatgtggatc tggcgcggca ttgacccacg cgaaatcctc 11220
gacgtccagg cacgtattgt gatgagcgat gccgaacgta ccgacgatga tttatacgat 11280
acggtgattg gctaccgtgg cggcaactgg atttatgagt gggccccgga tctttgtgaa 11340
ggaaccttac ttctgtggtg tgacataatt ggacaaacta cctacagaga tttaaagctc 11400
taaggtaaat ataaaatttt taagtgtata atgtgttaaa ctactgattc taattgtttg 11460
tgtattttag attccaacct atggaactga tgaatgggag cagtggtgga atgcctttaa 11520
tgaggaaaac ctgttttgct cagaagaaat gccatctagt gatgatgagg ctactgctga 11580
ctctcaacat tctactcctc caaaaaagaa gagaaaggta gaagacccca aggactttcc 11640
ttcagaattg ctaagttttt tgagtcatgc tgtgtttagt aatagaactc ttgcttgctt 11700
tgctatttac accacaaagg aaaaagctgc actgctatac aagaaaatta tggaaaaata 11760
ttctgtaacc tttataagta ggcataacag ttataatcat aacatactgt tttttcttac 11820
tccacacagg catagagtgt ctgctattaa taactatgct caaaaattgt gtacctttag 11880
ctttttaatt tgtaaagggg ttaataagga atatttgatg tatagtgcct tgactagaga 11940
tcataatcag ccataccaca tttgtagagg ttttacttgc tttaaaaaac ctccccacac 12000
ctccccctga acctgaaaca taaaatgaat gcaattgttg ttgttgggct gcagtatgaa 12060
t 12061
SEQ ID NO: 16 
catggccaag ttgaccagtg ccgttccggt gctcaccgcg cgcgacgtcg ccggagcggt 60
cgagttctgg accgaccggc tcgggttctc ccgggacttc gtggaggacg acttcgccgg 120
tgtggtccgg gacgacgtga ccctgttcat cagcgcggtc caggaccagg tggtgccgga 180
caacaccctg gcctgggtgt gggtgcgcgg cctggacgag ctgtacgccg agtggtcgga 240
ggtcgtgtcc acgaacttcc gggacgcctc cgggccggcc atgaccgaga tcggcgagca 300
gccgtggggg cgggagttcg ccctgcgcga cccggccggc aactgcgtgc acttcgtggc 360
cgaggagcag gactgacacg tgctacgaga tttcgattcc accgccgcct tctatgaaag 420
gttgggcttc ggaatcgttt tccgggacgc cggctggatg atcctccagc gcggggatct 480
catgctggag ttcttcgccc accccaactt gtttattgca gcttataatg gttacaaata 540
aagcaatagc atcacaaatt tcacaaataa agcatttttt tcactgcatt ctagttgtgg 600
tttgtccaaa ctcatcaatg tatcttatca tgtctgtata ccgtcgacct ctagctagag 660
cttggcgtaa tcatggtcat agctgtttcc tgtgtgaaat tgttatccgc tcacaattcc 720
acacaacata cgagccggaa gcataaagtg taaagcctgg ggtgcctaat gagtgagcta 780
actcacatta attgcgttgc gctcactgcc cgctttccag tcgggaaacc tgtcgtgcca 840
gctgcattaa tgaatcggcc aacgcgcggg gagaggcggt ttgcgtattg ggcgctcttc 900
cgcttcctcg ctcactgact cgctgcgctc ggtcgttcgg ctgcggcgag cggtatcagc 960
tcactcaaag gcggtaatac ggttatccac agaatcaggg gataacgcag gaaagaacat 1020
gtgagcaaaa ggccagcaaa aggccaggaa ccgtaaaaag gccgcgttgc tggcgttttt 1080
ccataggctc cgcccccctg acgagcatca caaaaatcga cgctcaagtc agaggtggcg 1140
aaacccgaca ggactataaa gataccaggc gtttccccct ggaagctccc tcgtgcgctc 1200
tcctgttccg accctgccgc ttaccggata cctgtccgcc tttctccctt cgggaagcgt 1260
ggcgctttct caatgctcac gctgtaggta tctcagttcg gtgtaggtcg ttcgctccaa 1320
gctgggctgt gtgcacgaac cccccgttca gcccgaccgc tgcgccttat ccggtaacta 1380
tcgtcttgag tccaacccgg taagacacga cttatcgcca ctggcagcag ccactggtaa 1440
caggattagc agagcgaggt atgtaggcgg tgctacagag ttcttgaagt ggtggcctaa 1500
ctacggctac actagaagga cagtatttgg tatctgcgct ctgctgaagc cagttacctt 1560
cggaaaaaga gttggtagct cttgatccgg caaacaaacc accgctggta gcggtggttt 1620
ttttgtttgc aagcagcaga ttacgcgcag aaaaaaagga tctcaagaag atcctttgat 1680
cttttctacg gggtctgacg ctcagtggaa cgaaaactca cgttaaggga ttttggtcat 1740
gagattatca aaaaggatct tcacctagat ccttttaaat taaaaatgaa gttttaaatc 1800
aatctaaagt atatatgagt aaacttggtc tgacagttac caatgcttaa tcagtgaggc 1860
acctatctca gcgatctgtc tatttcgttc atccatagtt gcctgactcc ccgtcgtgta 1920
gataactacg atacgggagg gcttaccatc tggccccagt gctgcaatga taccgcgaga 1980
 
cccacgctca ccggctccag atttatcagc aataaaccag ccagccggaa gggccgagcg 2040
cagaagtggt cctgcaactt tatccgcctc catccagtct attaattgtt gccgggaagc 2100
tagagtaagt agttcgccag ttaatagttt gcgcaacgtt gttgccattg ctacaggcat 2160
cgtggtgtca cgctcgtcgt ttggtatggc ttcattcagc tccggttccc aacgatcaag 2220
gcgagttaca tgatccccca tgttgtgcaa aaaagcggtt agctccttcg gtcctccgat 2280
cgttgtcaga agtaagttgg ccgcagtgtt atcactcatg gttatggcag cactgcataa 2340
ttctcttact gtcatgccat ccgtaagatg cttttctgtg actggtgagt actcaaccaa 2400
gtcattctga gaatagtgta tgcggcgacc gagttgctct tgcccggcgt caatacggga 2460
taataccgcg ccacatagca gaactttaaa agtgctcatc attggaaaac gttcttcggg 2520
gcgaaaactc tcaaggatct taccgctgtt gagatccagt tcgatgtaac ccactcgtgc 2580
acccaactga tcttcagcat cttttacttt caccagcgtt tctgggtgag caaaaacagg 2640
aaggcaaaat gccgcaaaaa agggaataag ggcgacacgg aaatgttgaa tactcatact 2700
cttccttttt caatattatt gaagcattta tcagggttat tgtctcatga gcggatacat 2760
atttgaatgt atttagaaaa ataaacaaat aggggttccg cgcacatttc cccgaaaagt 2820
gccacctgac gtcgacggat cgggagatct cccgatcccc tatggtcgac tctcagtaca 2880
atctgctctg atgccgcata gttaagccag tatctgctcc ctgcttgtgt gttggaggtc 2940
gctgagtagt gcgcgagcaa aatttaagct acaacaaggc aaggcttgac cgacaattgc 3000
atgaagaatc tgcttagggt taggcgtttt gcgctgcttc gcgatgtacg ggccagatat 3060
acgcgttgac attgattatt gactagttat taatagtaat caattacggg gtcattagtt 3120
catagcccat atatggagtt ccgcgttaca taacttacgg taaatggccc gcctggctga 3180
ccgcccaacg acccccgccc attgacgtca ataatgacgt atgttcccat agtaacgcca 3240
atagggactt tccattgacg tcaatgggtg gactatttac ggtaaactgc ccacttggca 3300
gtacatcaag tgtatcatat gccaagtacg ccccctattg acgtcaatga cggtaaatgg 3360
cccgcctggc attatgccca gtacatgacc ttatgggact ttcctacttg gcagtacatc 3420
tacgtattag tcatcgctat taccatggtg atgcggtttt ggcagtacat caatgggcgt 3480
ggatagcggt ttgactcacg gggatttcca agtctccacc ccattgacgt caatgggagt 3540
ttgttttggc accaaaatca acgggacttt ccaaaatgtc gtaacaactc cgccccattg 3600
acgcaaatgg gcggtaggcg tgtacggtgg gaggtctata taagcagagc tctctggcta 3660
actagagaac ccactgctta ctggcttatc gaaattaata cgactcacta tagggagacc 3720
caagctggct agcgtttaaa cttaagctta tgaatagagg attctttaac atgctcggcc 3780
gccgcccctt cccggccccc actgccatgt ggaggccgcg gagaaggagg caggcggccc 3840
cgatgcctgc ccgcaacggg ctggcttctc aaatccagca actgaccaca gccgtcagtg 3900
ccctagtcat tggacaggca actagacctc aacccccacg tccacgcccg ccaccgcgcc 3960
agaagaagca ggcgcccaag caaccaccga agccgaagaa accaaaaacg caggagaaga 4020
agaagaagca acctgcaaaa cccaaacccg gaaagagaca gcgcatggca cttaagttgg 4080
 
aggccgacag attgttcgac gtcaagaacg aggacggaga tgtcatcggg cacgcactgg 4140
ccatggaagg aaaggtaatg aaacctctgc acgtgaaagg aaccatcgac caccctgtgc 4200
tatcaaagct caaatttacc aagtcgtcag catacgacat ggagttcgca cagttgccag 4260
tcaacatgag aagtgaggca ttcacctaca ccagtgaaca ccccgaagga ttctataact 4320
ggcaccacgg agcggtgcag tatagtggag gtagatttac catccctcgc ggagtaggag 4380
gcagaggaga cagcggtcgt ccgatcatgg ataactccgg tcgggttgtc gcgatagtcc 4440
tcggtggagc tgatgaagga acacgaactg ccctttcggt cgtcacctgg aatagtaaag 4500
ggaagacaat taagacgacc ccggaaggga cagaagagtg gtccgcagca ccactggtca 4560
cggcaatgtg tttgctcgga aatgtgagct tcccatgcga ccgcccgccc acatgctata 4620
cccgcgaacc ttccagagcc ctcgacatcc ttgaagagaa cgtgaaccat gaggcctacg 4680
ataccctgct caatgccata ttgcggtgcg gatcgtctgg cagaagcaaa agaagcgtca 4740
ctgacgactt taccctgacc agcccctact tgggcacatg ctcgtactgc caccatactg 4800
aaccgtgctt cagccctgtt aagatcgagc aggtctggga cgaagcggac gataacacca 4860
tacgcataca gacttccgcc cagtttggat acgaccatag cggagcagca agcgcaaaca 4920
agtaccgcta catgtcgctt aagcaggtaa ccgacaacaa attcaacaaa gaacaacaaa 4980
acgcgttcta tgagatctta catttaccta acttaaacga agaacaacga aacgccttca 5040
tccaaagttt aaaagatgac ccaagccaaa gcgctaacct tttagcagaa gctaaaaagc 5100
taaatgatgc tcaggcgccg aaagtagaca acaaattcaa caaagaacaa caaaacgcgt 5160
tctatgagat cttacattta cctaacttaa acgaagaaca acgaaacgcc ttcatccaaa 5220
gtttaaaaga tgacccaagc caaagcgcta accttttagc agaagctaaa aagctaaatg 5280
atgctcaggc gccgaaagta gacgcgaatt cgagctcggt acccggggat ccggtaacca 5340
ccgttaaaga aggcaccatg gatgacatca agattagcac ctcaggaccg tgtagaaggc 5400
ttagctacaa aggatacttt ctcctcgcaa aatgccctcc aggggacagc gtaacggtta 5460
gcatagtgag tagcaactca gcaacgtcat gtacactggc ccgcaagata aaaccaaaat 5520
tcgtgggacg ggaaaaatat gatctacctc ccgttcacgg taaaaaaatt ccttgcacag 5580
tgtacgaccg tctgaaagaa acaactgcag gctacatcac tatgcacagg ccgggaccgc 5640
acgcttatac atcctacctg gaagaatcat cagggaaagt ttacgcaaag ccgccatctg 5700
ggaagaacat tacgtatgag tgcaagtgcg gcgactacaa gaccggaacc gtttcgaccc 5760
gcaccgaaat cactggttgc accgccatca agcagtgcgt cgcctataag agcgaccaaa 5820
cgaagtgggt cttcaactca ccggacttga tcagacatga cgaccacacg gcccaaggga 5880
aattgcattt gcctttcaag ttgatcccga gtacctgcat ggtccctgtt gcccacgcgc 5940
cgaatgtaat acatggcttt aaacacatca gcctccaatt agatacagac cacttgacat 6000
tgctcaccac caggagacta ggggcaaacc cggaaccaac cactgaatgg atcgtcggaa 6060
agacggtcag aaacttcacc gtcgaccgag atggcctgga atacatatgg ggaaatcatg 6120
agccagtgag ggtctatgcc caagagtcag caccaggaga ccctcacgga tggccacacg 6180
 
aaatagtaca gcattactac catcgccatc ctgtgtacac catcttagcc gtcgcatcag 6240
ctaccgtggc gatgatgatt ggcgtaactg ttgcagtgtt atgtgcctgt aaagcgcgcc 6300
gtgagtgcct gacgccatac gccctggccc caaacgccgt aatcccaact tcgctggcac 6360
tcttgtgctg cgttaggtcg gccaatgctg aaacgttcac cgagaccatg agttacttgt 6420
ggtcgaacag tcagccgttc ttctgggtcc agttgtgcat acctttggcc gctttcatcg 6480
ttctaatgcg ctgctgctcc tgctgcctgc cttttttagt ggttgccggc gcctacctgg 6540
cgaaggtaga cgcctacgaa catgcgacca ctgttccaaa tgtgccacag ataccgtata 6600
aggcacttgt tgaaagggca gggtatgccc cgctcaattt ggagatcact gtcatgtcct 6660
cggaggtttt gccttccacc aaccaagagt acattacctg caaattcacc actgtggtcc 6720
cctccccaaa aatcaaatgc tgcggctcct tggaatgtca gccggccgct catgcagact 6780
atacctgcaa ggtcttcgga ggggtctacc cctttatgtg gggaggagcg caatgttttt 6840
gcgacagtga gaacagccag atgagtgagg cgtacgtcga attgtcagca gattgcgcgt 6900
ctgaccacgc gcaggcgatt aaggtgcaca ctgccgcgat gaaagtagga ctgcgtatag 6960
tgtacgggaa cactaccagt ttcctagatg tgtacgtgaa cggagtcaca ccaggaacgt 7020
ctaaagactt gaaagtcata gctggaccaa tttcagcatc atttacgcca ttcgatcata 7080
aggtcgttat ccatcgcggc ctggtgtaca actatgactt cccggaatat ggagcgatga 7140
aaccaggagc gtttggagac attcaagcta cctccttgac tagcaaggat ctcatcgcca 7200
gcacagacat taggctactc aagccttccg ccaagaatgt gcatgtcccg tacacgcagg 7260
ccgcatcagg atttgagatg tggaaaaaca actcaggccg cccattgcag gaaaccgcac 7320
ctttcgggtg taagattgca gtaaatccgc tccgagcggt ggactgttca tacgggaaca 7380
ttcccatttc tattgacatc ccgaacgctg cctttatcag gacatcagat gcaccactgg 7440
tctcaacagt caaatgtgaa gtcagtgagt gcacttattc agcagacttc gacgggatgg 7500
ccaccctgca gtatgtatcc gaccgcgaag gtcaatgccc cgtacattcg cattcgagca 7560
cagcaactct ccaagagtcg acagtacatg tcctggagaa aggagcggtg acagtacact 7620
ttagcaccgc gagtccacag gcgaacttta tcgtatcgct gtgtgggaag aagacaacat 7680
gcaatgcaga atgtaaacca ccagctgacc atatcgtgag caccccgcac aaaaatgacc 7740
aagaatttca agccgccatc tcaaaaacat catggagttg gctgtttgcc cttttcggcg 7800
gcgcctcgtc gctattaatt ataggactta tgatttttgc ttgcagcatg atgctgacta 7860
gcacacgaag atgacgggcc cgtttaaacc cgctgatcag cctcgactgt gccttctagt 7920
tgccagccat ctgttgtttg cccctccccc gtgccttcct tgaccctgga aggtgccact 7980
cccactgtcc tttcctaata aaatgaggaa attgcatcgc attgtctgag taggtgtcat 8040
tctattctgg ggggtggggt ggggcaggac agcaaggggg aggattggga agacaatagc 8100
aggcatgctg gggatgcggt gggctctatg gcttctgagg cggaaagaac cagctggggc 8160
tctagggggt atccccacgc gccctgtagc ggcgcattaa gcgcggcggg tgtggtggtt 8220
acgcgcagcg tgaccgctac acttgccagc gccctagcgc ccgctccttt cgctttcttc 8280
 
ccttcctttc tcgccacgtt cgccggcttt ccccgtcaag ctctaaatcg gggcatccct 8340
ttagggttcc gatttagtgc tttacggcac ctcgacccca aaaaacttga ttagggtgat 8400
ggttcacgta gtgggccatc gccctgatag acggtttttc gccctttgac gttggagtcc 8460
acgttcttta atagtggact cttgttccaa actggaacaa cactcaaccc tatctcggtc 8520
tattcttttg atttataagg gattttgggg atttcggcct attggttaaa aaatgagctg 8580
atttaacaaa aatttaacgc gaattaattc tgtggaatgt gtgtcagtta gggtgtggaa 8640
agtccccagg ctccccaggc aggcagaagt atgcaaagca tgcatctcaa ttagtcagca 8700
accaggtgtg gaaagtcccc aggctcccca gcaggcagaa gtatgcaaag catgcatctc 8760
aattagtcag caaccatagt cccgccccta actccgccca tcccgcccct aactccgccc 8820
agttccgccc attctccgcc ccatggctga ctaatttttt ttatttatgc agaggccgag 8880
gccgcctctg cctctgagct attccagaag tagtgaggag gcttttttgg aggcctaggc 8940
ttttgcaaaa agctcccggg agcttgtata tccattttcg gatctgatca gcacgtgttg 9000
acaattaatc atcggcatag tatatcggca tagtataata cgacaaggtg aggaactaaa 9060
c 9061
SEQ ID NO: 17 
atgtccgcag caccactggt cacggcaatg tgtttgctcg gaaatgtgag cttcccatgc 60
gaccgcccgc ccacatgcta tacccgcgaa ccttccagag ccctcgacat ccttgaagag 120
aacgtgaacc atgaggccta cgataccctg ctcaatgcca tattgcggtg cggatcgtct 180
ggcagaagca aaaga 195
SEQ ID NO: 18 
agcgtcactg acgactttac cctgaccagc ccctacttgg gcacatgctc gtactgccac 60
catactgaac cgtgcttcag ccctgttaag atcgagcagg tctgggacga agcggacgat 120
aacaccatac gcatacagac ttccgcccag tttggatacg accatagcgg agcagcaagc 180
gcaaacaagt accgctacat gtcgcttaag caggtaacca acgtagacaa caaattcaac 240
aaagaacaac aaaacgcgtt ctatgagatc ttacatttac ctaacttaaa cgaagaacaa 300
cgaaacgcct tcatccaaag tttaaaagat gacccaagcc aaagcgctaa ccttttagca 360
gaagctaaaa agctaaatga tgctcaggcg ccgaaagtag acaacaaatt caacaaagaa 420
caacaaaacg cgttctatga gatcttacat ttacctaact taaacgaaga acaacgaaac 480
gccttcatcc aaagtttaaa agatgaccca agccaaagcg ctaacctttt agcagaagct 540
aaaaagctaa atgatgctca ggcgccgaaa gtagacgcga attcgagctc ggtacccgta 600
accaccgtta aagaaggcac catggatgac atcaagatta gcacctcagg accgtgtaga 660
aggcttagct acaaaggata ctttctcctc gcaaaatgcc ctccagggga cagcgtaacg 720
 
gttagcatag tgagtagcaa ctcagcaacg tcatgtacac tggcccgcaa gataaaacca 780
aaattcgtgg gacggaaaaa atatgatcta cctcccgttc acggtaaaaa aattccttgc 840
acagtgtacg accgtctgaa agaaacaact gcaggctaca tcactatgca caggccggga 900
ccgcacgctt atacatccta cctggaagaa tcatcaggga aagtttacgc aaagccgcca 960
tctgggaaga acattacgta tgagtgcaag tgcggcgact acaagaccgg aaccgtttcg 1020
acccgcaccg aaatcactgg ttgcaccgcc atcaagcagt gcgtcgccta taagagcgac 1080
caaacgaagt gggtcttcaa ctcaccggac ttgatcagac atgacgacca cacggcccaa 1140
gggaaattgc atttgccttt caagttgatc ccgagtacct gcatggtccc tgttgcccac 1200
gcgccgaatg taatacatgg ctttaaacac atcagcctcc aattagatac agaccacttg 1260
acattgctca ccaccaggag actaggggca aacccggaac caaccactga atggatcgtc 1320
ggaaagacgg tcagaaactt caccgtcgac cgagatggcc tggaatacat atggggaaat 1380
catgagccag tgagggtcta tgcccaagag tcagcaccag gagaccctca cggatggcca 1440
cacgaaatag tacagcatta ctaccatcgc catcctgtgt acaccatctt agccgtcgca 1500
tcagctaccg tggcgatgat gattggcgta actgttgcag tgttatgtgc ctgtaaagcg 1560
cgccgtgagt gcctgacgcc atacgccctg gccccaaacg ccgtaatccc aacttcgctg 1620
gcactcttgt gctgcgttag gtcggccaat gct 1653
SEQ ID NO: 19 
gaaacgttca ccgagaccat gagttacttg tggtcgaaca gtcagccgtt cttctgggtc 60

cagttgtgca tacctttggc cgctttcatc gttctaatgc gctgctgctc ctgctgcctg 120

ccttttttag tggttgccgg cgcctacctg gcgaaggtag acgcc 165
SEQ ID NO: 20 
tacgaacatg cgaccactgt tccaaatgtg ccacagatac cgtataaggc acttgttgaa 60
agggcagggt atgccccgct caatttggag atcactgtca tgtcctcgga ggttttgcct 120
tccaccaacc aagagtacat tacctgcaaa ttcaccactg tggtcccctc cccaaaaatc 180
aaatgctgcg gctccttgga atgtcagccg gccgctcatg cagactatac ctgcaaggtc 240
ttcggagggg tctacccctt tatgtgggga ggagcgcaat gtttttgcga cagtgagaac 300
agccagatga gtgaggcgta cgtcgaattg tcagcagatt gcgcgtctga ccacgcgcag 360
gcgattaagg tgcacactgc cgcgatgaaa gtaggactgc gtatagtgta cgggaacact 420
accagtttcc tagatgtgta cgtgaacgga gtcacaccag gaacgtctaa agacttgaaa 480
gtcatagctg gaccaatttc agcatcattt acgccattcg atcataaggt cgttatccat 540
cgcggcctgg tgtacaacta tgacttcccg gaatatggag cgatgaaacc aggagcgttt 600
ggagacattc aagctacctc cttgactagc aaggatctca tcgccagcac agacattagg 660
 
ctactcaagc cttccgccaa gaatgtgcat gtcccgtaca cgcaggccgc atcaggattt 720
gagatgtgga aaaacaactc aggccgccca ttgcaggaaa ccgcaccttt cgggtgtaag 780
attgcagtaa atccgctccg agcggtggac tgttcatacg ggaacattcc catttctatt 840
gacatcccga acgctgcctt tatcaggaca tcagatgcac cactggtctc aacagtcaaa 900
tgtgaagtca gtgagtgcac ttattcagca gacttcgacg ggatggccac cctgcagtat 960
gtatccgacc gcgaaggtca atgccccgta cattcgcatt cgagcacagc aactctccaa 1020
gagtcgacag tacatgtcct ggagaaagga gcggtgacag tacactttag caccgcgagt 1080
ccacaggcga actttatcgt atcgctgtgt gggaagaaga caacatgcaa tgcagaatgt 1140
aaaccaccag ctgaccatat cgtgagcacc ccgcacaaaa atgaccaaga atttcaagcc 1200
gccatctcaa aaacatcatg gagttggctg tttgcccttt tcggcggcgc ctcgtcgcta 1260
ttaattatag gacttatgat ttttgcttgc agcatgatgc tgactagcac acgaagatga 1320
SEQ ID NO: 21 
caggtggcacttttcggggaaatgtgcgcggaacccctatttgtttatttttctaaatacattcaaatatgtatccg
ctcatgagacaataaccctgataaatgcttcaataatattgaaaaaggaagagtatgagtattcaacatttccgtgt
cgcccttattcccttttttgcggcattttgccttcctgtttttgctcacccagaaacgctggtgaaagtaaaagatg
ctgaagatcagttgggtgcacgagtgggttacatcgaactggatctcaacagcggtaagatccttgagagttttcgc
cccgaagaacgttttccaatgatgagcacttttaaagttctgctatgtggcgcggtattatcccgtattgacgccgg
gcaagagcaactcggtcgccgcatacactattctcagaatgacttggttgagtactcaccagtcacagaaaagcatc
ttacggatggcatgacagtaagagaattatgcagtgctgccataaccatgagtgataacactgcggccaacttactt
ctgacaacgatcggaggaccgaaggagctaaccgcttttttgcacaacatgggggatcatgtaactcgccttgatcg
ttgggaaccggagctgaatgaagccataccaaacgacgagcgtgacaccacgatgcctgtagcaatggcaacaacgt
tgcgcaaactattaactggcgaactacttactctagcttcccggcaacaattaatagactggatggaggcggataaa
gttgcaggaccacttctgcgctcggcccttccggctggctggtttattgctgataaatctggagccggtgagcgtgg
gtctcgcggtatcattgcagcactggggccagatggtaagccctcccgtatcgtagttatctacacgacggggagtc
aggcaactatggatgaacgaaatagacagatcgctgagataggtgcctcactgattaagcattggtaactgtcagac
caagtttactcatatatactttagattgatttaaaacttcatttttaatttaaaaggatctaggtgaagatcctttt
tgataatctcatgaccaaaatcccttaacgtgagttttcgttccactgagcgtcagaccccgtagaaaagatcaaag
gatcttcttgagatcctttttttctgcgcgtaatctgctgcttgcaaacaaaaaaaccaccgctaccagcggtggtt
tgtttgccggatcaagagctaccaactctttttccgaaggtaactggcttcagcagagcgcagataccaaatactgt
ccttctagtgtagccgtagttaggccaccacttcaagaactctgtagcaccgcctacatacctcgctctgctaatcc
tgttaccagtggctgctgccagtggcgataagtcgtgtcttaccgggttggactcaagacgatagttaccggataag
gcgcagcggtcgggctgaacggggggttcgtgcacacagcccagcttggagcgaacgacctacaccgaactgagata
cctacagcgtgagctatgagaaagcgccacgcttcccgaagggagaaaggcggacaggtatccggtaagcggcaggg
tcggaacaggagagcgcacgagggagcttccagggggaaacgcctggtatctttatagtcctgtcgggtttcgccac
 
ctctgacttgagcgtcgatttttgtgatgctcgtcaggggggcggagcctatggaaaaacgccagcaacgcggcctt
tttacggttcctggccttttgctggccttttgctcacatgttctttcctgcgttatcccctgattctgtggataacc
gtattaccgcctttgagtgagctgataccgctcgccgcagccgaacgaccgagcgcagcgagtcagtgagcgaggaa
gcggaagagcgcccaatacgcaaaccgcctctccccgcgcgttggccgattcattaatgcagctggcacgacaggtt
tcccgactggaaagcgggcagtgagcgcaacgcaattaatgtgagttagctcactcattaggcaccccaggctttac
actttatgcttccggctcgtatgttgtgtggaattgtgagcggataacaatttcacacaggaaacagctatgaccat
gattacgccaagcgcgcaattaaccctcactaaagggaacaaaagctggagctgcaagcttggccattgcatacgtt
gtatccatatcataatatgtacatttatattggctcatgtccaacattaccgccatgttgacattgattattgacta
gttattaatagtaatcaattacggggtcattagttcatagcccatatatggagttccgcgttacataacttacggta
aatggcccgcctggctgaccgcccaacgacccccgcccattgacgtcaataatgacgtatgttcccatagtaacgcc
aatagggactttccattgacgtcaatgggtggagtatttacggtaaactgcccacttggcagtacatcaagtgtatc
atatgccaagtacgccccctattgacgtcaatgacggtaaatggcccgcctggcattatgcccagtacatgacctta
tgggactttcctacttggcagtacatctacgtattagtcatcgctattaccatggtgatgcggttttggcagtacat
caatgggcgtggatagcggtttgactcacggggatttccaagtctccaccccattgacgtcaatgggagtttgtttt
ggcaccaaaatcaacgggactttccaaaatgtcgtaacaactccgccccattgacgcaaatgggcggtaggcgtgta
cggtgggaggtctatataagcagagctcgtttagtgaaccggggtctctctggttagaccagatctgagcctgggag
ctctctggctaactagggaacccactgcttaagcctcaataaagcttgccttgagtgcttcaagtagtgtgtgcccg
tctgttgtgtgactctggtaactagagatccctcagacccttttagtcagtgtggaaaatctctagcagtggcgccc
gaacagggacctgaaagcgaaagggaaaccagaggagctctctcgacgcaggactcggcttgctgaagcgcgcacgg
caagaggcgaggggcggcgactggtgagtacgccaaaaattttgactagcggaggctagaaggagagagatgggtgc
gagagcgtcagtattaagcgggggagaattagatcgcgatgggaaaaaattcggttaaggccagggggaaagaaaaa
atataaattaaaacatatagtatgggcaagcagggagctagaacgattcgcagttaatcctggcctgttagaaacat
cagaaggctgtagacaaatactgggacagctacaaccatcccttcagacaggatcagaagaacttagatcattatat
aatacagtagcaaccctctattgtgtgcatcaaaggatagagataaaagacaccaaggaagctttagacaagataga
ggaagagcaaaacaaaagtaagaccaccgcacagcaagcggccgctgatcttcagacctggaggaggagatatgagg
gacaattggagaagtgaattatataaatataaagtagtaaaaattgaaccattaggagtagcacccaccaaggcaaa
gagaagagtggtgcagagagaaaaaagagcagtgggaataggagctttgttccttgggttcttgggagcagcaggaa
gcactatgggcgcagcctcaatgacgctgacggtacaggccagacaattattgtctggtatagtgcagcagcagaac
aatttgctgagggctattgaggcgcaacagcatctgttgcaactcacagtctggggcatcaagcagctccaggcaag
aatcctggctgtggaaagatacctaaaggatcaacagctcctggggatttggggttgctctggaaaactcatttgca
ccactgctgtgccttggaatgctagttggagtaataaatctctggaacagattggaatcacacgacctggatggagt
gggacagagaaattaacaattacacaagcttaatacactccttaattgaagaatcgcaaaaccagcaagaaaagaat
gaacaagaattattggaattagataaatgggcaagtttgtggaattggtttaacataacaaattggctgtggtatat
aaaattattcataatgatagtaggaggcttggtaggtttaagaatagtttttgctgtactttctatagtgaatagag
ttaggcagggatattcaccattatcgtttcagacccacctcccaaccccgaggggacccgacaggcccgaaggaata
 
gaagaagaaggtggagagagagacagagacagatccattcgattagtgaacggatctcgacggtatcgataagctaa
ttcacaaatggcagtattcatccacaattttaaaagaaaaggggggattggggggtacagtgcaggggaaagaatag
tagacataatagcaacagacatacaaactaaagaattacaaaaacaaattacaaaaattcaaaattttcgggtttat
tacagggacagcagagatccagtttgggaattagcttgatcgattagtccaatttgttaaagacaggatatcagtgg
tccaggctctagttttgactcaacaatatcaccagctgaagcctatagagtacgagccatagatagaataaaagatt
ttatttagtctccagaaaaaggggggaatgaaagaccccacctgtaggtttggcaagctaggatcaaggttaggaac
agagagacagcagaatatgggccaaacaggatatctgtggtaagcagttcctgccccggctcagggccaagaacagt
tggaacagcagaatatgggccaaacaggatatctgtggtaagcagttcctgccccggctcagggccaagaacagatg
gtccccagatgcggtcccgccctcagcagtttctagagaaccatcagatgtttccagggtgccccaaggacctgaaa
tgaccctgtgccttatttgaactaaccaatcagttcgcttctcgcttctgttcgcgcgcttctgctccccgagctca
ataaaagagcccacaacccctcactcggcgcgatctagatctcgaatcgaattcgcccttGATCCACCGGTCGCCAC
CATGGTGAGCAAGGGCGAGGAGCTGTTCACCGGGGTGGTGCCCATCCTGGTCGAGCTGGACGGCGACGTAAACGGCC
ACAAGTTCAGCGTGTCCGGCGAGGGCGAGGGCGATGCCACCTACGGCAAGCTGACCCTGAAGTTCATCTGCACCACC
GGCAAGCTGCCCGTGCCCTGGCCCACCCTCGTGACCACCCTGACCTACGGCGTGCAGTGCTTCAGCCGCTACCCCGA
CCACATGAAGCAGCACGACTTCTTCAAGTCCGCCATGCCCGAAGGCTACGTCCAGGAGCGCACCATCTTCTTCAAGG
ACGACGGCAACTACAAGACCCGCGCCGAGGTGAAGTTCGAGGGCGACACCCTGGTGAACCGCATCGAGCTGAAGGGC
ATCGACTTCAAGGAGGACGGCAACATCCTGGGGCACAAGCTGGAGTACAACTACAACAGCCACAACGTCTATATCAT
GGCCGACAAGCAGAAGAACGGCATCAAGGTGAACTTCAAGATCCGCCACAACATCGAGGACGGCAGCGTGCAGCTCG
CCGACCACTACCAGCAGAACACCCCCATCGGCGACGGCCCCGTGCTGCTGCCCGACAACCACTACCTGAGCACCCAG
TCCGCCCTGAGCAAAGACCCCAACGAGAAGCGCGATCACATGGTCCTGCTGGAGTTCGTGACCGCCGCCGGGATCAC
TCTCGGCATGGACGAGCTGTACAAGTAAAGCGGCCTACCGGGTAGGGGAGGCGCTTTTCCCAAGGCAGTCTGGAGCA
TGCGCTTTAGCAGCCCCGCTGGCACTTGGCGCTACACAAGTGGCCTCTGGCCTCGCACACATTCCACATCCACCGGT
AGGCGCCAACCGGCTCCGTTCTTTGGTGGCCCCTTCGCGCCACCTTCTACTCCTCCCCTAGTCAGGAAGTTCCCCCC
CGCCCCGCAGCTCGCGTCGTGCAGGACGTGACAAATGGAAGTAGCACGTCTCACTAGTCTCGTGCAGATGGACAGCA
CCGCTGAGCAATGGAAGCGGGTAGGCCTTTGGGGCAGCGGCCAATAGCAGCTTTGCTCCTTCGCTTTCTGGGCTCAG
AGGCTGGGAAGGGGTGGGTCCGGGGGCGGGCTCAGGGGCGGGCTCAGGGGCGGGGCGGGCGCCCGAAGGTCCTCCGG
AGGCCCGGCATTCTCGCACGCTTCAAAAGCGCACGTCTGCCGCGCTGTTCTCCTCTTCCTCATCTCCGGGCCTTTCG
ACCATCTAATGGATTCATACCTGCTGATGTGGGGACTGCTCACGTTCATCATGGTGCCTGGCTGCCAGGCAGAGCTC
TGTGACGATGACCCGCCAGAGATCCCACACGCCACATTCAAAGCCATGGCCTACAAGGAAGGAACCATGTTGAACTG
TGAATGCAAGAGAGGTTTCCGCAGAATAAAAAGCGGGTCACTCTATATGCTCTGTACAGGAAACTCTAGCCACTCGT
CCTGGGACAACCAATGTCAATGCACAAGCTCTGCCACTCGGAACACAACGAAACAAGTGACACCTCAACCTGAAGAA
CAGAAAGAAAGGAAAACCACAGAAATGCAAAGTCCAATGCAGCCAGTGGACCAAGCGAGCCTTCCAGGTCACTGCAG
GGAACCTCCACCATGGGAAAATGAAGCCACAGAGAGAATTTATCATTTCGTGGTGGGGCAGATGGTTTATTATCAGT
GCGTCCAGGGATACAGGGCTCTACACAGAGGTCCTGCTGAGAGCGTCTGCAAAATGACCCACGGGAAGACAAGGTGG
ACCCAGCCCCAGCTCATATGCACAGGTGAAATGGAGACCAGTCAGTTTCCAGGTGAAGAGAAGCCTCAGGCAAGCCC
 
CGAAGGCCGTCCTGAGAGTGAGACTTCCTGCCTCGTCACAACAACAGATTTCAAATACAGACAGAAATGGCTGCAA
CCATGGAGACGTCCATATTTACAACAGAGTACCAGGTAGCAGTGGCCGGCTGTGTTTTCCTGCTGATCAGCGTCCTC
CTCCTGAGTGGGCTCACCTGGCAGCGGAGACAGAGGAAGAGTAGAAGAACAATCTGATGAgttaacctcgagggatc
cccgggtcgactgatcaaattcgagctcggtacctttaagaccaatgacttacaaggcagctgtagatcttagcc
actttttaaaagaaaaggggggactggaagggctaattcactcccaacgaagacaagatctgctttttgcttgtact
gggtctctctggttagaccagatctgagcctgggagctctctggctaactagggaacccactgcttaagcctcaata
aagcttgccttgagtgcttcaagtagtgtgtgcccgtctgttgtgtgactctggtaactagagatccctcagaccct
tttagtcagtgtggaaaatctctagcagtagtagttcatgtcatcttattattcagtatttataacttgcaaagaaa
tgaatatcagagagtgagaggaacttgtttattgcagcttataatggttacaaataaagcaatagcatcacaaattt
cacaaataaagcatttttttcactgcattctagttgtggtttgtccaaactcatcaatgtatcttatcatgtctggc
tctagctatcccgcccctaactccgcccatcccgcccctaactccgcccagttccgcccattctccgccccatggct
gactaattttttttatttatgcagaggccgaggccgcctcggcctctgagctattccagaagtagtgaggaggcttt
tttggaggcctaggcttttgcgtcgagacgtacccaattcgccctatagtgagtcgtattacgcgcgctcactggcc
gtcgttttacaacgtcgtgactgggaaaaccctggcgttacccaacttaatcgccttgcagcacatccccctttcgc
cagctggcgtaatagcgaagaggcccgcaccgatcgcccttcccaacagttgcgcagcctgaatggcgaatggcgcg
acgcgccctgtagcggcgcattaagcgcggcgggtgtggtggttacgcgcagcgtgaccgctacacttgccagcgcc
ctagcgcccgctcctttcgctttcttcccttcctttctcgccacgttcgccggctttccccgtcaagctctaaatcg
ggggctccctttagggttccgatttagtgctttacggcacctcgaccccaaaaaacttgattagggtgatggttcac
gtagtgggccatcgccctgatagacggtttttcgccctttgacgttggagtccacgttctttaatagtggactcttg
ttccaaactggaacaacactcaaccctatctcggtctattcttttgatttataagggattttgccgatttcggccta
ttggttaaaaaatgagctgatttaacaaaaatttaacgcgaattttaacaaaatattaacgtttacaatttcc-
9897
SEQ ID NO: 22 
caggtggcacttttcggggaaatgtgcgcggaacccctatttgtttatttttctaaatacattcaaatatgtatccg
ctcatgagacaataaccctgataaatgcttcaataatattgaaaaaggaagagtatgagtattcaacatttccgtgt
cgcccttattcccttttttgcggcattttgccttcctgtttttgctcacccagaaacgctggtgaaagtaaaagatg
ctgaagatcagttgggtgcacgagtgggttacatcgaactggatctcaacagcggtaagatccttgagagttttcgc
cccgaagaacgttttccaatgatgagcacttttaaagttctgctatgtggcgcggtattatcccgtattgacgccgg
gcaagagcaactcggtcgccgcatacactattctcagaatgacttggttgagtactcaccagtcacagaaaagcatc
ttacggatggcatgacagtaagagaattatgcagtgctgccataaccatgagtgataacactgcggccaacttactt
ctgacaacgatcggaggaccgaaggagctaaccgcttttttgcacaacatgggggatcatgtaactcgccttgatcg
ttgggaaccggagctgaatgaagccataccaaacgacgagcgtgacaccacgatgcctgtagcaatggcaacaacgt
tgcgcaaactattaactggcgaactacttactctagcttcccggcaacaattaatagactggatggaggcggataaa
gttgcaggaccacttctgcgctcggcccttccggctggctggtttattgctgataaatctggagccggtgagcgtgg
 
gtctcgcggtatcattgcagcactggggccagatggtaagccctcccgtatcgtagttatctacacgacggggagtc
aggcaactatggatgaacgaaatagacagatcgctgagataggtgcctcactgattaagcattggtaactgtcagac
caagtttactcatatatactttagattgatttaaaacttcatttttaatttaaaaggatctaggtgaagatcctttt
tgataatctcatgaccaaaatcccttaacgtgagttttcgttccactgagcgtcagaccccgtagaaaagatcaaag
gatcttcttgagatcctttttttctgcgcgtaatctgctgcttgcaaacaaaaaaaccaccgctaccagcggtggtt
tgtttgccggatcaagagctaccaactctttttccgaaggtaactggcttcagcagagcgcagataccaaatactgt
ccttctagtgtagccgtagttaggccaccacttcaagaactctgtagcaccgcctacatacctcgctctgctaatcc
tgttaccagtggctgctgccagtggcgataagtcgtgtcttaccgggttggactcaagacgatagttaccggataag
gcgcagcggtcgggctgaacggggggttcgtgcacacagcccagcttggagcgaacgacctacaccgaactgagata
cctacagcgtgagctatgagaaagcgccacgcttcccgaagggagaaaggcggacaggtatccggtaagcggcaggg
tcggaacaggagagcgcacgagggagcttccagggggaaacgcctggtatctttatagtcctgtcgggtttcgccac
ctctgacttgagcgtcgatttttgtgatgctcgtcaggggggcggagcctatggaaaaacgccagcaacgcggcctt
tttacggttcctggccttttgctggccttttgctcacatgttctttcctgcgttatcccctgattctgtggataacc
gtattaccgcctttgagtgagctgataccgctcgccgcagccgaacgaccgagcgcagcgagtcagtgagcgaggaa
gcggaagagcgcccaatacgcaaaccgcctctccccgcgcgttggccgattcattaatgcagctggcacgacaggtt
tcccgactggaaagcgggcagtgagcgcaacgcaattaatgtgagttagctcactcattaggcaccccaggctttac
actttatgcttccggctcgtatgttgtgtggaattgtgagcggataacaatttcacacaggaaacagctatgaccat
gattacgccaagcgcgcaattaaccctcactaaagggaacaaaagctggagctgcaagcttggccattgcatacgtt
gtatccatatcataatatgtacatttatattggctcatgtccaacattaccgccatgttgacattgattattgacta
gttattaatagtaatcaattacggggtcattagttcatagcccatatatggagttccgcgttacataacttacggta
aatggcccgcctggctgaccgcccaacgacccccgcccattgacgtcaataatgacgtatgttcccatagtaacgcc
aatagggactttccattgacgtcaatgggtggagtatttacggtaaactgcccacttggcagtacatcaagtgtatc
atatgccaagtacgccccctattgacgtcaatgacggtaaatggcccgcctggcattatgcccagtacatgacctta
tgggactttcctacttggcagtacatctacgtattagtcatcgctattaccatggtgatgcggttttggcagtacat
caatgggcgtggatagcggtttgactcacggggatttccaagtctccaccccattgacgtcaatgggagtttgtttt
ggcaccaaaatcaacgggactttccaaaatgtcgtaacaactccgccccattgacgcaaatgggcggtaggcgtgta
cggtgggaggtctatataagcagagctcgtttagtgaaccggggtctctctggttagaccagatctgagcctgggag
ctctctggctaactagggaacccactgcttaagcctcaataaagcttgccttgagtgcttcaagtagtgtgtgcccg
tctgttgtgtgactctggtaactagagatccctcagacccttttagtcagtgtggaaaatctctagcagtggcgccc
gaacagggacctgaaagcgaaagggaaaccagaggagctctctcgacgcaggactcggcttgctgaagcgcgcacgg
caagaggcgaggggcggcgactggtgagtacgccaaaaattttgactagcggaggctagaaggagagagatgggtgc
gagagcgtcagtattaagcgggggagaattagatcgcgatgggaaaaaattcggttaaggccagggggaaagaaaaa
atataaattaaaacatatagtatgggcaagcagggagctagaacgattcgcagttaatcctggcctgttagaaacat
cagaaggctgtagacaaatactgggacagctacaaccatcccttcagacaggatcagaagaacttagatcattatat
aatacagtagcaaccctctattgtgtgcatcaaaggatagagataaaagacaccaaggaagctttagacaagataga
 
ggaagagcaaaacaaaagtaagaccaccgcacagcaagcggccgctgatcttcagacctggaggaggagatatgagg
gacaattggagaagtgaattatataaatataaagtagtaaaaattgaaccattaggagtagcacccaccaaggcaaa
gagaagagtggtgcagagagaaaaaagagcagtgggaataggagctttgttccttgggttcttgggagcagcaggaa
gcactatgggcgcagcctcaatgacgctgacggtacaggccagacaattattgtctggtatagtgcagcagcagaac
aatttgctgagggctattgaggcgcaacagcatctgttgcaactcacagtctggggcatcaagcagctccaggcaag
aatcctggctgtggaaagatacctaaaggatcaacagctcctggggatttggggttgctctggaaaactcatttgca
ccactgctgtgccttggaatgctagttggagtaataaatctctggaacagattggaatcacacgacctggatggagt
gggacagagaaattaacaattacacaagcttaatacactccttaattgaagaatcgcaaaaccagcaagaaaagaat
gaacaagaattattggaattagataaatgggcaagtttgtggaattggtttaacataacaaattggctgtggtatat
aaaattattcataatgatagtaggaggcttggtaggtttaagaatagtttttgctgtactttctatagtgaatagag
ttaggcagggatattcaccattatcgtttcagacccacctcccaaccccgaggggacccgacaggcccgaaggaata
gaagaagaaggtggagagagagacagagacagatccattcgattagtgaacggatctcgacggtatcgataagctaa
ttcacaaatggcagtattcatccacaattttaaaagaaaaggggggattggggggtacagtgcaggggaaagaatag
tagacataatagcaacagacatacaaactaaagaattacaaaaacaaattacaaaaattcaaaattttcgggtttat
tacagggacagcagagatccagtttgggaattagcttgatcgattagtccaatttgttaaagacaggatatcagtgg
tccaggctctagttttgactcaacaatatcaccagctgaagcctatagagtacgagccatagatagaataaaagatt
ttatttagtctccagaaaaaggggggaatgaaagaccccacctgtaggtttggcaagctaggatcaaggttaggaac
agagagacagcagaatatgggccaaacaggatatctgtggtaagcagttcctgccccggctcagggccaagaacagt
tggaacagcagaatatgggccaaacaggatatctgtggtaagcagttcctgccccggctcagggccaagaacagatg
gtccccagatgcggtcccgccctcagcagtttctagagaaccatcagatgtttccagggtgccccaaggacctgaaa
tgaccctgtgccttatttgaactaaccaatcagttcgcttctcgcttctgttcgcgcgcttctgctccccgagctca
ataaaagagcccacaacccctcactcggcgcgatctagatctcgaatcgaattcgcccttgctactATGGCTTCTGG
AATCCTGGTTAATGTAAAGGAGGAGGTGACCTGCCCCATCTGCCTGGAACTCCTGACACAACCCCTGAGCCTGGACT
GCGGCCACAGCTTCTGCCAAGCATGCCTCACTGCAAACCACAAGAAGTCCATGCTAGACAAAGGAGAGAGTAGCTGC
CCTGTGTGCCGGATCAGTTACCAGCCTGAGAACATACGGCCTAATCGGCATGTAGCCAACATAGTGGAGAAGCTCAG
GGAGGTCAAGTTGAGCCCAGAGGGGCAGAAAGTTGATCATTGTGCACGCCATGGAGAGAAACTTCTACTCTTCTGTC
AGGAGGACGGGAAGGTCATTTGCTGGCTTTGTGAGCGGTCTCAGGAGCACCGTGGTCACCACACGTTCCTCACAGAG
GAGGTTGCCCGGGAGTACCAAGTGAAGCTCCAGGCAGCTCTGGAGATGCTGAGGCAGAAGCAGCAGGAAGCTGAAGA
GTTAGAAGCTGACATCAGAGAAGAGAAAGCTTCCTGGAAGACTCAAATACAGTATGACAAAACCAACGTCTTGGCAG
ATTTTGAGCAACTGAGAGACATCCTGGACTGGGAGGAGAGCAATGAGCTGCAAAACCTGGAGAAGGAGGAGGAAGAC
ATTCTGAAAAGCCTTACGAACTCTGAAACTGAGATGGTGCAGCAGACCCAGTCCCTGAGAGAGCTCATCTCAGATCT
GGAGCATCGGCTGCAGGGGTCAGTGATGGAGCTGCTTCAGGGTGTGGATGGCGTCATAAAAAGGACGGAGAACGTGA
CCTTGAAGAAGCCAGAAACTTTTCCAAAAAATCAAAGGAGAGTGTTTCGAGCTCCTGATCTGAAAGGAATGCTAGAA
GTGTTTAGAGAGCTGACAGATGTCCGACGCTACTGGGTTGATGTGACAGTGGCTCCAAACAACATTTCATGTGCTGT
CATTTCTGAAGATAAGAGACAAGTGAGCTCTCCGAAACCACAGATAATATATcaggcaccagggacattatttacgt
 
ttccgtcactcacgAATTTCAATTATTGTACTGGCATCCTGGGCTCTCAAAGTATCACATCAGGGAAACATTACTGG
GAGGTAGACGTGTCCAAGAAAACTGCTTGGATCCTGGGGGTATGTGCTGGCTTCCAACCTGATGCAATGTGTAATAT
TGAAAAAAATGAAAATTATCAACCTAAATACGGCTACTGGGTTATAGGGTTAGAGGAAGGAGTTAAATGTAGTGCTT
TCCAGGATAGTTCCTTCCATACTCCTTCTGTTCCTTTCATTGTGCCCCTCTCTGTGATTATTTGTCCTGATCGTGTT
GGAGTTTTCCTAGACTATGAGGCTTGCACTGTCTCATTCTTCAATATCACAAACCATGGATTTCTCATCTATAAGTT
TTCTCACTGTTCTTTTTCTCAGCCTGTATTTCCATATTTAAATCCTAGAAAATGTGGAGTCCCCATGACTCTGTGCT
CACCAAGCTCTGTTAACAGATCCAGTTTGGGGATCCAAGGTCGGGCAGGAAGAGGGCCTATTTCCCATGATTCCTTC
ATATTTGCATATACGATACAAGGCTGTTAGAGAGATAATTAGAATTAATTTGACTGTAAACACAAAGATATTAGTAC
AAAATACGTGACGTAGAAAGTAATAATTTCTTGGGTAGTTTGCAGTTTTAAAATTATGTTTTAAAATGGACTATCAT
ATGCTTACCGTAACTTGAAAGTATTTCGATTTCTTGGCTTTATATATCTTGTGGAAAGGACGAAACACCGAGCATGA
CTGACATCTACTTCAAGAGAGTAGATGTCAGTCATGCTCTTTTTTACGCGTAGAATCGAGACCGAGGAGAGGGTTAG
GGATAGGCTTACCTTCGAACCGCGGGCCCTCTAGACTCGAGCGGCCGCCACTGTGCTGGATATCTGCAGAATTGCCC
TTGACGCGTCAGTTTGGGGATCCAAGGTCGGGCAGGAAGAGGGCCTATTTCCCATGATTCCTTCATATTTGCATATA
CGATACAAGGCTGTTAGAGAGATAATTAGAATTAATTTGACTGTAAACACAAAGATATTAGTACAAAATACGTGACG
TAGAAAGTAATAATTTCTTGGGTAGTTTGCAGTTTTAAAATTATGTTTTAAAATGGACTATCATATGCTTACCGTAA
CTTGAAAGTATTTCGATTTCTTGGCTTTATATATCTTGTGGAAAGGACGAAACACCGTCGACCTTGCAATGATGTCG
TAATTTGCGTCTTACTCTGTTCTCAGCGACAGCCAGATCTGAGCCTGGGAGCTCTCTGGCTGTCAGTAAGCTGGTAC
AGAAGGTTGACGAAAATTCTTACTGAGCAAGAAATTTTTACGCGTGTTAACAAGGGCGAATTCTACCGGGTAGGGG
AGGCGCTTTTCCCAAGGCAGTCTGGAGCATGCGCTTTAGCAGCCCCGCTGGCACTTGGCGCTACACAAGTGGCCTCT
GGCCTCGCACACATTCCACATCCACCGGTAGGCGCCAACCGGCTCCGTTCTTTGGTGGCCCCTTCGCGCCACCTTCT
ACTCCTCCCCTAGTCAGGAAGTTCCCCCCCGCCCCGCAGCTCGCGTCGTGCAGGACGTGACAAATGGAAGTAGCACG
TCTCACTAGTCTCGTGCAGATGGACAGCACCGCTGAGCAATGGAAGCGGGTAGGCCTTTGGGGCAGCGGCCAATAGC
AGCTTTGCTCCTTCGCTTTCTGGGCTCAGAGGCTGGGAAGGGGTGGGTCCGGGGGCGGGCTCAGGGGCGGGCTCAGG
GGCGGGGCGGGCGCCCGAAGGTCCTCCGGAGGCCCGGCATTCTCGCACGCTTCAAAAGCGCACGTCTGCCGCGCTGT
TCTCCTCTTCCTCATCTCCGGGCCTTTCGACCATCTAATGGATTCATACCTGCTGATGTGGGGACTGCTCACGTTCA
TCATGGTGCCTGGCTGCCAGGCAGAGCTCTGTGACGATGACCCGCCAGAGATCCCACACGCCACATTCAAAGCCATG
GCCTACAAGGAAGGAACCATGTTGAACTGTGAATGCAAGAGAGGTTTCCGCAGAATAAAAAGCGGGTCACTCTATAT
GCTCTGTACAGGAAACTCTAGCCACTCGTCCTGGGACAACCAATGTCAATGCACAAGCTCTGCCACTCGGAACACAA
CGAAACAAGTGACACCTCAACCTGAAGAACAGAAAGAAAGGAAAACCACAGAAATGCAAAGTCCAATGCAGCCAGTG
GACCAAGCGAGCCTTCCAGGTCACTGCAGGGAACCTCCACCATGGGAAAATGAAGCCACAGAGAGAATTTATCATTT
CGTGGTGGGGCAGATGGTTTATTATCAGTGCGTCCAGGGATACAGGGCTCTACACAGAGGTCCTGCTGAGAGCGTCT
GCAAAATGACCCACGGGAAGACAAGGTGGACCCAGCCCCAGCTCATATGCACAGGTGAAATGGAGACCAGTCAGTTT
CCAGGTGAAGAGAAGCCTCAGGCAAGCCCCGAAGGCCGTCCTGAGAGTGAGACTTCCTGCCTCGTCACAACAACAGA
TTTTCAAATACAGACAGAAATGGCTGCAACCATGGAGACGTCCATATTTACAACAGAGTACCAGGTAGCAGTGGCCG
GCTGTGTTTTCCTGCTGATCAGCGTCCTCCTCCTGAGTGGGCTCACCTGGCAGCGGAGACAGAGGAAGAGTAGAAGA
 
ACAATCTGATGAgttaacctcgaggatcccccgggtcgactgatcaaattcgagctcggtacctttaagaccaat
gacttacaaggcagctgtagatcttagccactttttaaaagaaaaggggggactggaagggctaattcactcccaac
gaagacaagatctgctttttgcttgtactgggtctctctggttagaccagatctgagcctgggagctctctggctaa
ctagggaacccactgcttaagcctcaataaagcttgccttgagtgcttcaagtagtgtgtgcccgtctgttgtgtga
ctctggtaactagagatccctcagacccttttagtcagtgtggaaaatctctagcagtagtagttcatgtcatctta
ttattcagtatttataacttgcaaagaaatgaatatcagagagtgagaggaacttgtttattgcagcttataatggt
tacaaataaagcaatagcatcacaaatttcacaaataaagcatttttttcactgcattctagttgtggtttgtccaa
actcatcaatgtatcttatcatgtctggctctagctatcccgcccctaactccgcccatcccgcccctaactccgcc
cagttccgcccattctccgccccatggctgactaattttttttatttatgcagaggccgaggccgcctcggcctctg
agctattccagaagtagtgaggaggcttttttggaggcctaggcttttgcgtcgagacgtacccaattcgccctata
gtgagtcgtattacgcgcgctcactggccgtcgttttacaacgtcgtgactgggaaaaccctggcgttacccaactt
aatcgccttgcagcacatccccctttcgccagctggcgtaatagcgaagaggcccgcaccgatcgcccttcccaaca
gttgcgcagcctgaatggcgaatggcgacgcgccctgtagcggcgcattaagcgcggcgggtgtggtggttacgc
gcagcgtgaccgctacacttgccagcgccctagcgcccgctcctttcgctttcttcccttcctttctcgccacgttc
gccggctttccccgtcaagctctaaatcgggggctccctttagggttccgatttagtgctttacggcacctcgaccc
caaaaaacttgattagggtgatggttcacgtagtgggccatcgccctgatagacggtttttcgccctttgacgttgg
agtccacgttctttaatagtggactcttgttccaaactggaacaacactcaaccctatctcggtctattcttttgat
ttataagggattttgccgatttcggcctattggttaaaaaatgagctgatttaacaaaaatttaacgcgaattttaa
caaaatattaacgtttacaatttcc-9897
SEQ ID NO: 23
   gagcatgactgacatctacttcaagagagtagatgtcagtcatgctc
SEQ ID NO: 24 
Gly Ala Arg Gly Thr Arg Tyr Gln Thr Phe Val
1               5                   10
SEQ ID NO: 25 
Gln Ala Pro Gly Thr Leu Phe Thr Phe Pro Ser Leu Thr
1               5                   10
SEQ ID NO: 26 
gctactatgg cttctggaat cctggttaat gtaaaggagg aggtgacctg ccccatctgc 60
ctggaactcc tgacacaacc cctgagcctg gactgcggcc acagcttctg ccaagcatgc 120
ctcactgcaa accacaagaa gtccatgcta gacaaaggag agagtagctg ccctgtgtgc 180
 
cggatcagtt accagcctga gaacatacgg cctaatcggc atgtagccaa catagtggag 240
aagctcaggg aggtcaagtt gagcccagag gggcagaaag ttgatcattg tgcacgccat 300
ggagagaaac ttctactctt ctgtcaggag gacgggaagg tcatttgctg gctttgtgag 360
cggtctcagg agcaccgtgg tcaccacacg ttcctcacag aggaggttgc ccgggagtac 420
caagtgaagc tccaggcagc tctggagatg ctgaggcaga agcagcagga agctgaagag 480
ttagaagctg acatcagaga agagaaagct tcctggaaga ctcaaataca gtatgacaaa 540
accaacgtct tggcagattt tgagcaactg agagacatcc tggactggga ggagagcaat 600
gagctgcaaa acctggagaa ggaggaggaa gacattctga aaagccttac gaactctgaa 660
actgagatgg tgcagcagac ccagtccctg agagagctca tctcagatct ggagcatcgg 720
ctgcaggggt cagtgatgga gctgcttcag ggtgtggatg gcgtcataaa aaggacggag 780
aacgtgacct tgaagaagcc agaaactttt ccaaaaaatc aaaggagagt gtttcgagct 840
cctgatctga aaggaatgct agaagtgttt agagagctga cagatgtccg acgctactgg 900
gttgatgtga cagtggctcc aaacaacatt tcatgtgctg tcatttctga agataagaga 960
caagtgagct ctccgaaacc acagataata tatcaggcac cagggacatt atttacgttt 1020
ccgtcactca cgaatttcaa ttattgtact ggcatcctgg gctctcaaag tatcacatca 1080
gggaaacatt actgggaggt agacgtgtcc aagaaaactg cttggatcct gggggtatgt 1140
gctggcttcc aacctgatgc aatgtgtaat attgaaaaaa atgaaaatta tcaacctaaa 1200
tacggctact gggttatagg gttagaggaa ggagttaaat gtagtgcttt ccaggatagt 1260
tccttccata ctccttctgt tcctttcatt gtgcccctct ctgtgattat ttgtcctgat 1320
cgtgttggag ttttcctaga ctatgaggct tgcactgtct cattcttcaa tatcacaaac 1380
catggatttc tcatctataa gttttctcac tgttcttttt ctcagcctgt atttccatat 1440
ttaaatccta gaaaatgtgg agtccccatg actctgtgct caccaagctc tggcggctac 1500
ccatacgacg tcccagacta cgcttgagcc tga 1533
SEQ ID NO: 27 
agatccagtt tggggatcca aggtcgggca ggaagagggc ctatttccca tgattccttc 60
atatttgcat atacgataca aggctgttag agagataatt agaattaatt tgactgtaaa 120
cacaaagata ttagtacaaa atacgtgacg tagaaagtaa taatttcttg ggtagtttgc 180
agttttaaaa ttatgtttta aaatggacta tcatatgctt accgtaactt gaaagtattt 240
cgatttcttg gctttatata tcttgtggaa aggacgaaac accgtcgacc ttgcaatgat 300
gtcgtaattt gcgtcttact ctgttctcag cgacagccag atctgagcct gggagctctc 360
tggctgtcag taagctggta cagaaggttg acgaaaattc ttactgagca agaaattttt 420
t 421
SEQ ID NO: 28
   5'-CTGGGTTGATGTGACAGTGG-3'
SEQ ID NO: 29
   5'-CGTGAGTGACGGAAACGTAA-3'
SEQ ID NO: 30
   5'-TCCATTCCGAGGCCACAGCAAG-3'
SEQ ID NO: 31
   5'-TCAGCTCGTTCCTCCTCTGACG-3'
SEQ ID NO: 32
   5'-AAGACCCTGAGAAGGAAAAGCG-3'
SEQ ID NO: 33
   5'-GTGTTGGTAATGCCTGCTGTCC-3'
SEQ ID NO: 34
   5'-ACTACCACTCACCCGCAGAC-3'
SEQ ID NO: 35
   5'-GACGGGAAGCCAGCCTTAC-3'
SEQ ID NO: 36
   5'-CCCCAAGATCCTGAAACAGA-3'
SEQ ID NO: 37
   5'-CCGTTGCTGGACTGGATTAT-3'
SEQ ID NO: 38

5'-
ATGGATTCATACCTGCTGATGTGGGGACTGCTCACGTTCATCATGGTGCCTGGCTGCCAGGCAGAGCTCTGTGACGA
TGACCCGCCAGAGATCCCACACGCCACATTCAAAGCCATGGCCTACAAGGAAGGAACCATGTTGAACTGTGAATGCA
AGAGAGGTTTCacCAGcATAAAAAGCGGGTCACTCTATATGCTCTGTACAGGAAACTCTAGCCACTCGTCCTGGGAC
AACCAATGTCAATGCACAAGCTCTGCCACTCGGAACACAACGAAACAAGTGACACCTCAACCTGAAGAACAGAAAGA

AAGGAAAACCACAGAAATGCAAAGTCCAATGCAGCCAGTGGACCAAGCGAGCCTTCCAGGTCACTGCAGGGAACCTC
CACCATGGGAAAATGAAGCCACAGAGAGAATTTATCATTTCGTGGTGGGGCAGATGGTTTATTATCAGTGCGTCCAG
GGATACAGGGCTCTACACAGAGGTCCTGCTGAGAGCGTCTGCAAAATGACCCACGGGAAGACAAGGTGGACCgcGCC
CggGCTCATATGCACAGGTGAAATGGAGACCAGTCAGTTTCCAGGTGAAGAGAAGCCTCAGGCAAGCCCCGAAGGCC
GTCCTGAGAGTGAGACTTCCTGCCTCGTCACAACAACAGATTTCAAATACAGACAGAAATGGCTGCAACCATGGAG
ACGTCCATATTTACAACAGAGTACCAGGTAGCAGTGGCCGGCTGTGTTTTCCTGCTGATCAGCGTCCTCCTCCTGAG
TGGGCTC - 3'

### REFERENCES

1. Anderson, J.S., Javien, J., Nolta, J.A., et al. (2009). Preintegration HIV-1 inhibition by a combination lentiviral vector containing a chimeric TRIM5alpha protein, a CCR5 shRNA, and a TAR decoy. Mol. Ther. 17, 2103-2114.
2. Baldanti, F., Paolucci, S., Gulminetti, R., et al. (2010). Early emergence of raltegravir resistance mutations in patients receiving HAART salvage regimens. J. Med. Virol. 82, 116-122.
3. Barouch, D.H. (2008). Challenges in the development of an HIV-1 vaccine. Nature 455, 613-619.
4. Bauer, G., Valdez, P., Kearns, K., et al. (1997). Inhibition of human immunodeficiency virus-1 (HIV-1) replication after transduction of granulocyte colony-stimulating factor-mobilized CD34+ cells from HIV-1-infected donors using retroviral vectors containing anti-HIV-1 genes. Blood 89, 2259-2267.
5. DiGiusto, D., Krishnan, A., Li, L., et al. (2010). RNA-based gene therapy for HIV with lentiviral vector-modified CD34(+) cells in patients undergoing transplantation for AIDS-related lymphoma. Sci. Transl. Med. 36, 36ra43.
6. Domingo, P., Estrada, V., Lopez-Aldeguer, J., et al. (2012). Fat redistribution syndromes associated with HIV-1 infection and combination antiretroviral therapy. AIDS Rev. 14, 112-123.
7. Edgeworth, R.L., San, J.H., Rosenweig, J.A., et al. (2002). Vaccine development against HIV-1: current perspectives and future directions. Immunol. Res. 25, 53-74.
8. Gazzola, L., Tincati, C., Bellistri, G.M., et al. (2009). The absence of CD4+ T cell count recovery despite receipt of virologically suppressive highly active antiretroviral therapy: clinical risk, immunological gaps, and therapeutic options. Clin. Infect. Dis. 48, 328-337.
9. Graham SM, Jalalian-Lechak Z, Shafi J, et al. (2012). Antiretroviral treatment interruptions predict female genital shedding of genotypically resistant HIV-1 RNA. J. Acquir. Immune. Defic. Syndr. [Epub ahead of print].
10. Grant, A.J., Roessler, E., Ju, G., et al. (1992). The interleukin 2 receptor (IL-2R) the IL-2R alpha subunit alters the function of the IL-2R beta subunit to enhance IL-2 binding and signaling by mechanisms that do not require binding of IL-2 to IL-2R alpha subunit. Proc. Natl. Acad. Sci. USA 89, 2165-2169.
11. Hutter, G., Nowak, D., Mossner, M., et al. (2009). Long-term control of HIV by CCR5 Delta32/Delta32 stem-cell transplantation. N. Engl. J. Med. 360, 692-698.
12. Johnson, V.A., Brun-Vezinet, F., Clotet, B., et al. (2010). Update of the drug resistance mutations in HIV-1: December 2010. Top. HIV Med. 18, 156-63.
13. Kalmar EM, Sanabani SS, Charlys da Costa A, et al. (2012). Evaluation of HIV-1 resistance to antiretroviral drugs among 150 patients after six months of therapeutic interruption. Int. J. STD. AIDS 23, 120-125.
14. Kohn, D.B., Bauer, G., Rice, C.R., et al. (1999). A clinical trial of retroviral-mediated transfer of a rev-responsive element decoy gene into CD34(+) cells from the bone marrow of human immunodeficiency virus-1-infected children. Blood 94, 368-371.
15. Kuritzkes, D.R. (2011). Drug Resistance in HIV-1. Curr. Opin. Virol. 1, 582-589.
16. Kuziel, W.A., and Greene, W.C. (1990). Interleukin-2 and IL-2 receptor: new insights intostructure and function. J. Invest. Dermatol. 94, 27S-32S.
17. Lewden, C., Chene, G., Morlat, P., et al. (2007). HIV-infected adults with a CD4 cell count greater than 500 cells/mm3 on long-term combination antiretroviral therapy reach same mortality rates as the general population. J. Acquir. Immune. Defic. Syndr. 46, 72-77.
18. Macias, J., Neukam, K., Mallolas, J., et al. (2012). Liver toxicity of initial antiretroviral drug regimens including two nucleoside analogs plus one non-nucleoside analog or one ritonavir-boosted protease inhibitor in HIV/HCV-coinfected patients. HIV Clin. Trials 13, 61-69.
19. Mehandru, S., Poles, M.A., Tenner-Racz, K., et al. (2006). Lack of mucosal immune reconstitution during prolonged treatment of acute and early HIV-1 infection. PLoS Med. 3, e484.
20. Minami, Y., Kono, T., Miyazaki, T., et al. (1993). The IL-2 receptor complex: Its structure, function, and target genes. Annu. Rev. Immunol. 11, 245-267.
21. Mitsuyasu, R.T., Merigan, T.C., Carr, A., et al. (2009). Phase 2 gene therapy trial of an anti-HIV ribozyme in autologous CD34+ cells. Nat. Med. 15, 285-292.
22. Podsakoff, G.M., Engel, B.C., Carbonaro, D.A., et al. (2005). Selective survival of peripheral blood lymphocytes in children with HIV-1 following delivery of an anti-HIV gene to bone marrow CD34(+) cells. Mol. Ther. 12, 77-86.
23. Shimizu, S., Hong, P., Arumugam, B., et al. (2010). A highly efficient short hairpin RNA potently down-regulates CCR5 expression in systemic lymphoid organs in the hu-BLT mouse model. Blood 115, 1534-1544.
24. Strayer, D.S., Akkina, R., Bunnel, B.A., et al. (2005). Current Status of Gene Therapy Strategies to Treat HIV/AIDS. Mol. Ther. 11,823-841.
25. ter Brake, O., Legrand, N., von Eije, K.J., et al. (2009). Evaluation of safety and efficacy of RNAi against HIV-1 in the human immune system (Rag-2(-/-)gammac(-/-)) mouse model. Gene Ther. 16, 148-153.
26. Tilton, J.C., Wilen, C.B., Didigu, C.A., et al. (2010). A maraviroc resistant HIV-1 with narrow cross-resistance to other CCR5 antagonists depends on both N-terminal and extracellular loop domains of drug-bound CCR5. J. Virol. 84, 10863-10876.
28. Trobridge, G.D., Wu, R.A., Beard, B.C., et al. (2009). Protection of stem cell-derived lymphocytes in a primate AIDS gene therapy model after in vivo selection. PLoS One 4, e7693.
27. Walker, J.E., Chen, R.X., McGee, J., et al. (2012). Generation of an HIV-1-resistant immune system with CD34(+) hematopoietic stem cells transduced with a triple-combination anti-HIV lentiviral vector. J. Virol. 86: 5719-5729.

### Sequence Listing

SEQ ID NO: 1 
caggtggcacttttcggggaaatgtgcgcggaacccctatttgtttatttttctaaatacattcaaatatgtatccg
ctcatgagacaataaccctgataaatgcttcaataatattgaaaaaggaagagtatgagtattcaacatttccgtgt
cgcccttattcccttttttgcggcattttgccttcctgtttttgctcacccagaaacgctggtgaaagtaaaagatg
ctgaagatcagttgggtgcacgagtgggttacatcgaactggatctcaacagcggtaagatccttgagagttttcgc
cccgaagaacgttttccaatgatgagcacttttaaagttctgctatgtggcgcggtattatcccgtattgacgccgg
gcaagagcaactcggtcgccgcatacactattctcagaatgacttggttgagtactcaccagtcacagaaaagcatc
ttacggatggcatgacagtaagagaattatgcagtgctgccataaccatgagtgataacactgcggccaacttactt
ctgacaacgatcggaggaccgaaggagctaaccgcttttttgcacaacatgggggatcatgtaactcgccttgatcg
ttgggaaccggagctgaatgaagccataccaaacgacgagcgtgacaccacgatgcctgtagcaatggcaacaacgt
tgcgcaaactattaactggcgaactacttactctagcttcccggcaacaattaatagactggatggaggcggataaa
gttgcaggaccacttctgcgctcggcccttccggctggctggtttattgctgataaatctggagccggtgagcgtgg
gtctcgcggtatcattgcagcactggggccagatggtaagccctcccgtatcgtagttatctacacgacggggagtc
aggcaactatggatgaacgaaatagacagatcgctgagataggtgcctcactgattaagcattggtaactgtcagac
caagtttactcatatatactttagattgatttaaaacttcatttttaatttaaaaggatctaggtgaagatcctttt
tgataatctcatgaccaaaatcccttaacgtgagttttcgttccactgagcgtcagaccccgtagaaaagatcaaag
gatcttcttgagatcctttttttctgcgcgtaatctgctgcttgcaaacaaaaaaaccaccgctaccagcggtggtt
tgtttgccggatcaagagctaccaactctttttccgaaggtaactggcttcagcagagcgcagataccaaatactgt
ccttctagtgtagccgtagttaggccaccacttcaagaactctgtagcaccgcctacatacctcgctctgctaatcc
tgttaccagtggctgctgccagtggcgataagtcgtgtcttaccgggttggactcaagacgatagttaccggataag
gcgcagcggtcgggctgaacggggggttcgtgcacacagcccagcttggagcgaacgacctacaccgaactgagata
cctacagcgtgagctatgagaaagcgccacgcttcccgaagggagaaaggcggacaggtatccggtaagcggcaggg
tcggaacaggagagcgcacgagggagcttccagggggaaacgcctggtatctttatagtcctgtcgggtttcgccac
ctctgacttgagcgtcgatttttgtgatgctcgtcaggggggcggagcctatggaaaaacgccagcaacgcggcctt
tttacggttcctggccttttgctggccttttgctcacatgttctttcctgcgttatcccctgattctgtggataacc
gtattaccgcctttgagtgagctgataccgctcgccgcagccgaacgaccgagcgcagcgagtcagtgagcgaggaa
gcggaagagcgcccaatacgcaaaccgcctctccccgcgcgttggccgattcattaatgcagctggcacgacaggtt
tcccgactggaaagcgggcagtgagcgcaacgcaattaatgtgagttagctcactcattaggcaccccaggctttac
actttatgcttccggctcgtatgttgtgtggaattgtgagcggataacaatttcacacaggaaacagctatgaccat
gattacgccaagcgcgcaattaaccctcactaaagggaacaaaagctggagctgcaagcttggccattgcatacgtt
gtatccatatcataatatgtacatttatattggctcatgtccaacattaccgccatgttgacattgattattgacta
gttattaatagtaatcaattacggggtcattagttcatagcccatatatggagttccgcgttacataacttacggta
aatggcccgcctggctgaccgcccaacgacccccgcccattgacgtcaataatgacgtatgttcccatagtaacgcc
aatagggactttccattgacgtcaatgggtggagtatttacggtaaactgcccacttggcagtacatcaagtgtatc
 
atatgccaagtacgccccctattgacgtcaatgacggtaaatggcccgcctggcattatgcccagtacatgacctta
tgggactttcctacttggcagtacatctacgtattagtcatcgctattaccatggtgatgcggttttggcagtacat
caatgggcgtggatagcggtttgactcacggggatttccaagtctccaccccattgacgtcaatgggagtttgtttt
ggcaccaaaatcaacgggactttccaaaatgtcgtaacaactccgccccattgacgcaaatgggcggtaggcgtgta
cggtgggaggtctatataagcagagctcgtttagtgaaccggggtctctctggttagaccagatctgagcctgggag
ctctctggctaactagggaacccactgcttaagcctcaataaagcttgccttgagtgcttcaagtagtgtgtgcccg
tctgttgtgtgactctggtaactagagatccctcagacccttttagtcagtgtggaaaatctctagcagtggcgccc
gaacagggacctgaaagcgaaagggaaaccagaggagctctctcgacgcaggactcggcttgctgaagcgcgcacgg
caagaggcgaggggcggcgactggtgagtacgccaaaaattttgactagcggaggctagaaggagagagatgggtgc
gagagcgtcagtattaagcgggggagaattagatcgcgatgggaaaaaattcggttaaggccagggggaaagaaaaa
atataaattaaaacatatagtatgggcaagcagggagctagaacgattcgcagttaatcctggcctgttagaaacat
cagaaggctgtagacaaatactgggacagctacaaccatcccttcagacaggatcagaagaacttagatcattatat
aatacagtagcaaccctctattgtgtgcatcaaaggatagagataaaagacaccaaggaagctttagacaagataga
ggaagagcaaaacaaaagtaagaccaccgcacagcaagcggccgctgatcttcagacctggaggaggagatatgagg
gacaattggagaagtgaattatataaatataaagtagtaaaaattgaaccattaggagtagcacccaccaaggcaaa
gagaagagtggtgcagagagaaaaaagagcagtgggaataggagctttgttccttgggttcttgggagcagcaggaa
gcactatgggcgcagcctcaatgacgctgacggtacaggccagacaattattgtctggtatagtgcagcagcagaac
aatttgctgagggctattgaggcgcaacagcatctgttgcaactcacagtctggggcatcaagcagctccaggcaag
aatcctggctgtggaaagatacctaaaggatcaacagctcctggggatttggggttgctctggaaaactcatttgca
ccactgctgtgccttggaatgctagttggagtaataaatctctggaacagattggaatcacacgacctggatggagt
gggacagagaaattaacaattacacaagcttaatacactccttaattgaagaatcgcaaaaccagcaagaaaagaat
gaacaagaattattggaattagataaatgggcaagtttgtggaattggtttaacataacaaattggctgtggtatat
aaaattattcataatgatagtaggaggcttggtaggtttaagaatagtttttgctgtactttctatagtgaatagag
ttaggcagggatattcaccattatcgtttcagacccacctcccaaccccgaggggacccgacaggcccgaaggaata
gaagaagaaggtggagagagagacagagacagatccattcgattagtgaacggatctcgacggtatcgataagctaa
ttcacaaatggcagtattcatccacaattttaaaagaaaaggggggattggggggtacagtgcaggggaaagaatag
tagacataatagcaacagacatacaaactaaagaattacaaaaacaaattacaaaaattcaaaattttcgggtttat
tacagggacagcagagatccagtttgggaattagcttgatcgattagtccaatttgttaaagacaggatatcagtgg
tccaggctctagttttgactcaacaatatcaccagctgaagcctatagagtacgagccatagatagaataaaagatt
ttatttagtctccagaaaaaggggggaatgaaagaccccacctgtaggtttggcaagctaggatcaaggttaggaac
agagagacagcagaatatgggccaaacaggatatctgtggtaagcagttcctgccccggctcagggccaagaacagt
tggaacagcagaatatgggccaaacaggatatctgtggtaagcagttcctgccccggctcagggccaagaacagatg
gtccccagatgcggtcccgccctcagcagtttctagagaaccatcagatgtttccagggtgccccaaggacctgaaa
tgaccctgtgccttatttgaactaaccaatcagttcgcttctcgcttctgttcgcgcgcttctgctccccgagctca
ataaaagagcccacaacccctcactcggcgcgatctagatctcgaatcgaattcgcccttgctactATGGCTTCTGG
AATCCTGGTTAATGTAAAGGAGGAGGTGACCTGCCCCATCTGCCTGGAACTCCTGACACAACCCCTGAGCCTGGACT
 
GCGGCCACAGCTTCTGCCAAGCATGCCTCACTGCAAACCACAAGAAGTCCATGCTAGACAAAGGAGAGAGTAGCTGC
CCTGTGTGCCGGATCAGTTACCAGCCTGAGAACATACGGCCTAATCGGCATGTAGCCAACATAGTGGAGAAGCTCAG
GGAGGTCAAGTTGAGCCCAGAGGGGCAGAAAGTTGATCATTGTGCACGCCATGGAGAGAAACTTCTACTCTTCTGTC
AGGAGGACGGGAAGGTCATTTGCTGGCTTTGTGAGCGGTCTCAGGAGCACCGTGGTCACCACACGTTCCTCACAGAG
GAGGTTGCCCGGGAGTACCAAGTGAAGCTCCAGGCAGCTCTGGAGATGCTGAGGCAGAAGCAGCAGGAAGCTGAAGA
GTTAGAAGCTGACATCAGAGAAGAGAAAGCTTCCTGGAAGACTCAAATACAGTATGACAAAACCAACGTCTTGGCAG
ATTTTGAGCAACTGAGAGACATCCTGGACTGGGAGGAGAGCAATGAGCTGCAAAACCTGGAGAAGGAGGAGGAAGAC
ATTCTGAAAAGCCTTACGAACTCTGAAACTGAGATGGTGCAGCAGACCCAGTCCCTGAGAGAGCTCATCTCAGATCT
GGAGCATCGGCTGCAGGGGTCAGTGATGGAGCTGCTTCAGGGTGTGGATGGCGTCATAAAAAGGACGGAGAACGTGA
CCTTGAAGAAGCCAGAAACTTTTCCAAAAAATCAAAGGAGAGTGTTTCGAGCTCCTGATCTGAAAGGAATGCTAGAA
GTGTTTAGAGAGCTGACAGATGTCCGACGCTACTGGGTTGATGTGACAGTGGCTCCAAACAACATTTCATGTGCTGT
CATTTCTGAAGATAAGAGACAAGTGAGCTCTCCGAAACCACAGATAATATATcaggcaccagggacattatttacgt
ttccgtcactcacgAATTTCAATTATTGTACTGGCATCCTGGGCTCTCAAAGTATCACATCAGGGAAACATTACTGG
GAGGTAGACGTGTCCAAGAAAACTGCTTGGATCCTGGGGGTATGTGCTGGCTTCCAACCTGATGCAATGTGTAATAT
TGAAAAAAATGAAAATTATCAACCTAAATACGGCTACTGGGTTATAGGGTTAGAGGAAGGAGTTAAATGTAGTGCTT
TCCAGGATAGTTCCTTCCATACTCCTTCTGTTCCTTTCATTGTGCCCCTCTCTGTGATTATTTGTCCTGATCGTGTT
GGAGTTTTCCTAGACTATGAGGCTTGCACTGTCTCATTCTTCAATATCACAAACCATGGATTTCTCATCTATAAGTT
TTCTCACTGTTCTTTTTCTCAGCCTGTATTTCCATATTTAAATCCTAGAAAATGTGGAGTCCCCATGACTCTGTGCT
CACCAAGCTCTggaagcggagctactaacttcagcctgctgaagcaggctggagacgtggaggagaaccctggacct
ATGGATTCATACCTGCTGATGTGGGGACTGCTCACGTTCATCATGGTGCCTGGCTGCCAGGCAGAGCTCTGTGACGA
TGACCCGCCAGAGATCCCACACGCCACATTCAAAGCCATGGCCTACAAGGAAGGAACCATGTTGAACTGTGAATGCA
AGAGAGGTTTCCGCAGAATAAAAAGCGGGTCACTCTATATGCTCTGTACAGGAAACTCTAGCCACTCGTCCTGGGAC
AACCAATGTCAATGCACAAGCTCTGCCACTCGGAACACAACGAAACAAGTGACACCTCAACCTGAAGAACAGAAAGA
AAGGAAAACCACAGAAATGCAAAGTCCAATGCAGCCAGTGGACCAAGCGAGCCTTCCAGGTCACTGCAGGGAACCTC
CACCATGGGAAAATGAAGCCACAGAGAGAATTTATCATTTCGTGGTGGGGCAGATGGTTTATTATCAGTGCGTCCAG
GGATACAGGGCTCTACACAGAGGTCCTGCTGAGAGCGTCTGCAAAATGACCCACGGGAAGACAAGGTGGACCCAGCC
CCAGCTCATATGCACAGGTGAAATGGAGACCAGTCAGTTTCCAGGTGAAGAGAAGCCTCAGGCAAGCCCCGAAGGCC
GTCCTGAGAGTGAGACTTCCTGCCTCGTCACAACAACAGATTTTCAAATACAGACAGAAATGGCTGCAACCATGGAG
ACGTCCATATTTACAACAGAGTACCAGGTAGCAGTGGCCGGCTGTGTTTTCCTGCTGATCAGCGTCCTCCTCCTGAG
TGGGCTCACCTGGCAGCGGAGACAGAGGAAGAGTAGAAGAACAATCTGATGAGTTAACAGATCCAGTTTGGGGATCC
AAGGTCGGGCAGGAAGAGGGCCTATTTCCCATGATTCCTTCATATTTGCATATACGATACAAGGCTGTTAGAGAGAT
AATTAGAATTAATTTGACTGTAAACACAAAGATATTAGTACAAAATACGTGACGTAGAAAGTAATAATTTCTTGGGT
AGTTTGCAGTTTTAAAATTATGTTTTAAAATGGACTATCATATGCTTACCGTAACTTGAAAGTATTTCGATTTCTTG
GCTTTATATATCTTGTGGAAAGGACGAAACACCGAGCATGACTGACATCTACTTCAAGAGAGTAGATGTCAGTCATG
CTCTTTTTTACGCGTAGAATCGAGACCGAGGAGAGGGTTAGGGATAGGCTTACCTTCGAACCGCGGGCCCTCTAGAC
TCGAGCGGCCGCCACTGTGCTGGATATCTGCAGAATTGCCCTTGACGCGTCAGTTTGGGGATCCAAGGTCGGGCAGG
 
AAGAGGGCCTATTTCCCATGATTCCTTCATATTTGCATATACGATACAAGGCTGTTAGAGAGATAATTAGAATTAAT
TTGACTGTAAACACAAAGATATTAGTACAAAATACGTGACGTAGAAAGTAATAATTTCTTGGGTAGTTTGCAGTTTT
AAAATTATGTTTTAAAATGGACTATCATATGCTTACCGTAACTTGAAAGTATTTCGATTTCTTGGCTTTATATATCT
TGTGGAAAGGACGAAACACCGTCGACCTTGCAATGATGTCGTAATTTGCGTCTTACTCTGTTCTCAGCGACAGCCAG
ATCTGAGCCTGGGAGCTCTCTGGCTGTCAGTAAGCTGGTACAGAAGGTTGACGAAAATTCTTACTGAGCAAGAAATT
TTTTACGCGTGTTAACAAGGGCGAATTCgttaacctcgaggatcccccgggtcgactgatcaaattcgagctcgg
tacctttaagaccaatgacttacaaggcagctgtagatcttagccactttttaaaagaaaaggggggactggaaggg
ctaattcactcccaacgaagacaagatctgctttttgcttgtactgggtctctctggttagaccagatctgagcctg
ggagctctctggctaactagggaacccactgcttaagcctcaataaagcttgccttgagtgcttcaagtagtgtgtg
cccgtctgttgtgtgactctggtaactagagatccctcagacccttttagtcagtgtggaaaatctctagcagtagt
agttcatgtcatcttattattcagtatttataacttgcaaagaaatgaatatcagagagtgagaggaacttgtttat
tgcagcttataatggttacaaataaagcaatagcatcacaaatttcacaaataaagcatttttttcactgcattcta
gttgtggtttgtccaaactcatcaatgtatcttatcatgtctggctctagctatcccgcccctaactccgcccatcc
cgcccctaactccgcccagttccgcccattctccgccccatggctgactaattttttttatttatgcagaggccgag
gccgcctcggcctctgagctattccagaagtagtgaggaggcttttttggaggcctaggcttttgcgtcgagacgta
cccaattcgccctatagtgagtcgtattacgcgcgctcactggccgtcgttttacaacgtcgtgactgggaaaaccc
tggcgttacccaacttaatcgccttgcagcacatccccctttcgccagctggcgtaatagcgaagaggcccgcaccg
atcgcccttcccaacagttgcgcagcctgaatggcgaatggcgacgcgccctgtagcggcgcattaagcgcggcg
ggtgtggtggttacgcgcagcgtgaccgctacacttgccagcgccctagcgcccgctcctttcgctttcttcccttc
ctttctcgccacgttcgccggctttccccgtcaagctctaaatcgggggctccctttagggttccgatttagtgctt
tacggcacctcgaccccaaaaaacttgattagggtgatggttcacgtagtgggccatcgccctgatagacggtttt
cgccctttgacgttggagtccacgttctttaatagtggactcttgttccaaactggaacaacactcaaccctatctc
ggtctattcttttgatttataagggattttgccgatttcggcctattggttaaaaaatgagctgatttaacaaaaat
ttaacgcgaattttaacaaaatattaacgtttacaatttcc-9897
SEQ ID NO: 2 
caggtggcacttttcggggaaatgtgcgcggaacccctatttgtttatttttctaaatacattcaaatatgtatccg
ctcatgagacaataaccctgataaatgcttcaataatattgaaaaaggaagagtatgagtattcaacatttccgtgt
cgcccttattcccttttttgcggcattttgccttcctgtttttgctcacccagaaacgctggtgaaagtaaaagatg
ctgaagatcagttgggtgcacgagtgggttacatcgaactggatctcaacagcggtaagatccttgagagttttcgc
cccgaagaacgttttccaatgatgagcacttttaaagttctgctatgtggcgcggtattatcccgtattgacgccgg
gcaagagcaactcggtcgccgcatacactattctcagaatgacttggttgagtactcaccagtcacagaaaagcatc
ttacggatggcatgacagtaagagaattatgcagtgctgccataaccatgagtgataacactgcggccaacttactt
ctgacaacgatcggaggaccgaaggagctaaccgcttttttgcacaacatgggggatcatgtaactcgccttgatcg
ttgggaaccggagctgaatgaagccataccaaacgacgagcgtgacaccacgatgcctgtagcaatggcaacaacgt
tgcgcaaactattaactggcgaactacttactctagcttcccggcaacaattaatagactggatggaggcggataaa
 
gttgcaggaccacttctgcgctcggcccttccggctggctggtttattgctgataaatctggagccggtgagcgtgg
gtctcgcggtatcattgcagcactggggccagatggtaagccctcccgtatcgtagttatctacacgacggggagtc
aggcaactatggatgaacgaaatagacagatcgctgagataggtgcctcactgattaagcattggtaactgtcagac
caagtttactcatatatactttagattgatttaaaacttcatttttaatttaaaaggatctaggtgaagatcctttt
tgataatctcatgaccaaaatcccttaacgtgagttttcgttccactgagcgtcagaccccgtagaaaagatcaaag
gatcttcttgagatcctttttttctgcgcgtaatctgctgcttgcaaacaaaaaaaccaccgctaccagcggtggtt
tgtttgccggatcaagagctaccaactctttttccgaaggtaactggcttcagcagagcgcagataccaaatactgt
ccttctagtgtagccgtagttaggccaccacttcaagaactctgtagcaccgcctacatacctcgctctgctaatcc
tgttaccagtggctgctgccagtggcgataagtcgtgtcttaccgggttggactcaagacgatagttaccggataag
gcgcagcggtcgggctgaacggggggttcgtgcacacagcccagcttggagcgaacgacctacaccgaactgagata
cctacagcgtgagctatgagaaagcgccacgcttcccgaagggagaaaggcggacaggtatccggtaagcggcaggg
tcggaacaggagagcgcacgagggagcttccagggggaaacgcctggtatctttatagtcctgtcgggtttcgccac
ctctgacttgagcgtcgatttttgtgatgctcgtcaggggggcggagcctatggaaaaacgccagcaacgcggcctt
tttacggttcctggccttttgctggccttttgctcacatgttctttcctgcgttatcccctgattctgtggataacc
gtattaccgcctttgagtgagctgataccgctcgccgcagccgaacgaccgagcgcagcgagtcagtgagcgaggaa
gcggaagagcgcccaatacgcaaaccgcctctccccgcgcgttggccgattcattaatgcagctggcacgacaggtt
tcccgactggaaagcgggcagtgagcgcaacgcaattaatgtgagttagctcactcattaggcaccccaggctttac
actttatgcttccggctcgtatgttgtgtggaattgtgagcggataacaatttcacacaggaaacagctatgaccat
gattacgccaagcgcgcaattaaccctcactaaagggaacaaaagctggagctgcaagcttggccattgcatacgtt
gtatccatatcataatatgtacatttatattggctcatgtccaacattaccgccatgttgacattgattattgacta
gttattaatagtaatcaattacggggtcattagttcatagcccatatatggagttccgcgttacataacttacggta
aatggcccgcctggctgaccgcccaacgacccccgcccattgacgtcaataatgacgtatgttcccatagtaacgcc
aatagggactttccattgacgtcaatgggtggagtatttacggtaaactgcccacttggcagtacatcaagtgtatc
atatgccaagtacgccccctattgacgtcaatgacggtaaatggcccgcctggcattatgcccagtacatgacctta
tgggactttcctacttggcagtacatctacgtattagtcatcgctattaccatggtgatgcggttttggcagtacat
caatgggcgtggatagcggtttgactcacggggatttccaagtctccaccccattgacgtcaatgggagtttgtttt
ggcaccaaaatcaacgggactttccaaaatgtcgtaacaactccgccccattgacgcaaatgggcggtaggcgtgta
cggtgggaggtctatataagcagagctcgtttagtgaaccggggtctctctggttagaccagatctgagcctgggag
ctctctggctaactagggaacccactgcttaagcctcaataaagcttgccttgagtgcttcaagtagtgtgtgcccg
tctgttgtgtgactctggtaactagagatccctcagacccttttagtcagtgtggaaaatctctagcagtggcgccc
gaacagggacctgaaagcgaaagggaaaccagaggagctctctcgacgcaggactcggcttgctgaagcgcgcacgg
caagaggcgaggggcggcgactggtgagtacgccaaaaattttgactagcggaggctagaaggagagagatgggtgc
gagagcgtcagtattaagcgggggagaattagatcgcgatgggaaaaaattcggttaaggccagggggaaagaaaaa
atataaattaaaacatatagtatgggcaagcagggagctagaacgattcgcagttaatcctggcctgttagaaacat
cagaaggctgtagacaaatactgggacagctacaaccatcccttcagacaggatcagaagaacttagatcattatat
aatacagtagcaaccctctattgtgtgcatcaaaggatagagataaaagacaccaaggaagctttagacaagataga
 
ggaagagcaaaacaaaagtaagaccaccgcacagcaagcggccgctgatcttcagacctggaggaggagatatgagg
gacaattggagaagtgaattatataaatataaagtagtaaaaattgaaccattaggagtagcacccaccaaggcaaa
gagaagagtggtgcagagagaaaaaagagcagtgggaataggagctttgttccttgggttcttgggagcagcaggaa
gcactatgggcgcagcctcaatgacgctgacggtacaggccagacaattattgtctggtatagtgcagcagcagaac
aatttgctgagggctattgaggcgcaacagcatctgttgcaactcacagtctggggcatcaagcagctccaggcaag
aatcctggctgtggaaagatacctaaaggatcaacagctcctggggatttggggttgctctggaaaactcatttgca
ccactgctgtgccttggaatgctagttggagtaataaatctctggaacagattggaatcacacgacctggatggagt
gggacagagaaattaacaattacacaagcttaatacactccttaattgaagaatcgcaaaaccagcaagaaaagaat
gaacaagaattattggaattagataaatgggcaagtttgtggaattggtttaacataacaaattggctgtggtatat
aaaattattcataatgatagtaggaggcttggtaggtttaagaatagtttttgctgtactttctatagtgaatagag
ttaggcagggatattcaccattatcgtttcagacccacctcccaaccccgaggggacccgacaggcccgaaggaata
gaagaagaaggtggagagagagacagagacagatccattcgattagtgaacggatctcgacggtatcgataagctaa
ttcacaaatggcagtattcatccacaattttaaaagaaaaggggggattggggggtacagtgcaggggaaagaatag
tagacataatagcaacagacatacaaactaaagaattacaaaaacaaattacaaaaattcaaaattttcgggtttat
tacagggacagcagagatccagtttgggaattagcttgatcgattagtccaatttgttaaagacaggatatcagtgg
tccaggctctagttttgactcaacaatatcaccagctgaagcctatagagtacgagccatagatagaataaaagatt
ttatttagtctccagaaaaaggggggaatgaaagaccccacctgtaggtttggcaagctaggatcaaggttaggaac
agagagacagcagaatatgggccaaacaggatatctgtggtaagcagttcctgccccggctcagggccaagaacagt
tggaacagcagaatatgggccaaacaggatatctgtggtaagcagttcctgccccggctcagggccaagaacagatg
gtccccagatgcggtcccgccctcagcagtttctagagaaccatcagatgtttccagggtgccccaaggacctgaaa
tgaccctgtgccttatttgaactaaccaatcagttcgcttctcgcttctgttcgcgcgcttctgctccccgagctca
ataaaagagcccacaacccctcactcggcgcgatctagatctcgaatcgaattcgcccttgctactATGGCTTCTGG
AATCCTGGTTAATGTAAAGGAGGAGGTGACCTGCCCCATCTGCCTGGAACTCCTGACACAACCCCTGAGCCTGGACT
GCGGCCACAGCTTCTGCCAAGCATGCCTCACTGCAAACCACAAGAAGTCCATGCTAGACAAAGGAGAGAGTAGCTGC
CCTGTGTGCCGGATCAGTTACCAGCCTGAGAACATACGGCCTAATCGGCATGTAGCCAACATAGTGGAGAAGCTCAG
GGAGGTCAAGTTGAGCCCAGAGGGGCAGAAAGTTGATCATTGTGCACGCCATGGAGAGAAACTTCTACTCTTCTGTC
AGGAGGACGGGAAGGTCATTTGCTGGCTTTGTGAGCGGTCTCAGGAGCACCGTGGTCACCACACGTTCCTCACAGAG
GAGGTTGCCCGGGAGTACCAAGTGAAGCTCCAGGCAGCTCTGGAGATGCTGAGGCAGAAGCAGCAGGAAGCTGAAGA
GTTAGAAGCTGACATCAGAGAAGAGAAAGCTTCCTGGAAGACTCAAATACAGTATGACAAAACCAACGTCTTGGCAG
ATTTTGAGCAACTGAGAGACATCCTGGACTGGGAGGAGAGCAATGAGCTGCAAAACCTGGAGAAGGAGGAGGAAGAC
ATTCTGAAAAGCCTTACGAACTCTGAAACTGAGATGGTGCAGCAGACCCAGTCCCTGAGAGAGCTCATCTCAGATCT
GGAGCATCGGCTGCAGGGGTCAGTGATGGAGCTGCTTCAGGGTGTGGATGGCGTCATAAAAAGGACGGAGAACGTGA
CCTTGAAGAAGCCAGAAACTTTTCCAAAAAATCAAAGGAGAGTGTTTCGAGCTCCTGATCTGAAAGGAATGCTAGAA
GTGTTTAGAGAGCTGACAGATGTCCGACGCTACTGGGTTGATGTGACAGTGGCTCCAAACAACATTTCATGTGCTGT
CATTTCTGAAGATAAGAGACAAGTGAGCTCTCCGAAACCACAGATAATATATcaggcaccagggacattatttacgt
ttccgtcactcacgAATTTCAATTATTGTACTGGCATCCTGGGCTCTCAAAGTATCACATCAGGGAAACATTACTGG
 
GAGGTAGACGTGTCCAAGAAAACTGCTTGGATCCTGGGGTATGTGCTGGCTTCCAACCTGATGCAATGTGTAATAT
TGAAAAAAATGAAAATTATCAACCTAAATACGGCTACTGGGTTATAGGGTTAGAGGAAGGAGTTAAATGTAGTGCTT
TCCAGGATAGTTCCTTCCATACTCCTTCTGTTCCTTTCATTGTGCCCCTCTCTGTGATTATTTGTCCTGATCGTGTT
GGAGTTTTCCTAGACTATGAGGCTTGCACTGTCTCATTCTTCAATATCACAAACCATGGATTTCTCATCTATAAGTT
TTCTCACTGTTCTTTTTCTCAGCCTGTATTTCCATATTTAAATCCTAGAAAATGTGGAGTCCCCATGACTCTGTGCT
CACCAAGCTCTggaagcggagctactaacttcagcctgctgaagcaggctggagacgtggaggagaaccctggacct
ATGGATTCATACCTGCTGATGTGGGGACTGCTCACGTTCATCATGGTGCCTGGCTGCCAGGCAGAGCTCTGTGACGA
TGACCCGCCAGAGATCCCACACGCCACATTCAAAGCCATGGCCTACAAGGAAGGAACCATGTTGAACTGTGAATGCA
AGAGAGGTTTCCGCAGAATAAAAAGCGGGTCACTCTATATGCTCTGTACAGGAAACTCTAGCCACTCGTCCTGGGAC
AACCAATGTCAATGCACAAGCTCTGCCACTCGGAACACAACGAAACAAGTGACACCTCAACCTGAAGAACAGAAAGA
AAGGAAAACCACAGAAATGCAAAGTCCAATGCAGCCAGTGGACCAAGCGAGCCTTCCAGGTCACTGCAGGGAACCTC
CACCATGGGAAAATGAAGCCACAGAGAGAATTTATCATTTCGTGGTGGGGCAGATGGTTTATTATCAGTGCGTCCAG
GGATACAGGGCTCTACACAGAGGTCCTGCTGAGAGCGTCTGCAAAATGACCCACGGGAAGACAAGGTGGACCCAGCC
CCAGCTCATATGCACAGGTGAAATGGAGACCAGTCAGTTTCCAGGTGAAGAGAAGCCTCAGGCAAGCCCCGAAGGCC
GTCCTGAGAGTGAGACTTCCTGCCTCGTCACAACAACAGATTTTCAAATACAGACAGAAATGGCTGCAACCATGGAG
ACGTCCATATTTACAACAGAGTACCAGGTAGCAGTGGCCGGCTGTGTTTTCCTGCTGATCAGCGTCCTCCTCCTGAG
TGGGCTCACCTGGCAGCGGTGATGAGTTAACAGATCCAGTTTGGGGATCCAAGGTCGGGCAGGAAGAGGGCCTATTT
CCCATGATTCCTTCATATTTGCATATACGATACAAGGCTGTTAGAGAGATAATTAGAATTAATTTGACTGTAAACAC
AAAGATATTAGTACAAAATACGTGACGTAGAAAGTAATAATTTCTTGGGTAGTTTGCAGTTTTAAAATTATGTTTTA
AAATGGACTATCATATGCTTACCGTAACTTGAAAGTATTTCGATTTCTTGGCTTTATATATCTTGTGGAAAGGACGA
AACACCGAGCATGACTGACATCTACTTCAAGAGAGTAGATGTCAGTCATGCTCTTTTTTACGCGTAGAATCGAGACC
GAGGAGAGGGTTAGGGATAGGCTTACCTTCGAACCGCGGGCCCTCTAGACTCGAGCGGCCGCCACTGTGCTGGATAT
CTGCAGAATTGCCCTTGACGCGTCAGTTTGGGGATCCAAGGTCGGGCAGGAAGAGGGCCTATTTCCCATGATTCCTT
CATATTTGCATATACGATACAAGGCTGTTAGAGAGATAATTAGAATTAATTTGACTGTAAACACAAAGATATTAGTA
CAAAATACGTGACGTAGAAAGTAATAATTTCTTGGGTAGTTTGCAGTTTTAAAATTATGTTTTAAAATGGACTATCA
TATGCTTACCGTAACTTGAAAGTATTTCGATTTCTTGGCTTTATATATCTTGTGGAAAGGACGAAACACCGTCGACC
TTGCAATGATGTCGTAATTTGCGTCTTACTCTGTTCTCAGCGACAGCCAGATCTGAGCCTGGGAGCTCTCTGGCTGT
CAGTAAGCTGGTACAGAAGGTTGACGAAAATTCTTACTGAGCAAGAAATTTTTTACGCGTGTTAACAAGGGCGAATT
Cgttaacctcgagggatcccccgggtcgactgatcaaattcgagctcggtacctttaagaccaatgacttacaagg
cagctgtagatcttagccactttttaaaagaaaaggggggactggaagggctaattcactcccaacgaagacaagat
ctgctttttgcttgtactgggtctctctggttagaccagatctgagcctgggagctctctggctaactagggaaccc
actgcttaagcctcaataaagcttgccttgagtgcttcaagtagtgtgtgcccgtctgttgtgtgactctggtaact
agagatccctcagacccttttagtcagtgtggaaaatctctagcagtagtagttcatgtcatcttattattcagtat
ttataacttgcaaagaaatgaatatcagagagtgagaggaacttgtttattgcagcttataatggttacaaataaag
caatagcatcacaaatttcacaaataaagcatttttttcactgcattctagttgtggtttgtccaaactcatcaatg
tatcttatcatgtctggctctagctatcccgcccctaactccgcccatcccgcccctaactccgcccagttccgccc
 
attctccgccccatggctgactaattttttttatttatgcagaggccgaggccgcctcggcctctgagctattccag
aagtagtgaggaggcttttttggaggcctaggcttttgcgtcgagacgtacccaattcgccctatagtgagtcgtat
tacgcgcgctcactggccgtcgttttacaacgtcgtgactgggaaaaccctggcgttacccaacttaatcgccttgc
agcacatccccctttcgccagctggcgtaatagcgaagaggcccgcaccgatcgcccttcccaacagttgcgcagcc
tgaatggcgaatggcgcgacgcgccctgtagcggcgcattaagcgcggcgggtgtggtggttacgcgcagcgtgacc
gctacacttgccagcgccctagcgcccgctcctttcgctttcttcccttcctttctcgccacgttcgccggctttcc
ccgtcaagctctaaatcgggggctccctttagggttccgatttagtgctttacggcacctcgaccccaaaaaacttg
attagggtgatggttcacgtagtgggccatcgccctgatagacggtttttcgccctttgacgttggagtccacgttc
tttaatagtggactcttgttccaaactggaacaacactcaaccctatctcggtctattcttttgatttataagggat
tttgccgatttcggcctattggttaaaaaatgagctgatttaacaaaaatttaacgcgaattttaacaaaatattaa
cgtttacaatttcc-9897
SEQ ID NO: 3 
caggtggcacttttcggggaaatgtgcgcggaacccctatttgtttatttttctaaatacattcaaatatgtatccg
ctcatgagacaataaccctgataaatgcttcaataatattgaaaaaggaagagtatgagtattcaacatttccgtgt
cgcccttattcccttttttgcggcattttgccttcctgtttttgctcacccagaaacgctggtgaaagtaaaagatg
ctgaagatcagttgggtgcacgagtgggttacatcgaactggatctcaacagcggtaagatccttgagagttttcgc
cccgaagaacgttttccaatgatgagcacttttaaagttctgctatgtggcgcggtattatcccgtattgacgccgg
gcaagagcaactcggtcgccgcatacactattctcagaatgacttggttgagtactcaccagtcacagaaaagcatc
ttacggatggcatgacagtaagagaattatgcagtgctgccataaccatgagtgataacactgcggccaacttactt
ctgacaacgatcggaggaccgaaggagctaaccgcttttttgcacaacatgggggatcatgtaactcgccttgatcg
ttgggaaccggagctgaatgaagccataccaaacgacgagcgtgacaccacgatgcctgtagcaatggcaacaacgt
tgcgcaaactattaactggcgaactacttactctagcttcccggcaacaattaatagactggatggaggcggataaa
gttgcaggaccacttctgcgctcggcccttccggctggctggtttattgctgataaatctggagccggtgagcgtgg
gtctcgcggtatcattgcagcactggggccagatggtaagccctcccgtatcgtagttatctacacgacggggagtc
aggcaactatggatgaacgaaatagacagatcgctgagataggtgcctcactgattaagcattggtaactgtcagac
caagtttactcatatatactttagattgatttaaaacttcatttttaatttaaaaggatctaggtgaagatcctttt
tgataatctcatgaccaaaatcccttaacgtgagttttcgttccactgagcgtcagaccccgtagaaaagatcaaag
gatcttcttgagatcctttttttctgcgcgtaatctgctgcttgcaaacaaaaaaaccaccgctaccagcggtggtt
tgtttgccggatcaagagctaccaactctttttccgaaggtaactggcttcagcagagcgcagataccaaatactgt
ccttctagtgtagccgtagttaggccaccacttcaagaactctgtagcaccgcctacatacctcgctctgctaatcc
tgttaccagtggctgctgccagtggcgataagtcgtgtcttaccgggttggactcaagacgatagttaccggataag
gcgcagcggtcgggctgaacggggggttcgtgcacacagcccagcttggagcgaacgacctacaccgaactgagata
cctacagcgtgagctatgagaaagcgccacgcttcccgaagggagaaaggcggacaggtatccggtaagcggcaggg
tcggaacaggagagcgcacgagggagcttccagggggaaacgcctggtatctttatagtcctgtcgggtttcgccac
ctctgacttgagcgtcgatttttgtgatgctcgtcaggggggcggagcctatggaaaaacgccagcaacgcggcctt
 
tttacggttcctggccttttgctggccttttgctcacatgttctttcctgcgttatcccctgattctgtggataacc
gtattaccgcctttgagtgagctgataccgctcgccgcagccgaacgaccgagcgcagcgagtcagtgagcgaggaa
gcggaagagcgcccaatacgcaaaccgcctctccccgcgcgttggccgattcattaatgcagctggcacgacaggtt
tcccgactggaaagcgggcagtgagcgcaacgcaattaatgtgagttagctcactcattaggcaccccaggctttac
actttatgcttccggctcgtatgttgtgtggaattgtgagcggataacaatttcacacaggaaacagctatgaccat
gattacgccaagcgcgcaattaaccctcactaaagggaacaaaagctggagctgcaagcttggccattgcatacgtt
gtatccatatcataatatgtacatttatattggctcatgtccaacattaccgccatgttgacattgattattgacta
gttattaatagtaatcaattacggggtcattagttcatagcccatatatggagttccgcgttacataacttacggta
aatggcccgcctggctgaccgcccaacgacccccgcccattgacgtcaataatgacgtatgttcccatagtaacgcc
aatagggactttccattgacgtcaatgggtggagtatttacggtaaactgcccacttggcagtacatcaagtgtatc
atatgccaagtacgccccctattgacgtcaatgacggtaaatggcccgcctggcattatgcccagtacatgacctta
tgggactttcctacttggcagtacatctacgtattagtcatcgctattaccatggtgatgcggttttggcagtacat
caatgggcgtggatagcggtttgactcacggggatttccaagtctccaccccattgacgtcaatgggagtttgtttt
ggcaccaaaatcaacgggactttccaaaatgtcgtaacaactccgccccattgacgcaaatgggcggtaggcgtgta
cggtgggaggtctatataagcagagctcgtttagtgaaccgggtctctctggttagaccagatctgagcctgggag
ctctctggctaactagggaacccactgcttaagcctcaataaagcttgccttgagtgcttcaagtagtgtgtgcccg
tctgttgtgtgactctggtaactagagatccctcagacccttttagtcagtgtggaaaatctctagcagtggcgccc
gaacagggacctgaaagcgaaagggaaaccagaggagctctctcgacgcaggactcggcttgctgaagcgcgcacgg
caagaggcgaggggcggcgactggtgagtacgccaaaaattttgactagcggaggctagaaggagagagatgggtgc
gagagcgtcagtattaagcgggggagaattagatcgcgatgggaaaaaattcggttaaggccagggggaaagaaaaa
atataaattaaaacatatagtatgggcaagcagggagctagaacgattcgcagttaatcctggcctgttagaaacat
cagaaggctgtagacaaatactgggacagctacaaccatcccttcagacaggatcagaagaacttagatcattatat
aatacagtagcaaccctctattgtgtgcatcaaaggatagagataaaagacaccaaggaagctttagacaagataga
ggaagagcaaaacaaaagtaagaccaccgcacagcaagcggccgctgatcttcagacctggaggaggagatatgagg
gacaattggagaagtgaattatataaatataaagtagtaaaaattgaaccattaggagtagcacccaccaaggcaaa
gagaagagtggtgcagagagaaaaaagagcagtgggaataggagctttgttccttgggttcttgggagcagcaggaa
gcactatgggcgcagcctcaatgacgctgacggtacaggccagacaattattgtctggtatagtgcagcagcagaac
aatttgctgagggctattgaggcgcaacagcatctgttgcaactcacagtctggggcatcaagcagctccaggcaag
aatcctggctgtggaaagatacctaaaggatcaacagctcctggggatttggggttgctctggaaaactcatttgca
ccactgctgtgccttggaatgctagttggagtaataaatctctggaacagattggaatcacacgacctggatggagt
gggacagagaaattaacaattacacaagcttaatacactccttaattgaagaatcgcaaaaccagcaagaaaagaat
gaacaagaattattggaattagataaatgggcaagtttgtggaattggtttaacataacaaattggctgtggtatat
aaaattattcataatgatagtaggaggcttggtaggtttaagaatagtttttgctgtactttctatagtgaatagag
ttaggcagggatattcaccattatcgtttcagacccacctcccaaccccgaggggacccgacaggcccgaaggaata
gaagaagaaggtggagagagagacagagacagatccattcgattagtgaacggatctcgacggtatcgataagctaa
ttcacaaatggcagtattcatccacaattttaaaagaaaaggggggattggggggtacagtgcaggggaaagaatag
 
tagacataatagcaacagacatacaaactaaagaattacaaaaacaattacaaaaattcaaaattttcgggtttat
tacagggacagcagagatccagtttgggaattagcttgatcgattagtccaatttgttaaagacaggatatcagtgg
tccaggctctagttttgactcaacaatatcaccagctgaagcctatagagtacgagccatagatagaataaaagatt
ttatttagtctccagaaaaaggggggaatgaaagaccccacctgtaggtttggcaagctaggatcaaggttaggaac
agagagacagcagaatatgggccaaacaggatatctgtggtaagcagttcctgccccggctcagggccaagaacagt
tggaacagcagaatatgggccaaacaggatatctgtggtaagcagttcctgccccggctcagggccaagaacagatg
gtccccagatgcggtcccgccctcagcagtttctagagaaccatcagatgtttccagggtgccccaaggacctgaaa
tgaccctgtgccttatttgaactaaccaatcagttcgcttctcgcttctgttcgcgcgcttctgctccccgagctca
ataaaagagcccacaacccctcactcggcgcgatctagatctcgaatcgaattcgcccttgctactATGGCTTCTGG
AATCCTGGTTAATGTAAAGGAGGAGGTGACCTGCCCCATCTGCCTGGAACTCCTGACACAACCCCTGAGCCTGGACT
GCGGCCACAGCTTCTGCCAAGCATGCCTCACTGCAAACCACAAGAAGTCCATGCTAGACAAAGGAGAGAGTAGCTGC
CCTGTGTGCCGGATCAGTTACCAGCCTGAGAACATACGGCCTAATCGGCATGTAGCCAACATAGTGGAGAAGCTCAG
GGAGGTCAAGTTGAGCCCAGAGGGGCAGAAAGTTGATCATTGTGCACGCCATGGAGAGAAACTTCTACTCTTCTGTC
AGGAGGACGGGAAGGTCATTTGCTGGCTTTGTGAGCGGTCTCAGGAGCACCGTGGTCACCACACGTTCCTCACAGAG
GAGGTTGCCCGGGAGTACCAAGTGAAGCTCCAGGCAGCTCTGGAGATGCTGAGGCAGAAGCAGCAGGAAGCTGAAGA
GTTAGAAGCTGACATCAGAGAAGAGAAAGCTTCCTGGAAGACTCAAATACAGTATGACAAAACCAACGTCTTGGCAG
ATTTTGAGCAACTGAGAGACATCCTGGACTGGGAGGAGAGCAATGAGCTGCAAAACCTGGAGAAGGAGGAGGAAGAC
ATTCTGAAAAGCCTTACGAACTCTGAAACTGAGATGGTGCAGCAGACCCAGTCCCTGAGAGAGCTCATCTCAGATCT
GGAGCATCGGCTGCAGGGGTCAGTGATGGAGCTGCTTCAGGGTGTGGATGGCGTCATAAAAAGGACGGAGAACGTGA
CCTTGAAGAAGCCAGAAACTTTTCCAAAAAATCAAAGGAGAGTGTTTCGAGCTCCTGATCTGAAAGGAATGCTAGAA
GTGTTTAGAGAGCTGACAGATGTCCGACGCTACTGGGTTGATGTGACAGTGGCTCCAAACAACATTTCATGTGCTGT
CATTTCTGAAGATAAGAGACAAGTGAGCTCTCCGAAACCACAGATAATATATcaggcaccagggacattatttacgt
ttccgtcactcacgAATTTCAATTATTGTACTGGCATCCTGGGCTCTCAAAGTATCACATCAGGGAAACATTACTGG
GAGGTAGACGTGTCCAAGAAAACTGCTTGGATCCTGGGGGTATGTGCTGGCTTCCAACCTGATGCAATGTGTAATAT
TGAAAAAAATGAAAATTATCAACCTAAATACGGCTACTGGGTTATAGGGTTAGAGGAAGGAGTTAAATGTAGTGCTT
TCCAGGATAGTTCCTTCCATACTCCTTCTGTTCCTTTCATTGTGCCCCTCTCTGTGATTATTTGTCCTGATCGTGTT
GGAGTTTTCCTAGACTATGAGGCTTGCACTGTCTCATTCTTCAATATCACAAACCATGGATTTCTCATCTATAAGTT
TTCTCACTGTTCTTTTTCTCAGCCTGTATTTCCATATTTAAATCCTAGAAAATGTGGAGTCCCCATGACTCTGTGCT
CACCAAGCTCTggaagcggagctactaacttcagcctgctgaagcaggctggagacgtggaggagaaccctggacct
ATGGATTCATACCTGCTGATGTGGGGACTGCTCACGTTCATCATGGTGCCTGGCTGCCAGGCAGAGCTCTGTGACGA
TGACCCGCCAGAGATCCCACACGCCACATTCAAAGCCATGGCCTACAAGGAAGGAACCATGTTGAACTGTGAATGCA
AGAGAGGTTTCCGCAGAATAAAAAGCGGGTCACTCTATATGCTCTGTACAGGAAACTCTAGCCACTCGTCCTGGGAC
AACCAATGTCAATGCACAAGCTCTGCCACTCGGAACACAACGAAACAAGTGACACCTCAACCTGAAGAACAGAAAGA
AAGGAAAACCACAGAAATGCAAAGTCCAATGCAGCCAGTGGACCAAGCGAGCCTTCCAGGTCACTGCAGGGAACCTC
CACCATGGGAAAATGAAGCCACAGAGAGAATTTATCATTTCGTGGTGGGGCAGATGGTTTATTATCAGTGCGTCCAG
GGATACAGGGCTCTACACAGAGGTCCTGCTGAGAGCGTCTGCAAAATGACCCACGGGAAGACAAGGTGGACCCAGCC
 
CCAGCTCATATGCACAGGTGAAATGGAGACCAGTCAGTTTCCAGGTGAAGAGAAGCCTCAGGCAAGCCCCGAAGGCC
GTCCTGAGAGTGAGACTTCCTGCCTCGTCACAACAACAGATTTTCAAATACAGACAGAAATGGCTGCAACCATGGAG
ACGTCCATATTTACAACAGAGTACCAGGTAGCAGTGGCCGGCTGTGTTTTCCTGCTGATCAGCGTCCTCCTCCTGAG
TGGGCTCTGATGAGTTAACAGATCCAGTTTGGGGATCCAAGGTCGGGCAGGAAGAGGGCCTATTTCCCATGATTCCT
TCATATTTGCATATACGATACAAGGCTGTTAGAGAGATAATTAGAATTAATTTGACTGTAAACACAAAGATATTAGT
ACAAAATACGTGACGTAGAAAGTAATAATTTCTTGGGTAGTTTGCAGTTTTAAAATTATGTTTTAAAATGGACTATC
ATATGCTTACCGTAACTTGAAAGTATTTCGATTTCTTGGCTTTATATATCTTGTGGAAAGGACGAAACACCGAGCAT
GACTGACATCTACTTCAAGAGAGTAGATGTCAGTCATGCTCTTTTTTACGCGTAGAATCGAGACCGAGGAGAGGGTT
AGGGATAGGCTTACCTTCGAACCGCGGGCCCTCTAGACTCGAGCGGCCGCCACTGTGCTGGATATCTGCAGAATTGC
CCTTGACGCGTCAGTTTGGGGATCCAAGGTCGGGCAGGAAGAGGGCCTATTTCCCATGATTCCTTCATATTTGCATA
TACGATACAAGGCTGTTAGAGAGATAATTAGAATTAATTTGACTGTAAACACAAAGATATTAGTACAAAATACGTGA
CGTAGAAAGTAATAATTTCTTGGGTAGTTTGCAGTTTTAAAATTATGTTTTAAAATGGACTATCATATGCTTACCGT
AACTTGAAAGTATTTCGATTTCTTGGCTTTATATATCTTGTGGAAAGGACGAAACACCGTCGACCTTGCAATGATGT
CGTAATTTGCGTCTTACTCTGTTCTCAGCGACAGCCAGATCTGAGCCTGGGAGCTCTCTGGCTGTCAGTAAGCTGGT
ACAGAAGGTTGACGAAAATTCTTACTGAGCAAGAAATTTTTTACGCGTGTTAACAAGGGCGAATTCgttaacctcga
gggatcccccggggtcgactgatcaaattcgagctcggtacctttaagaccaatgacttacaaggcagctgtagatc
ttagccactttttaaaagaaaaggggggactggaagggctaattcactcccaacgaagacaagatctgctttttgct
tgtactgggtctctctggttagaccagatctgagcctgggagctctctggctaactagggaacccactgcttaagcc
tcaataaagcttgccttgagtgcttcaagtagtgtgtgcccgtctgttgtgtgactctggtaactagagatccctca
gacccttttagtcagtgtggaaaatctctagcagtagtagttcatgtcatcttattattcagtatttataacttgca
aagaaatgaatatcagagagtgagaggaacttgtttattgcagcttataatggttacaaataaagcaatagcatcac
aaatttcacaaataaagcatttttttcactgcattctagttgtggtttgtccaaactcatcaatgtatcttatcatg
tctggctctagctatcccgcccctaactccgcccatcccgcccctaactccgcccagttccgcccattctccgcccc
atggctgactaattttttttatttatgcagaggccgaggccgcctcggcctctgagctattccagaagtagtgagga
ggcttttttggaggcctaggcttttgcgtcgagacgtacccaattcgccctatagtgagtcgtattacgcgcgctca
ctggccgtcgttttacaacgtcgtgactgggaaaaccctggcgttacccaacttaatcgccttgcagcacatccccc
tttcgccagctggcgtaatagcgaagaggcccgcaccgatcgcccttcccaacagttgcgcagcctgaatggcgaat
ggcgcgacgcgccctgtagcggcgcattaagcgcggcgggtgtggtggttacgcgcagcgtgaccgctacacttgcc
agcgccctagcgcccgctcctttcgctttcttcccttcctttctcgccacgttcgccggctttccccgtcaagctct
aaatcgggggctccctttagggttccgatttagtgctttacggcacctcgaccccaaaaaacttgattagggtgatg
gttcacgtagtgggccatcgccctgatagacggtttttcgccctttgacgttggagtccacgttctttaatagtgga
ctcttgttccaaactggaacaacactcaaccctatctcggtctattcttttgatttataaggatttgccgatttc
ggcctattggttaaaaaatgagctgatttaacaaaaatttaacgcgaattttaacaaaatattaacgtttacaattt
cc-9897
SEQ ID NO: 4 
ATGGATTCATACCTGCTGATGTGGGGACTGCTCACGTTCATCATGGTGCCTGGCTGCCAGGCAGAGCTCTGTGACGA
TGACCCGCCAGAGATCCCACACGCCACATTCAAAGCCATGGCCTACAAGGAAGGAACCATGTTGAACTGTGAATGCA
AGAGAGGTTTCCGCAGAATAAAAAGCGGGTCACTCTATATGCTCTGTACAGGAAACTCTAGCCACTCGTCCTGGGAC
AACCAATGTCAATGCACAAGCTCTGCCACTCGGAACACAACGAAACAAGTGACACCTCAACCTGAAGAACAGAAAGA
AAGGAAAACCACAGAAATGCAAAGTCCAATGCAGCCAGTGGACCAAGCGAGCCTTCCAGGTCACTGCAGGGAACCTC
CACCATGGGAAAATGAAGCCACAGAGAGAATTTATCATTTCGTGGTGGGGCAGATGGTTTATTATCAGTGCGTCCAG
GGATACAGGGCTCTACACAGAGGTCCTGCTGAGAGCGTCTGCAAAATGACCCACGGGAAGACAAGGTGGACCCAGCC
CCAGCTCATATGCACAGGTGAAATGGAGACCAGTCAGTTTCCAGGTGAAGAGAAGCCTCAGGCAAGCCCCGAAGGCC
GTCCTGAGAGTGAGACTTCCTGCCTCGTCACAACAACAGATTTTCAAATACAGACAGAAATGGCTGCAACCATGGAG
ACGTCCATATTTACAACAGAGTACCAGGTAGCAGTGGCCGGCTGTGTTTTCCTGCTGATCAGCGTCCTCCTCCTGAG
TGGGCTCACCTGGCAGCGGAGACAGAGGAAGAGTAGAAGAACAATCTGATGA
SEQ ID NO: 5 
ATGGATTCATACCTGCTGATGTGGGGACTGCTCACGTTCATCATGGTGCCTGGCTGCCAGGCAGAGCTCTGTGACGA
TGACCCGCCAGAGATCCCACACGCCACATTCAAAGCCATGGCCTACAAGGAAGGAACCATGTTGAACTGTGAATGCA
AGAGAGGTTTCCGCAGAATAAAAAGCGGGTCACTCTATATGCTCTGTACAGGAAACTCTAGCCACTCGTCCTGGGAC
AACCAATGTCAATGCACAAGCTCTGCCACTCGGAACACAACGAAACAAGTGACACCTCAACCTGAAGAACAGAAAGA
AAGGAAAACCACAGAAATGCAAAGTCCAATGCAGCCAGTGGACCAAGCGAGCCTTCCAGGTCACTGCAGGGAACCTC
CACCATGGGAAAATGAAGCCACAGAGAGAATTTATCATTTCGTGGTGGGGCAGATGGTTTATTATCAGTGCGTCCAG
GGATACAGGGCTCTACACAGAGGTCCTGCTGAGAGCGTCTGCAAAATGACCCACGGGAAGACAAGGTGGACCCAGCC
CCAGCTCATATGCACAGGTGAAATGGAGACCAGTCAGTTTCCAGGTGAAGAGAAGCCTCAGGCAAGCCCCGAAGGCC
GTCCTGAGAGTGAGACTTCCTGCCTCGTCACAACAACAGATTTTCAAATACAGACAGAAATGGCTGCAACCATGGAG
ACGTCCATATTTACAACAGAGTACCAGGTAGCAGTGGCCGGCTGTGTTTTCCTGCTGATCAGCGTCCTCCTCCTGAG
TGGGCTCACCTGGCAGCGGTGATGA
SEQ ID NO: 6 
ATGGATTCATACCTGCTGATGTGGGGACTGCTCACGTTCATCATGGTGCCTGGCTGCCAGGCAGAGCTCTGTGACGA
TGACCCGCCAGAGATCCCACACGCCACATTCAAAGCCATGGCCTACAAGGAAGGAACCATGTTGAACTGTGAATGCA
AGAGAGGTTTCCGCAGAATAAAAAGCGGGTCACTCTATATGCTCTGTACAGGAAACTCTAGCCACTCGTCCTGGGAC
AACCAATGTCAATGCACAAGCTCTGCCACTCGGAACACAACGAAACAAGTGACACCTCAACCTGAAGAACAGAAAGA
AAGGAAAACCACAGAAATGCAAAGTCCAATGCAGCCAGTGGACCAAGCGAGCCTTCCAGGTCACTGCAGGGAACCTC
CACCATGGGAAAATGAAGCCACAGAGAGAATTTATCATTTCGTGGTGGGGCAGATGGTTTATTATCAGTGCGTCCAG
GGATACAGGGCTCTACACAGAGGTCCTGCTGAGAGCGTCTGCAAAATGACCCACGGGAAGACAAGGTGGACCCAGCC
CCAGCTCATATGCACAGGTGAAATGGAGACCAGTCAGTTTCCAGGTGAAGAGAAGCCTCAGGCAAGCCCCGAAGGCC
GTCCTGAGAGTGAGACTTCCTGCCTCGTCACAACAACAGATTTTCAAATACAGACAGAAATGGCTGCAACCATGGAG
 
ACGTCCATATTTACAACAGAGTACCAGGTAGCAGTGGCCGGCTGTGTTTTCCTGCTGATCAGCGTCCTCCTCCTGAG
TGGGCTCTGATGA
SEQ ID NO: 7
   gtgtcaagtccaatctatgacatcaattatatgtgaattgatgtcatagattggacttga
SEQ ID NO: 8
   gttcagaaactacctcttaatatgtgtaagaggtagtttctgaac
SEQ ID NO: 9
   gttcagaaactacctcttagtcttcttcatatgtggaagaagactaagagaac
SEQ ID NO: 10
   Gagcatgactgacatctacctgctcaacatatgtggttgagcaggtagatgtcagtcatgctc

SEQ ID NO: 11

gacatccagatgacccagtctccatcctccctgtctgcatctgtaggagacagagtcaccatcacttgccaggcgag

tcagggcattcgcaaatatttaaattggtatcagcaaaaaccagggaaagtccctaagctcctgatctacgatgcat

ccaatttggaaacaggggtcccatcaaggttcagtggaagtggatcaggacagattttactttcgccatcagcagc

ctgcagccggaagatactgcaacatattactgtcaacaatatgatgatttccccttcaccttcggccaggggacacg

actggagattaaacgt

SEQ ID NO: 12
5'-cgacttaaaatcgctagccagatctgagcctgggagctctctggctag-3'
SEQ ID NO: 13
5'-gggtctctctggttagaccagatttgagcctgggagctctctggctaactagggaaccc-3'
SEQ ID NO: 14
5'-acgaagcttgatcccgtttgccggtcgatcgcttcga-3'
SEQ ID NO: 15 
taattcgagc tcgcccgaca ttgattattg actagttatt aatagtaatc aattacgggg 60
tcattagttc atagcccata tatggagttc cgcgttacat aacttacggt aaatggcccg 120
cctggctgac cgcccaacga cccccgccca ttgacgtcaa taatgacgta tgttcccata 180
gtaacgccaa tagggacttt ccattgacgt caatgggtgg agtatttacg gtaaactgcc 240
 
cacttggcag tacatcaagt gtatcatatg ccaagtacgc cccctattga cgtcaatgac 300
ggtaaatggc ccgcctggca ttatgcccag tacatgacct tatgggactt tcctacttgg 360
cagtacatct acgtattagt catcgctatt accatggtga tgcggttttg gcagtacatc 420
aatgggcgtg gatagcggtt tgactcacgg ggattccaag tctcacccca ttgacgtcaa 480
tgggagtttg ttttggcacc aaaatcaacg ggactttcca aaatgtcgta acaactccgc 540
cccattgacg caaatgggcg gtaggcgtgt acggtgggag gtctatataa gcagagctcg 600
tttagtgaac cgtcagatcg cctggagacg ccatccacgc tgttttgacc tccatagaag 660
acaccgggac cgatcctagc ctccgcggcc gggaacggtg cattggaacg cggattcccc 720
gtgccaagag tgacgtaagt accgcctata gagtctatag gcccaccccc ttggcttctt 780
atgcgacgga tcgatcccgt aataagcttc gaggtccgcg gccggccgcg ttgacgcgca 840
cggcagaggc gatggggctg gcgactggtg agagatgggt gcgagagcgt cagtattaag 900
cgggggagaa ttagatcgat gggaaaaaat tcggttaagg ccagggggaa agaaaaaata 960
taaattaaaa catatagtat gggcaagcag ggagctagaa cgattcgcag ttaatcctgg 1020
cctgttagaa acatcagaag gctgtagaca aatactggga cagctacaac catcccttca 1080
gacaggatca gaagaactta gatcattata taatacagta gcaaccctct attgtgtgca 1140
tcaaaggata gagataaaag acaccaagga agctttagac aagatagagg aagagcaaaa 1200
caaaagtaag aaaaaagcac agcaagcagc agctgacaca ggacacagca atcaggtcag 1260
ccaaaattac cctatagtgc agaacatcca ggggcaaatg gtacatcagg ccatatcacc 1320
tagaacttta aatgcatggg taaaagtagt agaagagaag gctttcagcc cagaagtgat 1380
acccatgttt tcagcattat cagaaggagc caccccacaa gatttaaaca ccatgctaac 1440
acagtggggg gacatcaagc agccatgcaa atgttaaaag agaccatcaa tgaggaagct 1500
gcagaatggg atagagtgca tccagtgcat gcagggccta ttgcaccagg ccagatgaga 1560
gaaccaaggg gaagtgacat agcaggaact actagtaccc ttcaggaaca aataggatgg 1620
atgacacata atccacctat cccagtagga gaaatctata aaagatggat aatcctggga 1680
ttaaataaaa tagtaagaat gtatagccct accagcattc tggacataag acaaggacca 1740
aaggaaccct ttagagacta tgtagaccga ttctataaaa ctctaagagc cgagcaagct 1800
tcacaagagg taaaaaattg gatgacagaa accttgttgg tccaaaatgc gaacccagat 1860
tgtaagacta ttttaaaagc attgggacca ggagcgacac tagaagaaat gatgacagca 1920
tgtcagggag tggggggacc cggccataaa gcaagagttt tggctgaagc aatgagccaa 1980
gtaacaaatc cagctaccat aatgatacag aaaggcaatt ttaggaacca aagaaagact 2040
gttaagtgtt tcaattgtgg caaagaaggg cacatagcca aaaattgcag ggcccctagg 2100
aaaagggctg ttggaaatgt ggaaaggaag gacacccaat gaaagattgt actgagagac 2160
aggctatttt ttagggaaga tctggccttc cacagggaag gccagggaat tttcttcaga 2220
gcagaccaga gccaacagcc ccaccagaag agagcttcag gtttggggaa gagacaacaa 2280
ctccctctca gaagcaggag ccgatagaca aggaactgtg tcctttagct tccctcagat 2340
cactctttgg cagcgacccc tcgtcacaat aaagataggg gggcaattaa aggaagctct 2400
 
attagataca ggagcagatg atacagtatt agaagaaatg aatttgccag gaagatggaa 2460
accaaaaatg atagggggaa ttggaggttt tatcaaagta ggacagtatg atcagatact 2520
catagaaatc tgcggacata aagctatagg tacagtatta gtaggaccta cacctgtcaa 2580
cataattgga agaaatctgt tgactcagat tggctgcact ttaaattttc ccattagtcc 2640
tattgagact gtaccagtaa aattaaagcc aggaatggat ggcccaaaag ttaaacaatg 2700
gccattgaca gaagaaaaaa taaaagcatt agtagaaatt tgtacagaaa tggaaaagga 2760
aggaaaaatt tcaaaaattg ggcctgaaaa tccatacaat actccagtat ttgccataaa 2820
gaaaaaagac agtactaaat ggagaaaatt agtagatttc agagaactta ataagagaac 2880
tcaagatttc tgggaagttc aattaggaat accacatcct gcagggttaa aacagaaaaa 2940
atcagtaaca gtactggatg tgggcgatgc atatttttca gttcccttag ataaagactt 3000
caggaagtat actgcattta ccatacctag tataaacaat gagnacacca gggnattagn 3060
atatcagtac aatgtgcttc cacagggnat ggaaaggatc accagcaata ttccagtgta 3120
gcatgacaaa aatcttagag ccttttagaa aacaaaatcc agacatagtc atctatcaat 3180
acatggatga tttgtatgta ggatctgact tagaaatagg gcagcataga acaaaaatag 3240
aggaactgag acaacatctg ttgaggtggg gatttaccac accagacaaa aaacatcaga 3300
aagaacctcc attcctttgg atgggttatg aactccatcc tgataaatgg acagtacagc 3360
ctatagtgct gccagaaaag gacagctgga ctgtcaatga catacagaaa ttagtgggaa 3420
aattgaattg ggcaagtcag atttatgcag ggattaaagt aaggcaatta tgtaaacttc 3480
ttaggggaac caaagcacta acagaagtag taccactaac agaagaagca gagctagaac 3540
tggcagaaaa cagggagatt ctaaaagaac cggtacatgg agtgtattat gacccatcaa 3600
aagacttaat agcagaaata cagaagcagg ggcaaggcca atggacatat caaatttatc 3660
aagagccatt taaaaatctg aaaacaggaa aatatgcaag aatgaagggt gcccacacta 3720
atgatgtgaa acaattaaca gaggcagtac aaaaaatagc cacagaaagc atagtaatat 3780
ggggaaagac tcctaaattt aaattaccca tacaaaagga aacatgggaa gcatggtgga 3840
cagagtattg gcaagccacc tggattcctg agtgggagtt tgtcaatacc cctcccttag 3900
tgaagttatg gtaccagtta gagaaagaac ccataatagg agcagaaact ttctatgtag 3960
atggggcagc caatagggaa actaaattag gaaaagcagg atatgtaact gacagaggaa 4020
gacaaaaagt tgtcccccta acggacacaa caaatcagaa gactgagtta caagcaattc 4080
atctagcttt gcaggattcg ggattagaag taaacatagt gacagactca caatatgcat 4140
tgggaatcat tcaagcacaa ccagataaga gtgaatcaga gttagtcagt caaataatag 4200
agcagttaat aanaaaagga aaaagtctac ctggcatggg taccagcaca caaaggaatt 4260
ggaggaaatg aacagtagat aaattggtca gtgctggaat caggaaagta ctatttttag 4320
atggaataga taaggcccaa gaagaacatg agaaatatca cagtaattgg agagcaatgg 4380
ctagtgattt taacctacca cctgtagtag caaaagaaat agtagccagc tgtgataaat 4440
gtcagctaaa aggggaagcc atgcatggac aagtagactg tagcccagga atatggcagc 4500
tagattgtac acatttagaa ggaaaagtta tcttggtagc agttcatgta gccagtggat 4560
 
atatagaagc agaagtaatt ccagcagaga cagggcaaga aacagcatac ttcctcttaa 4620
aattagcagg aagatggcca gtaaaaacag tacatacaga caatggcagc aatttcacca 4680
gtactacagt taaggccgcc tgttggtggg cggggatcag caggaatttg gcattcccta 4740
caatccccaa agtcaaggag taatagaatc tatgaataaa gaattaaaga aaattatagg 4800
acaggtaaga gatcaggctg aacatcttaa gacagcagta caaatggcag tattcatcca 4860
caattttaaa agaaaagggg ggattggggg gtacagtgca ggggaaagaa tagtagacat 4920
aatagcaaca gacatacaaa ctaaagaatt acaaaaacaa attacaaaaa ttcaaaattt 4980
tcgggtttat tacagggaca gcagagatcc agtttggaaa ggaccagcaa agctcctctg 5040
gaaaggtgaa ggggcagtag taatacaaga taatagtgac ataaaagtag tgccaagaag 5100
aaaagcaaag atcatcaggg attatggaaa acagatggca ggtgatgatt gtgtggcaag 5160
tagacaggat gaggattaac acatggaatt ctgcaacaac tgctgtttat ccatttcaga 5220
attgggtgtc gacatagcag aataggcgtt actcgacaga ggagagcaag aaatggagcc 5280
agtagatcct agactagagc cctggaagca tccaggaagt cagcctaaaa ctgcttgtac 5340
caattgctat tgtaaaaagt gttgctttca ttgccaagtt tgtttcatga caaaagcctt 5400
aggcatctcc tatggcagga agaagcggag acagcgacga agagctcatc agaacagtca 5460
gactcatcaa gcttctctat caaagcagta agtagtacat gtaatgcaac ctataatagt 5520
agcaatagta gcattagtag tagcaataat aatagcaata gttgtgtggt ccatagtaat 5580
catagaatat aggaaaatgg ccgctgatct tcagacctgg aggaggagat atgagggaca 5640
attggagaag tgaattatat aaatataaag tagtaaaaat tgaaccatta ggagtagcac 5700
ccaccaaggc aaagagaaga gtggtgcaga gagaaaaaag agcagtggga ataggagctt 5760
tgttccttgg gttcttggga gcagcaggaa gcactatggg cgcagcgtca atgacgctga 5820
cggtacaggc cagacaatta ttgtctggtn tagtgcagca gcagaacaat ttgctgaggg 5880
ctattgaggc gcaacagcat ctgttgcaac tcacagtctg gggcatcaag cagctccagg 5940
caagaatcct ggctgtggaa agatacctaa aggatcaaca gctcctgggg atttggggtt 6000
gctctggaaa actcatttgc accactgctg tgccttggaa tgctagttgg agtaataaat 6060
ctctggaaca gatttggaat cacacgacct ggatggagtg ggacagagaa attaacaatt 6120
acacaagctt aatacactcc ttaattgaag aatcgcaaaa ccagcaagaa aagaatgaac 6180
aagaattatt ggaattagat gaatgggcaa gtttgtggaa ttggtttaac ataacaaatt 6240
ggctgtggta tataaaatta ttcataatga tagtaggagg cttggtaggt ttaagaatag 6300
tttttgctgt actttctata gtgaatagag ttaggcaggg atattcacca ttatcgtttc 6360
agacccacct cccaaccccg aggggacccg acaggcccga aggaatagaa gaagaaggtg 6420
gagagagaga cagagacaga tccattcgat tagtgaacgg atccttggca cttatctggg 6480
acgatctgcg gagcctgtgc ctcttcagct accaccgctt gagagactta ctcttgattg 6540
taacgaggat tgtggaactt ctgggacgca gggggtggga agccctcaaa tattggtgga 6600
atctcctaca atattggagt caggagctaa agaatagtgc tgttagcttg ctcaatgcca 6660
cagccatagc agtagctgag gggacagata gggttataga agtagtacaa ggagcttgta 6720
 
gagctattcg ccacatacct agaagaataa gacagggctt ggaaaggatt ttgctataag 6780
ctcgaggccg ccccggtgac cttcagacct tggcactgga ggtggcccgg cagaagcgcg 6840
gcatcgtgga tcagtgctgc accagcatct gctctctcta ccaactggag aactactgca 6900
actaggccca ccactaccct gtccacccct ctgcaatgaa taaaaccttt gaaagagcac 6960
tacaagttgt gtgtacatgc gtgcatgtgc atatgtggtg cggggggaac atgagtgggg 7020
ctggctggag tggcgatgat aagctgtcaa acatgagaat taattcttga agacgaaagg 7080
gcctcgtgat acgcctattt ttataggtta atgtcatgat aataatggtt tcttagtcta 7140
gaattaattc cgtgtattct atagtgncac ctaaatcgta tgtgtatgat acataaggtt 7200
atgtattaat tgtagccgcg ttctaacgac aatatgtaca agcctaattg tgtagcatct 7260
ggcttactga agcagaccct atcatctctc tcgtaaactg ccgtcagagt cggtttggtt 7320
ggacgaacct tctgagtttc tggtaacgcc gtcccgcacc cgcacccggn aaatggtcag 7380
cgaaccaatc agcagggtca tcgctagcca gatcctctac gccggacgca tcgtggccgg 7440
catcaccggc gccacaggtg cggttgcatg gcgcctatat cgccgacatc accgatgggg 7500
aagatcgggc tcgccacttc gggctcatga gcgcttgttt cggcgtgggt atggtggcag 7560
gccccgtggc cgggggactg ttgggcgcca tctccttgca tgcaccattc cttgcggcgg 7620
cggtgctcaa cggcctcaac ctactactgg gctgcttcct aatgcaggag tcgcataagg 7680
gagagcgtcg aatggtgcac tctcagtaca atctgctctg atgccgcata gttaagccag 7740
ccccgacacc cgccaacacc cgctgacgcg ccctgacggg cttgtctgct cccggcatcc 7800
gcttacagac aagctgtgac cgtctccggg agctgcatgt gtcagaggtn ntcaccgtca 7860
tcaccgaaac gcgcgagacg aaagggcctc gtgatacgcc tatttttata ggttaatgtc 7920
atgataataa tggtttctta gacgtcaggt ggcacttttc ggggaaatgt gcgcggaacc 7980
cctatttgtt tatttttcta aatacattca aatatgtatc cgctcatgag acaataaccc 8040
tgataaatgc ttcaataata ttgaaaaagg aagagtatga gtattcaaca tttccgtgtc 8100
gcccttattc ccttttttgc ggcattttgc cttcctgttt ttgctcaccc agaaacgctg 8160
gtgaaagtaa aagatgctga agatcagttg ggtgcacgag tgggttacat cgaactggat 8220
ctcaacagcg gtaagatcct tgagagtttt cgccccgaag aacgttttcc aatgatgagc 8280
acttttaaag ttctgctatg tggcgcggta ttatcccgta ttgacgccgg gcaagagcaa 8340
ctcggtcgcc gcatacacta ttctcagaat gacttggttg agtactcacc agtcacagaa 8400
aagcatctta cggatggcat gacagtaaga gaattatgca gtgctgccat aaccatgagt 8460
gataacactg cggccaactt acttctgaca acgatcggag gaccgaagga gctaaccgct 8520
tttttgcaca acatggggga tcatgtaact cgccttgatc gttgggaacc ggagctgaat 8580
gaagccatac caaacgacga gcgtgacacc acgatgcctg tagcaatggc aacaacgttg 8640
cgcaaactat taactggcga actacttact ctagcttccc ggcaacaatt aatagactgg 8700
atggaggcgg ataaagttgc aggaccactt ctgcgctcgg cccttccggc tggctggttt 8760
attgctgata aatctggagc cggtgagcgt gggtctcgcg gtatcattgc agcactgggg 8820
ccagatggta agccctcccg tatcgtagtt atctacacga cggggagtca ggcaactatg 8880
 
gatgaacgaa atagacagat cgctgagata ggtgcctcac tgattaagca ttggtaactg 8940
tcagaccaag tttactcata tatactttag attgatttaa aacttcattt ttaatttaaa 9000
aggatctagg tgaagatcct ttttgataat ctcatgacca aaatccctta acgtgagttt 9060
tcgttccact gagcgtcaga ccccgtagaa aagatcaaag gatcttcttg agatcctttt 9120
tttctgcgcg taatctgctg cttgcaaaca aaaaaaccac cgctaccagc ggtggtttgt 9180
ttgccggatc aagagctacc aactcttttt ccgaaggtaa ctggcttcag cagagcgcag 9240
ataccaaata ctgtccttct agtgtagccg tagttaggcc accacttcaa gaactctgta 9300
gcaccgccta catacctcgc tctgctaatc ctgttaccag tggctgctgc cagtggcgat 9360
aagtcgtgtc ttaccgggtt ggactcaaga cgatagttac cggataaggc gcagcggtcg 9420
ggctgaacgg ggggttcgtg cacacagccc agcttggagc gaacgaccta caccgaactg 9480
agatacctac agcgtgagca ttgagaaagc gccacgcttc ccgaagggag aaaggcggac 9540
aggtatccgg taagcggcag ggtcggaaca ggagagcgca cgagggagct tccaggggga 9600
aacgcctggt atctttatag tcctgtcggg tttcgccacc tctgacttga gcgtcgattt 9660
ttgtgatgct cgtcaggggg gcggagccta tggaaaaacg ccagcaacgc ggccttttta 9720
cggttcctgg ccttttgctg gccttttgct cacatgttct ttcctgcgtt atcccctgat 9780
tctgtggata accgtattac cgcctttgag tgagctgata ccgctcgccg cagccgaacg 9840
accgagcgca gcgagtcagt gagcgaggaa gcggaagagc gcccaatacg caaaccgcct 9900
ctccccgcgc gttggccgat tcattaatgc agctgtggaa tgtgtgtcag ttagggtgtg 9960
gaaagtcccc aggctcccca gcaggcagaa gtatgcaaag catgcatctc aattagtcag 10020
caaccaggtg tggaaagtcc ccaggctccc cagcaggcag aagtatgcaa agcatgcatc 10080
tcaattagtc agcaaccata gtcccgcccc taactccgcc catcccgccc ctaactccgc 10140
ccagttccgc ccattctccg ccccatggct gactaatttt ttttatttat gcagaggccg 10200
aggccgcctc ggcctctgag ctattccaga agtagtgagg aggctttttt ggaggcctag 10260
gcttttgcaa aaagcttgga cacaagacag gcttgcgaga tatgtttgag aataccactt 10320
tatcccgcgt cagggagagg cagtgcgtaa aaagacgcgg actcatgtga aatactggtt 10380
tttagtgcgc cagatctcta taatctcgcg caacctattt tcccctcgaa cactttttaa 10440
gccgtagata aacaggctgg gacacttcac atgagcgaaa aatacatcgt cacctgggac 10500
atgttgcaga tccatgcacg taaactcgca agccgactga tgccttctga acaatggaaa 10560
ggcattattg ccgtaagccg tggcggtctg taccgggtgc gttactggcg cgtgaactgg 10620
gtattcgtca tgtcgatacc gtttgtattt ccagctacga tcacgacaac cagcgcgagc 10680
ttaaagtgct gaaacgcgca gaaggcgatg gcgaaggctt catcgttatt gatgacctgg 10740
tggataccgg tggtactgcg gttgcgattc gtgaaatgta tccaaaagcg cactttgtca 10800
ccatcttcgc aaaaccggct ggtcgtccgc tggttgatga ctatgttgtt gatatcccgc 10860
aagatacctg gattgaacag ccgtgggata tgggcgtcgt attcgtcccg ccaatctccg 10920
gtcgctaatc ttttcaacgc ctggcactgc cgggcgttgt tctttttaac ttcaggcggg 10980
ttacaatagt ttccagtaag tattctggag gctgcatcca tgacacaggc aaacctgagc 11040
 
gaaaccctgt tcaaaccccg ctttaaacat cctgaaacct cgacgctagt ccgccgcttt 11100
aatcacggcg cacaaccgcc tgtgcagtcg gcccttgatg gtaaaaccat ccctcactgg 11160
tatcgcatga ttaaccgtct gatgtggatc tggcgcggca ttgacccacg cgaaatcctc 11220
gacgtccagg cacgtattgt gatgagcgat gccgaacgta ccgacgatga tttatacgat 11280
acggtgattg gctaccgtgg cggcaactgg atttatgagt gggccccgga tctttgtgaa 11340
ggaaccttac ttctgtggtg tgacataatt ggacaaacta cctacagaga tttaaagctc 11400
taaggtaaat ataaaatttt taagtgtata atgtgttaaa ctactgattc taattgtttg 11460
tgtattttag attccaacct atggaactga tgaatgggag cagtggtgga atgcctttaa 11520
tgaggaaaac ctgttttgct cagaagaaat gccatctagt gatgatgagg ctactgctga 11580
ctctcaacat tctactcctc caaaaaagaa gagaaaggta gaagacccca aggactttcc 11640
ttcagaattg ctaagttttt tgagtcatgc tgtgtttagt aatagaactc ttgcttgctt 11700
tgctatttac accacaaagg aaaaagctgc actgctatac aagaaaatta tggaaaaata 11760
ttctgtaacc tttataagta ggcataacag ttataatcat aacatactgt tttttcttac 11820
tccacacagg catagagtgt ctgctattaa taactatgct caaaaattgt gtacctttag 11880
ctttttaatt tgtaaagggg ttaataagga atatttgatg tatagtgcct tgactagaga 11940
tcataatcag ccataccaca tttgtagagg ttttacttgc tttaaaaaac ctccccacac 12000
ctccccctga acctgaaaca taaaatgaat gcaattgttg ttgttgggct gcagtatgaa 12060
t 12061
SEQ ID NO: 16 
catggccaag ttgaccagtg ccgttccggt gctcaccgcg cgcgacgtcg ccggagcggt 60
cgagttctgg accgaccggc tcgggttctc ccgggacttc gtggaggacg acttcgccgg 120
tgtggtccgg gacgacgtga ccctgttcat cagcgcggtc caggaccagg tggtgccgga 180
caacaccctg gcctgggtgt gggtgcgcgg cctggacgag ctgtacgccg agtggtcgga 240
ggtcgtgtcc acgaacttcc gggacgcctc cgggccggcc atgaccgaga tcggcgagca 300
gccgtggggg cgggagttcg ccctgcgcga cccggccggc aactgcgtgc acttcgtggc 360
cgaggagcag gactgacacg tgctacgaga tttcgattcc accgccgcct tctatgaaag 420
gttgggcttc ggaatcgttt tccgggacgc cggctggatg atcctccagc gcggggatct 480
catgctggag ttcttcgccc accccaactt gtttattgca gcttataatg gttacaaata 540
aagcaatagc atcacaaatt tcacaaataa agcatttttt tcactgcatt ctagttgtgg 600
tttgtccaaa ctcatcaatg tatcttatca tgtctgtata ccgtcgacct ctagctagag 660
cttggcgtaa tcatggtcat agctgtttcc tgtgtgaaat tgttatccgc tcacaattcc 720
acacaacata cgagccggaa gcataaagtg taaagcctgg ggtgcctaat gagtgagcta 780
actcacatta attgcgttgc gctcactgcc cgctttccag tcgggaaacc tgtcgtgcca 840
gctgcattaa tgaatcggcc aacgcgcggg gagaggcggt ttgcgtattg ggcgctcttc 900
cgcttcctcg ctcactgact cgctgcgctc ggtcgttcgg ctgcggcgag cggtatcagc 960
 
tcactcaaag gcggtaatac ggttatccac agaatcaggg gataacgcag gaaagaacat 1020
gtgagcaaaa ggccagcaaa aggccaggaa ccgtaaaaag gccgcgttgc tggcgttttt 1080
ccataggctc cgcccccctg acgagcatca caaaaatcga cgctcaagtc agaggtggcg 1140
aaacccgaca ggactataaa gataccaggc gtttccccct ggaagctccc tcgtgcgctc 1200
tcctgttccg accctgccgc ttaccggata cctgtccgcc tttctccctt cgggaagcgt 1260
ggcgctttct caatgctcac gctgtaggta tctcagttcg gtgtaggtcg ttcgctccaa 1320
gctgggctgt gtgcacgaac cccccgttca gcccgaccgc tgcgccttat ccggtaacta 1380
tcgtcttgag tccaacccgg taagacacga cttatcgcca ctggcagcag ccactggtaa 1440
caggattagc agagcgaggt atgtaggcgg tgctacagag ttcttgaagt ggtggcctaa 1500
ctacggctac actagaagga cagtatttgg tatctgcgct ctgctgaagc cagttacctt 1560
cggaaaaaga gttggtagct cttgatccgg caaacaaacc accgctggta gcggtggttt 1620
ttttgtttgc aagcagcaga ttacgcgcag aaaaaaagga tctcaagaag atcctttgat 1680
cttttctacg gggtctgacg ctcagtggaa cgaaaactca cgttaaggga ttttggtcat 1740
gagattatca aaaaggatct tcacctagat ccttttaaat taaaaatgaa gttttaaatc 1800
aatctaaagt atatatgagt aaacttggtc tgacagttac caatgcttaa tcagtgaggc 1860
acctatctca gcgatctgtc tatttcgttc atccatagtt gcctgactcc ccgtcgtgta 1920
gataactacg atacgggagg gcttaccatc tggccccagt gctgcaatga taccgcgaga 1980
cccacgctca ccggctccag atttatcagc aataaaccag ccagccggaa gggccgagcg 2040
cagaagtggt cctgcaactt tatccgcctc catccagtct attaattgtt gccgggaagc 2100
tagagtaagt agttcgccag ttaatagttt gcgcaacgtt gttgccattg ctacaggcat 2160
cgtggtgtca cgctcgtcgt ttggtatggc ttcattcagc tccggttccc aacgatcaag 2220
gcgagttaca tgatccccca tgttgtgcaa aaaagcggtt agctccttcg gtcctccgat 2280
cgttgtcaga agtaagttgg ccgcagtgtt atcactcatg gttatggcag cactgcataa 2340
ttctcttact gtcatgccat ccgtaagatg cttttctgtg actggtgagt actcaaccaa 2400
gtcattctga gaatagtgta tgcggcgacc gagttgctct tgcccggcgt caatacggga 2460
taataccgcg ccacatagca gaactttaaa agtgctcatc attggaaaac gttcttcggg 2520
gcgaaaactc tcaaggatct taccgctgtt gagatccagt tcgatgtaac ccactcgtgc 2580
acccaactga tcttcagcat cttttacttt caccagcgtt tctgggtgag caaaaacagg 2640
aaggcaaaat gccgcaaaaa agggaataag ggcgacacgg aaatgttgaa tactcatact 2700
cttccttttt caatattatt gaagcattta tcagggttat tgtctcatga gcggatacat 2760
atttgaatgt atttagaaaa ataaacaaat aggggttccg cgcacatttc cccgaaaagt 2820
gccacctgac gtcgacggat cgggagatct cccgatcccc tatggtcgac tctcagtaca 2880
atctgctctg atgccgcata gttaagccag tatctgctcc ctgcttgtgt gttggaggtc 2940
gctgagtagt gcgcgagcaa aatttaagct acaacaaggc aaggcttgac cgacaattgc 3000
atgaagaatc tgcttagggt taggcgtttt gcgctgcttc gcgatgtacg ggccagatat 3060
acgcgttgac attgattatt gactagttat taatagtaat caattacggg gtcattagtt 3120
 
catagcccat atatggagtt ccgcgttaca taacttacgg taaatggccc gcctggctga 3180
ccgcccaacg acccccgccc attgacgtca ataatgacgt atgttcccat agtaacgcca 3240
atagggactt tccattgacg tcaatgggtg gactatttac ggtaaactgc ccacttggca 3300
gtacatcaag tgtatcatat gccaagtacg ccccctattg acgtcaatga cggtaaatgg 3360
cccgcctggc attatgccca gtacatgacc ttatgggact ttcctacttg gcagtacatc 3420
tacgtattag tcatcgctat taccatggtg atgcggtttt ggcagtacat caatgggcgt 3480
ggatagcggt ttgactcacg gggatttcca agtctccacc ccattgacgt caatgggagt 3540
ttgttttggc accaaaatca acgggacttt ccaaaatgtc gtaacaactc cgccccattg 3600
acgcaaatgg gcggtaggcg tgtacggtgg gaggtctata taagcagagc tctctggcta 3660
actagagaac ccactgctta ctggcttatc gaaattaata cgactcacta tagggagacc 3720
caagctggct agcgtttaaa cttaagctta tgaatagagg attctttaac atgctcggcc 3780
gccgcccctt cccggccccc actgccatgt ggaggccgcg gagaaggagg caggcggccc 3840
cgatgcctgc ccgcaacggg ctggcttctc aaatccagca actgaccaca gccgtcagtg 3900
ccctagtcat tggacaggca actagacctc aacccccacg tccacgcccg ccaccgcgcc 3960
agaagaagca ggcgcccaag caaccaccga agccgaagaa accaaaaacg caggagaaga 4020
agaagaagca acctgcaaaa cccaaacccg gaaagagaca gcgcatggca cttaagttgg 4080
aggccgacag attgttcgac gtcaagaacg aggacggaga tgtcatcggg cacgcactgg 4140
ccatggaagg aaaggtaatg aaacctctgc acgtgaaagg aaccatcgac caccctgtgc 4200
tatcaaagct caaatttacc aagtcgtcag catacgacat ggagttcgca cagttgccag 4260
tcaacatgag aagtgaggca ttcacctaca ccagtgaaca ccccgaagga ttctataact 4320
ggcaccacgg agcggtgcag tatagtggag gtagatttac catccctcgc ggagtaggag 4380
gcagaggaga cagcggtcgt ccgatcatgg ataactccgg tcgggttgtc gcgatagtcc 4440
tcggtggagc tgatgaagga acacgaactg ccctttcggt cgtcacctgg aatagtaaag 4500
ggaagacaat taagacgacc ccggaaggga cagaagagtg gtccgcagca ccactggtca 4560
cggcaatgtg tttgctcgga aatgtgagct tcccatgcga ccgcccgccc acatgctata 4620
cccgcgaacc ttccagagcc ctcgacatcc ttgaagagaa cgtgaaccat gaggcctacg 4680
ataccctgct caatgccata ttgcggtgcg gatcgtctgg cagaagcaaa agaagcgtca 4740
ctgacgactt taccctgacc agcccctact tgggcacatg ctcgtactgc caccatactg 4800
aaccgtgctt cagccctgtt aagatcgagc aggtctggga cgaagcggac gataacacca 4860
tacgcataca gacttccgcc cagtttggat acgaccatag cggagcagca agcgcaaaca 4920
agtaccgcta catgtcgctt aagcaggtaa ccgacaacaa attcaacaaa gaacaacaaa 4980
acgcgttcta tgagatctta catttaccta acttaaacga agaacaacga aacgccttca 5040
tccaaagttt aaaagatgac ccaagccaaa gcgctaacct tttagcagaa gctaaaaagc 5100
taaatgatgc tcaggcgccg aaagtagaca acaaattcaa caaagaacaa caaaacgcgt 5160
tctatgagat cttacattta cctaacttaa acgaagaaca acgaaacgcc ttcatccaaa 5220
gtttaaaaga tgacccaagc caaagcgcta accttttagc agaagctaaa aagctaaatg 5280
 
atgctcaggc gccgaaagta gacgcgaatt cgagctcggt acccggggat ccggtaacca 5340
ccgttaaaga aggcaccatg gatgacatca agattagcac ctcaggaccg tgtagaaggc 5400
ttagctacaa aggatacttt ctcctcgcaa aatgccctcc aggggacagc gtaacggtta 5460
gcatagtgag tagcaactca gcaacgtcat gtacactggc ccgcaagata aaaccaaaat 5520
tcgtgggacg ggaaaaatat gatctacctc ccgttcacgg taaaaaaatt ccttgcacag 5580
tgtacgaccg tctgaaagaa acaactgcag gctacatcac tatgcacagg ccgggaccgc 5640
acgcttatac atcctacctg gaagaatcat cagggaaagt ttacgcaaag ccgccatctg 5700
ggaagaacat tacgtatgag tgcaagtgcg gcgactacaa gaccggaacc gtttcgaccc 5760
gcaccgaaat cactggttgc accgccatca agcagtgcgt cgcctataag agcgaccaaa 5820
cgaagtgggt cttcaactca ccggacttga tcagacatga cgaccacacg gcccaaggga 5880
aattgcattt gcctttcaag ttgatcccga gtacctgcat ggtccctgtt gcccacgcgc 5940
cgaatgtaat acatggcttt aaacacatca gcctccaatt agatacagac cacttgacat 6000
tgctcaccac caggagacta ggggcaaacc cggaaccaac cactgaatgg atcgtcggaa 6060
agacggtcag aaacttcacc gtcgaccgag atggcctgga atacatatgg ggaaatcatg 6120
agccagtgag ggtctatgcc caagagtcag caccaggaga ccctcacgga tggccacacg 6180
aaatagtaca gcattactac catcgccatc ctgtgtacac catcttagcc gtcgcatcag 6240
ctaccgtggc gatgatgatt ggcgtaactg ttgcagtgtt atgtgcctgt aaagcgcgcc 6300
gtgagtgcct gacgccatac gccctggccc caaacgccgt aatcccaact tcgctggcac 6360
tcttgtgctg cgttaggtcg gccaatgctg aaacgttcac cgagaccatg agttacttgt 6420
ggtcgaacag tcagccgttc ttctgggtcc agttgtgcat acctttggcc gctttcatcg 6480
ttctaatgcg ctgctgctcc tgctgcctgc cttttttagt ggttgccggc gcctacctgg 6540
cgaaggtaga cgcctacgaa catgcgacca ctgttccaaa tgtgccacag ataccgtata 6600
aggcacttgt tgaaagggca gggtatgccc cgctcaattt ggagatcact gtcatgtcct 6660
cggaggtttt gccttccacc aaccaagagt acattacctg caaattcacc actgtggtcc 6720
cctccccaaa aatcaaatgc tgcggctcct tggaatgtca gccggccgct catgcagact 6780
atacctgcaa ggtcttcgga ggggtctacc cctttatgtg gggaggagcg caatgttttt 6840
gcgacagtga gaacagccag atgagtgagg cgtacgtcga attgtcagca gattgcgcgt 6900
ctgaccacgc gcaggcgatt aaggtgcaca ctgccgcgat gaaagtagga ctgcgtatag 6960
tgtacgggaa cactaccagt ttcctagatg tgtacgtgaa cggagtcaca ccaggaacgt 7020
ctaaagactt gaaagtcata gctggaccaa tttcagcatc atttacgcca ttcgatcata 7080
aggtcgttat ccatcgcggc ctggtgtaca actatgactt cccggaatat ggagcgatga 7140
aaccaggagc gtttggagac attcaagcta cctccttgac tagcaaggat ctcatcgcca 7200
gcacagacat taggctactc aagccttccg ccaagaatgt gcatgtcccg tacacgcagg 7260
ccgcatcagg atttgagatg tggaaaaaca actcaggccg cccattgcag gaaaccgcac 7320
ctttcgggtg taagattgca gtaaatccgc tccgagcggt ggactgttca tacgggaaca 7380
ttcccatttc tattgacatc ccgaacgctg cctttatcag gacatcagat gcaccactgg 7440
 
tctcaacagt caaatgtgaa gtcagtgagt gcacttattc agcagacttc gacgggatgg 7500
ccaccctgca gtatgtatcc gaccgcgaag gtcaatgccc cgtacattcg cattcgagca 7560
cagcaactct ccaagagtcg acagtacatg tcctggagaa aggagcggtg acagtacact 7620
ttagcaccgc gagtccacag gcgaacttta tcgtatcgct gtgtgggaag aagacaacat 7680
gcaatgcaga atgtaaacca ccagctgacc atatcgtgag caccccgcac aaaaatgacc 7740
aagaatttca agccgccatc tcaaaaacat catggagttg gctgtttgcc cttttcggcg 7800
gcgcctcgtc gctattaatt ataggactta tgatttttgc ttgcagcatg atgctgacta 7860
gcacacgaag atgacggccc cgtttaaacc cgctgatcag cctcgactgt gccttctagt 7920
tgccagccat ctgttgtttg cccctccccc gtgccttcct tgaccctgga aggtgccact 7980
cccactgtcc tttcctaata aaatgaggaa attgcatcgc attgtctgag taggtgtcat 8040
tctattctgg ggggtggggt ggggcaggac agcaaggggg aggattggga agacaatagc 8100
aggcatgctg gggatgcggt gggctctatg gcttctgagg cggaaagaac cagctggggc 8160
tctagggggt atccccacgc gccctgtagc ggcgcattaa gcgcggcggg tgtggtggtt 8220
acgcgcagcg tgaccgctac acttgccagc gccctagcgc ccgctccttt cgctttcttc 8280
ccttcctttc tcgccacgtt cgccggcttt ccccgtcaag ctctaaatcg gggcatccct 8340
ttagggttcc gatttagtgc tttacggcac ctcgacccca aaaaacttga ttagggtgat 8400
ggttcacgta gtgggccatc gccctgatag acggtttttc gccctttgac gttggagtcc 8460
acgttcttta atagtggact cttgttccaa actggaacaa cactcaaccc tatctcggtc 8520
tattcttttg atttataagg gattttgggg atttcggcct attggttaaa aaatgagctg 8580
atttaacaaa aatttaacgc gaattaattc tgtggaatgt gtgtcagtta gggtgtggaa 8640
agtccccagg ctccccaggc aggcagaagt atgcaaagca tgcatctcaa ttagtcagca 8700
accaggtgtg gaaagtcccc aggctcccca gcaggcagaa gtatgcaaag catgcatctc 8760
aattagtcag caaccatagt cccgccccta actccgccca tcccgcccct aactccgccc 8820
agttccgccc attctccgcc ccatggctga ctaatttttt ttatttatgc agaggccgag 8880
gccgcctctg cctctgagct attccagaag tagtgaggag gcttttttgg aggcctaggc 8940
ttttgcaaaa agctcccggg agcttgtata tccattttcg gatctgatca gcacgtgttg 9000
acaattaatc atcggcatag tatatcggca tagtataata cgacaaggtg aggaactaaa 9060
c 9061
SEQ ID NO: 17 
atgtccgcag caccactggt cacggcaatg tgtttgctcg gaaatgtgag cttcccatgc 60
gaccgcccgc ccacatgcta tacccgcgaa ccttccagag ccctcgacat ccttgaagag 120
aacgtgaacc atgaggccta cgataccctg ctcaatgcca tattgcggtg cggatcgtct 180
ggcagaagca aaaga 195
SEQ ID NO: 18 
agcgtcactg acgactttac cctgaccagc ccctacttgg gcacatgctc gtactgccac 60
catactgaac cgtgcttcag ccctgttaag atcgagcagg tctgggacga agcggacgat 120
aacaccatac gcatacagac ttccgcccag tttggatacg accatagcgg agcagcaagc 180
gcaaacaagt accgctacat gtcgcttaag caggtaacca acgtagacaa caaattcaac 240
aaagaacaac aaaacgcgtt ctatgagatc ttacatttac ctaacttaaa cgaagaacaa 300
cgaaacgcct tcatccaaag tttaaaagat gacccaagcc aaagcgctaa ccttttagca 360
gaagctaaaa agctaaatga tgctcaggcg ccgaaagtag acaacaaatt caacaaagaa 420
caacaaaacg cgttctatga gatcttacat ttacctaact taaacgaaga acaacgaaac 480
gccttcatcc aaagtttaaa agatgaccca agccaaagcg ctaacctttt agcagaagct 540
aaaaagctaa atgatgctca ggcgccgaaa gtagacgcga attcgagctc ggtacccgta 600
accaccgtta aagaaggcac catggatgac atcaagatta gcacctcagg accgtgtaga 660
aggcttagct acaaaggata ctttctcctc gcaaaatgcc ctccagggga cagcgtaacg 720
gttagcatag tgagtagcaa ctcagcaacg tcatgtacac tggcccgcaa gataaaacca 780
aaattcgtgg gacgggaaaa atatgatcta cctcccgttc acggtaaaaa aattccttgc 840
acagtgtacg accgtctgaa agaaacaact gcaggctaca tcactatgca caggccggga 900
ccgcacgctt atacatccta cctggaagaa tcatcaggga aagtttacgc aaagccgcca 960
tctgggaaga acattacgta tgagtgcaag tgcggcgact acaagaccgg aaccgtttcg 1020
acccgcaccg aaatcactgg ttgcaccgcc atcaagcagt gcgtcgccta taagagcgac 1080
caaacgaagt gggtcttcaa ctcaccggac ttgatcagac atgacgacca cacggcccaa 1140
gggaaattgc atttgccttt caagttgatc ccgagtacct gcatggtccc tgttgcccac 1200
gcgccgaatg taatacatgg ctttaaacac atcagcctcc aattagatac agaccacttg 1260
acattgctca ccaccaggag actaggggca aacccggaac caaccactga atggatcgtc 1320
ggaaagacgg tcagaaactt caccgtcgac cgagatggcc tggaatacat atggggaaat 1380
catgagccag tgagggtcta tgcccaagag tcagcaccag gagaccctca cggatggcca 1440
cacgaaatag tacagcatta ctaccatcgc catcctgtgt acaccatctt agccgtcgca 1500
tcagctaccg tggcgatgat gattggcgta actgttgcag tgttatgtgc ctgtaaagcg 1560
cgccgtgagt gcctgacgcc atacgccctg gccccaaacg ccgtaatccc aacttcgctg 1620
gcactcttgt gctgcgttag gtcggccaat gct 1653
SEQ ID NO: 19 
gaaacgttca ccgagaccat gagttacttg tggtcgaaca gtcagccgtt cttctgggtc 60
cagttgtgca tacctttggc cgctttcatc gttctaatgc gctgctgctc ctgctgcctg 120
ccttttttag tggttgccgg cgcctacctg gcgaaggtag acgcc 165
SEQ ID NO: 20 
tacgaacatg cgaccactgt tccaaatgtg ccacagatac cgtataaggc acttgttgaa 60
agggcagggt atgccccgct caatttggag atcactgtca tgtcctcgga ggttttgcct 120
tccaccaacc aagagtacat tacctgcaaa ttcaccactg tggtcccctc cccaaaaatc 180
aaatgctgcg gctccttgga atgtcagccg gccgctcatg cagactatac ctgcaaggtc 240
ttcggagggg tctacccctt tatgtgggga ggagcgcaat gtttttgcga cagtgagaac 300
agccagatga gtgaggcgta cgtcgaattg tcagcagatt gcgcgtctga ccacgcgcag 360
gcgattaagg tgcacactgc cgcgatgaaa gtaggactgc gtatagtgta cgggaacact 420
accagtttcc tagatgtgta cgtgaacgga gtcacaccag gaacgtctaa agacttgaaa 480
gtcatagctg gaccaatttc agcatcattt acgccattcg atcataaggt cgttatccat 540
cgcggcctgg tgtacaacta tgacttcccg gaatatggag cgatgaaacc aggagcgttt 600
ggagacattc aagctacctc cttgactagc aaggatctca tcgccagcac agacattagg 660
ctactcaagc cttccgccaa gaatgtgcat gtcccgtaca cgcaggccgc atcaggattt 720
gagatgtgga aaaacaactc aggccgccca ttgcaggaaa ccgcaccttt cgggtgtaag 780
attgcagtaa atccgctccg agcggtggac tgttcatacg ggaacattcc catttctatt 840
gacatcccga acgctgcctt tatcaggaca tcagatgcac cactggtctc aacagtcaaa 900
tgtgaagtca gtgagtgcac ttattcagca gacttcgacg ggatggccac cctgcagtat 960
gtatccgacc gcgaaggtca atgccccgta cattcgcatt cgagcacagc aactctccaa 1020
gagtcgacag tacatgtcct ggagaaagga gcggtgacag tacactttag caccgcgagt 1080
ccacaggcga actttatcgt atcgctgtgt gggaagaaga caacatgcaa tgcagaatgt 1140
aaaccaccag ctgaccatat cgtgagcacc ccgcacaaaa atgaccaaga atttcaagcc 1200
gccatctcaa aaacatcatg gagttggctg tttgcccttt tcggcggcgc ctcgtcgcta 1260
ttaattatag gacttatgat ttttgcttgc agcatgatgc tgactagcac acgaagatga 1320
SEQ ID NO: 21 
caggtggcacttttcggggaaatgtgcgcggaacccctatttgtttatttttctaaatacattcaaatatgtatccg
ctcatgagacaataaccctgataaatgcttcaataatattgaaaaaggaagagtatgagtattcaacatttccgtgt
cgcccttattcccttttttgcggcattttgccttcctgtttttgctcacccagaaacgctggtgaaagtaaaagatg
ctgaagatcagttgggtgcacgagtgggttacatcgaactggatctcaacagcggtaagatccttgagagttttcgc
cccgaagaacgttttccaatgatgagcacttttaaagttctgctatgtggcgcggtattatcccgtattgacgccgg
gcaagagcaactcggtcgccgcatacactattctcagaatgacttggttgagtactcaccagtcacagaaaagcatc
ttacggatggcatgacagtaagagaattatgcagtgctgccataaccatgagtgataacactgcggccaacttactt
ctgacaacgatcggaggaccgaaggagctaaccgcttttttgcacaacatgggggatcatgtaactcgccttgatcg
ttgggaaccggagctgaatgaagccataccaaacgacgagcgtgacaccacgatgcctgtagcaatggcaacaacgt
tgcgcaaactattaactggcgaactacttactctagcttcccggcaacaattaatagactggatggaggcggataaa
gttgcaggaccacttctgcgctcggcccttccggctggctggtttattgctgataaatctggagccggtgagcgtgg
 
gtctcgcggtatcattgcagcactggggccagatggtaagccctcccgtatcgtagttatctacacgacggggagtc
aggcaactatggatgaacgaaatagacagatcgctgagataggtgcctcactgattaagcattggtaactgtcagac
caagtttactcatatatactttagattgatttaaaacttcatttttaatttaaaaggatctaggtgaagatcctttt
tgataatctcatgaccaaaatcccttaacgtgagttttcgttccactgagcgtcagaccccgtagaaaagatcaaag
gatcttcttgagatcctttttttctgcgcgtaatctgctgcttgcaaacaaaaaaaccaccgctaccagcggtggtt
tgtttgccggatcaagagctaccaactctttttccgaaggtaactggcttcagcagagcgcagataccaaatactgt
ccttctagtgtagccgtagttaggccaccacttcaagaactctgtagcaccgcctacatacctcgctctgctaatcc
tgttaccagtggctgctgccagtggcgataagtcgtgtcttaccgggttggactcaagacgatagttaccggataag
gcgcagcggtcgggctgaacggggggttcgtgcacacagcccagcttggagcgaacgacctacaccgaactgagata
cctacagcgtgagctatgagaaagcgccacgcttcccgaagggagaaaggcggacaggtatccggtaagcggcaggg
tcggaacaggagagcgcacgagggagcttccagggggaaacgcctggtatctttatagtcctgtcgggtttcgccac
ctctgacttgagcgtcgatttttgtgatgctcgtcaggggggcggagcctatggaaaaacgccagcaacgcggcctt
tttacggttcctggccttttgctggccttttgctcacatgttctttcctgcgttatcccctgattctgtggataacc
gtattaccgcctttgagtgagctgataccgctcgccgcagccgaacgaccgagcgcagcgagtcagtgagcgaggaa
gcggaagagcgcccaatacgcaaaccgcctctccccgcgcgttggccgattcattaatgcagctggcacgacaggtt
tcccgactggaaagcgggcagtgagcgcaacgcaattaatgtgagttagctcactcattaggcaccccaggctttac
actttatgcttccggctcgtatgttgtgtggaattgtgagcggataacaatttcacacaggaaacagctatgaccat
gattacgccaagcgcgcaattaaccctcactaaagggaacaaaagctggagctgcaagcttggccattgcatacgtt
gtatccatatcataatatgtacatttatattggctcatgtccaacattaccgccatgttgacattgattattgacta
gttattaatagtaatcaattacggggtcattagttcatagcccatatatggagttccgcgttacataacttacggta
aatggcccgcctggctgaccgcccaacgacccccgcccattgacgtcaataatgacgtatgttcccatagtaacgcc
aatagggactttccattgacgtcaatgggtggagtatttacggtaaactgcccacttggcagtacatcaagtgtatc
atatgccaagtacgccccctattgacgtcaatgacggtaaatggcccgcctggcattatgcccagtacatgacctta
tgggactttcctacttggcagtacatctacgtattagtcatcgctattaccatggtgatgcggttttggcagtacat
caatgggcgtggatagcggtttgactcacggggatttccaagtctccaccccattgacgtcaatgggagtttgtttt
ggcaccaaaatcaacgggactttccaaaatgtcgtaacaactccgccccattgacgcaaatgggcggtaggcgtgta
cggtgggaggtctatataagcagagctcgtttagtgaaccgggtctctctggttagaccagatctgagcctgggag
ctctctggctaactagggaacccactgcttaagcctcaataaagcttgccttgagtgcttcaagtagtgtgtgcccg
tctgttgtgtgactctggtaactagagatccctcagacccttttagtcagtgtggaaaatctctagcagtggcgccc
gaacagggacctgaaagcgaaagggaaaccagaggagctctctcgacgcaggactcggcttgctgaagcgcgcacgg
caagaggcgaggggcggcgactggtgagtacgccaaaaattttgactagcggaggctagaaggagagagatgggtgc
gagagcgtcagtattaagcgggggagaattagatcgcgatgggaaaaaattcggttaaggccagggggaaagaaaaa
atataaattaaaacatatagtatgggcaagcagggagctagaacgattcgcagttaatcctggcctgttagaaacat
cagaaggctgtagacaaatactgggacagctacaaccatcccttcagacaggatcagaagaacttagatcattatat
aatacagtagcaaccctctattgtgtgcatcaaaggatagagataaaagacaccaaggaagctttagacaagataga
ggaagagcaaaacaaaagtaagaccaccgcacagcaagcggccgctgatcttcagacctggaggaggagatatgagg
 
gacaattggagaagtgaattatataaatataaagtagtaaaaattgaaccattaggagtagcacccaccaaggcaaa
gagaagagtggtgcagagagaaaaaagagcagtgggaataggagctttgttccttgggttcttgggagcagcaggaa
gcactatgggcgcagcctcaatgacgctgacggtacaggccagacaattattgtctggtatagtgcagcagcagaac
aatttgctgagggctattgaggcgcaacagcatctgttgcaactcacagtctggggcatcaagcagctccaggcaag
aatcctggctgtggaaagatacctaaaggatcaacagctcctggggatttggggttgctctggaaaactcatttgca
ccactgctgtgccttggaatgctagttggagtaataaatctctggaacagattggaatcacacgacctggatggagt
gggacagagaaattaacaattacacaagcttaatacactccttaattgaagaatcgcaaaaccagcaagaaaagaat
gaacaagaattattggaattagataaatgggcaagtttgtggaattggtttaacataacaaattggctgtggtatat
aaaattattcataatgatagtaggaggcttggtaggtttaagaatagtttttgctgtactttctatagtgaatagag
ttaggcagggatattcaccattatcgtttcagacccacctcccaaccccgaggggacccgacaggcccgaaggaata
gaagaagaaggtggagagagagacagagacagatccattcgattagtgaacggatctcgacggtatcgataagctaa
ttcacaaatggcagtattcatccacaattttaaaagaaaaggggggattggggggtacagtgcaggggaaagaatag
tagacataatagcaacagacatacaaactaaagaattacaaaaacaaattacaaaaattcaaaattttcgggtttat
tacagggacagcagagatccagtttgggaattagcttgatcgattagtccaatttgttaaagacaggatatcagtgg
tccaggctctagttttgactcaacaatatcaccagctgaagcctatagagtacgagccatagatagaataaaagatt
ttatttagtctccagaaaaaggggggaatgaaagaccccacctgtaggtttggcaagctaggatcaaggttaggaac
agagagacagcagaatatgggccaaacaggatatctgtggtaagcagttcctgccccggctcagggccaagaacagt
tggaacagcagaatatgggccaaacaggatatctgtggtaagcagttcctgccccggctcagggccaagaacagatg
gtccccagatgcggtcccgccctcagcagtttctagagaaccatcagatgtttccagggtgccccaaggacctgaaa
tgaccctgtgccttatttgaactaaccaatcagttcgcttctcgcttctgttcgcgcgcttctgctccccgagctca
ataaaagagcccacaacccctcactcggcgcgatctagatctcgaatcgaattcgcccttGATCCACCGGTCGCCAC
CATGGTGAGCAAGGGCGAGGAGCTGTTCACCGGGGTGGTGCCCATCCTGGTCGAGCTGGACGGCGACGTAAACGGCC
ACAAGTTCAGCGTGTCCGGCGAGGGCGAGGGCGATGCCACCTACGGCAAGCTGACCCTGAAGTTCATCTGCACCACC
GGCAAGCTGCCCGTGCCCTGGCCCACCCTCGTGACCACCCTGACCTACGGCGTGCAGTGCTTCAGCCGCTACCCCGA
CCACATGAAGCAGCACGACTTCTTCAAGTCCGCCATGCCCGAAGGCTACGTCCAGGAGCGCACCATCTTCTTCAAGG
ACGACGGCAACTACAAGACCCGCGCCGAGGTGAAGTTCGAGGGCGACACCCTGGTGAACCGCATCGAGCTGAAGGGC
ATCGACTTCAAGGAGGACGGCAACATCCTGGGGCACAAGCTGGAGTACAACTACAACAGCCACAACGTCTATATCAT
GGCCGACAAGCAGAAGAACGGCATCAAGGTGAACTTCAAGATCCGCCACAACATCGAGGACGGCAGCGTGCAGCTCG
CCGACCACTACCAGCAGAACACCCCCATCGGCGACGGCCCCGTGCTGCTGCCCGACAACCACTACCTGAGCACCCAG
TCCGCCCTGAGCAAAGACCCCAACGAGAAGCGCGATCACATGGTCCTGCTGGAGTTCGTGACCGCCGCCGGGATCAC
TCTCGGCATGGACGAGCTGTACAAGTAAAGCGGCCTACCGGGTAGGGGAGGCGCTTTTCCCAAGGCAGTCTGGAGCA
TGCGCTTTAGCAGCCCCGCTGGCACTTGGCGCTACACAAGTGGCCTCTGGCCTCGCACACATTCCACATCCACCGGT
AGGCGCCAACCGGCTCCGTTCTTTGGTGGCCCCTTCGCGCCACCTTCTACTCCTCCCCTAGTCAGGAAGTTCCCCCC
CGCCCCGCAGCTCGCGTCGTGCAGGACGTGACAAATGGAAGTAGCACGTCTCACTAGTCTCGTGCAGATGGACAGCA
CCGCTGAGCAATGGAAGCGGGTAGGCCTTTGGGGCAGCGGCCAATAGCAGCTTTGCTCCTTCGCTTTCTGGGCTCAG
AGGCTGGGAAGGGGTGGGTCCGGGGGCGGGCTCAGGGGCGGGCTCAGGGGCGGGGCGGGCGCCCGAAGGTCCTCCGG
 
AGGCCCGGCATTCTCGCACGCTTCAAAAGCGCACGTCTGCCGCGCTGTTCTCCTCTTCCTCATCTCCGGGCCTTTCG
ACCATCTAATGGATTCATACCTGCTGATGTGGGGACTGCTCACGTTCATCATGGTGCCTGGCTGCCAGGCAGAGCTC
TGTGACGATGACCCGCCAGAGATCCCACACGCCACATTCAAAGCCATGGCCTACAAGGAAGGAACCATGTTGAACTG
TGAATGCAAGAGAGGTTTCCGCAGAATAAAAAGCGGGTCACTCTATATGCTCTGTACAGGAAACTCTAGCCACTCGT
CCTGGGACAACCAATGTCAATGCACAAGCTCTGCCACTCGGAACACAACGAAACAAGTGACACCTCAACCTGAAGAA
CAGAAAGAAAGGAAAACCACAGAAATGCAAAGTCCAATGCAGCCAGTGGACCAAGCGAGCCTTCCAGGTCACTGCAG
GGAACCTCCACCATGGGAAAATGAAGCCACAGAGAGAATTTATCATTTCGTGGTGGGGCAGATGGTTTATTATCAGT
GCGTCCAGGGATACAGGGCTCTACACAGAGGTCCTGCTGAGAGCGTCTGCAAAATGACCCACGGGAAGACAAGGTGG
ACCCAGCCCCAGCTCATATGCACAGGTGAAATGGAGACCAGTCAGTTTCCAGGTGAAGAGAAGCCTCAGGCAAGCCC
CGAAGGCCGTCCTGAGAGTGAGACTTCCTGCCTCGTCACAACAACAGATTTTCAAATACAGACAGAAATGGCTGCAA
CCATGGAGACGTCCATATTTACAACAGAGTACCAGGTAGCAGTGGCCGGCTGTGTTTTCCTGCTGATCAGCGTCCTC
CTCCTGAGTGGGCTCACCTGGCAGCGGAGACAGAGGAAGAGTAGAAGAACAATCTGATGAgttaacctcgagggatc
cccggggtcgactgatcaaattcgagctcggtacctttaagaccaatgacttacaaggcagctgtagatcttagcc
actttttaaaagaaaaggggggactggaagggctaattcactcccaacgaagacaagatctgctttttgcttgtact
gggtctctctggttagaccagatctgagcctgggagctctctggctaactagggaacccactgcttaagcctcaata
aagcttgccttgagtgcttcaagtagtgtgtgcccgtctgttgtgtgactctggtaactagagatccctcagaccct
tttagtcagtgtggaaaatctctagcagtagtagttcatgtcatcttattattcagtatttataacttgcaaagaaa
tgaatatcagagagtgagaggaacttgtttattgcagcttataatggttacaaataaagcaatagcatcacaaattt
cacaaataaagcatttttttcactgcattctagttgtggtttgtccaaactcatcaatgtatcttatcatgtctggc
tctagctatcccgcccctaactccgcccatcccgcccctaactccgcccagttccgcccattctccgccccatggct
gactaattttttttatttatgcagaggccgaggccgcctcggcctctgagctattccagaagtagtgaggaggcttt
tttggaggcctaggcttttgcgtcgagacgtacccaattcgccctatagtgagtcgtattacgcgcgctcactggcc
gtcgttttacaacgtcgtgactgggaaaaccctggcgttacccaacttaatcgccttgcagcacatccccctttcgc
cagctggcgtaatagcgaagaggcccgcaccgatcgcccttcccaacagttgcgcagcctgaatggcgaatggcgcg
acgcgccctgtagcggcgcattaagcgcggcgggtgtggtggttacgcgcagcgtgaccgctacacttgccagcgcc
ctagcgcccgctcctttcgctttcttcccttcctttctcgccacgttcgccggctttccccgtcaagctctaaatcg
ggggctccctttagggttccgatttagtgctttacggcacctcgaccccaaaaaacttgattagggtgatggttcac
gtagtgggccatcgccctgatagacggtttttcgccctttgacgttggagtccacgttctttaatagtggactcttg
ttccaaactggaacaacactcaaccctatctcggtctattcttttgatttataaggatttgccgatttcggccta
ttggttaaaaaatgagctgatttaacaaaaatttaacgcgaattttaacaaaatattaacgtttacaatttcc-
9897
SEQ ID NO: 22 
caggtggcacttttcggggaaatgtgcgcggaacccctatttgtttatttttctaaatacattcaaatatgtatccg
ctcatgagacaataaccctgataaatgcttcaataatattgaaaaaggaagagtatgagtattcaacatttccgtgt
cgcccttattcccttttttgcggcattttgccttcctgtttttgctcacccagaaacgctggtgaaagtaaaagatg
 
ctgaagatcagttgggtgcacgagtgggttacatcgaactggatctcaacagcggtaagatccttgagagttttcgc
cccgaagaacgttttccaatgatgagcacttttaaagttctgctatgtggcgcggtattatcccgtattgacgccgg
gcaagagcaactcggtcgccgcatacactattctcagaatgacttggttgagtactcaccagtcacagaaaagcatc
ttacggatggcatgacagtaagagaattatgcagtgctgccataaccatgagtgataacactgcggccaacttactt
ctgacaacgatcggaggaccgaaggagctaaccgcttttttgcacaacatgggggatcatgtaactcgccttgatcg
ttgggaaccggagctgaatgaagccataccaaacgacgagcgtgacaccacgatgcctgtagcaatggcaacaacgt
tgcgcaaactattaactggcgaactacttactctagcttcccggcaacaattaatagactggatggaggcggataaa
gttgcaggaccacttctgcgctcggcccttccggctggctggtttattgctgataaatctggagccggtgagcgtgg
gtctcgcggtatcattgcagcactggggccagatggtaagccctcccgtatcgtagttatctacacgacggggagtc
aggcaactatggatgaacgaaatagacagatcgctgagataggtgcctcactgattaagcattggtaactgtcagac
caagtttactcatatatactttagattgatttaaaacttcatttttaatttaaaaggatctaggtgaagatcctttt
tgataatctcatgaccaaaatcccttaacgtgagttttcgttccactgagcgtcagaccccgtagaaaagatcaaag
gatcttcttgagatcctttttttctgcgcgtaatctgctgcttgcaaacaaaaaaaccaccgctaccagcggtggtt
tgtttgccggatcaagagctaccaactctttttccgaaggtaactggcttcagcagagcgcagataccaaatactgt
ccttctagtgtagccgtagttaggccaccacttcaagaactctgtagcaccgcctacatacctcgctctgctaatcc
tgttaccagtggctgctgccagtggcgataagtcgtgtcttaccgggttggactcaagacgatagttaccggataag
gcgcagcggtcgggctgaacggggggttcgtgcacacagcccagcttggagcgaacgacctacaccgaactgagata
cctacagcgtgagctatgagaaagcgccacgcttcccgaagggagaaaggcggacaggtatccggtaagcggcaggg
tcggaacaggagagcgcacgagggagcttccagggggaaacgcctggtatctttatagtcctgtcgggtttcgccac
ctctgacttgagcgtcgatttttgtgatgctcgtcaggggggcggagcctatggaaaaacgccagcaacgcggcctt
tttacggttcctggccttttgctggccttttgctcacatgttctttcctgcgttatcccctgattctgtggataacc
gtattaccgcctttgagtgagctgataccgctcgccgcagccgaacgaccgagcgcagcgagtcagtgagcgaggaa
gcggaagagcgcccaatacgcaaaccgcctctccccgcgcgttggccgattcattaatgcagctggcacgacaggtt
tcccgactggaaagcgggcagtgagcgcaacgcaattaatgtgagttagctcactcattaggcaccccaggctttac
actttatgcttccggctcgtatgttgtgtggaattgtgagcggataacaatttcacacaggaaacagctatgaccat
gattacgccaagcgcgcaattaaccctcactaaagggaacaaaagctggagctgcaagcttggccattgcatacgtt
gtatccatatcataatatgtacatttatattggctcatgtccaacattaccgccatgttgacattgattattgacta
gttattaatagtaatcaattacggggtcattagttcatagcccatatatggagttccgcgttacataacttacggta
aatggcccgcctggctgaccgcccaacgacccccgcccattgacgtcaataatgacgtatgttcccatagtaacgcc
aatagggactttccattgacgtcaatgggtggagtatttacggtaaactgcccacttggcagtacatcaagtgtatc
atatgccaagtacgccccctattgacgtcaatgacggtaaatggcccgcctggcattatgcccagtacatgacctta
tgggactttcctacttggcagtacatctacgtattagtcatcgctattaccatggtgatgcggttttggcagtacat
caatgggcgtggatagcggtttgactcacggggatttccaagtctccaccccattgacgtcaatgggagtttgtttt
ggcaccaaaatcaacgggactttccaaaatgtcgtaacaactccgccccattgacgcaaatgggcggtaggcgtgta
cggtgggaggtctatataagcagagctcgtttagtgaaccggggtctctctggttagaccagatctgagcctgggag
ctctctggctaactagggaacccactgcttaagcctcaataaagcttgccttgagtgcttcaagtagtgtgtgcccg
 
tctgttgtgtgactctggtaactagagatccctcagacccttttagtcagtgtggaaaatctctagcagtggcgccc
gaacagggacctgaaagcgaaagggaaaccagaggagctctctcgacgcaggactcggcttgctgaagcgcgcacgg
caagaggcgaggggcggcgactggtgagtacgccaaaaattttgactagcggaggctagaaggagagagatgggtgc
gagagcgtcagtattaagcgggggagaattagatcgcgatgggaaaaaattcggttaaggccagggggaaagaaaaa
atataaattaaaacatatagtatgggcaagcagggagctagaacgattcgcagttaatcctggcctgttagaaacat
cagaaggctgtagacaaatactgggacagctacaaccatcccttcagacaggatcagaagaacttagatcattatat
aatacagtagcaaccctctattgtgtgcatcaaaggatagagataaaagacaccaaggaagctttagacaagataga
ggaagagcaaaacaaaagtaagaccaccgcacagcaagcggccgctgatcttcagacctggaggaggagatatgagg
gacaattggagaagtgaattatataaatataaagtagtaaaaattgaaccattaggagtagcacccaccaaggcaaa
gagaagagtggtgcagagagaaaaaagagcagtgggaataggagctttgttccttgggttcttgggagcagcaggaa
gcactatgggcgcagcctcaatgacgctgacggtacaggccagacaattattgtctggtatagtgcagcagcagaac
aatttgctgagggctattgaggcgcaacagcatctgttgcaactcacagtctggggcatcaagcagctccaggcaag
aatcctggctgtggaaagatacctaaaggatcaacagctcctggggatttggggttgctctggaaaactcatttgca
ccactgctgtgccttggaatgctagttggagtaataaatctctggaacagattggaatcacacgacctggatggagt
gggacagagaaattaacaattacacaagcttaatacactccttaattgaagaatcgcaaaaccagcaagaaaagaat
gaacaagaattattggaattagataaatgggcaagtttgtggaattggtttaacataacaaattggctgtggtatat
aaaattattcataatgatagtaggaggcttggtaggtttaagaatagtttttgctgtactttctatagtgaatagag
ttaggcagggatattcaccattatcgtttcagacccacctcccaaccccgaggggacccgacaggcccgaaggaata
gaagaagaaggtggagagagagacagagacagatccattcgattagtgaacggatctcgacggtatcgataagctaa
ttcacaaatggcagtattcatccacaattttaaaagaaaaggggggattggggggtacagtgcaggggaaagaatag
tagacataatagcaacagacatacaaactaaagaattacaaaaacaaattacaaaaattcaaaattttcgggtttat
tacagggacagcagagatccagtttgggaattagcttgatcgattagtccaatttgttaaagacaggatatcagtgg
tccaggctctagttttgactcaacaatatcaccagctgaagcctatagagtacgagccatagatagaataaaagatt
ttatttagtctccagaaaaaggggggaatgaaagaccccacctgtaggtttggcaagctaggatcaaggttaggaac
agagagacagcagaatatgggccaaacaggatatctgtggtaagcagttcctgccccggctcagggccaagaacagt
tggaacagcagaatatgggccaaacaggatatctgtggtaagcagttcctgccccggctcagggccaagaacagatg
gtccccagatgcggtcccgccctcagcagtttctagagaaccatcagatgtttccagggtgccccaaggacctgaaa
tgaccctgtgccttatttgaactaaccaatcagttcgcttctcgcttctgttcgcgcgcttctgctccccgagctca
ataaaagagcccacaacccctcactcggcgcgatctagatctcgaatcgaattcgcccttgctactATGGCTTCTGG
AATCCTGGTTAATGTAAAGGAGGAGGTGACCTGCCCCATCTGCCTGGAACTCCTGACACAACCCCTGAGCCTGGACT
GCGGCCACAGCTTCTGCCAAGCATGCCTCACTGCAAACCACAAGAAGTCCATGCTAGACAAAGGAGAGAGTAGCTGC
CCTGTGTGCCGGATCAGTTACCAGCCTGAGAACATACGGCCTAATCGGCATGTAGCCAACATAGTGGAGAAGCTCAG
GGAGGTCAAGTTGAGCCCAGAGGGGCAGAAAGTTGATCATTGTGCACGCCATGGAGAGAAACTTCTACTCTTCTGTC
AGGAGGACGGGAAGGTCATTTGCTGGCTTTGTGAGCGGTCTCAGGAGCACCGTGGTCACCACACGTTCCTCACAGAG
GAGGTTGCCCGGGAGTACCAAGTGAAGCTCCAGGCAGCTCTGGAGATGCTGAGGCAGAAGCAGCAGGAAGCTGAAGA
GTTAGAAGCTGACATCAGAGAAGAGAAAGCTTCCTGGAAGACTCAAATACAGTATGACAAAACCAACGTCTTGGCAG
 
ATTTTGAGCAACTGAGAGACATCCTGGACTGGGAGGAGAGCAATGAGCTGCAAAACCTGGAGAAGGAGGAGGAAGAC
ATTCTGAAAAGCCTTACGAACTCTGAAACTGAGATGGTGCAGCAGACCCAGTCCCTGAGAGAGCTCATCTCAGATCT
GGAGCATCGGCTGCAGGGGTCAGTGATGGAGCTGCTTCAGGGTGTGGATGGCGTCATAAAAAGGACGGAGAACGTGA
CCTTGAAGAAGCCAGAAACTTTTCCAAAAAATCAAAGGAGAGTGTTTCGAGCTCCTGATCTGAAAGGAATGCTAGAA
GTGTTTAGAGAGCTGACAGATGTCCGACGCTACTGGGTTGATGTGACAGTGGCTCCAAACAACATTTCATGTGCTGT
CATTTCTGAAGATAAGAGACAAGTGAGCTCTCCGAAACCACAGATAATATATcaggcaccagggacattatttacgt
ttccgtcactcacgAATTTCAATTATTGTACTGGCATCCTGGGCTCTCAAAGTATCACATCAGGGAAACATTACTGG
GAGGTAGACGTGTCCAAGAAAACTGCTTGGATCCTGGGGGTATGTGCTGGCTTCCAACCTGATGCAATGTGTAATAT
TGAAAAAAATGAAAATTATCAACCTAAATACGGCTACTGGGTTATAGGGTTAGAGGAAGGAGTTAAATGTAGTGCTT
TCCAGGATAGTTCCTTCCATACTCCTTCTGTTCCTTTCATTGTGCCCCTCTCTGTGATTATTTGTCCTGATCGTGTT
GGAGTTTTCCTAGACTATGAGGCTTGCACTGTCTCATTCTTCAATATCACAAACCATGGATTTCTCATCTATAAGTT
TTCTCACTGTTCTTTTTCTCAGCCTGTATTTCCATATTTAAATCCTAGAAAATGTGGAGTCCCCATGACTCTGTGCT
CACCAAGCTCTGTTAACAGATCCAGTTTGGGGATCCAAGGTCGGGCAGGAAGAGGGCCTATTTCCCATGATTCCTTC
ATATTTGCATATACGATACAAGGCTGTTAGAGAGATAATTAGAATTAATTTGACTGTAAACACAAAGATATTAGTAC
AAAATACGTGACGTAGAAAGTAATAATTTCTTGGGTAGTTTGCAGTTTTAAAATTATGTTTTAAAATGGACTATCAT
ATGCTTACCGTAACTTGAAAGTATTTCGATTTCTTGGCTTTATATATCTTGTGGAAAGGACGAAACACCGAGCATGA
CTGACATCTACTTCAAGAGAGTAGATGTCAGTCATGCTCTTTTTTACGCGTAGAATCGAGACCGAGGAGAGGGTTAG
GGATAGGCTTACCTTCGAACCGCGGGCCCTCTAGACTCGAGCGGCCGCCACTGTGCTGGATATCTGCAGAATTGCCC
TTGACGCGTCAGTTTGGGGATCCAAGGTCGGGCAGGAAGAGGGCCTATTTCCCATGATTCCTTCATATTTGCATATA
CGATACAAGGCTGTTAGAGAGATAATTAGAATTAATTTGACTGTAAACACAAAGATATTAGTACAAAATACGTGACG
TAGAAAGTAATAATTTCTTGGGTAGTTTGCAGTTTTAAAATTATGTTTTAAAATGGACTATCATATGCTTACCGTAA
CTTGAAAGTATTTCGATTTCTTGGCTTTATATATCTTGTGGAAAGGACGAAACACCGTCGACCTTGCAATGATGTCG
TAATTTGCGTCTTACTCTGTTCTCAGCGACAGCCAGATCTGAGCCTGGGAGCTCTCTGGCTGTCAGTAAGCTGGTAC
AGAAGGTTGACGAAAATTCTTACTGAGCAAGAAATTTTTTACGCGTGTTAACAAGGGCGAATTCTACCGGGTAGGGG
AGGCGCTTTTCCCAAGGCAGTCTGGAGCATGCGCTTTAGCAGCCCCGCTGGCACTTGGCGCTACACAAGTGGCCTCT
GGCCTCGCACACATTCCACATCCACCGGTAGGCGCCAACCGGCTCCGTTCTTTGGTGGCCCCTTCGCGCCACCTTCT
ACTCCTCCCCTAGTCAGGAAGTTCCCCCCCGCCCCGCAGCTCGCGTCGTGCAGGACGTGACAAATGGAAGTAGCACG
TCTCACTAGTCTCGTGCAGATGGACAGCACCGCTGAGCAATGGAAGCGGGTAGGCCTTTGGGGCAGCGGCCAATAGC
AGCTTTGCTCCTTCGCTTTCTGGGCTCAGAGGCTGGGAAGGGGTGGGTCCGGGGGCGGGCTCAGGGGCGGGCTCAGG
GGCGGGGCGGGCGCCCGAAGGTCCTCCGGAGGCCCGGCATTCTCGCACGCTTCAAAAGCGCACGTCTGCCGCGCTGT
TCTCCTCTTCCTCATCTCCGGGCCTTTCGACCATCTAATGGATTCATACCTGCTGATGTGGGGACTGCTCACGTTCA
TCATGGTGCCTGGCTGCCAGGCAGAGCTCTGTGACGATGACCCGCCAGAGATCCCACACGCCACATTCAAAGCCATG
GCCTACAAGGAAGGAACCATGTTGAACTGTGAATGCAAGAGAGGTTTCCGCAGAATAAAAAGCGGGTCACTCTATAT
GCTCTGTACAGGAAACTCTAGCCACTCGTCCTGGGACAACCAATGTCAATGCACAAGCTCTGCCACTCGGAACACAA
CGAAACAAGTGACACCTCAACCTGAAGAACAGAAAGAAAGGAAAACCACAGAAATGCAAAGTCCAATGCAGCCAGTG
GACCAAGCGAGCCTTCCAGGTCACTGCAGGGAACCTCCACCATGGAAAATGAAGCCACAGAGAGAATTTATCATTT
 
CGTGGTGGGGCAGATGGTTTATTATCAGTGCGTCCAGGATACAGGGCTCTACACAGAGGTCCTGCTGAGAGCGTCT
GCAAAATGACCCACGGGAAGACAAGGTGGACCCAGCCCCAGCTCATATGCACAGGTGAAATGGAGACCAGTCAGTTT
CCAGGTGAAGAGAAGCCTCAGGCAAGCCCCGAAGGCCGTCCTGAGAGTGAGACTTCCTGCCTCGTCACAACAACAGA
TTTTCAAATACAGACAGAAATGGCTGCAACCATGGAGACGTCCATATTTACAACAGAGTACCAGGTAGCAGTGGCCG
GCTGTGTTTTCCTGCTGATCAGCGTCCTCCTCCTGAGTGGGCTCACCTGGCAGCGGAGACAGAGGAAGAGTAGAAGA
ACAATCTGATGAgttaacctcgaggatcccccgggtcgactgatcaaattcgagctcggtacctttaagaccaat
gacttacaaggcagctgtagatcttagccactttttaaaagaaaaggggggactggaagggctaattcactcccaac
gaagacaagatctgctttttgcttgtactgggtctctctggttagaccagatctgagcctgggagctctctggctaa
ctagggaacccactgcttaagcctcaataaagcttgccttgagtgcttcaagtagtgtgtgcccgtctgttgtgtga
ctctggtaactagagatccctcagacccttttagtcagtgtggaaaatctctagcagtagtagttcatgtcatctta
ttattcagtatttataacttgcaaagaaatgaatatcagagagtgagaggaacttgtttattgcagcttataatggt
tacaaataaagcaatagcatcacaaatttcacaaataaagcatttttttcactgcattctagttgtggtttgtccaa
actcatcaatgtatcttatcatgtctggctctagctatcccgcccctaactccgcccatcccgcccctaactccgcc
cagttccgcccattctccgccccatggctgactaattttttttatttatgcagaggccgaggccgcctcggcctctg
agctattccagaagtagtgaggaggcttttttggaggcctaggcttttgcgtcgagacgtacccaattcgccctata
gtgagtcgtattacgcgcgctcactggccgtcgttttacaacgtcgtgactgggaaaaccctggcgttacccaactt
aatcgccttgcagcacatccccctttcgccagctggcgtaatagcgaagaggcccgcaccgatcgcccttcccaaca
gttgcgcagcctgaatggcgaatggcgcgacgcgccctgtagcggcgcattaagcgcggcgggtgtggtggttacgc
gcagcgtgaccgctacacttgccagcgccctagcgcccgctcctttcgctttcttcccttcctttctcgccacgttc
gccggctttccccgtcaagctctaaatcgggggctccctttagggttccgatttagtgctttacggcacctcgaccc
caaaaaacttgattagggtgatggttcacgtagtgggccatcgccctgatagacggtttttcgccctttgacgttgg
agtccacgttctttaatagtggactcttgttccaaactggaacaacactcaaccctatctcggtctattcttttgat
ttataagggattttgccgatttcggcctattggttaaaaaatgagctgatttaacaaaaatttaacgcgaatttaa
caaaatattaacgtttacaatttcc-9897
SEQ ID NO: 23
   gagcatgactgacatctacttcaagagagtagatgtcagtcatgctc
SEQ ID NO: 24 
Gly Ala Arg Gly Thr Arg Tyr Gln Thr Phe Val
1               5                   10
SEQ ID NO: 25 
Gln Ala Pro Gly Thr Leu Phe Thr Phe Pro Ser Leu Thr
1               5                   10
SEQ ID NO: 26 
gctactatgg cttctggaat cctggttaat gtaaaggagg aggtgacctg ccccatctgc 60
ctggaactcc tgacacaacc cctgagcctg gactgcggcc acagcttctg ccaagcatgc 120
ctcactgcaa accacaagaa gtccatgcta gacaaaggag agagtagctg ccctgtgtgc 180
cggatcagtt accagcctga gaacatacgg cctaatcggc atgtagccaa catagtggag 240
aagctcaggg aggtcaagtt gagcccagag gggcagaaag ttgatcattg tgcacgccat 300
ggagagaaac ttctactctt ctgtcaggag gacggaaggg tcatttgctg gctttgtgag 360
cggtctcagg agcaccgtgg tcaccacacg ttcctcacag aggaggttgc ccgggagtac 420
caagtgaagc tccaggcagc tctggagatg ctgaggcaga agcagcagga agctgaagag 480
ttagaagctg acatcagaga agagaaagct tcctggaaga ctcaaataca gtatgacaaa 540
accaacgtct tggcagattt tgagcaactg agagacatcc tggactggga ggagagcaat 600
gagctgcaaa acctggagaa ggaggaggaa gacattctga aaagccttac gaactctgaa 660
actgagatgg tgcagcagac ccagtccctg agagagctca tctcagatct ggagcatcgg 720
ctgcaggggt cagtgatgga gctgcttcag ggtgtggatg gcgtcataaa aaggacggag 780
aacgtgacct tgaagaagcc agaaactttt ccaaaaaatc aaaggagagt gtttcgagct 840
cctgatctga aaggaatgct agaagtgttt agagagctga cagatgtccg acgctactgg 900
gttgatgtga cagtggctcc aaacaacatt tcatgtgctg tcatttctga agataagaga 960
caagtgagct ctccgaaacc acagataata tatcaggcac cagggacatt atttacgttt 1020
ccgtcactca cgaatttcaa ttattgtact ggcatcctgg gctctcaaag tatcacatca 1080
gggaaacatt actgggaggt agacgtgtcc aagaaaactg cttggatcct gggggtatgt 1140
gctggcttcc aacctgatgc aatgtgtaat attgaaaaaa atgaaaatta tcaacctaaa 1200
tacggctact gggttatagg gttagaggaa ggagttaaat gtagtgcttt ccaggatagt 1260
tccttccata ctccttctgt tcctttcatt gtgcccctct ctgtgattat ttgtcctgat 1320
cgtgttggag ttttcctaga ctatgaggct tgcactgtct cattcttcaa tatcacaaac 1380
catggatttc tcatctataa gttttctcac tgttcttttt ctcagcctgt atttccatat 1440
ttaaatccta gaaaatgtgg agtccccatg actctgtgct caccaagctc tggcggctac 1500
ccatacgacg tcccagacta cgcttgagcc tga 1533
SEQ ID NO: 27 
agatccagtt tggggatcca aggtcgggca ggaagagggc ctatttccca tgattccttc 60
atatttgcat atacgataca aggctgttag agagataatt agaattaatt tgactgtaaa 120
cacaaagata ttagtacaaa atacgtgacg tagaaagtaa taatttcttg ggtagtttgc 180
agttttaaaa ttatgtttta aaatggacta tcatatgctt accgtaactt gaaagtattt 240
cgatttcttg gctttatata tcttgtggaa aggacgaaac accgtcgacc ttgcaatgat 300
gtcgtaattt gcgtcttact ctgttctcag cgacagccag atctgagcct gggagctctc 360
tggctgtcag taagctggta cagaaggttg acgaaaattc ttactgagca agaaattttt 420
 t                                                421
SEQ ID NO: 28
   5'-CTGGGTTGATGTGACAGTGG-3'
SEQ ID NO: 29
   5'-CGTGAGTGACGGAAACGTAA-3'
SEQ ID NO: 30
   5'-TCCATTCCGAGGCCACAGCAAG-3'
SEQ ID NO: 31
   5'-TCAGCTCGTTCCTCCTCTGACG-3'
SEQ ID NO: 32
   5'-AAGACCCTGAGAAGGAAAAGCG-3'
SEQ ID NO: 33
   5'-GTGTTGGTAATGCCTGCTGTCC-3'
SEQ ID NO: 34
   5'-ACTACCACTCACCCGCAGAC-3'
SEQ ID NO: 35
   5'-GACGGGAAGCCAGCCTTAC-3'
SEQ ID NO: 36
   5'-CCCCAAGATCCTGAAACAGA-3'
SEQ ID NO: 37
   5'-CCGTTGCTGGACTGGATTAT-3'
SEQ ID NO: 38

5′-
ATGGATTCATACCTGCTGATGTGGGGACTGCTCACGTTCATCATGGTGCCTGGCTGCCAGGCAGAGCTCTGTGACGA

TGACCCGCCAGAGATCCCACACGCCACATTCAAAGCCATGGCCTACAAGGAAGGAACCATGTTGAACTGTGAATGCA
AGAGAGGTTTCacCAGcATAAAAGCGGGTCACTCTATATGCTCTGTACAGGAAACTCTAGCCACTCGTCCTGGGAC
AACCAATGTCAATGCACAAGCTCTGCCACTCGGAACACAACGAAACAAGTGACACCTCAACCTGAAGAACAGAAAGA
AAGGAAAACCACAGAAATGCAAAGTCCAATGCAGCCAGTGGACCAAGCGAGCCTTCCAGGTCACTGCAGGGAACCTC
CACCATGGGAAAATGAAGCCACAGAGAGAATTTATCATTTCGTGGTGGGGCAGATGGTTTATTATCAGTGCGTCCAG
GGATACAGGGCTCTACACAGAGGTCCTGCTGAGAGCGTCTGCAAAATGACCCACGGGAAGACAAGGTGGACCgcGCC
CggGCTCATATGCACAGGTGAAATGGAGACCAGTCAGTTTCCAGGTGAAGAGAAGCCTCAGGCAAGCCCCGAAGGCC
GTCCTGAGAGTGAGACTTCCTGCCTCGTCACAACAACAGATTTTCAAATACAGACAGAAATGGCTGCAACCATGGAG
ACGTCCATATTTACAACAGAGTACCAGGTAGCAGTGGCCGGCTGTGTTTTCCTGCTGATCAGCGTCCTCCTCCTGAG
TGGGCTC - 3'

## Claims

1. A vector comprising:
(a) a backbone comprising essential sequences for integration into a target cell genome;
(b) a nucleic acid encoding a CCR5 RNAi;
(c) a first expression control element that regulates expression of the nucleic acid encoding the RNAi of element (b);
(d) a nucleic acid encoding at least the extracellular domain of CD25; and
(e) a second expression control element that regulates expression of the nucleic acid encoding at least the extracellular domain of CD25.

2. The vector of claim 1, further comprising a nucleic acid encoding a TRIM5 alpha sequence under the control of the second expression control element or a third expression control element.

3. The vector of claim 1 or 2, further comprising a nucleic acid encoding an HIV TAR sequence and a promoter that regulates expression of the nucleic acid.

4. The vector of any one of claim 1 to 3, wherein:
a) the nucleic acid encoding the CCR5 RNAi is the polynucleotide shown in SEQ ID NO 23 or a polynucleotide having at least 80% identity thereto, and
b) the nucleic acid encoding CD25 is selected from a nucleic acid comprises the polynucleotide selected from the polynucleotide of SEQ ID NO. 4, SEQ ID NO. 5 and SEQ ID NO. 6, or a polynucleotide having at least 80% identity to each thereto, and
c) the nucleic acid encoding TRIM5alpha comprises the polynucleotide shown in SEQ ID NO: 1 from nucleotide 5226 to 6710 or a polynucleotide having at least 80% identity thereto, and optionally
d) the nucleic acid encoding TAR comprises nucleotide 8337 to 8468 of SEQ ID NO. 1, or a polynucleotide having at least 80% identity thereto.

5. The vector of any one of claims 1 to 4, wherein the vector is a retroviral vector.

6. The vector of claim 5, wherein the retroviral vector is a lentiviral vector.

7. A viral packaging system comprising:
(a) the vector of any one of claims 1 to 6, wherein the backbone is derived from a virus;
(b) a packaging plasmid; and
(c) an envelope plasmid.

8. A method for producing a pseudotyped viral particle, comprising transducing a packaging cell line with the system of claim 7 under conditions suitable to package the viral vector.

9. A pseudotyped viral particle comprising the vector of any one of claims 1 to 6.

10. An isolated cell comprising the vector of any one of claims 1 to 6, or the pseudotyped viral particle of claim 9.

11. The isolated cell of claim 10, wherein the cell is selected from the group consisting of a macrophage, a lymphocyte, a stem cell and a hematopoietic progenitor cell.

12. An isolated cell of claim 11, wherein the isolated cell expresses the CD25 on the surface of the cell.

13. The isolated cell of claim 11, wherein the isolated stem cell is a hematopoietic stem cell or a hematopoietic progenitor cell.

14. The isolated cell of claim 12 or 13 for use in a method to inhibit HIV replication comprising administering to a subject in need thereof an effective amount of one or more of the cell of claim 12 or 13.

15. The isolated cell of claim 12 or 13 for use in a method to inhibit HIV infection of cells in a subject in need thereof comprising administering to the subject in need thereof an effective amount of the isolated cell of claim 12 or 13.

16. The isolated cell of claim 12 or 13 for use in a method to treat an HIV infection in a subject in need thereof, comprising administering to the subject an effective amount of the isolated cell of claim 12 or 13.

17. A composition comprising the isolated cell of any one of claims 10-13 and a carrier, optionally wherein the carrier is a pharmaceutically acceptable carrier.

18. A method of producing the isolated cell of claim 13, comprising transducing an isolated hematopoietic stem cell or hematopoietic progenitor cell with the vector of any one of claims 1 to 6 or the pseudotyped viral particle of claim 9.

## Patentansprüche

1. Vektor, umfassend:
(a) ein Rückgrat, umfassend wesentliche Sequenzen zum Einbau in ein Zielzellgenom;
(b) eine Nukleinsäure, kodierend für eine CCR5-RNAi;
(c) ein erstes Expressionskontrollelement, das die Expression der für die RNAi von Element (b) kodierenden Nukleinsäure reguliert;
(d) eine Nukleinsäure, kodierend für mindestens die extrazelluläre Domäne von CD25; und
(e) ein zweites Expressionskontrollelement, das die Expression der für mindestens die extrazelluläre Domäne von CD25 kodierenden Nukleinsäure reguliert.

2. Vektor nach Anspruch 1, des Weiteren umfassend eine Nukleinsäure, kodierend für eine TRIM5-alpha-Sequenz unter der Kontrolle des zweiten Expressionskontrollelements oder eines dritten Expressionskontrollelements.

3. Vektor nach Anspruch 1 oder 2, des Weiteren umfassend eine Nukleinsäure, kodierend für eine HIV-TAR-Sequenz und einen Promotor, der die Expression der Nukleinsäure reguliert.

4. Vektor nach einem der Ansprüche 1 bis 3, wobei:
a) die für die CCR5-RNAi kodierende Nukleinsäure ein in SEQ ID NO: 23 gezeigtes Polynukleotid oder ein Polynukleotid mit mindestens 80 % Identität dazu ist, und
b) die für CD25 kodierende Nukleinsäure aus einer Nukleinsäure ausgewählt ist, die das Polynukleotid, ausgewählt aus dem Polynukleotid von SEQ ID NO: 4, SEQ ID NO: 5 und SEQ ID NO: 6, oder ein Polynukleotid mit mindestens 80 % Identität zu jedem davon umfasst, und
c) die für TRIM5-alpha kodierende Nukleinsäure das in SEQ ID NO: 1 von Nukleotid 5226 bis 6710 gezeigte Polynukleotid oder ein Polynukleotid mit mindestens 80 % Identität dazu umfasst, und gegebenenfalls
d) die für TAR kodierende Nukleinsäure Nukleotid 8337 bis 8468 von SEQ ID NO: 1 oder ein Polynukleotid mit mindestens 80 % Identität dazu umfasst.

5. Vektor nach einem der Ansprüche 1 bis 4, wobei der Vektor ein retroviraler Vektor ist.

6. Vektor nach Anspruch 5, wobei der retrovirale Vektor ein Lentivirus-Vektor ist.

7. Virusverpackungssystem, umfassend:
(a) Vektor nach einem der Ansprüche 1 bis 6, wobei das Rückgrat von einem Virus abgeleitet ist;
(b) ein Verpackungsplasmid; und
(c) ein Hüllplasmid.

8. Verfahren zum Herstellen eines pseudotypisierten Viruspartikels, umfassend das Transduzieren einer Verpackungszelllinie mit dem System nach Anspruch 7 unter Bedingungen, die zum Verpacken des viralen Vektors geeignet sind.

9. Pseudotypisiertes Viruspartikel, umfassend den Vektor nach einem der Ansprüche 1 bis 6.

10. Isolierte Zelle, umfassend den Vektor nach einem der Ansprüche 1 bis 6 oder das pseudotypisierte Viruspartikel nach Anspruch 9.

11. Isolierte Zelle nach Anspruch 10, wobei die Zelle aus der Gruppe bestehend aus einem Makrophagen, einem Lymphozyten, einer Stammzelle und einer hämatopoetischen Vorläuferzelle ausgewählt ist.

12. Isolierte Zelle nach Anspruch 11, wobei die isolierte Zelle das CD25 auf der Oberfläche der Zelle exprimiert.

13. Isolierte Zelle nach Anspruch 11, wobei die isolierte Stammzelle eine hämatopoetische Stammzelle oder eine hämatopoetische Vorläuferzelle ist.

14. Isolierte Zelle nach Anspruch 12 oder 13 zur Verwendung in einem Verfahren zum Hemmen der HIV-Replikation, umfassend das Verabreichen einer wirksamen Menge von einer oder mehreren der Zellen nach Anspruch 12 oder 13 an ein dieses benötigende Subjekt.

15. Isolierte Zelle nach Anspruch 12 oder 13 zur Verwendung in einem Verfahren zum Hemmen einer HIV-Infektion von Zellen bei einem dieses benötigenden Subjekt, umfassend das Verabreichen einer wirksamen Menge der isolierten Zelle nach Anspruch 12 oder 13 an ein dieses benötigende Subjekt.

16. Isolierte Zelle nach Anspruch 12 oder 13 zur Verwendung in einem Verfahren zum Behandeln einer HIV-Infektion bei einem dieses benötigenden Subjekt, umfassend das Verabreichen einer wirksamen Menge der isolierten Zelle nach Anspruch 12 oder 13 an ein dieses benötigende Subjekt.

17. Zusammensetzung, umfassend die isolierte Zelle nach einem der Ansprüche 10-13 und einen Träger, gegebenenfalls wobei der Träger ein pharmazeutisch verträglicher Träger ist.

18. Verfahren zum Herstellen der isolierten Zelle nach Anspruch 13, umfassend das Transduzieren einer isolierten hämatopoetischen Stammzelle oder hämatopoetischen Vorläuferzelle mit dem Vektor nach einem der Ansprüche 1 bis 6 oder dem pseudotypisierten Viruspartikel nach Anspruch 9.

## Revendications

1. Vecteur comprenant :
(a) un squelette comprenant des séquences essentielles pour l'intégration dans un génome de cellule cible ;
(b) un acide nucléique codant pour un ARNi CCR5 ;
(c) un premier élément de contrôle d'expression qui régule l'expression de l'acide nucléique codant pour l'ARNi de l'élément (b) ;
(d) un acide nucléique codant pour au moins le domaine extracellulaire de CD25 ; et
(e) un second élément de contrôle d'expression qui régule l'expression de l'acide nucléique codant pour au moins le domaine extracellulaire de CD25.

2. Vecteur selon la revendication 1, comprenant en outre un acide nucléique codant pour une séquence TRIM5 alpha sous le contrôle du second élément de contrôle d'expression ou d'un troisième élément de contrôle d'expression.

3. Vecteur selon l'une des revendications 1 ou 2, comprenant en outre un acide nucléique codant pour une séquence TAR du VIH et un promoteur qui régule l'expression de l'acide nucléique.

4. Vecteur selon l'une quelconque des revendications 1 à 3, dans lequel :
a) l'acide nucléique codant pour l'ARNi CCR5 est le polynucléotide représenté dans SEQ ID NO. 23
ou un polynucléotide ayant au moins 80 % d'identité avec celui-ci, et
b) l'acide nucléique codant pour CD25 est choisi parmi un acide nucléique qui comprend le polynucléotide choisi parmi le polynucléotide de SEQ ID NO. 4, SEQ ID NO. 5 et SEQ ID NO. 6, ou un polynucléotide ayant au moins 80 % d'identité avec chacun d'eux ; et
c) l'acide nucléique codant pour TRIM5alpha comprend le polynucléotide représenté dans SEQ ID NO. 1 à partir du nucléotide 5226 à 6710 ou un polynucléotide ayant au moins 80 % d'identité avec celui-ci, et facultativement
d) l'acide nucléique codant pour TAR comprend le nucléotide 8337 à 8468 de SEQ ID NO. 1, ou un polynucléotide ayant au moins 80 % d'identité avec celui-ci.

5. Vecteur selon l'une quelconque des revendications 1 à 4, dans lequel le vecteur est un vecteur rétroviral.

6. Vecteur selon la revendication 5, dans lequel le vecteur rétroviral est un vecteur lentiviral.

7. Système d'encapsidation virale comprenant :
(a) le vecteur de l'une quelconque des revendications 1 à 6, dans lequel le squelette est dérivé d'un virus ;
(b) un plasmide d'encapsidation ; et
(c) un plasmide d'enveloppe.

8. Procédé de production d'une particule virale pseudotypée, comprenant la transduction d'une lignée cellulaire d'encapsidation avec le système de la revendication 7 dans des conditions appropriées pour encapsider le vecteur viral.

9. Particule virale pseudotypée comprenant le vecteur de l'une quelconque des revendications 1 à 6.

10. Cellule isolée comprenant le vecteur de l'une quelconque des revendications 1 à 6, ou la particule virale pseudotypée de la revendication 9.

11. Cellule isolée selon la revendication 10, dans laquelle la cellule est choisie dans le groupe consistant en un macrophage, un lymphocyte, une cellule souche et une cellule progénitrice hématopoïétique.

12. Cellule isolée selon la revendication 11, dans laquelle la cellule isolée exprime le CD25 sur la surface de la cellule.

13. Cellule isolée selon la revendication 11, dans laquelle la cellule souche isolée est une cellule souche hématopoïétique ou une cellule progénitrice hématopoïétique.

14. Cellule isolée selon l'une des revendications 12 ou 13 pour utilisation dans un procédé pour inhiber la réplication du VIH, comprenant l'administration à un sujet en ayant besoin d'une quantité efficace d'une ou plusieurs des cellules des revendications 12 ou 13.

15. Cellule isolée selon l'une des revendications 12 ou 13 pour utilisation dans un procédé pour inhiber l'infection par le VIH de cellules chez un sujet en ayant besoin, comprenant l'administration au sujet en ayant besoin d'une quantité efficace de la cellule isolée de l'une des revendications 12 ou 13.

16. Cellule isolée selon l'une des revendications 12 ou 13 pour utilisation dans une méthode pour traiter une infection par le VIH chez un sujet en ayant besoin, comprenant l'administration au sujet d'une quantité efficace de la cellule isolée de l'une des revendications 12 ou 13.

17. Composition comprenant la cellule isolée de l'une quelconque des revendications 10 à 13 et un support, facultativement dans laquelle le support est un support pharmaceutiquement acceptable.

18. Procédé de production de la cellule isolée de la revendication 13, comprenant la transduction d'une cellule souche hématopoïétique isolée ou d'une cellule progénitrice hématopoïétique avec le vecteur de l'une quelconque des revendications 1 à 6 ou la particule virale pseudotypée de la revendication 9.
